(19) Europäisches Patentamt
European Patent Office
Office européen des brevets

(11) EP 1 640 021 A1

(12) **EUROPEAN PATENT APPLICATION**
published in accordance with Art. 158(3) EPC

(43) Date of publication:
**29.03.2006 Bulletin 2006/13**

(21) Application number: **04746955.6**

(22) Date of filing: **29.06.2004**

(51) Int Cl.:
***A61K 45/00*** (1985.01) ***A61P 13/02*** (2000.01)
***A61P 13/10*** (2000.01) ***A61P 43/00*** (2000.01)

(86) International application number:
**PCT/JP2004/009486**

(87) International publication number:
**WO 2005/000354 (06.01.2005 Gazette 2005/01)**

(84) Designated Contracting States:
**AT BE BG CH CY CZ DE DK EE ES FI FR GB GR HU IE IT LI LU MC NL PL PT RO SE SI SK TR**

(30) Priority: **30.06.2003 JP 2003188761**

(71) Applicant: **Takeda Pharmaceutical Company Limited**
**Osaka-shi,**
**Osaka 541-0045 (JP)**

(72) Inventors:
- **DOI, Takayuki**
**Osaka-shi,**
**Osaka 5450042 (JP)**
- **NAGABUKURO, Hiroshi**
**Osaka-shi,**
**Osaka 5330003 (JP)**

(74) Representative: **von Kreisler, Alek et al**
**Patentanwälte,**
**von Kreisler-Selting-Werner,**
**Deichmannhaus am Dom,**
**Bahnhofsvorplatz 1**
**50667 Köln (DE)**

(54) **PREVENTIVE/REMEDY FOR URINARY DISTURBANCE**

(57) It is intended to provide a preventive/remedy for urinary disturbance containing a compound, which shows an acetylcholine esterase inhibitory activity but substantially has no butyrylcholine esterase inhibitory activity, showing no side effect and being safe and efficacious without inhibiting the urine collection function; a preventive/remedy for dry mouth induced by the administration of a remedy for urinary disturbance and a preventive or remedy for hyperactive bladder not accompanied by dry mouth; and a method of screening a substance preventing/treating urinary disturbance without inhibiting the urine collection function **characterized by** comprising measuring and comparing the acetylcholine esterase inhibitory activity and the butyrylcholine esterase inhibitory activity of a test compound.



Printed by Jouve, 75001 PARIS (FR)

## Description

Technical Field

The present invention relates to a medicament, more specifically, a preventive or remedy for urinary disturbance, a preventive or remedy for dry mouth induced by the administration of a remedy for urinary disturbance, a preventive or remedy for overactive bladder not accompanied by dry mouth, and a method of screening them. Herein, the overactive bladder means symptoms or disorders whose main symptom is the urgent feeling of urination and which are often accompanied with frequent urination and frequent urination at night, and cause urinary incontinence in some cases.

Technical Background

Urinary disturbance is a generic term of subjective or objective abnormalities during the process from the accumulation of urine (collection of urine) to elimination (urination), and classified as the collection disturbance of urine (urinary incontinence, frequent urination and the like) and elimination disturbance (dysuria, pain on urination, urinary obstruction and the like). In accordance with the progress of aging society, the lower urinary tract disease of an aged person, in particular, urinary disturbance has been recently a great social problem.

The urinary disturbance (dysuria) is caused by the obstruction of urinary tract, the contraction insufficiency of detrusor muscle, the coordination disorder of detrusor and orbicular muscle or the complex occurrence thereof, and includes various background diseases or causes such as brain diseases and spiral cord diseases, prostate-gland enlargement, diabetes, intrapelvic operation, aging and the like. For physically enhancing the elimination function of the bladder, there can be considered a method for relaxing the urinary tract to lower the resistance of the urinary tract and a method for enhancing the contractive force of the bladder at urination. An $\alpha_1$ blocker is widely used for relaxation of the urinary tract and is the first drug of choice in the symptom of urinary obstruction accompanied with prostate-gland enlargement.

Distigmine bromide and neostigigmine methyl sulfate which are carbamate acetylcholine esterase inhibitors, bethanechol chloride which is a muscarine receptor agonist, and the like are used for the contraction insufficiency of the detrusor muscle to enhance the contractive force of the bladder. Since the acetylcholine esterase inhibitor enhances the action of acetylcholine released from the terminal of the pelvic nerve at urination, considering the biological mechanism of urination, such an inhibitor is an excellent drug which enhances the contraction of the bladder at urination.

However, for example, as for distigmine, such a risk that residual urine is increased has been pointed out because distigmine contracts the bladder, while it raises the pressure of the bladder outlet portion. As for neostigmine, it is not used for a remedy because of its short durability of activity (for example, "Diagnosis and Treatment of Neurogenic Bladder" the second edition edited by Takamichi Hattori and Kousaku Yasuda, published by Igaku Shoin pages 105 to 106). Further, in view of reports disclosing that neostigmine decreases a bladder volume and, at the same time, induces micturition desire, distigmine induces the rhythmic contraction of the bladder, and the like, these acetylcholine esterase inhibitors are considered to also contract the bladder at the time of collection of urine to lower the compliance of the collection of urine (the flexibility of the bladder) (for example, "Urology" Vol.10, (1977), pages 83 to 89). Furthermore, since bethanechol is an agonist of muscarine receptor, it is considered to promote the contraction of the bladder irrespective of the timing of the collection of urine and urination, thereby similarly lowering the compliance of the bladder. It is one of important functions of the lower urinary tract in the collection of urine to maintain inside of the bladder at low pressure at the time of collection of urine so as to accumulate a sufficient amount of urine in the bladder. Then, if the function of the collection of urine is damaged by a drug used for treatment of the contraction insufficiency of the detrusor muscle, it can be a cause narrowing the application range of the drug. Thus, the pharmacotherapy of urinary disturbance caused by the contraction insufficiency of the detrusor muscle is not widely approved because of its low clinical activity and less side effects.

Among urinary disturbance, fundamental research as well as research and development for novel drugs for urinary disturbance accompanied with the overactive bladder and prostate-gland enlargement are actively carried out. On the other hand, there is a history that urinary disturbance caused by the contraction insufficiency of the detrusor muscle has been taken off from the objective of development for novel drugs in accordance with the prevalence of clean intermittent self-catherterization. However, considering the psychological burdens of patients, pharmacotherapy which is truly effective is still required.

Acetylcholine is inactivated in an organism by being hydrolyzed with acetylcholine esterase and nonspecific choline esterase (butyrylcholine esterase) which is a free protein existing on the membrane. Acetylcholine esterase and butyrylcholine esterase also coexist in the smooth muscle of the bladder and both enzymes are considered to contribute the decomposition of acetylcholine released (for example, refer to "The Anatomical Record" 1996, Vol.245, pages 645 to 641). The activity of acetylcholine esterase and butyrylcholine esterase can be measured in vitro, and many drugs inhibiting their activity have been reported. The selectivity of inhibitory activity in both enzymes varies (Table 1; The

Japanese Journal of Pharmacology, 2002, Vol.89, pages 7 to 20; Biochemical Pharmacology, 1998, Vol.55, pages 1733 to 1737; The Journal of Pharmacology and Experimental Therapeutics, 1997, Vol.280, pages 1261 to 1269; and CNS Drug Reviews, 2002, Vol.8 pages 53 to 69.)

Table 1

| Drug | Acetylcholine esterase inhibitory activity ($IC_{50}$, $\mu M$) (animal species) | Butyrylcholine esterase inhibitory activity ($IC_{50}$, $\mu M$) (animal species) | Selectivity (butyrylcholine esterase/acetyl -choline esterase) | Literature |
|---|---|---|---|---|
| Physostigmine | 0.00067 (Rat) | 0.016 (Rat) | 24 | 4 |
| | 10 (Electric eel) | 0.03 (Horse) | 0.0030 | 5 |
| | 0.13 (Rat) | 0.015 (Rat) | 0.12 | 6 |
| Donepezil | 0.0067 (Rat) | 7.4 (Rat) | 1100 | 4 |
| | 30 (Electric eel) | 3.8 (Horse) | 0.13 | 5 |
| Tacrine | 0.077 (Rat) | 0.069 (Rat) | 0.90 | 4 |
| | 30 (Electric eel) | 0.01 (Horse) | 0.00033 | 6 |
| | 0.15 (Rat) | 0.54 (Rat) | 0.38 | 6 |
| Rivastigmine | 0.0043 (Rat) | 0.031 (Rat) | 7.2 | 4 |
| Neostigmine | 5 | 0.1 | 0.02 | 5 |
| TAK-147 | 0.051 (Rat) | 24 (Rat) | 460 | 6 |
| Ganstigmine | | | 115 | 7 |

It is suggested that acetylcholine esterase among the above-mentioned two choline esterases has a more important role in the adjustment of contraction of the bladder by acetylcholine from the study using a non-selective choline esterase inhibitor and a butyrylcholine esterase selective inhibitor (Urological Research, opened by on-line on May 8, 2003,
(http://link.springer.de/link/service/journals/00240/conten ts/03/00326/paper/s00240)). However, the details of the relation between the enzyme selectivity of the choline esterase inhibitor and its medical effects and side effects, and the selectivity of a drug required for optimum practical use are still unclear.

On the other hand, WO 00/18391 discloses a non-carbamate amine compound having acetylcholine esterase inhibitory activity improves the excreting force of the bladder and is effective for urinary disturbance, in particular, the treatment of difficulty of urination.

WO 03/57254 discloses a compound having acetylcholine esterase inhibitory activity together with $\alpha_1$ antagonistic activity, and effectiveness thereof for the treatment of urinary disturbance.

Incidentally, there are many reports showing that dry mouth is a side effect caused by the administration of a remedy for urinary disturbance, mainly a remedy for the overactive bladder having anticholine activity such as oxybutynine hydrochloride, propiverine hydrochloride and tolterodine (Urology 2000, Vol. 55, pages 33 to 46), and this is one of the biggest causes which stops the administration of the drug. Dry mouth is caused by an antagonistic reaction of an anticholinergic agent with acetylcholine to promote salivation in salivary glands (submaxilla gland and parotid gland). The muscarine $M_3$ receptor of acetylcholine is a receptor which plays a great role in contraction of the bladder but, at the same time, it is also a receptor which greatly participates in salivation of salivary glands. Accordingly, it is difficult to

create an anticholinergic agent as a novel compound with aiming at dissociation of overactive bladder inhibitory activity from salivation inhibitory activity from a viewpoint of pharmacological activity. At present, although alleviation of the side effect by changing a dosage form to, for example, a sustained release form or a transcutaneous absorbing form is reported, an onset rate of dry mouth is high yet.

Objects of the present invention are to provide a preventive or remedy substance for urinary disturbance, in particular, the difficulty of urination which has a higher treatment effect and convenience as compared with conventional acetylcholine esterase inhibitors known to have the treatment effect of urinary disturbance and has less side effects, and a method of screening the substance, as well as to provide a preventive or remedy means for urinary disturbance using the substance.

Still other objects of the present invention are to provide a preventive or remedy for dry mouth induced by the administration of a preventive or remedy for urinary disturbance as well as a preventive or remedy for the overactive bladder not accompanied by dry mouth.

Disclosure of the Invention

The present inventors have intensively studied in order to solve the fore-mentioned problems and, as a result, have found that a compound substantially free from butyrylcholine esterase inhibitory activity among compounds having acetylcholine esterase inhibitory activity enhances the elimination function of the bladder without influencing the collection function of urine of the bladder in pressure/flow study using guinea pigs anesthetized with urethane. Further, they have found that distigmine and neostigigmine which are acetylcholine esterase inhibitors also having butyrylcholine esterase inhibitory activity reduce the collection function of urine of the bladder due to the lowering of the bladder compliance, and further reduce the elimination function of the bladder due to the increase in the resistance of the urinary tract by the contraction of the orbicular muscle of the external urinary tract. Furthermore, they have unexpectedly found that, as a result of their study of the action of various choline esterase inhibitors on basic tension (stationary tension) in a specimen of the bladder muscle removed from guinea pig, any of non-selective choline esterase inhibitors significantly increase the basic tension, while non-carbamate amine compounds which are acetylcholine esterase selective inhibitors do not influence the basic tension. Moreover, they have found that each of the above amine compound and tetraisopropylpyrophosphoramide (hereinafter, abbreviated as iso-OMPA) which is a butyrylcholine esterase selective inhibitor alone does not influence the basic tension, while they increase the basic tension, as with distigmine and neostigigmine, when they are acted simultaneously.

These results show that a drug inhibiting both acetylcholine esterase and butyrylcholine esterase reduces the collection function of urine due to the increase in the basic tension of the bladder and further the internal pressure of the bladder at the collection of urine, while the acetylcholine esterase selective inhibitor including the above-mentioned amine compound enhances only the contraction of the bladder at urination without influence on the collection function of urine. Therefore such a drug has been shown to be a safe and effective preventive or remedy for urinary disturbance free from a side effect.

Further, it has been found that the above-mentioned acetylcholine esterase selective inhibitor is a preventive or remedy effective for dry mouth induced by the administration of a preventive or remedy for urinary disturbance. That is, it has been found that the above-mentioned acetylcholine esterase selective inhibitor acts antagonistically for dry mouth (salivation suppressing action) induced by the administration of a preventive or remedy for urinary disturbance, for example an anticholinergic agent. On the other hand, it has been found that the above-mentioned acetylcholine esterase selective inhibitor does not influence the increasing action of the bladder volume by a preventive or remedy substance for urinary disturbance and therefore is also useful as a preventive or remedy for the overactive bladder not accompanied by dry mouth.

In addition, as described above, it has been shown that the screening of a preventive or remedy substance for urinary disturbance free from influence on the collection function of urine, the screening of a preventive or remedy substance for dry mouth induced by the administration of a preventive or remedy for urinary disturbance, and the screening of a preventive or remedy substance for the overactive bladder not accompanied by dry mouth can be rapidly, simply and easily carried out by measuring and comparing the acetylcholine esterase inhibitory activity and the butyrylcholine esterase inhibitory activity of a tested compound.

The present inventors have further studied based on these findings and, as a result, have completed the present invention.

That is, the present invention relates to:

[1] A preventive or remedy for urinary disturbance comprising a compound having acetylcholine esterase inhibitory activity and being substantially free from butyrylcholine esterase inhibitory activity, which does not inhibit the collection function of urine;

[2] A preventive or remedy for dry mouth induced by the administration of a remedy for urinary disturbance, comprising

a compound having acetylcholine esterase inhibitory activity and being substantially free from butyrylcholine esterase inhibitory activity;

[3] A preventive or remedy for overactive bladder not accompanied by dry mouth comprising a combination of a compound having acetylcholine esterase inhibitory activity and being substantially free from butyrylcholine esterase inhibitory activity, and an anticholinergic agent;

[4] The preventive or remedy according to the above [1], [2] or [3], wherein the ratio of a butyrylcholine esterase 50% inhibitory concentration to an acetylcholine esterase 50% inhibitory concentration of the compound is 20 or more;

[5] The preventive or remedy according to the above [1], [2] or [3], wherein the ratio of a butyrylcholine esterase 50% inhibitory concentration to an acetylcholine esterase 50% inhibitory concentration of the compound is 100 or more;

[6] The preventive or remedy according to the above [1], wherein urinary disturbance is dysuria;

[7] The preventive or remedy according to the above [2], wherein the remedy for urinary disturbance is an anticholinergic agent;

[8] A method for screening a preventive or remedy substance for urinary disturbance not inhibiting the collection function of urine, which comprises measuring and comparing the acetylcholine esterase inhibitory activity and the butyrylcholine esterase inhibitory activity of a test compound;

[9] A method for preventing or treating urinary disturbance without inhibiting the collection function of urine, which comprises administering an effective amount of a compound having acetylcholine esterase inhibitory activity and being substantially free from butyrylcholine esterase inhibitory activity to a mammal;

[10] A method for preventing or treating dry mouth induced by the administration of a remedy for urinary disturbance, which comprises administering an effective amount of a compound having acetylcholine esterase inhibitory activity and being substantially free from butyrylcholine esterase inhibitory activity to a mammal;

[11] A method for preventing or treating overactive bladder without being accompanied by dry mouth, which comprises administering a combination of effective amounts of a compound having acetylcholine esterase inhibitory activity and being substantially free from butyrylcholine esterase inhibitory activity, and an anticholinergic agent to a mammal;

[12] Use of a compound having acetylcholine esterase inhibitory activity and being substantially free from butyrylcholine esterase inhibitory activity for manufacturing a preventive or remedy for urinary disturbance which does not inhibit the collection function of urine;

[13] Use of a compound having acetylcholine esterase inhibitory activity and being substantially free from butyrylcholine esterase inhibitory activity for manufacturing a preventive or remedy for dry mouth induced by the administration of a remedy for urinary disturbance;

[14] Use of a compound having acetylcholine esterase inhibitory activity and being substantially free from butyrylcholine esterase inhibitory activity and an anticholinergic agent for manufacturing a preventive or remedy for overactive bladder not accompanied by dry mouth;

[15] A preventive or remedy for overactive bladder not accompanied by dry mouth comprising a compound having acetylcholine esterase inhibitory activity and being substantially free from butyrylcholine esterase inhibitory activity;

[16] A method for preventing or treating overactive bladder without being accompanied by dry mouth, which comprises administering an effective amount of a compound having acetylcholine esterase inhibitory activity and being substantially free from butyrylcholine esterase inhibitory activity to a mammal; and

[17] Use of a compound having acetylcholine esterase inhibitory activity and being substantially free from butyrylcholine esterase inhibitory activity for manufacturing a preventive or remedy for overactive bladder not accompanied by dry mouth.

Since a compound having AChE inhibitory activity and free from BuChE inhibitory activity enhances the contraction action of the detrusor muscle only at urination without lowering the bladder compliance at the collection of urine, the compound is extremely useful as a safe and effective preventive or remedy for urinary disturbance free from a side effect. In addition, the compound is also extremely useful as a preventive or remedy for dry mouth induced by the administration of a remedy for urinary disturbance as well as a preventive or remedy for the overactive bladder not accompanied by dry mouth.

The "compound having acetylcholine esterase (hereinafter, sometimes, abbreviated as AChE) inhibitory activity and being substantially free from butyrylcholine esterase (hereinafter, sometimes, abbreviated as BuChE) inhibitory activity" (hereinafter, sometimes, abbreviated as "the compound of the present invention") used in the present invention means a compound which has AChE inhibitory activity adequate for improving the constriction insufficiency of the detrusor muscle at urination and is substantially free from BuChE inhibitory activity, that is, has no BuChE inhibitory activity or has only low BuChE inhibitory activity which does not increase the basic tension of the detrusor muscle at urination.

Preferably, the compound of the present invention has AChE inhibitory activity as a 50% inhibitory concentration ($IC_{50}$) value of AChE of about 0.5 $\mu$M or less which is obtained by a thiocholine method (Ellman method: a measurement method used in the in vitro enzyme inhibitory test in Test Example 1 hereinafter), and more preferably about 0.2 $\mu$M or less.

Further, the compound of the present invention preferably has BuChE inhibitory activity as a 50% inhibitory concentration ($IC_{50}$) value of BuChE of about 1 μM or more which is obtained by the thiocholine method (the same as above), and more preferably about 10 μM or more.

The balance of inhibitory activities of both enzymes in the compound of the present invention is such that a ratio of the $IC_{50}$ value of AChE to the $IC_{50}$ value of BuChE which are obtained by the thiocholine method (the same as above) is at least about 1 : 20 or more, more preferably about 1 : 100 or more, further preferably about 1 : 1000 or more, and most preferably about 1 : 10000 or more.

The preventive or remedy for urinary disturbance comprising the compound of the present invention is characterized in that it does not inhibit the collection function of urine. Herein, the phrase "not inhibiting the collection function of urine" means that the period of time for inducing initial desire for urination is not shortened by administration of the drug than before administration, or the bladder compliance and/or the bladder volume is not significantly lowered.

The preventive or remedy for dry mouth induced by the administration of a remedy for urinary disturbance comprising the compound of the present invention is characterized in that it prevents and/or treats dry mouth induced by a preventive or remedy for urinary disturbance, for example, administration of an anticholinergic agent.

The preventive or remedy for overactive bladder not accompanied by dry mouth which comprises the compound of the present invention is characterized in that dry mouth is not generated when the compound of the present invention itself is used as the preventive or remedy for overactive bladder, or when it is used in combination with another preventive or remedy for overactive bladder (an anticholinergic agent, etc.).

The compound of the present invention may be any compound having any molecular structure in so far as it is a compound having AChE inhibitory activity and being substantially free from BuChE inhibitory activity as defined herein. Among these, amine compounds in which hydrogen atom(s) of ammonia are substituted with hydrocarbon group (s) are preferable, and primary amine compounds, secondary amine compounds and tertiary amine compounds are more preferable.

One of such amine compounds includes, for example, amine compounds without carbamate structure (-OCON-) in their molecules such as 1) to 49) described hereinafter (hereinafter, sometimes, referred to as the compound A). Among these compounds, those which contain at least one 5-to 7-membered nitrogen-containing heterocycle as a partial structure are preferred, and in particular, compounds 1), 20), 23), 41), 42) and 43) as described hereinafter are especially preferred. Among them, particularly preferred is compound 1) as described hereinafter.

Further, examples of other preferable amine compounds include compounds which are converted into compounds having acetylcholine esterase inhibitory activity and α1 antagonistic activity in combination by forming salts, such as salts of prodrugs of the compounds (II), (IIa), (IIb), (IIc) or (IId) as described hereinafter; and compounds which are converted into compounds having acetylcholine esterase inhibitory activity and α1 antagonistic activity in combination by reactions with enzymes and gastric acid under physiological conditions in an organism (hereinafter, sometimes, referred to as the compound B).

Specifically, the compounds described hereinafter are preferable.

The compound A is a compound represented by the formula (I):

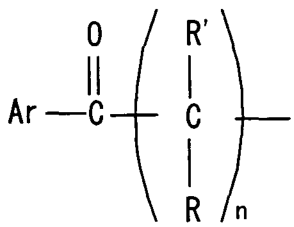

(I)

wherein Ar is an optionally condensed phenyl group in which the phenyl moiety may be substituted, n is an integer of 1 to 10, each of R and R' is a hydrogen atom, a halogen atom or a hydrocarbon group which may be substituted, and Y is an optionally substituted amino group, or an optionally substituted nitrogen-containing saturated heterocyclic group, or a salt thereof (hereinafter, sometimes, abbreviated as the compound (I)).

In the above-mentioned formula, the "substituent" in the "optionally condensed phenyl in which the phenyl moiety may be substituted" represented by Ar includes, for example, (i) optionally halogenated lower alkyl, (ii) halogen (e.g., fluoro, chloro, bromo, iodo, etc.), (iii) lower alkylenedioxy (e.g., $C_{1-3}$ alkylenedioxy such as methylenedioxy, ethylenedioxy, etc.), (iv) nitro, (v) cyano, (vi) hydroxy, (vii) optionally halogenated lower alkoxy, (viii) cycloalkyl (e.g., $C_{3-6}$ cycloalkyl such as cyclopropyl, cyclobutyl, cyclopentyl, cyclohexyl, etc.), (ix) optionally halogenated lower alkylthio, (x) amino, (xi) mono-lower alkylamino (e.g., mono-$C_{1-6}$ alkylamino such as methylamino, ethylamino, propylamino, etc.), (xii) di-lower alkylamino (e.g., di-$C_{1-6}$ alkylamino such as dimethylamino, diethylamino, etc.), (xiii) 5- to 7-membered

cyclic amino (e.g., 5- to 7-membered cyclic amino which may contain 1 to 3 heteroatoms selected from nitrogen, oxygen and sulfur, etc., in addition to one nitrogen atom (e.g., pyrrolidino, piperidino, piperazino, morpholino, thiomorpholino, etc.)), (xiv) lower alkyl-carbonylamino (e.g., $C_{1-6}$ alkyl-carbonylamino such as acetylamino, propionylamino, butyrylamino, etc.), (xv) lower alkyl-sulfonylamino (e.g., $C_{1-6}$ alkylsulfonylamino such as methylsulfonylamino, ethylsulfonylamino, propylsulfonylamino, etc.), (xvi) lower alkoxycarbonyl (e.g., $C_{1-6}$ alkoxy-carbonyl such as methoxycarbonyl, ethoxycarbonyl, propoxycarbonyl, isobutoxycarbonyl, etc.), (xvii) carboxy, (xviii) lower alkylcarbonyl (e.g., $C_{1-6}$ alkylcarbonyl such as methylcarbonyl, ethylcarbonyl, butylcarbonyl, etc.), (xix) cycloalkylcarbonyl (e.g., $C_{3-6}$ cycloalkylcarbonyl such as cyclopropylcarbonyl, cyclobutylcarbonyl, cyclopentylcarbonyl, cyclohexylcarbonyl, etc.), (xx) carbamoyl, thiocarbamoyl, (xxi) mono-lower alkyl-carbamoyl (e.g., mono-$C_{1-6}$ alkyl-carbamoyl such as methylcarbamoyl, ethylcarbamoyl, propylcarbamoyl, butylcarbamoyl, etc.), (xxii) di-lower alkyl-carbamoyl (e.g., di-$C_{1-6}$ alkyl-carbamoyl such as diethylcarbamoyl, dibutylcarbamoyl, etc.), (xxiii) lower alkylsulfonyl (e.g., $C_{1-6}$ alkylsulfonyl such as methylsulfonyl, ethylsulfonyl, propylsulfonyl, etc.), (xxiv) cycloalkylsulfonyl (e.g., $C_{3-6}$ cycloalkylsulfonyl such as cyclopentylsulfonyl, cyclohexylsulfonyl, etc.), (xxv) phenyl, (xxvi) naphthyl, (xxvii) mono-phenyl-lower alkyl (e.g., mono-phenyl-$C_{1-6}$ alkyl such as benzyl, phenylethyl, etc.), (xxviii) di-phenyl-lower alkyl (e.g., di-phenyl-$C_{1-6}$ alkyl such as diphenylmethyl, diphenylethyl, etc.), (xxix) mono-phenyl-lower alkyl-carbonyloxy (e.g., mono-phenyl-$C_{1-6}$ alkyl-carbonyloxy such as phenylmethylcarbonyloxy, phenylethylcarbonyloxy, etc.), (xxx) di-phenyl-lower alkyl-carbonyloxy (e.g., diphenyl-$C_{1-6}$ alkyl-carbonyloxy such as diphenylmethylcarbonyloxy, diphenylethylcarbonyloxy, etc.), (xxxi) phenoxy, (xxxii) mono-phenyl-lower alkyl-carbonyl (e.g., mono-phenyl-$C_{1-6}$ alkyl-carbonyl such as phenylmethylcarbonyl, phenylethylcarbonyl, etc.), (xxxiii) di-phenyl-lower alkyl-carbonyl (e.g., di-phenyl-$C_{1-6}$ alkyl-carbonyl such as diphenylmethylcarbonyl, diphenylethylcarbonyl, etc.), (xxxiv) benzoyl, (xxxv) phenoxycarbonyl, (xxxvi) phenyl-lower alkyl-carbamoyl (e.g., phenyl-$C_{1-6}$ alkyl-carbamoyl such as phenyl-methylcarbamoyl, phenyl-ethylcarbamoyl, etc.), (xxxvii) phenylcarbamoyl, (xxxviii) phenyl-lower alkyl-carbonylamino (e.g., phenyl-$C_{1-6}$ alkyl-carbonylamino such as phenyl-methylcarbonylamino, phenyl-ethylcarbonylamino, etc.), (xxxix) phenyl-lower alkylamino (e.g., phenyl-$C_{1-6}$ alkylamino such as phenyl-methylamino, phenyl-ethylamino, etc.), (xxxx) phenyl-lower alkylsulfonyl (e.g., phenyl-$C_{1-6}$ alkylsulfonyl such as phenyl-methylsulfonyl, phenyl-ethylsulfonyl, etc.), (xxxxi) phenylsulfonyl, (xxxxii) phenyl-lower alkylsulfinyl (e.g., phenyl-$C_{1-6}$ alkylsulfinyl such as phenyl-methylsulfinyl, phenyl-ethylsulfinyl, etc.), (xxxxiii) phenyl-lower alkylsulfonylamino (e.g., phenyl-$C_{1-6}$ alkylsulfonylamino such as phenyl-methylsulfonylamino, phenyl-ethylsulfonylamino, etc.), and (xxxxiv) phenylsulfonylamino (wherein the phenyl, naphthyl, mono-phenyl-lower alkyl, di-phenyl-lower alkyl, mono-phenyl-lower alkyl-carbonyloxy, di-phenyl-lower alkyl-carbonyloxy, phenoxy, mono-phenyl-lower alkyl-carbonyl, di-phenyl-lower alkyl-carbonyl, benzoyl, phenoxycarbonyl, phenyl-lower alkyl-carbamoyl, phenylcarbamoyl, phenyl-lower alkyl-carbonylamino, phenyl-lower alkylamino, phenyl-lower alkylsulfonyl, phenylsulfonyl, phenyl-lower alkylsulfinyl, phenyl-lower alkylsulfonylamino and phenylsulfonylamino as mentioned above in (xxv) to (xxxxiv) may further be substituted by 1 to 4 substituents selected from lower alkyl (e.g., $C_{1-6}$ alkyl such as methyl, ethyl, propyl, isopropyl, butyl, sec-butyl, tert-butyl, pentyl, hexyl, etc.), lower alkoxy (e.g., $C_{1-6}$ alkoxy such as methoxy, ethoxy, propoxy, isopropoxy, butoxy, isobutoxy, sec-butoxy, tert-butoxy, etc.), halogen (e.g., chloro, bromo, iodo, etc.), hydroxy, benzyloxy, amino, mono-lower alkylamino (e.g., mono-$C_{1-6}$ alkylamino such as methylamino, ethylamino, propylamino, etc.), di-lower alkylamino (e.g., di-$C_{1-6}$ alkylamino such as dimethylamino, diethylamino, etc.), nitro, lower alkyl-carbonyl (e.g., $C_{1-6}$ alkyl-carbonyl such as methylcarbonyl, ethylcarbonyl, butylcarbonyl, etc.), benzoyl, and the like). Said phenyl may be substituted by 1 to 4 of these substituents.

The "optionally halogenated lower alkyl " as mentioned above includes, for example, lower alkyl (e.g., $C_{1-6}$ alkyl such as methyl, ethyl, propyl, isopropyl, butyl, sec-butyl, tert-butyl, pentyl, hexyl, etc.) which may have 1 to 3 halogen atoms (e.g. chloro, bromo, iodo, etc.), and is exemplified by methyl, chloromethyl, difluoromethyl, trichloromethyl, trifluoromethyl, ethyl, 2-bromoethyl, 2,2,2-trifluoroethyl, propyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl, 6,6,6-trifluorohexyl, and the like.

The "optionally halogenated lower alkoxy " as mentioned above includes, for example, lower alkoxy (e.g., $C_{1-6}$ alkoxy such as methoxy, ethoxy, propoxy, isopropoxy, butoxy, isobutoxy, sec-butoxy, tert-butoxy, etc.) which may have 1 to 3 halogen atoms (e.g. chloro, bromo, iodo, etc.), and is exemplified by methoxy, difluoromethoxy, trifluoromethoxy, ethoxy, 2,2,2-trifluoroethoxy, propoxy, isopropoxy, butoxy, 4,4,4-trifluorobutoxy, isobutoxy, sec-butoxy, pentyloxy, hexyloxy, and the like.

The "optionally halogenated lower alkylthio " as mentioned above includes, for example, lower alkylthio (e.g., $C_{1-6}$ alkylthio such as methylthio, ethylthio, propylthio, isopropylthio, butylthio, isobutylthio, sec-butylthio, tert-butylthio, etc.) which may have 1 to 3 halogen atoms (e.g. chloro, bromo, iodo, etc.), and is exemplified by methylthio, difluoromethylthio, trifluoromethylthio, ethylthio, propylthio, isopropylthio, butylthio, 4,4,4-trifluorobutylthio, isobutylthio, sec-butylthio, tert-butylthio, pentylthio, hexylthio, and the like.

The "substituent" in the "optionally condensed phenyl which may be substituted" includes preferably, (i) amino, (ii) mono-lower alkylamino (e.g., mono-$C_{1-6}$ alkylamino such as methylamino, ethylamino, propylamino, etc.), (iii) di-lower alkylamino (e.g., di-$C_{1-6}$ alkylamino such as dimethylamino, diethylamino, etc.), (iv) 5- to 7-membered cyclic amino which may contain, for example, 1 to 3 heteroatoms selected from nitrogen, oxygen and sulfur, etc., in addition to one nitrogen atom (e.g., pyrrolidino, piperidino, piperazino, morpholino, thiomorpholino, etc.), (v) lower alkyl-carbonylamino (e.g., $C_{1-6}$

alkyl-carbonylamino such as acetylamino, propionylamino, butyrylamino, etc.), (vi) lower alkyl-sulfonylamino (e.g., $C_{1-6}$ alkylsulfonylamino such as methylsulfonylamino, ethylsulfonylamino, propylsulfonylamino etc.), (vii) phenyl-lower alkylamino (e.g., phenyl-$C_{1-6}$ alkylamino such as phenyl-methylamino, phenyl-ethylamino, etc.), (viii) phenyl-lower alkylsulfonylamino (e.g., phenyl-lower $C_{1-6}$ alkylsulfonylamino such as phenyl-methylsulfonylamino, phenyl-ethylsulfonylamino, etc.), (ix) phenylsulfonylamino, (x) halogen (e.g. fluoro, chloro, etc.), (xi) optionally halogenated lower alkyl (e.g., methyl, ethyl, isopropyl, tert-butyl, trifluoromethyl, etc.) and (xii) optionally halogenated lower alkoxy (e.g., methoxy, ethoxy, isopropoxy, tert-butoxy, trifluoromethoxy, etc.). Particularly preferred are di-lower alkylamino (e.g., di-$C_{1-6}$ alkylamino such as dimethylamino, di-ethylamino, etc.), 5- to 7-membered cyclic amino which may contain 1 to 3 heteroatoms selected from nitrogen, oxygen and sulfur, etc., in addition to one nitrogen atom (e.g., pyrrolidino, piperidino, piperazino, morpholino, thiomorpholino, etc.), and the like.

The condensed "phenyl" of "optionally condensed phenyl which may be substituted" is exemplified by, for example,

(1) an example in which the phenyl is condensed with an optionally substituted mono-cyclic heterocycle;
(2) an example in which the phenyl is condensed with an optionally substituted bicyclic heterocycle or with two same or different mono-cyclic groups (provided that at least one of two is a mono-cyclic heterocycle); and
(3) an example in which the phenyl is condensed with an optionally substituted tricyclic heterocycle.

When the phenyl of "optionally condensed phenyl which may be substituted" is condensed with a mono-cyclic heterocycle, a group of the formula:

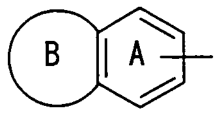

wherein A ring is an optionally substituted benzene ring; and B ring is an optionally substituted heterocycle; is exemplified.

As for the substituent on the A ring, the "substituent" of "optionally condensed phenyl which may be substituted" is exemplified. The number of the substituent is 1 to 3.

The "heterocycle" of "optionally substituted heterocycle" represented by the B ring includes 4- to 14-membered (preferably 5- to 9-membered) aromatic or non-aromatic heterocycles which contain 1 to 4 heteroatoms selected from, for example, nitrogen, oxygen and sulfur. Such heterocycles are exemplified by pyridine, pyrazine, pyrimidine, imidazole, furan, thiophene, dihydropyridine, diazepine, oxazepine, pyrrolidine, piperidine, hexamethylenimine, heptamethylenimine, tetrahydrofuran, piperazine, homopiperazine, tetrahydrooxazepine, morpholine, thiomorpholine, pyrrole, pyrazole, 1,2,3-triazole, oxazole, oxazolidine, thiazole, thiazolidine, isoxazole, imidazoline, and the like. Among these heterocylces, 5- to 9-membered non-aromatic heterocycles containing 1 heteroatom or 2 identical or different heteroatoms (e.g., pyrrolidine, piperidine, hexamethylenimine, heptamethylenimine, tetrahydrofuran, piperazine, homopiperazine, tetrahydrooxazepine, morpholine, thiomorpholine, etc.) are preferred. Particularly preferred are (1) non-aromatic heterocycles containing 1 heteroatom selected from, for example, nitrogen, oxygen and sulfur, and (2) non-aromatic heterocycles containing 1 nitrogen atom and 1 heteroatom selected from nitrogen, oxygen and sulfur.

As the "substituent" of "optionally substituted heterocycle" represented by the B ring, the following 1 to 5 substituents may be used: (i) halogen (e.g., fluoro, chloro, bromo, iodo, etc.), (ii) nitro, (iii) cyano, (iv) oxo, (v) hydroxy, (vi) lower alkyl (e.g., $C_{1-6}$ alkyl such as methyl, ethyl, propyl, isopropyl, butyl, isobutyl, tert-butyl, sec-butyl, etc.), (vii) lower alkoxy (e.g., $C_{1-6}$ alkoxy such as methoxy, ethoxy, propyloxy, isopropyloxy, butyloxy, etc.), (viii) lower alkylthio (e.g., $C_{1-6}$ alkylthio such as methylthio, ethylthio, propylthio, etc.), (ix) amino, (x) mono-lower alkylamino (e.g., mono-$C_{1-6}$ alkylamino such as methylamino, ethylamino, propylamino, etc.), (xi) di-lower alkylamino (e.g., di-$C_{1-6}$ alkylamino such as dimethylamino, diethylamino, etc.), (xii) 5- to 7-membered cyclic amino which may contain 1 to 3 heteroatoms selected from, for example, nitrogen, oxygen and sulfur, in addition to carbon atoms and one nitrogen atom (e.g., pyrrolidino, piperidino, piperazino, morpholino, thiomorpholino, etc.), (xiii) lower alkyl-carbonylamino (e.g., $C_{1-6}$ alkyl-carbonylamino such as acetylamino, propionylamino, butyrylamino, etc.), (xiv) lower alkylsulfonylamino (e.g., $C_{1-6}$ alkylsulfonylamino such as methylsulfonylamino, ethylsulfonylamino, etc.), (xv) lower alkoxy-carbonyl (e.g., $C_{1-6}$ alkoxy-carbonyl such as methoxycarbonyl, ethoxycarbonyl, propoxycarbonyl, etc.), (xvi) carboxy, (xvii) lower alkylcarbonyl (e.g., $C_{1-6}$ alkylcarbonyl such as methylcarbonyl, ethylcarbonyl, propylcarbonyl, etc.), (xviii) carbamoyl, (xix) mono-lower alkylcarbamoyl (e.g., mono-$C_{1-6}$ alkyl-carbamoyl such as methylcarbamoyl, ethylcarbamoyl, etc.), (xx) di-lower alkylcarbamoyl (e.g., di-$C_{1-6}$ alkyl-carbamoyl such as dimethylcarbamoyl, diethylcarbamoyl, etc.), (xxi) lower alkylsulfonyl (e.g., $C_{1-6}$ alkylsulfonyl such as methylsulfonyl, ethylsulfonyl, propylsulfonyl, etc.), and the like. Among these substituents, oxo, lower alkyl (e.g., $C_{1-6}$ alkyl such as methyl, ethyl, propyl, isopropyl, butyl, isobutyl, tert-butyl, sec-butyl, etc.), and the like are preferred.

Particularly preferred is oxo.

When the B ring contains a nitrogen atom in the ring, it may have a group of the formula:

>N-$R^1$

wherein $R^1$ is hydrogen, optionally substituted hydrocarbon group, acyl, or optionally substituted heterocyclic group; in the ring. In addition, the B ring may contain 1 to 3 of the above-mentioned substituents (i) to (xxi).

The "hydrocarbon group" of the "optionally substituted hydrocarbon group" represented by $R^1$ indicates a group which is formed from a hydrocarbon compound by removing one hydrogen atom, and is exemplified, for example, by the following alkyl, alkenyl, alkynyl, cycloalkyl, aryl, aralkyl, a combination of these groups, and the like. Among these groups, $C_{1-16}$ hydro-carbon group is preferred.

(1) Alkyl (e.g., $C_{1-6}$ alkyl such as methyl, ethyl, propyl, isopropyl, butyl, isobutyl, tert-butyl, sec-butyl, pentyl, hexyl, etc.)
(2) Alkenyl (e.g., $C_{2-6}$ alkenyl such as vinyl, allyl, isopropenyl, butenyl, isobutenyl, sec-butenyl, etc.)
(3) Alkynyl (e.g., $C_{2-6}$ alkynyl such as propargyl, ethynyl, butynyl, 1-hexynyl, etc.)
(4) Cycloalkyl (e.g., $C_{3-6}$ cycloalkyl such as cyclopropyl, cyclobutyl, cyclopentyl, cyclohexyl, etc.)
(5) Crosslinked cyclic lower saturated hydrocarbon group (e.g., Crosslinked cyclic $C_{8-14}$ saturated hydrocarbon group such as bicyclo[3.2.1]oct-2-yl, bicyclo[3.3.1]non-2-yl, adamantan-1-yl, etc.)
(6) Aryl (e.g., $C_{6-14}$ aryl such as phenyl, 1-naphthyl, 2-naphthyl, biphenyl, 2-indenyl, 2-anthryl, etc. Phenyl is preferred.)
(7) Aralkyl (e.g., $C_{7-16}$ aralkyl such as: phenyl-$C_{1-10}$ alkyl such as benzyl, phenylethyl, phenylpropyl, phenylbutyl, phenylpentyl, phenylhexyl, etc.; naphthyl-$C_{1-6}$ alkyl such as $\alpha$-naphthylmethyl, etc.; diphenyl-$C_{1-3}$ alkyl such as diphenylmethyl, diphenylethyl, etc.)
(8) Aryl-alkenyl (e.g., $C_{6-14}$ aryl-$C_{2-12}$ alkenyl such as: phenyl-$C_{2-12}$ alkenyl such as styryl, cinnamyl, 4-phenyl-2-butenyl, 4-phenyl-3-butenyl, etc.)
(9) Aryl-$C_{2-12}$ alkynyl (e.g., $C_{6-14}$ aryl-$C_{2-12}$ alkynyl such as: phenyl-$C_{2-12}$ alkynyl such as phenylethynyl, 3-phenyl-2-propynyl, 3-phenyl-1-propynyl, etc.)
(10) Cycloalkyl-alkyl (e.g., $C_{3-7}$ cycloalkyl-$C_{1-6}$ alkyl such as cyclopropylmethyl, cyclobutylmethyl, cyclopentylmethyl, cyclohexylmethyl, cycloheptylmethyl, cyclopropylethyl, cyclobutylethyl, cyclopentylethyl, cyclohexylethyl, cycloheptylethyl, cyclopropylpropyl, cyclobutylpropyl, cyclopentylpropyl, cyclohexylpropyl, cycloheptylpropyl, cyclopropylbutyl, cyclobutylbutyl, cyclopentylbutyl, cyclohexylbutyl, cycloheptylbutyl, cyclopropylpentyl, cyclobutylpentyl, cyclopentylpentyl, cyclohexylpentyl, cycloheptylpentyl, cyclopropylhexyl, cyclobutylhexyl, cyclopentylhexyl, cyclohexylhexyl, etc.)
(11) Aryl-aryl-$C_{1-10}$ alkyl (e.g., biphenylmethyl, biphenylethyl, etc.)

The "hydrocarbon group" of "optionally substituted hydrocarbon group" represented by $R^1$ preferably includes, for example, $C_{1-6}$ alkyl, $C_{3-6}$ cycloalkyl, $C_{7-16}$ aralkyl, and the like. Particularly preferred are $C_{7-10}$ aralkyl (e.g., phenyl-$C_{1-4}$ alkyl such as benzyl, phenylethyl, phenylpropyl, etc.), and the like.

As the "substituent" of "optionally substituted hydrocarbon group" represented by $R^1$, the following 1 to 5 substituents (preferably, 1 to 3 substituents) may be used: (i) halogen (e.g., fluoro, chloro, bromo, iodo, etc.), (ii) nitro, (iii) cyano, (iv) oxo, (v) hydroxy, (vi) optionally halogenated lower alkyl, (vii) optionally halogenated lower alkoxy, (viii) optionally halogenated lower alkylthio, (ix) amino, (x) mono-lower alkylamino (e.g., mono-$C_{1-6}$ alkylamino such as methylamino, ethylamino, propylamino, etc.), (xi) di-lower alkylamino (e.g., di-$C_{1-6}$ alkylamino such as dimethylamino, diethylamino, etc.), (xii) 5- to 7-membered cyclic amino which may contain 1 to 3 heteroatoms selected from, for example, nitrogen, oxygen and sulfur, etc., in addition to carbon atoms and one nitrogen atom (e.g., pyrrolidino, piperidino, piperazino, morpholino, thiomorpholino, etc.), (xiii) lower alkyl-carbonylamino (e.g., $C_{1-6}$ alkyl-carbonylamino such as acetylamino, propionylamino, butyrylamino, etc.), (xiv) lower alkyl-sulfonylamino (e.g., $C_{1-6}$ alkyl-sulfonylamino such as methylsulfonylamino, ethylsulfonylamino, etc.), (xv) lower alkoxy-carbonyl (e.g., $C_{1-6}$ alkoxy-carbonyl such as methoxycarbonyl, ethoxycarbonyl, propoxycarbonyl, etc.), (xvi) carboxy, (xvii) lower alkyl-carbonyl (e.g., $C_{1-6}$ alkyl-carbonyl such as methylcarbonyl, ethylcarbonyl, propylcarbonyl, etc.), (xviii) carbamoyl, thiocarbamoyl, (xix) mono-lower alkyl-carbamoyl (e.g., mono-$C_{1-6}$ alkyl-carbamoyl such as methylcarbamoyl, ethylcarbamoyl, etc.), (xx) di-lower alkyl-carbamoyl (e.g., di-$C_{1-6}$ alkyl-carbamoyl such as dimethylcarbamoyl, diethylcarbamoyl, etc.), (xxi) lower alkylsulfonyl (e.g., $C_{1-6}$ alkylsulfonyl such as methylsulfonyl, ethylsulfonyl, propylsulfonyl, etc.), (xxii) lower alkoxy-carbonyl-lower alkyl (e.g., $C_{1-6}$ alkyl-carbonyl-$C_{1-6}$ alkyl such as methoxycarbonylmethyl, ethoxycarbonylmethyl, tert-butoxycarbonylmethyl, methoxycarbonylethyl, methoxycarbonylmethyl, methoxycarbonyl(dimethyl)methyl, ethoxycarbonyl(dimethyl)methyl, tert-butoxycarbonyl(dimethyl)methyl, etc.), (xxiii) carboxy-lower alkyl (e.g., carboxy-$C_{1-6}$ alkyl such as carboxylmethyl, carboxylethyl, carboxyl(dimethyl)methyl, etc.), (xxiv) optionally substituted heterocycle, (xxv) $C_{6-14}$ aryl (e.g., phenyl, naphthyl, etc.), (xxvi) $C_{7-16}$ aralkyl (e.g., benzyl, etc.), (xxvii) optionally substituted ureido (e.g., ureido, 3-methylureido, 3-ethylureido, 3-phenylureido, 3-(4-fluorophenyl)ureido, 3-(2-methylphenyl)ureido, 3-(4-methoxyphenyl)ureido, 3-(2,4-difluorophenyl)ureido, 3-[3,5-bis(trifluoromethyl)phenyl]ureido, 3-benzylureido, 3-(1-naphthyl)ureido, 3-(2-biphenylyl)ureido, etc.), (xxviii) optionally substituted thioureido (e.g., thioureido, 3-methylthioureido, 3-ethylthioureido, 3-phenylthioureido, 3-(4-fluorophenyl)thioureido, 3-(4-methylphenyl)thioureido, 3-(4-methoxyphenyl)thioureido, 3-(2,4-dichlorophenyl) thioureido, 3-benzylthioureido, 3-(1-naphthyl)thioureido, etc.), (xxix) optionally substituted amidino (e.g., amidino, $N^1$-methylamidino, $N^1$-ethylamidino, $N^1$-phenylamidino, $N^1$, $N^1$-dimethylamidino, $N^1$,$N^2$-dimethylamidino, $N^1$-methyl-$N^1$-ethylamidino, $N^1$,$N^1$-diethylamidino, $N^1$-methyl-$N^1$-phenylamidino., $N^1$,$N^1$-di (4-nitrophenyl)amidino, etc.), (xxx) optionally substituted guanidino (e.g., guanidino, 3-methylguanidino, 3,3-dimethylguanidino, 3,3-diethylguanidino, etc.), (xxxi) optionally substituted cyclic aminocarbonyl (e.g., pyrrolidinocarbonyl, piperidinocarbonyl, (4-methylpiperidino) carbonyl, (4-phenylpiperidino) carbonyl, (4-benzylpiperidino)carbonyl, (4-benzoylpiperidino) carbonyl, [4-(4-fluorobenzoyl) piperidino]carbonyl, (4-methylpiperazino)carbonyl, (4-phenylpiperazino)carbonyl, [4-(4-nitrophenyl)piperazino]-carbonyl, (4-benzylpiperazino)carbonyl, morpholinocarbonyl, thiomorpholinocarbonyl, etc.), (xxxii) optionally substituted aminothiocarbonyl (e.g., aminothiocarbonyl, methylaminothiocarbonyl, dimethylaminothiocarbonyl, etc.), (xxxiii) optionally substituted aminosulfonyl (e.g., aminosulfonyl, methylaminosulfonyl, dimethylaminosulfonyl, etc.), (xxxiv) optionally substituted phenylsulfonylamino (e.g., phenylsulfonylamino, (4-methylphenyl)sulfonylamino, (4-chlorophenyl)sulfonylamino, (2,5-dichlorophenyl)sulfonylamino, (4-methoxyphenyl)sulfonylamino, (4-acetylaminophenyl)sulfonylamino, (4-nitrophenyl)phenylsulfonylamino, etc.), (xxxv) sulfo, (xxxvi) sulfino, (xxxvii) sulfeno, (xxxviii) $C_{1-6}$ alkylsulfo (e.g., methylsulfo, ethylsulfo, propylsulfo, etc.), (xxxix) $C_{1-6}$ alkylsulfino (e.g., methylsulfino, ethylsulfino, propylsulfino, etc.), (xxxx) $C_{1-6}$ alkylsulfeno (e.g., methylsulfeno, ethylsulfeno, propylsulfeno, etc.), (xxxxi) phosphono, (xxxxii) di-$C_{1-6}$ alkoxyphosphoryl (e.g., dimethoxyphosphoryl, diethoxyphosphoryl, dipropoxyphosphoryl, etc.).

Among these substituents, preferred ones include halogen, optionally halogenated alkyl, optionally halogenated alkoxy, hydroxy, nitro, cyano, carboxy, $C_{1-6}$ alkoxy-carbonyl, carbamoyl, aminothiocarbonyl, mono-$C_{1-6}$ alkyl-carbamoyl, di-$C_{1-6}$ alkyl-carbamoyl, amino, mono-$C_{1-6}$ alkylamino, di-$C_{1-6}$ alkylamino, 5- to 7-membered cyclic amino, $C_{1-6}$ alkyl-carbonylamino, phenylsulfonylamino, $C_{1-6}$ alkylsulfonylamino, and the like.

As the "heterocyclic group" of "optionally substituted heterocyclic group" above-mentioned, for example, a group may be used which is formed from a 5- to 14-membered (monocyclic or bi- to tetra-cyclic) heterocycle containing 1 to 6 (preferably, 1 to 4) heteroatoms selected from nitrogen, oxygen, sulfur and the like by removing one hydrogen atom.

The monocyclic heterocyclic group includes those derived from the following monocyclic heterocycles by removing one hydrogen atom: pyridine, pyrazine, pyrimidine, imidazole, furan, thiophene, dihydropyridine, diazepine, oxazepine, pyrrolidine, piperidine, hexamethylenimine, heptamethylenimine, tetrahydrofuran, piperazine, homopiperazine, tetrahydrooxazepine, morpholine, thiomorpholine, pyrrole, pyrazole, 1,2,3-triazole, oxazole, oxazolidine, thiazole, thiazolidine, isoxazole, imidazoline, triazole, thiadiazole, oxadiazole, oxathiadiazole, triazine, tetrazole, and the like.

The bicyclic heterocyclic group includes those derived from the following bicyclic heterocycles by removing one hydrogen atom: indole, dihydroindole, isoindole, dihydroisoindole, benzofuran, dihydrobenzofuran, benzimidazole, benzoxazole, benzisoxazole, benzothiazole, indazole, quinoline, tetrahydroquinoline, isoquinoline, tetrahydroisoquinoline, tetrahydro-1H-1-benzazepine, tetrahydro-1H-2-benzazepine, tetrahydro-1H-3-benzazepine, tetrahydrobenzoxazepine, quinazoline, tetrahydroquinazoline, quinoxaline, tetrahydroquinoxaline, benzodioxane, benzodioxole, benzothiazine, imidazopyridine, and the like.

The tri- or tetra-cyclic heterocyclic group includes those derived from the following tri- or tetra-cyclic heterocycles by removing one hydrogen atom: acridine, tetrahydroacridine, pyrroloquinoline, pyrroloindole, cyclopentaindole, isoindolobenzazepine, and the like.

The "heterocyclic group" preferably includes those derived from monocyclic or bicyclic heterocycles by removing one hydrogen atom.

As for the "substituent" in said "optionally substituted heterocyclic group", those of "optionally substituted heterocycles" represented by the above-mentioned B ring are exemplified. The number of the substituents is 1 to 5.

The "optionally substituted hydrocarbon group" represented by $R^1$ preferably includes $C_{7-16}$ aralkyl (preferably, benzyl, etc.) which may contain 1 to 5 of substituents selected from halogen, $C_{1-6}$ alkyl, $C_{1-6}$ alkoxy, nitro, cyano and hydroxy.

The "acyl" represented by the above-mentioned $R^1$ includes those indicated by the formula: $-(C=0)-R^2$, $-(C=0)-OR^2$, $-(C=O)-NR^2R^3$, $-SO_2-R^2$, $-SO-R^2$, $-(C=S)-OR^2$ or $-(C=S)\ NR^2R^3$ [wherein $R^2$ and $R^3$ each is (i) hydrogen atom, (ii) optionally substituted hydrocarbon group or (iii) optionally substituted heterocyclic group, or $R^2$ and $R^3$ taken each other together with the adjacent nitrogen atom may form an optionally substituted nitrogen-containing cyclic group].

Among these groups, the acyl of the formula $-(C=O)-R^2$ or $-(C=O)-NR^2R^3$, wherein each symbol is as defined above, is preferred.

The "optionally substituted hydrocarbon group" and "optionally substituted heterocyclic group" represented by $R^2$ or $R^3$, respectively, include the same groups as the "optionally substituted hydrocarbon group" and the "optionally substituted heterocyclic group" represented by the above-mentioned $R^1$.

The "optionally substituted nitrogen-containing cyclic group" formed by $R^2$ and $R^3$ includes 5- to 9-membered

(preferably, 5- to 7-membered) nitrogen-containing saturated heterocyclic group which may contain 1 to 3 heteroatoms selected from, for example, nitrogen, oxygen and sulfur, in addition to carbon atoms and one nitrogen atom. Such a group is exemplified, for example, by groups of the formulae:

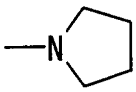,
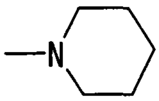,
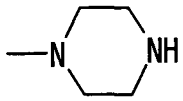,
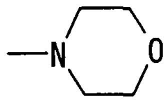,

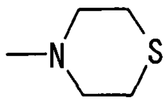,
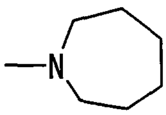,
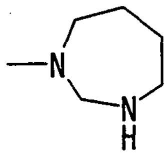,
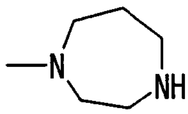

As for the "substituent" of the "optionally substituted nitrogen-containing cyclic group", the same substituent as that of the "optionally substituted heterocycle" represented by the above-mentioned B ring is exemplified. The number of the substituent is 1 to 5.

$R^2$ and $R^3$ preferably includes (i) hydrogen, (ii) optionally halogenated $C_{1-6}$ alkyl, (iii) $C_{6-10}$ aryl optionally substituted by 1 to 3 substituents selected from $C_{1-6}$ alkyl and $C_{1-6}$ alkoxy, (iv) $C_{7-16}$ aralkyl (e.g., benzyl, etc.), (v) 5- or 6-membered heterocyclic group (e.g., pyridyl, thienyl, furyl, etc.), and the like.

The "acyl " represented by the above-mentioned $R^1$, preferably, includes formyl, optionally halogenated $C_{1-6}$ alkyl- carbonyl (e.g., acetyl, trifluoroacetyl, propionyl, etc.), 5- or 6-membered heterocycle-carbonyl (e.g., pyridylcarbonyl, thienylcarbonyl, furylcarbonyl, etc.), $C_{6-14}$ aryl-carbonyl (e.g., benzoyl, 1-naphthoyl, 2-naphthoyl, etc.), $C_{7-16}$ aralkyl-carbonyl (e.g., phenylacetyl, 3-phenylpropionyl, etc.), $C_{6-10}$ aryl-sulfonyl (e.g., benzenesulfonyl, naphthylsulfonyl, etc.), and the like.

$R^1$ is, preferably, hydrogen, $C_{1-6}$ alkyl, $C_{1-6}$ alkyl-carbonyl, $C_{6-14}$ aryl-carbonyl, and the like.

The group of the above-mentioned formula:

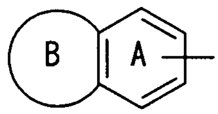

includes groups derived from bicyclic condensed benzene rings by removing one hydrogen atom, which are exemplified by 2,3-dihydrobenzofuran; 3,4-dihydro-2H-1-benzothiopyran; 2,3-dihydro-1H-indole; 1,2,3,4-tetrahydroquinoline; 2,3-dihydro-1H-isoindole; 1,2,3,4-tetrahydroisoquinoline; benzazepine such as 2,3,4,5-tetrahydro-1H-1-benzazepine, 2,3,4,5-tetrahydro-1H-2-benzazepine, 2,3,4,5-tetrahydro-1H-3-benzazepine, etc.; benzazocine such as 1,2,3,4,5,6-hexahydro-1-benzazocine, 1,2,3,4,5,6-hexahydro-2-benzazocine, 1,2,3,4,5,6-hexahydro-3-benzazocine, etc.; benzazonine such as 2,3,4,5,6,7-hexahydro-1H-1-benzazonine, 2,3,4,5,6,7-hexahydro-1H-2-benzazonine, 2,3,4,5,6,7-hexahydro-1H-3-benzazonine, 2,3,4,5,6,7-hexahydro-1H-4-benzazonine, etc.; benzoxazole such as 2,3-dihydrobenzoxazole, etc.; benzothiazole such as 2,3-dihydrobenzothiazole, etc.; benzimidazole such as 2,3-dihydro-1H-benzimidazole, etc.; benzoxazine such as 3,4-dihydro-1H-2,1-benzoxazine, 3,4-dihydro-1H-2,3-benzoxazine, 3,4-dihydro-2H-1,2-benzoxazine, 3,4-dihydro-2H-1,4-benzoxazine, 3,4-dihydro-2H-1,3-benzoxazine, 3,4-dihydro-2H-3,1-benzoxazine, etc.; benzothiazine such as 3,4-dihydro-1H-2,1-benzothiazine, 3,4-dihydro-1H-2,3-benzothiazine, 3,4-dihydro-2H-1,2-benzothiazine, 3,4-dihydro-2H-1,4-benzothiazine, 3,4-dihydro-2H-1,3-benzothiazine, 3,4-dihydro-2H-3,1-benzothiazine, etc.; benzodiazine such as 1,2,3,4-tetrahydrocinnoline, 1,2,3,4-tetrahydrophthalazine, 1,2,3,4-tetrahydroquinazoline, 1,2,3,4-tetrahydroquinoxaline, etc.; benzoxathiin such as 3,4-dihydro-1,2-benzoxathiin, 3,4-dihydro-2,1-benzoxathiin, 2,3-dihydro-1,4-benzoxathiin, 1,4-dihydro-2,3-benzoxathiin, 4H-1,3-benzoxathiin, 4H-3,1-benzoxathiin, etc.; benzodioxin such as 3,4-dihydro-1,2-benzodioxin, 2,3-dihydro-1,4-benzodioxin, 1,4-dihydro-2,3-benzodioxin, 4H-1,3-benzodioxin, etc.; benzdithiin such as 3,4-dihydro-1,2-benzdithiin, 2,3-dihydro-1,4-benzdithiin, 1,4-dihydro-2,3-benzdithiin, 4H-1,3-benzdithiin, etc.; benzoxazepine such as 2,3,4,5-tetrahydro-1,2-benzoxazepine, 2,3,4,5-tetrahydro-1,3-benzoxazepine, 2,3,4,5-tetrahydro-1,4-benzoxazepine, 2,3,4,5-tetrahydro-1,5-benzoxazepine, 1,3,4,5-tetrahydro-2,1-benzoxazepine, 1,3,4,5-tetrahydro-2,3-benzoxazepine, 1,3,4,5-tetrahydro-2,4-benzoxazepine, 1,2,4,5-tetrahydro-3,1-benzoxazepine, 1,2,4,5-tetrahydro-3,2-benzoxazepine, 1,2,3,5-tetrahydro-4,1-benzoxazepine, etc.; benzothiazepine such as 2,3,4,5-tetrahydro-1,2-benzothiazepine, 2,3,4,5-tetrahydro-1,4-benzothiazepine, 2,3,4,5-tetrahydro-1,5-benzothiazepine, 1,3,4,5-tetrahydro-2,1-benzothiazepine, 1,3,4,5-tetrahydro-2,4-benzothiazepine, 1,2,4,5-tetrahydro-3,1-benzothiazepine, 1,2,4,5-tetrahydro-3,2-benzothiazepine, 1,2,3,5-tetrahydro-4,1-benzothiazepine, etc.; benzodiazepine such as 2,3,4,5-tetrahydro-1H-1,2-benzodiazepine, 2,3,4,5-tetrahydro-1H-1,3-benzodiazepine, 2,3,4,5-tetrahydro-1H-1,4-benzodiazepine, 2,3,4,5-tetrahydro-1H-1,5-benzodiazepine, 2,3,4,5-tetrahydro-1H-2,3-benzodiazepine, 2,3,4,5-tetrahydro-1H-2,4-benzodiazepine, etc.; benzodioxepine such as 4,5-dihydro-1,3-benzodioxepine, 4,5-dihydro-3H-1,2-benzodioxepine, 2,3-dihydro-5H-1,4-benzodioxepine, 3,4-dihydro-1H-1,5-benzodioxepine, 4,5-dihydro-1H-2,3-benzodioxepine, 1,5-dihydro-2,4-benzodioxepine, etc.; benzothiepine such as 4,5-dihydro-1H-2,3-benzothiepine, 1,5-dihydro-2,4-benzothiepine, 3,4-dihydro-2H-1,5-benzothiepine, 2,3-dihydro-5H-1,4-benzothiepine, etc.; benzoxazocine such as 3,4,5,6-tetrahydro-2H-1,5-benzoxazocine, 3,4,5,6-tetrahydro-2H-1,6-benzoxazocine, etc.; benzothiazocine such as 3,4,5,6-tetrahydro-2H-1,5-benzothiazocine, 3,4,5,6-tetrahydro-2H-1,6-benzothiazocine, etc.; benzodiazocine such as 1,2,3,4,5, 6-hexahydro-1,6-benzodiazocine, etc.; benzoxathiocine such as 2,3,4,5-tetrahydro-1,6-benzoxathiocine, etc.; benzodioxocine such as 2,3,4,5-tetrahydro-1,6-benzodioxocine, etc.; benzotrioxepine such as 1,3,5-benzotrioxepine, 5H-1,3,4-benzotrioxepine, etc.; benzoxathiazepine such as 3,4-dihydro-2H-5,2,1-benzoxathiazepine, 3,4-dihydro-2H-5,1,2-benzoxathiazepine, 4,5-dihydro-3,1,4-benzoxathiazepine, 4,5-dihydro-3H-1,2,5-benzoxathiazepine, etc.; benzoxadiazepine such as 2,3,4,5-tetrahydro-1,3,4-benzoxadiazepine, etc.; benzthiadiazepine such as 2,3,4,5-tetrahydro-1,3,5-benzthiadiazepine, etc.; benzotriazepine such as 2,3,4,5-tetrahydro-1H-1,2,5-benzotriazepine, etc.; 4,5-dihydro-1,3,2-benzoxathiepine, 4,5-dihydro-1H-2,3-benzoxathiepine, 3,4-dihydro-2H-1,5-benzoxathiepine, 4,5-dihydro-3H-1,2-benzoxathiepine, 4,5-dihydro-3H-2,1-benzoxathiepine, 2,3-dihydro-5H-1,4-benzoxathiepine, 2,3-dihydro-5H-4,1-benzoxathiepine, etc.; particularly, 2,3,4,5-tetrahydro-1H-3-benzazepine, 2,3,4,5-tetrahydro-1H-2-benzazepine, 2,3-dihydro-1H-indole, 2,3,4,5-tetrahydro-1,4-benzoxazepine, and the like.

Among these groups, preferred ones are exemplified by groups of the formula:

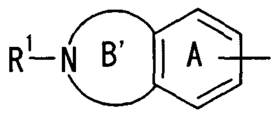

wherein the B' ring is a 5- to 9-membered nitrogen-containing heterocycle which may further be substituted; and the other symbols are as defined above.

The "5- to 9-membered nitrogen-containing heterocycle" of said "5- to 9-membered nitrogen-containing heterocycle which may further be substituted" includes 5- to 9-membered nitrogen-containing heterocycles which may contain 1 to 3 heteroatoms selected from, for example, nitrogen, oxygen and sulfur, in addition to carbon atoms and one nitrogen atom. Preferably, 5- to 9-membered non-aromatic nitrogen-containing heterocycles (e.g., pyrrolidine, piperidine, hexamethylenimine, heptamethylenimine, piperazine, homopiperazine, tetrahydrooxazepine, morpholine, thiomorpholine, etc.) is used. The "substituent" includes 1 to 3 of substituents selected from the same "substituents" as those of the "optionally substituted heterocycle" represented by the above-mentioned B ring, but oxo is preferred.

Among these heterocycles, particularly preferred ones are exemplified by the groups of the formula:

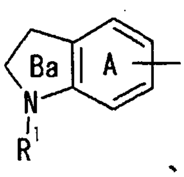 , 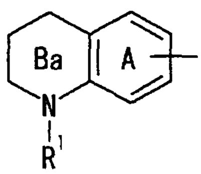 , 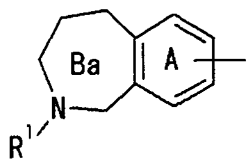 , 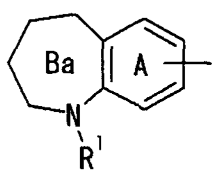 ,

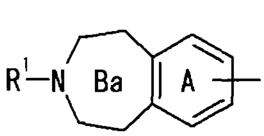 , 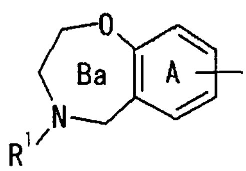 or 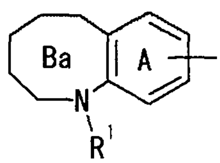

wherein the Ba ring is optionally substituted heterocycle and the other symbols are as defined above. Particularly preferred are the groups represented by the formula:

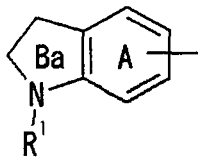 or 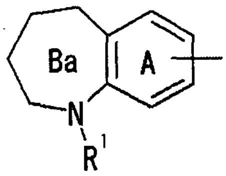

wherein the symbols are as defined above.

The "substituent" in the "optionally substituted heterocycle" represented by the Ba ring includes 1 to 3 of substituents selected from the same "substituents" as those of the "optionally substituted heterocycle" represented by the above-mentioned B ring, but oxo is preferred.

When the phenyl of "optionally condensed phenyl which may be substituted" in the above-mentioned item (2), is condensed with an optionally substituted bicyclic heterocycle or with two identical or different monocycles (provided that at least one of them is a monocyclic heterocycle), such groups are exemplified by those of the formula:

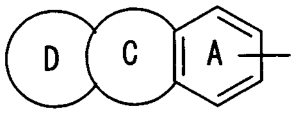 , 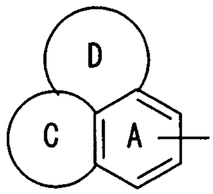 ,

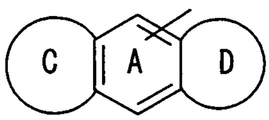 or 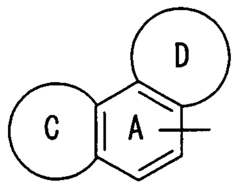

wherein the A ring is as defined above; one of the C and D rings is an optionally substituted heterocycle and the other is an optionally substituted 5- to 9-membered ring.

As for the "heterocycle" of "optionally substituted heterocycle" represented by the C or D ring, those of "optionally substituted heterocycle" represented by the B ring are exemplified. The "5- to 9-membered ring" of "optionally substituted 5- to 9-membered ring" represented by the C or D ring may have 1 to 3 heteroatoms selected from nitrogen, oxygen and sulfur, and includes, for example, 5- to 9-membered heterocycles (e.g., pyridine, pyrazine, pyrimidine, imidazole, furan, thiophene, dihydropyridine, diazepine, oxazepine, pyrrolidine, piperidine, hexamethylenimine, heptamethylenimine, tetrahydrofuran, piperazine, homopiperazine, tetrahydrooxazepine, morpholine, thiomorpholine, etc.), 5- to 9-membered carbocycles (e.g., benzene, cyclopentane, cyclopentene, cyclohexane, cyclohexene, cyclohexadiene, cycloheptane, cycloheptene, cycloheptadiene, etc.), and the like. Among them, those of the 5- to 7-membered rings are preferred. Benzene, cyclohexane, and the like are particularly preferred.

As for the "substituent" of "optionally substituted 5-to 9-membered ring", the same "substituent" as that of the "optionally substituted heterocycle" represented by the above-mentioned B ring can be exemplified.

The group of the above-mentioned formula:

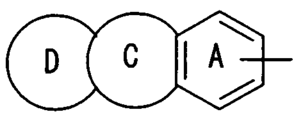

wherein each symbol is as defined above, includes the groups derived from tricyclic condensed benzene rings by

removing one hydrogen atom, which are exemplified by carbazole, 1,2,3,4,4a,9a-hexahydrocarbazole, 9,10-dihydroacridine, 1,2,3,4-tetrahydroacridine, 10, 11-dihydro-5H-dibenz[b,f]azepine, 5,6,7,12-tetraydrodibenz[b,g]azocine, 6,11-dihydro-5H-dibenz[b,e]azepine, 6,7-dihydro-5H-dibenz[c,e]azepine, 5,6,11,12-tetrahydrodibenz[b,f]azocine, dibenzofuran, 9H-xanthene, 10,11-dihydrodibenz[b,f]oxepine, 6,11-dihydrodibenz[b,e]oxepine, 6,7-dihydro-5H-dibenz[b,g]oxocine, dibenzothiophene, 9H-thioxanthene, 10,11-dihydro-dibenzo[b,f]thiepine, 6,11-dihydrodibenzo[b,e]thiepine, 6,7-dihydro-5H-dibenzo[b,g]thiocine, 10H-phenothiazine, 10H-phenoxazine, 5,10-dihydrophenazine, 10,11-dibenzo[b,f][1,4]thiazepine, 10,11-dihydrodibenz[b,f][1,4]oxazepine, 2,3,5,6,11,11a-hexahydro-1H-pyrrolo[2,1-b][3]benzazepine, 10,11-dihydro-5H-dibenzo[b,e][1,4]diazepine, 5,11-dihydrodibenz[b,e][1,4]oxazepine, 5,11-dihydrodibenzo[b,f][1,4]thiazepine, 10,11-dihydro-5H-dibenzo[b,e][1,4]diazepine, 1,2,3,3a,8,8a-hexahydropyrrolo[2,3-b]indole, and the like.

The group of the above-mentioned formula:

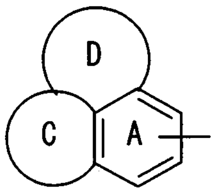

wherein each symbol is as defined above, includes the groups derived from tricyclic condensed benzene rings by removing one hydrogen atom, which are exemplified by 1H,3H-naphtho[1,8-cd][1,2]oxazine, naphtho[1,8-de]-1,3-oxazine, naphtho[1,8-de]-1,2-oxazine, 1,2,2a,3,4,5-hexahydrobenz[cd]indole, 2,3,3a,4,5,6-hexahydro-1H-benzo[de]quinoline, 4H-pyrrolo[3,2,1-ij]quinoline, 1,2,5,6-tetrahydro-4H-pyrrolo[3,2,1-ij]quinoline, 5,6-dihydro-4H-pyrrolo-[3,2,1-ij]quinoline, 1H,5H-benzo[ij]quinolizine, azepino[3,2,1-hj]indole, 1,2,4,5,6,7-hexahydroazepino[3,2,1-hi]indole, 1H-pyrido[3,2,1-jk][1]benzazepine, 5,6,7,8-tetrahydro-1H-pyrido[3,2,1-jk][1]benzazepine, 1,2,5,6,7,8-hexahydro-1H-pyrido[3,2,1-jk] [1]benzazepine, 2,3-dihydro-1H-benz [de] isoquinoline, 1,2,3,4,4a,5,6,7-octahydronaphtho-[1,8-bc]azepine, 2,3,5,6,7,8-hexahydro-1H-pyrido[3,2,1-jk] [1] benzazepine, and the like.

The group of the above-mentioned formula:

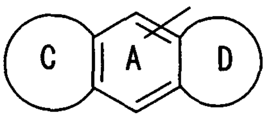

wherein each symbol is as defined above, includes the groups derived from tricyclic condensed benzene rings by removing one hydrogen atom, which are exemplified by 1,2,3,5,6,7-hexahydrobenzo[1,2-b:4,5-b']dipyrrole, 1,2,3,5,6,7-hexahydrocyclopent[f]indole, and the like.

The group of the above-mentioned formula:

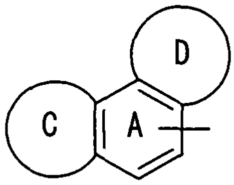

wherein each symbol is as defined above, includes the groups derived from tricyclic condensed benzene rings by removing one hydrogen atom, which are exemplified by 1,2,3,6,7,8-hexahydrocyclopent[e] indole, 2,3,4,7,8,9-hexahydro-1H-cyclopenta[f]quinoline, and the like.

Among these groups, preferred ones are exemplified by the groups of the formula:

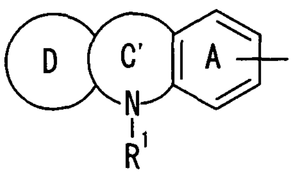,
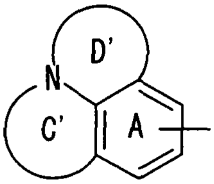
or
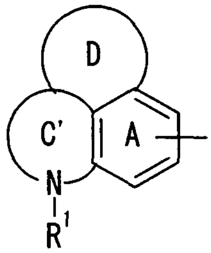

wherein each of the C' and D' rings is a 5- to 9-membered nitrogen-containing heterocycle which may further be substituted; the other symbols are as defined above. Among them, the groups of the formula:

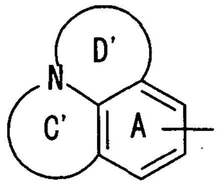

wherein each symbol is as defined above, are particularly preferred.

As for the "5- to 9-membered nitrogen-containing heterocycle which may further be substituted" represented by the C' or D' ring, the same heterocycle as the "5- to 9-membered nitrogen-containing heterocycle which may further be substituted" represented by the B' ring may be exemplified.

Among them, the group of the formula:

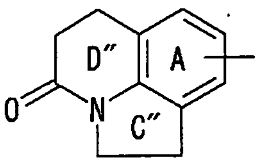
or
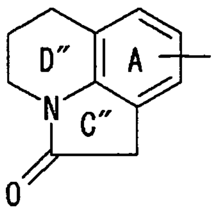

wherein each of the C" and D" rings is optionally substituted nitrogen-containing heterocycle; the other symbols are as defined above, is particularly preferred.

The "substituent" of the "optionally substituted nitrogen-containing heterocycle" represented by the C" and D" rings includes 1 or 2 substituents selected from the same "substituent" as that of the "optionally substituted heterocycle" represented by the B ring except for oxo.

When the phenyl group of the "optionally condensed phenyl group which may be substituted" in the above-mentioned item (3), is condensed with an optionally substituted tricyclic heterocycle, such groups are exemplified by those of the formula:

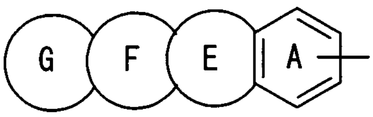
or
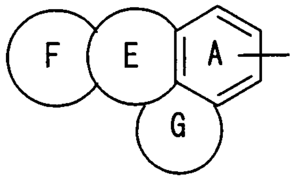

wherein the A ring is as defined above; at least one of the E, F and G rings is an optionally substituted heterocycle and the other is an optionally substituted 5- to 9-membered ring.

The "optionally substituted heterocycle" and the "optionally substituted 5- to 9-membered ring" represented by the E, F or G ring are exemplified by the "optionally substituted heterocycle" and the "optionally substituted 5-to 9-membered ring" represented by the B or C ring.

Among them, the followings are preferred:

(i) Groups of the formula:

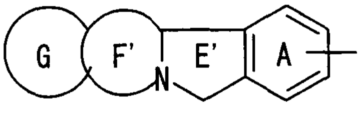,
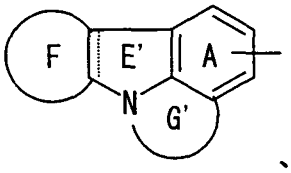,
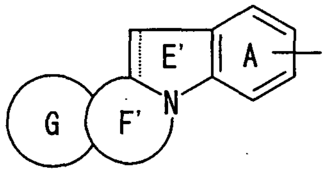,

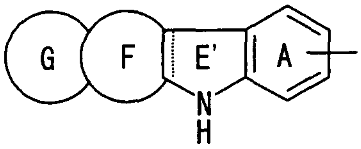
or
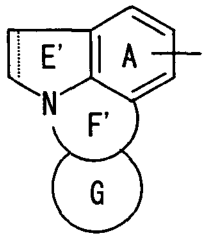

wherein the A ring is as defined above; the E', F' and G' rings each is an optionally substituted 5- to 9-membered nitrogen-containing heterocycle; and --- indicates a single bond or a double bond; the other symbols are as defined above;

(ii) Groups derived from cyclic compounds by removing one hydrogen atom, which are exemplified by fluoranthene, acephenanthrylene, aceanthrylene, triphenylene, pyrene, chrysene, naphthacene, pleiadene, benzo[a]anthracene, indeno[1,2-a]indene, cyclopenta[a]phenanthrene, pyrido-[1',2':1,2]imidazo[4,5-b]quinoxaline, 1H-2-oxapyrene, spiro[piperidin-4,9'-xanthene], and the like; and their dihydro-, tetrahydro-, hexahydro-, octahydro-, decahydro-derivatives, etc.

As for the "optionally substituted 5- to 9-membered nitrogen-containing heterocycle" represented by the E', F' or G' ring, the same heterocycle as that of the "optionally substituted 5- to 9-membered nitrogen-containing heterocycle" represented by the B' ring can be exemplified. As the substituent thereof, oxo is preferred.

The group of the above-mentioned formula:

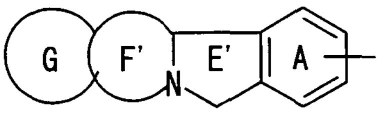

wherein each symbol is as defined above, includes the groups derived from tetracyclic condensed benzene rings by removing one hydrogen atom, which are exemplified by 2H-isoindolo[2,1-e]purine, 1H-pyrazolo[4',3':3,4]pyrido[2,1-a]isoindole, 1H-pyrido[2',3':4,5]imidazo[2,1-a]isoindole, 2H,6H-pyrido[1',2':3,4]imidazo[5,1-a]isoindole, 1H-isoindolo[2,1-a]benzimidazole, 1H-pyrido[3',4':4,5]pyrrolo[2,1-a]isoindole, 2H-pyrido[4',3':4,5]pyrrolo[2,1-a]isoindole, 1H-isoindolo[2,1-a]indole, 2H-isoindolo[1,2-a]isoindole, 1H-cyclopenta[4,5]pyrimido[2,1-a]isoindole, 2H,4H-pyrano [4',3':4,5] [1,3] oxazino [2,3-a]isoindole, 2H-isoindolo[2,1-a][3,1]benzoxazine, 7H-isoindolo-[1,2-b][1,3]benzoxazine, 2H-pyrido[2',1':3,4]pyrazino[2,1-a]isoindole, pyrido[2',3':4,5]pyrimido[2,1-a]isoindole, pyrido[3',2':5,6]pyrimido[2,1-a]isoindole, 1H-pyrido[1',2':3,4]pyrimido[2,1-a]isoindole, isoindolo[2,1-a]quinazoline, isoindolo[2,1-a]quinoxaline, isoindolo[1,2-a]isoquinoline, isoindolo[2,1-b]isoquinoline, isoindolo[2,1-a]quinoline, 6H-oxazino-[3',4':3,4][1,4]diazepino[2,1-a]isoindole, azepino[2' ,1' : 3,4]pyrazino[2,1-a]isoindole, 2H,6H-pyrido[2',1':3,4][1,4]-diazepino[2,1-a]isoindole, 1H-isoindolo[1,2-b][1,3,4]benzotriazepine, 2H-isoindolo[2,1-a][1,3,4]benzotriazepine, isoindolo[2,1-d][1,4]benzoxazepine, 1H-isoindolo[2,1-b][2,4]-benzodiazepine, 1H-isoindolo[2,1-c][2,3]benzodiazepine, 2H-isoindolo[1,2-a][2,4]benzodiazepine, 2H-isoindolo[2,1-d]-[1,4]benzodiazepine, 5H-indolo[2,1-b][3]benzazepine, 2H-isoindolo[1,2-a][2]benzazepine, 2H-isoindolo[1,2-b]

[3]-benzazepine, 2H-isoindolo[2,1-b][2]benzazepine, 2H-iso-indolo[1,2-b][1,3,4]benzoxazocine, isoindolo[2,1-b][1,2, 6] benzotriazocine, 5H-4,8-methano-1H-[1,5]diazacyclo-undecino[1,11-a]indole, and the like.

The group of the above-mentioned formula:

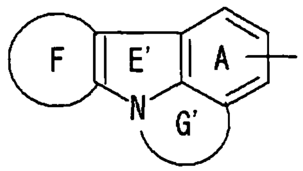

wherein each symbol is as defined above, includes the groups derived from tetracyclic condensed benzene rings by removing one hydrogen atom, which are exemplified by 1H,4H-pyrrolo[3',2':4,5]pyrrolo[3,2,1-ij]quinoline, pyrrolo[3,2,1-jk] carbazole, 1H-furo[2',3':4,5]pyrrolo[3,2,1-ij]-quinoline, 1H,4H-cyclopenta[4,5]pyrrolo[1,2,3-de]quinoxaline, 1H,4H-cyclopenta[4,5]pyrrolo[3,2,1-ij]quinoline, pyrido[3',4':4,5]pyrrolo[1,2,3-de]benzoxazine, [1,4]oxazino[2,3,4-jk]carbazole, 1H,3H-[1,3]oxazino[5,4,3-jk]carbazole, pyrido[3',4':4,5]pyrrolo[1,2,3-de][1,4]benzothiazine, 4H-pyrro[3,2,1-de]phenanthridine, 4H,5H-pyrido[3,2,1-de]phenanthridine, 1H,4H-3a,6a-diazafluoroanthene, 1-oxa-4,6a-diazafluoroanthene, 4-oxa-2,10b-diazafluoroanthene, 1-thia-4,6a-diazafluoroanthene, 1H-pyrazino[3,2,1-jk]carbazole, 1H-indolo[3,2,1-de] [1,5]naphthyridine, benzo[b]pyrano[2,3,4-hi]indolizine, 1H,3H-benzo[b]pyrano[3,4,5-hi]indolizine, 1H,4H-pyrano[2',3': 4,5]pyrrolo[3,2,1-ij]quinoline, 1H,3H-benzo[b]thiopyrano[3,4,5-hi]indolizine, 1H-pyrido[3,2,1-jk]carbazole, 4H-3-oxa-11b-azacyclohepta[jk]fluorene, 2H-azepino[1',2':1,2]pyrimidino[4,5-b]indole, 1H,4H-cyclohepta[4,5]pyrrolo[1,2,3-de] quinoxaline, 5H-pyrido[3',4':4,5]pyrrolo[1,2,3-ef][1,5]benzoxazepine, 4H-pyrido[3',4':4,5]pyrrolo[3,2,1-jk][4,1]benzothiazepine, 5H-pyrido[3',4':4,5]pyrrolo[1,2,3-ef][1,5]benzothiazepine, 5H-pyrido[4',3':4,5]pyrrolo[1,2,3-ef][1,5]benzothiazepine, [1,2,4]triazepino[6,5,4-jk]carbazole, [1,2,4]triazepino[6,7,1-jk]carbazole, [1,2,5]triazepino[3,4,5-jk]carbazole, 5H-[1,4]oxazepino-[2,3,4-jk]carbzole, 5H-[1,4]thiazepino[2,3,4-jk]carbazole, [1,4]diazepino[3,2,1-jk]carbazole, [1,4]diazepino[6,7,1-jk]carbazole, azepino[3,2,1-jk]carbazole, 1H-cycloocta[4,5]pyrrolo[1,2,3-de]quinoxaline, 1H-cycloocta [4,5]pyrrolo[3,2,1-ij]quinoline, and the like.

The group of the above-mentioned formula:

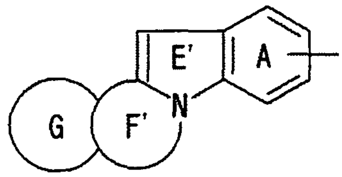

wherein each symbol is as defined above, includes the groups derived from tetracyclic condensed benzene rings by removing one hydrogen atom, which are exemplified by 1H-indolo[1,2-a]benzimidazole, 1H-indolo[1,2-b]indazole, pyrrolo [2',1':3,4]pyrazino[1,2-a]indole, 1H,5H-pyrrolo[1',2':4,5]pyrazino[1,2-a]indole, 2H-pyrido[2',3':3,4]pyrrolo[1,2-a]indole, 1H-pyrrolo[2',3':3,4]pyrido[1,2-a]indole, 1H-indolo[1,2-a]indole, 6H-isoindolo[2,1-a]indole, 6H-indolo[1,2-c][1,3]benzoxazine, 1H-indolo[1,2-b][1,2]benzothiazine, pyrimido[4',5':4,5]pyrimido[1,6-a]indole, pyrazino[2',3':3,4]pyrrido[1,2-a]indole, 6H-pyrido[1',2': 3,4]pyrimido[1,6-a]indole, indolo[1,2-b]cinnoline, indolo-[1,2-a]quinazoline, indolo[1,2-c]quinazoline, indolo[2,1-b]quinazoline, indolo[1,2-a]quinoxaline, indolo[1,2-a]-[1,8]naphthyridine, indolo[1,2-b]-2,6-naphthyridine, indolo[1,2-b][2,7]naphthyridine, indolo[1,2-h]-1,7-naphthyridine, indolo[1,2-b]isoquinoline, indolo[1,2-a]isoquinoline, indolo [1,2-a] quinoline, 2H,6H-pyrido[2',1':3,4][1,4]diazepino[1,2-a]indole, 1H-indolo[2,1-c][1,4]benzodiazepine, 2H-indolo[1,2-d][1,4]benzodiazepine, 2H-indolo[2,1-a][2,3]benzodiazepine, 2H-indolo[2,1-b][1,3]benzodiazepine, 1H-indolo [1,2-b][2]benzazepine, 2H-indolo[1,2-a][1]benzazepine, 2H-indolo[2,1-a][2]benzazepine, indolo[1,2-e][1,5]benzodiazocine, indolo[2,1-b][3]benzazocine, and the like.

The group of the above-mentioned formula:

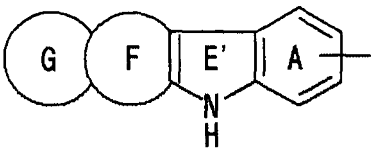

wherein each symbol is as defined above, includes the groups derived from tetracyclic condensed benzene rings by removing one hydrogen atom, which are exemplified by 1H-imidazo[1',2':1,2]pyrido[3,4-b]indole, 1H-imidazo[1',2':1,6]pyrido[4,3-b]indole, 1H-imidazo[1',5':1,2]pyrido[3,4-b]indole, 1H-imidazo[1',5':1,6]pyrido[4,3-b]indole, 1H-imidazo-[2',1':2,3]pyrido[4,5-b]indole, imidazo[4,5-a]-carbazole, imidazo[4,5-c]carbazole, pyrazolo[3,4-c]carbazole, 2H-pyrazino[1',2':1,5]pyrrolo[2,3-b]indole, 1H-pyrrolo[1',2':1, 2]pyrimido[4,5-b]indole, 1H-indolizino[6,7-b]indole, 1H-indolizino[8,7-b]indole, indolo[2,3-b]indole, indolo[3,2-b]indole, pyrrolo[2,3-a]carbazole, pyrrolo[2,3-b]carbazole, pyrrolo[2,3-c]carbazole, pyrrolo[3,2-a]carbazole, pyrrolo-[3,2-b]carbazole, pyrrolo[3,2-c]carbazole, pyrrolo[3,4-a]-carbazole, pyrrolo[3,4-b]carbazole, pyrrolo[3,4-c]carbazole, 1H-pyrido[3',4':4,5]furo[3,2-b]indole, 1H-furo[3,4-a]-carbazole, 1H-furo[3,4-b]carbazole, 1H-furo[3,4-c]carbazole, 2H-furo[2,3-a]carbazole, 2H-furo[2,3-c]carbazole, 2H-furo-[3,2-a]carbazole, 2H-furo[3,2-c]carbazole, 1H-pyrido[3',4':4,5]thieno[2,3-b]indole, thieno[3',2':5,6]thiopyrano[4,3-b]indole, thieno[3',4':5,6]thiopyrano[4,3-b]indole, 1H-[1]-benzothieno[2,3-b]indole, 1H-[1]-benzothieno[3,2-b]indole, 1H-thieno[3,4-a]carbazole, 2H-thieno[2,3-b]carbazole, 2H-thieno[3,2-a]carbazole, 2H-thieno[3,2-b]carbazole, cyclopenta[4,5]pyrrolo[2,3-f]quinoxaline, cyclopenta[5,6]pyrido[2,3-b]indole, pyrido[2',3':3,4]cyclopenta[1,2-b]indole, pyrido[2',3':4,5]cyclopenta[1,2-b]indole, pyrido[3',4':3,4]cyclopenta[1,2-b]indole, pyrido[3',4':4,5]cyclopenta[1,2-b]indole, pyrido[4',3':4,5]cyclopenta[1,2-b]indole, 1H-cyclopenta[5,6]pyrano[2,3-b]indole, 1H-cyclopenta[5,6]thiopyrano[4,3-b]indole, cyclopenta[a]carbazole, cyclopenta[c]-carbazole, indeno[1,2-b]indole, indeno[2,1-b]indole, [1,2,4]triazino[4',3':1,2]pyrido[3,4-b]indole, 1,3,5-triazino[1',2':1,1]pyrido[3,4-b]indole, 1H-[1,4]oxazino-[4',3':1,2]pyrido[3,4-b]indole, 1H-[1,4]oxazino[4',3':1,6]-pyrido[3,4-b]indole, 4H-[1,3]oxazino[3',4':1,2]pyrido[3,4-b]indole, indo-lo[3,2-b][1,4]benzoxazine, 1,3-oxazino[6,5-b]carbazole, 2H-pyrimido[2',1':2,3][1,3]thiazino[5,6-b]indole, 2H-[1,3]thiazino[3',2':1,2]pyrido[3,4-b]indole, 4H-[1,3]thiazino[3',4':1,2]pyrido[3,4-b]indole, indolo[2,3-b][1,4]benzothiazine, indolo[3,2-b][1,4]benzothiazine, indolo[3,2-c][2,1]benzothiazine, 1,4-thiazino[2,3-a]carbazole, [1,4]-thiazino[2,3-b]carbazole, [1,4]thiazino[2,3-c]carbazole, 1,4-thiazino[3,2-b]carbazole, 1,4-thiazino[3,2-c]carbazole, 1H-indolo[2,3-g]pteridine, 1H-indolo[3,2-g]pteridine, pyrazino[1',2':1,2]pyrido[3,4-b]indole, pyrazino[1',2':1,2]pyrido[4,3-b]indole, 1H-pyrido[2',3':5,6]pyrazino[2,3-b]indole, 1H-pyrido[3',2':5,6]pyrazino[2,3-b]indole, 1H-pyrido[3',4':5,6]pyrazino[2,3-b]indole, pyrido[1',2':1,2]-pyrimido[4,5-b]indole, pyrido[1',2':1,2]pyrimido[5,4-b]-indole, pyrido[2',1':2,3]pyrimido[4,5-b]indole, pyrido-[1',2':1,2]pyrido[3,4-b]indole, pyrimido[1',2':1,6]pyrido-[3,4-b]indole, pyrimido[5',4':5,6]pyrano[2,3-b]indole, pyridazino[4',5':5,6]thiopyrano[4,5-b]indole, 1H-indolo-[3,2-c]cinnoline, 1H-indolo[2,3-b]quinoxaline, 1H-pyrazino-[2,3-a]carbazole, 1H-pyrazino[2,3-b]carbazole, 1H-pyrazino-[2,3-c]carbazole, 1H-pyridazino[3,4-c]carbazole, 1H-pyridazino[4,5-b]carbazole, 1H-pyrimido[4,5-a]carbazole, 1H-pyrimido[4,5-c]carbazole, 1H-pyrimido[5,4-a]carbazole, 1H-pyrimido[5,4-b]carbazole, 1H-pyrimido[5,4-c]carbazole, 7H-1,4-dioxino[2',3':5,6][1,2]dioxino[3,4-b]indole, 6H-[1,4]benzodioxino[2,3-b]indole, 6H-[1,4]benzodithiino[2,3-b]indole, 1H-indolo[2,3-b]-1,5-naphthyridine, 1H-indolo-[2,3-b][1,6]naphthyridine, 1H-indolo [2,3-b] [1,8] naphthyridine, 1H-indolo[2,3-c]-1,5-naphthyridine, 1H-indolo[2,3-c] [1,6]naphthyridine, 1H-indolo [2, 3-c] [1, 7] naphthyridine, 1H-indolo[2,3-c][1,8]naphthyridine, 1H-indolo[3,2-b]-1,5-naphthyridine, 1H-indolo[3,2-b] [1,7]naphthyridine, 1H-indolo[3,2-b][1,8]naphthyridine, 1H-indolo[3,2-c][1,8]naphthyridine, indolo[2,3-a]quinolizine, indolo[2,3-b]quinolizine, indolo[3,2-a]quinolizine, indolo[3,2-b]quinolizine, pyrano[4',3' :5,6]pyrido[3,4-b]indole, pyrido[4',3':4,5]pyrano[3,2-b]indole, pyrido[4',3':5,6]pyrano[2,3-b]indole, pyrido[4',3':5,6]pyrano[3,4-b]indole, 1H-indolo[2,3-c]isoquinoline, 1H-indolo[3,2-c]isoquinoline, 1H-indolo[2,3-c]quinoline, 1H-indolo[3,2-c]quinoline, 1H-pyrido[2,3-a]carbazole, 1H-pyrido[2,3-b]carbazole, 1H-pyrido[2,3-c]carbazole, 1H-pyrido[3,2-a]carbazole, 1H-pyrido[3,2-b]carbazole, 1H-pyrido[3,2-c]carbazole, 1H-pyrido[3,4-a]carbazole, 1H-pyrido[3,4-b]carbazole, 1H-pyrido[3,4-c]carbazole, 1H-pyrido[4,3-a]carbazole, 1H-pyrido[4,3-b]carbazole, 1H-pyrido[4,3-c]carbazole, 1H-quindoline, 1H-quinindoline, 1H-pyrano[3',4':5,6]pyrano[4,3-b]indole, [1]-benzopyrano[2,3-b]indole, [1]benzopyrano[3,2-b]indole, [1]-benzopyrano[3,4-b]indole, [1]benzopyrano[4,3-b]indole, [2]-benzopyrano[4,3-b]indole, pyrano[2,3-a]carbazole, pyrano[2,3-b]carbazole, pyrano[2,3-c]carbazole, pyrano[3,2-a]-carbazole, pyrano[3,2-c]carbazole, pyrano[3,4-a]carbazole, 1H-phosphinolino[4,3-b]indole, [1]benzothiopyrano[2,3-b]indole, [1]benzothiopyrano[3,2-b]indole, [1]benzothiopyrano[3,4-b]indole, [1]benzothiopyrano[4,3-b]indole, [2]benzothiopyrano[4,3-b]indole, 1H-benzo[a]carbazole, 1H-benzo[b]carbazole, 1H-benzo[c]carbazole, [1,6,2]oxathiazepino[2',3':1,2]pyrido[3,4-b]indole, 1H-azepino[1',2':1,2]pyrido[3,4-b]indole, 1H-pyrido[1',2':1,2]azepino[4,5-b]indole, 2H-pyrido[1',2':1,2]azepino[3,4-b]indole, 1H-pyrido[3',2':5,6]oxepino[3,2-b]indole, 1H-pyrido[4',3':5,6]oxepino[3,2-b]indole, 2H-pyrido[2',3':5,6]oxepino[2,3-b]indole, 2H-pyrido[2',3':5,6]oxepino[3,2-b]indole, 2H-pyrido-[3',4':5,6]oxepino[3,2-b]indole, pyrido[2',3':4,5]cyclohepta[1,2-b]indole, pyrido[3',2':3,4]cyclohepta[1,2-b]indole, pyrido[3',4':4,5]cyclohepta[1,2-b]indole, pyrido[3',4':5,6]cyclohepta[1,2-b]indole, 2H-pyrano[3',2':2,3]azepino[4,5-b]indole, 1H-indolo[3,2-b][1,5]benzoxazepine, 1H-indolo[3,2-d][1,2]benzoxazepine,

1H-indolo[2,3-c][1,5]benzothiazepine, [1,4]diazepino[2,3-a]carbazole, indolo[2,3-b][1,5]benzodiazepine, indolo[2,3-d][1,3]benzodiazepine, indolo[3,2-b][1,4]benzodiazepine, indolo[3,2-b][1,5]benzodiazepine, indolo[3,2-d][1,3]benzodiazepine, indolo[3,2-d][2,3]benzodiazepine, indolo[2,3-a][3]benzazepine, indolo[2,3-c][1]benzazepine, indolo[2,3-d][1]benzazepine, indolo[2,3-d][2]benzazepine, indolo[3,2-b][1]benzazepine, indolo[3,2-c][1]benzazepine, indolo[3,2-d][1]benzazepine, 1H-indolo[2,1-b][3]benzazepine, 1H-[1]benzoxepino[5,4-b]indole, 1H-[2]benzoxepino[4,3-b]indole, 1H-[1]benzothiepino[4,5-b]-indole, 1H-[1]benzothiepino[5,4-b]indole, benzo[3,4]cyclohepta[1,2-b]indole, benzo[4,5]cyclohepta[1,2-b]indole, benzo[5,6]cyclohepta[1,2-b]indole, benzo[6,7]cyclohepta[1,2-b]indole, cyclohepta[b]carbazole, 4H-[1,5]oxazocino[5',4':1,6]pyrido[3,4-b]indole, azocino[1',2':1,2]pyrido[3,4-b]indole, 2,6-methano-2H-azecino[4,3-b]indole, 3,7-methano-3H-azecino-[5,4-b]indole, pyrido[1',2':1,8]azocino[5,4-b]indole, pyrido-[4',3':6,7]oxocino[2,3-b]indole, pyrido-[4',3':6,7]oxocino[4,3-b]indole, 1,5-methano-1H-azecino[3,4-b]indole, 2,6-methano-1H-azecino[5,4-b]indole, 1H-pyrido[3',4':5,6]cycloocta[1,2-b]indole, 1,4-ethanooxocino[3,4-b]indole, pyrano[3',4':5,6]cycloocta[1,2-b]indole, 1H-indolo[2,3-c][1,2,5,6]benzotetrazocine, 1H-indolo[2,3-c][1,6]benzodiazocine, 6,13b-methano-13bH-azecino[5,4-b]indole, oxocino[3,2-a]carbazole, 1H-benzo[g]cycloocta[b]indole, 6,3-(iminomethano)-2H-1,4-thiazonino[9,8-b]indole, 1H,3H-[1,4]oxazonino[4',3':1,2]pyrido[3,4-b]indole, 2H-3,6-ethanoazonino[5,4-b]indole, 2H-3,7-methanoazacycloundecino[5,4-b]indole, 1H-6,12b-ethanoazonino[5,4-b]indole, indolo[3,2-e] [2]benzazonine, 5,9-methanoazacycloundecino[5,4-b]indole, 3,6-ethano-3H-azecino[5,4-b]indole, 3,7-methano-3H-azacycloundecino[5,4-b]indole, pyrano[4',3':8,9]azecino[5,4-b]-indole, 1H-indolo[2,3-c][1,7]benzodiazecine, 1H-indolo[3,2-e][2]benzazecine, benzo[e]pyrrolo[3,2-b]indole, benzo[e]-pyrrolo[3,2-g]indole, benzo[e]pyrrolo[3,2,1-hi]indole, benzo[e]pyrrolo[3,4-b]indole, benzo[g]pyrrolo[3,4-b]indole, 1H-benzo[f]pyrrolo[1,2-a]indole, 1H-benzo[g]pyrrolo[1,2-a]indole, 2H-benzo[e]pyrrolo[1,2-a]indole, 1H-benzo[f]-pyrrolo[2,1-a]isoindole, 1H-benzo[g]pyrrolo[2,1-a]isoindole, 2H-benzo[e]pyrrolo[2,1-a]isoindole, isoindolo[6,7,1-cde]-indole, spiro[cyclohexane-1,5'-[5H]pyrrolo[2,1-a]isoindole], isoindolo[7,1,2-hij]quinoline, 7,11-methanoazocino [1,2-a]-indole, 7,11-methanoazocino[2,1-a]isoindole, dibenz[cd,f]-indole, dibenz[cd,g]indole, dibenz[d,f]indole, 1H-dibenz-[e,g]indole, 1H-dibenz[e,g]isoindole, naphtho[1,2,3-cd]-indole, naphtho[1,8-ef]indole, naphtho[1,8-fg] indole, naphtho[3,2,1-cd]indole, 1H-naphtho[1,2-e]indole, 1H-naphtho[1,2-f]indole, 1H-naphtho[1,2-g]indole, 1H-naphtho-[2,1-e]indole, 1H-naphtho[2,3-e]indole, 1H-naphtho[1,2-f]-isoindole, 1H-naphtho[2,3-e]isoindole, spiro[1H-carbazole-1,1'-cyclohexane], spiro[2H-carbazol-2,1'-cyclohexane], spiro[3H-carbazol-3,1'-cyclohexane], cyclohepta[4,5]pyrrolo[3,2-f]quinoline, cyclohepta[4,5]pyrrolo[3,2-h]quinoline, azepino[4,5-b]benz[e]indole, 1H-azepino[1,2-a]benz[f]indole, 1H-azepino[2,1-a]benz[f]isoindole, benzo[e]cyclohepta[b]-indole, benzo[g]cyclohepta[b]indole, and the like.

The group of the above-mentioned formula:

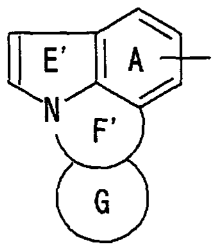

wherein each symbol is as defined above, includes the groups derived from tetracyclic condensed benzene rings by removing one hydrogen atom, which are exemplified by 1H-dipyrrolo[2,3-b:3',2',1'-hi]indole, spiro[cyclopentane-1,2'(1'H)-pyrrolo[3,2,1-hi]indole], spiro[imidazolizine-4,1'(2'H)-[4H]pyrrolo[3,2,1-ij]quinoline], pyrido[2,3-b]pyrrolo[3,2,1-hi]indole, pyrido[4,3-b]pyrrolo[3,2,1-hi]indole, benzo[de]pyrrolo[3,2,1-ij]quinoline, 3H-pyrrolo[3,2,1-de]acridine, 1H-pyrrolo[3,2,1-de]phenanthridine, spiro[cyclohexane-1,6'-[6H]pyrrolo[3,2,1-ij]quinoline], 4,9-methanopyrrolo[3,2,1-lm] [1]benzazocine, spiro[cycloheptane-1,6'-[6H]pyrrolo[3,2,1-ij] quinoline], 1H-pyrrano[3,4-d]pyrrolo[3,2,1-jk][1]benzazepine, 3H-benzo[b]pyrrolo[3,2,1-jk][4,1]benzoxazepine, 7H-indolo[1,7ab][4,1]benzoxazepine, benzo[b]pyrrolo[3,2,1-jk] [1,4]benzodiazepine, indolo [1,7-ab] [1,4] benzodiazepine, indolo[1,7-ab][1]benzazepine, indolo[7,1-ab][3]benzazepine, 1H-cyclohepta[d][3,2,1-jk][1]benzazepine, spiro[azepino[3,2,1-hi]indole-7(4H),1'-cycloheptane], 4H-5,11-methanopyrrolo[3,2,1-no][1]benzazacycloundecine, spiro[azepino[3,2,1-hi]indole-7(4H),1'-cyclooctane], and the like.

Among them, particularly preferred is a group of the formula:

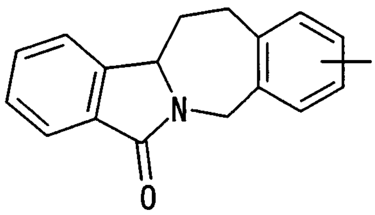

The "optionally condensed phenyl which may be substituted" represented by Ar preferably includes, for example, optionally substituted groups of formula:

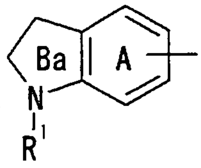
,
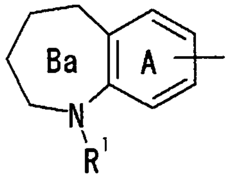
,
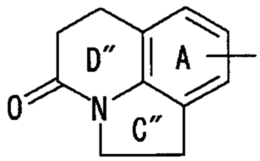

or

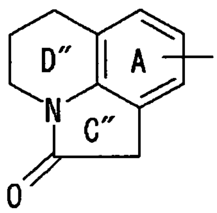

wherein each symbol is as defined above. Particularly preferred is a group of formula:

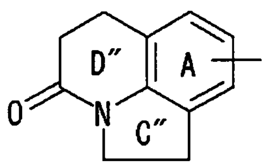

wherein each symbol is as defined above.
n is, preferably, an integer of 1 to 6. Particularly preferred are 2 to 6, and especially preferred is 2.
Each of R and R' is hydrogen, halogen or optionally substituted hydrocarbon group, which may be different according to repetition of n.
The "halogen" represented by R and R' includes fluorine, chlorine, bromine, iodine and the like and fluorine is preferable in particular.
As for the "optionally substituted hydrocarbon group" represented by R and R', the same hydrocarbon group as that of the "optionally substituted hydrocarbon group" represented by $R^1$ can be exemplified.
R and R' are preferably hydrogen of fluorine. As R and R', hydrogen is further preferred.
The "optionally substituted amino" represented by Y includes, for example, groups of the formula:

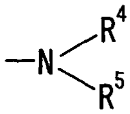

wherein each of $R^4$ and $R^5$ is hydrogen, optionally substituted hydrocarbon group, or acyl.

As for the "optionally substituted hydrocarbon group" and the "acyl" represented by $R^4$ and $R^5$, the same groups as the "optionally substituted hydrocarbon group" and the "acyl" represented by $R^1$ can be exemplified.

The "nitrogen-containing saturated heterocyclic group" of the "optionally substituted nitrogen-containing saturated heterocyclic group" represented by Y includes 5-to 9-membered (preferably, 5- to 7-membered) nitrogen-containing saturated heterocyclic group which may contain 1 to 3 heteroatoms selected from nitrogen, oxygen and sulfur, in addition to carbon atoms and one nitrogen atom. Such a group is exemplified, for example, by groups of the formula:

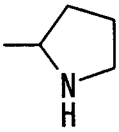 , 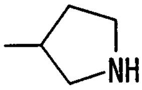 , 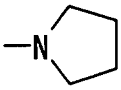 , 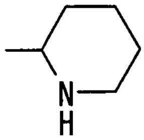 , 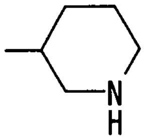 ,

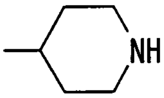 , 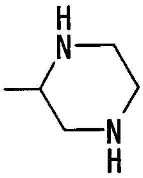 , 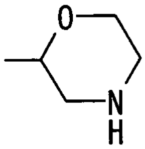 , 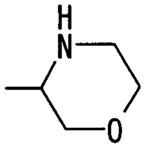 , 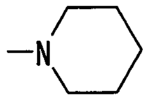 ,

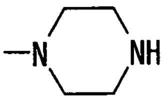 , 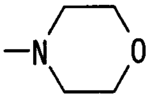 , 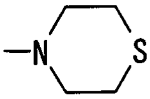 , 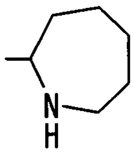 , 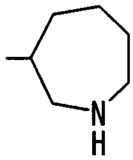 ,

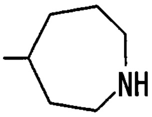 , 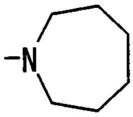 or 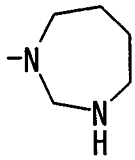

Among them, the 6-membered cyclic groups are preferred. Particularly preferred is a group of the formula:

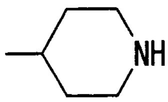

As for the "substituent" of the "optionally substituted nitrogen-containing saturated heterocyclic group", the same "substituent" as that of the "optionally substituted nitrogen-containing saturated heterocyclic group" represented by the above-mentioned B ring can be exemplified. The number of the substituent is 1 to 5. In addition, the nitrogen atom on the "nitrogen-containing saturated heterocyclic group" of the "optionally substituted nitrogen-containing saturated heterocyclic group" may have the same group as that represented by the above-mentioned $R^1$.

Preferably, Y is a group of the formula:

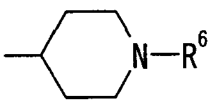, 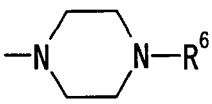 or 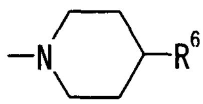

wherein $R^6$ is as defined above for $R^1$. Particularly preferred is a group of the formula:

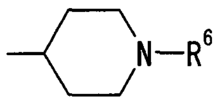

wherein $R^6$ is as defined above.

$R^6$ is preferably hydrogen or an optionally substituted hydrocarbon group. Particularly preferred are $C_{7-16}$ aralkyl (preferably, benzyl) and the like, which may be substituted by 1 to 3 substituents selected from halogen (preferably, fluoro, etc.), $C_{1-6}$ alkyl (preferably, methyl, etc.), $C_{1-6}$ alkoxy (preferably, methoxy, etc.), cyano, nitro and hydroxy.

The compound (I) preferably includes that in which Ar is a group of the formula:

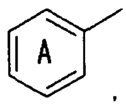, 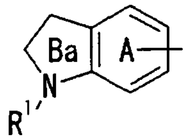, 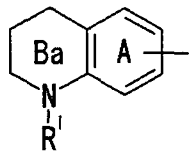, 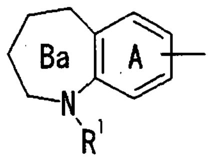, 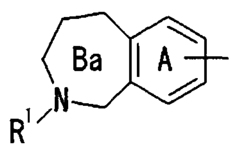,

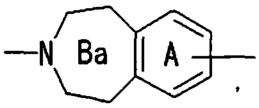, 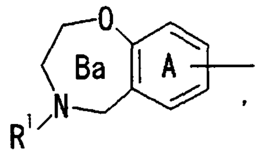, 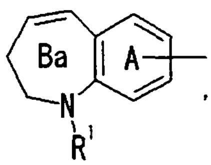, 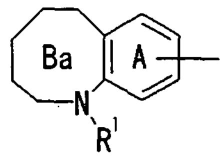,

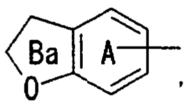, 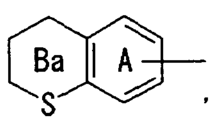, 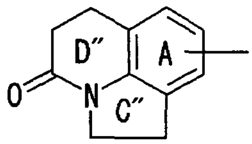 or 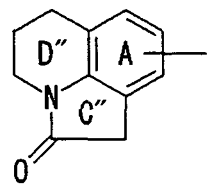

wherein each symbol is as defined above. Among them, particular examples include those wherein A ring is a benzene ring which may have 1 to 4 of substituents selected from (i) halogen (fluoro and the like), (ii) $C_{1-6}$ alkoxy (methoxy, and the like), (iii) halogeno $C_{1-6}$ alkoxy (trifluoromethoxy, and the like), (iv) amino, (v) (mono or di) $C_{1-6}$ alkylamino (methylamino, ethylamino, dimethylamino, diethylamino, and the like), (vi) 1-pyrrolidinyl, (vii) piperidino, (viii) 1-piperazinyl, (ix) N-methyl-1-piperazinyl, (x) N-acetyl-1-piperazinyl, (xi) morpholino, (xii) hexamethyleneimino, (xiii) imidazolyl, (xiv) $C_{1-6}$ alkyl (propyl and the like) which may be substituted with carboxyl which may be esterified with $C_{1-6}$ alkyl (methyl and the like), (xv) lower alkyl-carbonylamino (acetylamino and the like), (xvi) lower alkylsulfonylamino (methylsulfonylamino and the like), (xvii) aminosulfonyl, (xviii) (mono or di) $C_{1-6}$ alkylaminosulfonyl, (xix) 5 to 7-membered cyclic amino-sulfonyl ((1-pyrrolidinyl)sulfonyl, piperidinosulfonyl, (1-piperazinyl)sulfonyl, morpholinosulfonyl and the like), (xx) carbamoyl, (xxi) (mono or di) $C_{1-6}$ alkylcarbamoyl, (xxi) 5 to 7-membered amino-carbonyl ((1-pyrrolidinyl)carbonyl, piperidinocarbonyl, (1-piperazinyl)carbonyl, morpholinocarbonyl and the like), (xxii) cyano, and the like [more preferably, the A ring is a benzene ring which may have 1 or 2 substituents selected from aminosulfonyl, (mono or di) $C_{1-6}$ alkylaminosulfonyl, carbamoyl and (mono or di) $C_{1-6}$ alkyl-carbamoyl];

each of the Ba, C" and D" rings may have 1 or 2 substituents selected from $C_{1-6}$ alkyl, $C_{1-6}$ alkyl-carbonylamino and $C_{1-6}$ alkylsulfonylamino;

$R^1$ is (1) hydrogen, (2) $C_{1-6}$ alkyl group or $C_{7-16}$ aralkyl which may have 1 or 2 substituents selected from hydroxy and $C_{1-6}$ alkoxy-carbonyl, or (3) a group represented by - (C=O) -$R^{2'}$, - (C=O) -$NR^{2'}R^{3'}$, -$SO_2$-$R^{2'}$ (wherein each of $R^{2'}$ and $R^{31}$ is hydrogen, or optionally halogenated $C_{1-6}$ alkyl or $C_{6-10}$ aryl];

n is 2;

each of R and R' is hydrogen or fluorine (more preferably hydrogen); that is,

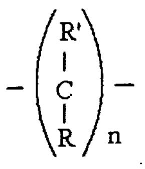

is -$CH_2CH_2$-, -$CHFCH_2$- or -$CF_2CH_2$-;

Y is a group represented by the formula:

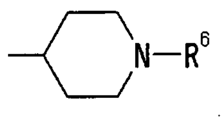

wherein the symbol is as defined above, and

$R^6$ is (1) hydrogen, (2) $C_{1-6}$ alkyl (methyl, ethyl, isopropyl and the like) which may have a substituent selected from cyano, hydroxy, (mono or di) $C_{1-6}$ alkylamino (diethylamino and the like), pyridyl and carboxyl which may be esterified with $C_{1-6}$ alkyl (ethyl and the like), (3) $C_{7-16}$ aralkyl (benzyl, $\alpha$-methylbenzyl, phenylethyl and the like) which may have a substituent selected from halogen (fluoro, chloro and the like), $C_{1-6}$ alkyl (methyl, t-butyl and the like), halogeno $C_{1-6}$ alkyl (trifluoromethyl and the like), hydroxy, $C_{1-6}$ alkoxy (methoxy and the like), nitro, amino, cyano, carbamoyl, $C_{1-6}$ alkoxy which may be esterified with ($C_{1-6}$ alkyl and the like) ($OCH_2COOH$, $OCH_2CO_2Et$ and the like), amino which may be substituted with carbamoyl or formyl which may be substituted with $C_{1-6}$ alkyl (NHCHO, $NHCONH_2$, NHCONHMe and the like), and $C_{1-3}$ alkylenedioxy (methylenedioxy and the like), (4) $C_{1-6}$ alkyl (methyl, propyl and the like) which may be substituted with carboxyl which may be esterified with ($C_{1-6}$ alkyl (ethyl and the like)), or (5) $C_{1-6}$ alkyl-carbonyl (acetyl and the like) which may be substituted with (mono or di) $C_{1-6}$ alkylamino (dimethylamino and the like).

The particularly preferred compound (I) includes that wherein Ar is a group of the formula:

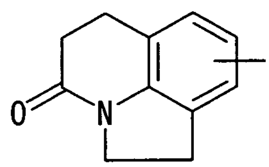

n is 2;

each of R and R' is hydrogen or fluorine (more preferably hydrogen); that is,

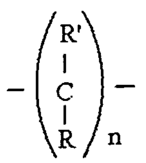

is -$CH_2CH_2$-, -$CHFCH_2$- or -$CF_2CH_2$-;

Y is a group of the formula:

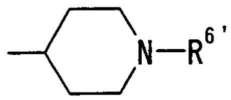

wherein $R^{6'}$ is benzyl which may be substituted by 1 or 2 substituents selected from halogen, $C_{1-3}$ alkyl, $C_{1-3}$ alkoxy, cyano, nitro and hydroxy.

Specifically, as the compound A, the compounds of Reference Examples 1 to 30 described in WO00/18391 are used.

Particularly preferred are:

8-[3-[1-[(3-fluorophenyl)methyl]-4-piperidinyl]-1-oxopropyl]-1,2,5,6-tetrahydro-4H-pyrrolo[3,2,1-ij] quinolin-4-one;
8-[3-[1-(phenylmethyl)-4-piperidinyl]-1-oxopropyl]-1,2,5,6-tetrahydro-4H-pyrrolo[3,2,1-ij]quinolin-4-one;
8-[3-[1-[(2-hydroxyphenyl)methyl)-4-piperidinyl]-1-oxopropyl]-1,2,5,6-tetrahydro-4H-pyrrolo[3,2,1-ij]quinolin-4-one; and
8-[2-fluoro-3-[1-[(3-fluorophenyl)methyl]-4-piperidinyl]-1-oxopropyl]-1,2,5,6-tetrahydro-4H-pyrrolo[3,2,1-ij]quinolin-4-one; or salts thereof. The crystals of the present invention are most preferable from the viewpoint of the stability and effectiveness of an effective ingredient.

Next, the following compounds can be disclosed as the compound B used in the present invention.

A compound represented by the formula:

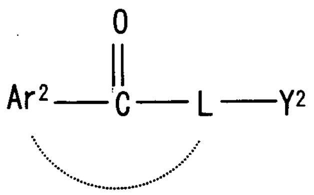 (II)

wherein $Ar^2$ represents an optionally condensed 5- or 6-membered aromatic ring group and said aromatic ring group may have a substituent, L represents a spacer having a main chain of 1 to 10 atoms which may have a substituent, or may form a ring between $Ar^2$, and $Y^2$ represents an amino group which may have a substituent or a nitrogen-containing heterocyclic ring which may have a substituent (hereinafter, sometimes, abbreviated as the compound (II)), or a salt thereof.

In the aforementioned formula, examples of the "substituent" in the "optionally condensed 5-or 6-membered aromatic ring group and said aromatic ring group may have a substituent" represented by $Ar^2$ include (i) an optionally halogenated lower alkyl group, (ii) a halogen atom (e.g. fluoro, chloro, bromo, iodo etc.), (iii) a lower alkylenedioxy group (e.g. $C_{1-3}$ alkylenedioxy group such as methylenedioxy, ethylenedioxy etc.), (iv) a nitro group, (v) a cyano group, (vi) a hydroxy group, (vii) an optionally halogenated lower alkoxy group, (viii) a cycloalkyl group (e.g. $C_{3-6}$ cycloalkyl group such as cyclopropyl, cyclobutyl, cyclopentyl, cyclohexyl etc.), (ix) an optionally halogenated lower alkylthio group, (x) an amino group, (xi) a mono-lower alkylamino group (e.g. mono-$C_{1-6}$ alkylamino group such as methylamino, ethylamino, propylamino etc.), (xii) a di-lower alkylamino group (e.g. di-$C_{1-6}$ alkylamino group such as dimethylamino, diethylamino etc.), (xiii) a 5- to 7-membered cyclic amino group (e.g. 5- to 7-membered cyclic amino group which may have 1 to 3 hetero atom(s) selected from a nitrogen atom, an oxygen atom and a sulfur atom in addition to one nitrogen atom (e.g. 1-pyrrolidinyl, piperidino, 1-piperazinyl, morpholino, thiomorpholino etc.) etc.), (xiv) a lower alkyl-carbonylamino group (e.g. $C_{1-6}$ alkyl-carbonylamino group such as acetylamino, propionylamino, butyrylamino etc.), (xv) a lower alkylsulfonylamino group (e.g. $C_{1-6}$ alkylsulfonylamino group such as methylsulfonylamino, ethylsulfonylamino, propylsulfonylamino etc.), (xvi) a lower alkoxy-carbonyl group (e.g. $C_{1-6}$ alkoxy-carbonyl group such as methoxycarbonyl, ethoxycarbonyl, propoxycarbonyl, isobutoxycarbonyl etc.), (xvii) a carboxy group, (xviii) a lower alkyl-carbonyl group (e.g. $C_{1-6}$ alkyl-carbonyl group such as methylcarbonyl, ethylcarbonyl, butylcarbonyl etc.), (xix) a cycloalkyl-carbonyl group (e.g. $C_{3-6}$ cycloalkyl-carbonyl group such as cyclopropylcarbonyl, cyclobutylcarbonyl, cyclopentylcarbonyl, cyclohexylcarbonyl etc.), (xx) a carbamoyl group, a thiocarbamoyl group, (xxi) a mono-lower alkyl-carbamoyl group (e.g. mono-$C_{1-6}$alkyl-carbamoyl group such as methylcarbamoyl, ethylcarbamoyl, propylcarbamoyl, butylcarbamoyl etc.), (xxii) a di-lower alkyl-carbamoyl group (e.g. di-$C_{1-6}$ alkyl-carbamoyl group such as diethylcarbamoyl, dibutylcarbamoyl etc.), (xxiii) a lower alkylsulfonyl group (e.g. $C_{1-6}$ alkylsulfonyl group such as methylsulfonyl, ethylsulfonyl, propylsulfonyl etc.), (xxiv) a cycloalkylsulfonyl group (e.g. $C_{3-6}$ cycloalkylsulfonyl such as cyclopentylsulfonyl, cyclohexylsulfonyl etc.), (xxv) a phenyl group, (xxvi) a naphthyl group, (xxvii) a mono-phenyl-lower alkyl group (e.g. mono-phenyl-$C_{1-6}$ alkyl group such as

benzyl, phenylethyl etc.), (xxviii) a di-phenyl-lower alkyl group (e.g. di-phenyl-$C_{1-6}$ alkyl group such as diphenylmethyl, diphenylethyl etc.), (xxix) a mono-phenyl-lower alkyl-carbonyloxy group (e.g. mono-phenyl-$C_{1-6}$ alkyl-carbonyloxy group such as phenylmethylcarbonyloxy, phenylethylcarbonyloxy etc.), (xxx) a di-phenyl-lower alkyl-carbonyloxy group (e.g. di-phenyl-$C_{1-6}$ alkyl-carbonyloxy group such as diphenylmethylcarbonyloxy, diphenylethylcarbonyloxy etc.), (xxxi) a phenoxy group, (xxxii) a mono-phenyl-lower alkyl-carbonyl group (e.g. mono-phenyl-$C_{1-6}$ alkyl-carbonyl group such as phenylmethylcarbonyl, phenylethylcarbonyl etc.), (xxxiii) a di-phenyl-lower alkyl-carbonyl group (e.g. di-phenyl-$C_{1-6}$ alkyl-carbonyl group such as diphenylmethylcarbonyl, diphenylethylcarbonyl etc.), (xxxiv) a benzoyl group, (xxxv) a phenoxycarbonyl group, (xxxvi) a phenyl-lower alkyl-carbamoyl group (e.g. phenyl-$C_{1-6}$ alkyl-carbamoyl group such as phenyl-methylcarbamoyl, phenyl-ethylcarbamoyl etc.), (xxxvii) a phenylcarbamoyl group, (xxxviii) a phenyl-lower alkyl-carbonylamino group (e.g. phenyl-$C_{1-6}$ alkyl-carbonylamino group such as phenyl-methylcarbonylamino, phenyl-ethylcarbonylamino etc.), (xxxix) a phenyl-lower alkylamino group (e.g. phenyl-$C_{1-6}$ alkylamino group such as phenyl-methylamino, phenyl-ethylamino etc.), (xxxx) a phenyl-lower alkylsulfonyl group (e.g. phenyl-$C_{1-6}$ alkylsulfonyl group such as phenyl-methylsulfonyl, phenyl-ethylsulfonyl etc.), (xxxxi) a phenylsulfonyl group, (xxxxii) a phenyl-lower alkylsulfinyl group (e.g. phenyl-$C_{1-6}$ alkylsulfinyl group such as phenyl-methylsulfinyl, phenyl-ethylsulfinyl etc.), (xxxxiii) a phenyl-lower alkylsulfonylamino group (e.g. phenyl-$C_{1-6}$ alkylsulfonylamino group such as phenyl-methylsulfonylamino, phenyl-ethylsulfonylamino etc.), (xxxxiv) a phenylsulfonylamino group, (xxxxv) a 5- to 7-membered cyclic amino-carbonyl group (e.g. 5- to 7-membered cyclic amino-carbonyl group which may have 1 to 3 hetero atom(s) selected from a nitrogen atom, an oxygen atom and a sulfur atom in addition to one nitrogen atom (e.g. (1-pyrrolidinyl)carbonyl, piperidinocarbonyl, (1-piperazinyl)carbonyl, morpholinocarbonyl, thiomorpholinocarbonyl group etc.) etc.), (xxxxvi) an aminosulfonyl group, (xxxxvii) a mono-lower alkylaminosulfonyl group (e.g. mono-$C_{1-6}$ alkylaminosulfonyl group such as methylaminosulfonyl, ethylaminosulfonyl, propylaminosulfonyl, butylaminosulfonyl etc.), (xxxxviii) a di-lower alkylaminosulfonyl group (e.g. di-$C_{1-6}$ alkylaminosulfonyl group such as diethylaminosulfonyl, dibutylaminosulfonyl etc.), (xxxxix) a 5- to 7-membered cyclic amino-sulfonyl group (e.g. 5- to 7-membered cyclic amino-sulfonyl group which may have 1 to 3 hetero atom(s) selected from a nitrogen atom, an oxygen atom and a sulfur atom in addition to one nitrogen atom (e.g. (1-pyrrolidinyl) sulfonyl, piperidinosulfonyl, (1-piperazinyl)sulfonyl, morpholinosulfonyl, thiomorpholinosulfonyl group etc.), (xxxxx) an aminocarbonyloxy group, (xxxxxi) a mono-lower alkylaminocarbonyloxy group (e.g. mono-$C_{1-6}$ alkylaminocarbonyloxy group such as methylaminocarbonyloxy, ethylaminocarbonyloxy, propylaminocarbonyloxy etc.), (xxxxxii) a di-lower alkylaminocarbonyloxy group (e.g. di-$C_{1-6}$ alkylaminocarbonyloxy group such as dimethylaminocarbonyloxy, diethylaminocarbonyloxy etc.) and (xxxxxiii) a 5- to 7-membered cyclic amino-carbonyloxy group (e.g. 5- to 7-membered cyclic amino-carbonyloxy group which may have 1 to 3 hetero atom(s) selected from a nitrogen atom, an oxygen atom and a sulfur atom in addition to one nitrogen atom (e.g. (1-pyrrolidinyl)carbonyloxy, piperidinocarbonyloxy, (1-piperazinyl) carbonyloxy, morpholinocarbonyloxy, thiomorpholinocarbonyloxy group etc.) etc.) (the phenyl group moiety in phenyl group, naphthyl group, mono-phenyl-lower alkyl group, di-phenyl-lower alkyl group, mono-phenyl-lower alkyl-carbonyloxy group, di-phenyl-lower alkyl-carbonyloxy group, phenoxy group, mono-phenyl-lower alkyl-carbonyl group, di-phenyl-lower alkyl-carbonyl group, benzoyl group, phenoxycarbonyl group, phenyl-lower alkyl-carbamoyl group, phenylcarbamoyl group, phenyl-lower alkyl-carbonylamino group, phenyl-lower alkylamino group, phenyl-lower alkylsulfonyl group, phenylsulfonyl group, phenyl-lower alkylsulfinyl group, phenyl-lower alkylsulfonylamino group and phenylsulfonylamino group of the aforementioned (xxv) to (xxxxiv) may further have 1 to 4 substituent(s) selected from, for example, a lower alkyl group (e.g. $C_{1-6}$ alkyl such as methyl, ethyl, propyl, isopropyl, butyl, sec-butyl, tert-butyl, pentyl, hexyl etc.), a lower alkoxy group (e.g. $C_{1-6}$ alkoxy such as methoxy, ethoxy, propoxy, isopropoxy, butoxy, isobutoxy, sec-butoxy, tert-butoxy etc.), a halogen atom (e.g. chloro, bromo, iodo etc.), a hydroxy group, a benzyloxy group, an amino group, a mono-lower alkylamino group (e.g. mono-$C_{1-6}$ alkylamino such as methylamino, ethylamino, propylamino etc.), a di-lower alkylamino group (e.g. di-$C_{1-6}$ alkylamino such as dimethylamino, diethylamino etc.), a nitro group, a lower alkyl-carbonyl group (e.g. $C_{1-6}$ alkyl-carbonyl such as methylcarbonyl, ethylcarbonyl, butylcarbonyl etc.), and a benzoyl group). The "optionally condensed 5-or 6-membered aromatic ring group" represented by Ar may have 1 to 4, preferably 1 or 2 of these substituent (s) of (i) to (xxxxxiii).

Examples of the above-mentioned "optionally halogenated lower alkyl group" include a lower alkyl group (e.g. $C_{1-6}$ alkyl group such as methyl, ethyl, propyl, isopropyl, butyl, sec-butyl, tert-butyl, pentyl, hexyl etc.) which may have 1 to 3 halogen atom(s) (e.g. chloro, bromo, iodo, etc.), specifically, methyl, chloromethyl, difluoromethyl, trichloromethyl, trifluoromethyl, ethyl, 2-bromoethyl, 2,2,2-trifluoroethyl, propyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl, and 6,6,6-trifluorohexyl.

Examples of the above-mentioned "optionally halogenated lower alkoxy group" include a lower alkoxy group (e.g. $C_{1-6}$alkoxy group such as methoxy, ethoxy, propoxy, isopropxy, butoxy, isobutoxy, sec-butoxy, tert-butoxy etc.) which may have 1 to 3 halogen atom(s) (e.g. chloro, bromo, iodo etc.), specifically, methoxy, difluoromethoxy, trifluoromethoxy, ethoxy, 2,2,2-trifluoroethoxy, propoxy, isopropoxy, butoxy, 4,4,4-trifluorobutoxy, isobutoxy, sec-butoxy, pentyloxy, and hexyloxy.

Examples of the above-mentioned "optionally halogenated lower alkylthio group" include a lower alkylthio group (e.g. $C_{1-6}$ alkylthio group such as methylthio, ethylthio, propylthio, isopropylthio, butylthio, isobutylthio, sec-butylthio,

tert-butylthio etc.) which may have 1 to 3 halogen atom(s) (e.g. chloro, bromo, iodo etc.), specifically, methylthio, difluoromethylthio, trifluoromethylthio, ethylthio, propylthio, isopropylthio, butylthio, 4,4,4-trifluorobutylthio, isobutylthio, sec-butylthio, tert-butylthio, pentylthio, and hexylthio.

Preferable examples of the "substituent" in the "optionally condensed 5-or 6-membered aromatic ring group and said aromatic ring group may have a substituent" include (i) an amino group, (ii) a mono-lower alkylamino group (e.g. mono-$C_{1-6}$ alkylamino group such as methylamino, ethylamino, propylamino etc.), (iii) a di-lower alkylamino group (e.g. di-$C_{1-6}$ alkylamino group such as dimethylamino, diethylamino etc.), (iv) a 5- to 7-membered cyclic amino group which may have 1 to 3 hetero atom(s) selected from a nitrogen atom, an oxygen atom and sulfur atom in addition to one nitrogen atom (e.g. 1-pyrrolidinyl, piperidino, 1-piperazinyl, morpholino, thiomorpholino etc.), (v) a lower alkyl-carbonylamino group (e.g. $C_{1-6}$ alkyl-carbonylamino group such as acetylamino, propionylamino, butyrylamino etc.), (vi) a lower alkylsulfonylamino group (e.g. $C_{1-6}$ alkylsulfonylamino group such as methylsulfonylamino, ethylsulfonylamino, propylsulfonylamino etc.), (vii) a phenyl-lower alkylamino (e.g. phenyl-$C_{1-6}$ alkylamino such as phenyl-methylamino, phenyl-ethylamino etc.), (viii) a phenyl-lower alkylsulfonylamino group (e.g. phenyl-$C_{1-6}$ alkyl-sulfonylamino group such as phenyl-methylsulfonylamino, phenyl-ethylsulfonylamino etc.), (ix) a phenylsulfonylamino, (x) a halogen atom (e.g. fluoro, chloro etc.), (xi) an optionally halogenated lower alkyl group, (e.g. methyl, ethyl, isopropyl, tert-butyl, trifluoromethyl etc.), (xii) an optionally halogenated lower alkoxy group (e.g. methoxy, ethoxy, isopropoxy, tert-butoxy, trifluoromethoxy etc.), (xiii) an aminosulfonyl group, (xiv) a mono-lower alkylaminosulfonyl group (e.g. mono-$C_{1-6}$ alkylaminosulfonyl group such as methylaminosulfonyl, ethylaminosulfonyl, propylaminosulfonyl, butylaminosulfonyl etc.), (xv) a di-lower alkylaminosulfonyl group (e.g. di-$C_{1-6}$ alkylaminosulfonyl group such as diethylaminosulfonyl, dibutylaminosulfonyl etc.), (xvi) a carbamoyl group, (xvii) a mono-lower alkyl-carbamoyl group (e.g. mono-$C_{1-6}$ alkyl-carbamoyl group such as methylcarbamoyl, ethylcarbamoyl, propylcarbamoyl, butylcarbamoyl etc.), and (xviii) a di-lower alkyl-carbamoyl group (e.g. di-$C_{1-6}$ alkyl-carbamoyl group such as diethylcarbamoyl, dibutylcarbamoyl etc.). Particularly, a di-lower alkylamino group (e.g. di-$C_{1-6}$ alkylamino group such as dimethylamino, diethylamino etc.), a 5- to 7-membered cyclic amino group which may have 1 to 3 hetero atom(s) selected from a nitrogen atom, an oxygen atom and a sulfur atom in addition to one nitrogen atom (e.g. 1-pyrrolidinyl, piperidino, 1-piperazinyl, morpholino, thiomorpholino etc.), an optionally halogenated lower alkoxy group (e.g. methoxy, ethoxy, isopropoxy, tert-butoxy, trifluoromethoxy etc.), an aminosulfonyl group, a mono-lower alkylaminosulfonyl group (e.g. mono-$C_{1-6}$ alkylaminosulfonyl group such as methylaminosulfonyl, ethylaminosulfonyl, propylaminosulfonyl, butylaminosulfonyl etc.), a di-lower alkyl-aminosulfonyl group (e.g. di-$C_{1-6}$ alkylaminosulfonyl group such as diethylaminosulfonyl, dibutylaminosulfonyl etc.), a carbamoyl group, a mono-lower alkyl-carbamoyl group (e.g. mono-$C_{1-6}$ alkyl-carbamoyl group such as methylcarbamoyl, ethylcarbamoyl, propylcarbamoyl, butylcarbamoyl etc.), and a di-lower alkyl-carbamoyl group (e.g. di-$C_{1-6}$ alkyl-carbamoyl group such as diethylcarbamoyl, dibutylcarbamoyl etc.) are preferred.

Examples of the "5-or 6-membered aromatic ring group" in the "optionally condensed 5- or 6-membered aromatic ring group" represented by $Ar^2$ include a phenyl group (benzene ring group), and a 5- or 6-membered aromatic heterocyclic group.

Examples of the "5- or 6-membered aromatic heterocyclic group" include 5- or 6-membered aromatic heterocyclic groups containing 1 or more (e.g. 1 to 3) hetero atom(s) selected from a nitrogen atom, a sulfur atom and an oxygen atom in addition to carbon atoms. Specific examples include thienyl, furyl, pyrrolyl, imidazolyl, pyrazolyl, thiazolyl, isothiazolyl, oxazolyl, isooxazolyl, pyridyl, pyrazinyl, pyrimidinyl, pyridazinyl, oxadiazolyl, thiadiazolyl, and furazanyl.

When the "5- or 6-membered aromatic ring group" in the "optionally condensed 5- or 6-membered aromatic ring group" represented by $Ar^2$ is, for example, "a phenyl group which may have a substituent", examples of condensing of the "phenyl group" include:

(a) the case where the phenyl group is condensed with a monocyclic homocyclic ring or heterocyclic ring, which may have a substituent,
(b) the case where the phenyl group is condensed with a bicyclic homocyclic ring or heterocyclic ring, which may have a substituent, or is condensed with two same or different monocyclic homocyclic rings or heterocyclic rings, and
(c) the case where the phenyl group is condensed with a tricyclic homocyclic ring or heterocyclic ring which may have a substituent.

Examples of the case where a phenyl group in the "optionally condensed phenyl group, and said phenyl group may have a substituent" of the above-mentioned (a) is condensed with a monocyclic homocyclic ring or heterocyclic ring include a group represented by the formula:

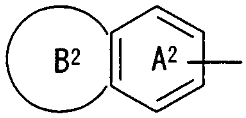

wherein $A^2$ ring represents a benzene ring which may have a substituent, and $B^2$ ring represents a homocyclic ring or a heterocyclic ring which may have a substituent.

Examples of the substituent of $A^2$ ring include the aforementioned "substituents" of the "optionally condensed 5 or 6-membered aromatic ring group" represented by $Ar^2$, and the number of substituent(s) is 1 to 3.

Examples of the "homocyclic ring" in the "homocyclic ring which may have a substituent" represented by $B^2$ ring include 5- to 9-membered carbocyclic rings (e.g. benzene, cyclopentane, cyclopentene, cyclohexane, cyclohexene, cyclohexadiene, cycloheptane, cycloheptene, cycloheptadiene etc.).

Examples of the "heterocyclic ring" in the "heterocyclic ring which may have a substituent" represented by $B^2$ ring include 4- to 14-membered (preferably 5- to 9-membered) aromatic or non-aromatic heterocyclic rings containing 1 to 4 hetero atom(s) selected from a nitrogen atom, an oxygen atom and a sulfur atom. Specific examples include pyridine, pyrazine, pyrimidine, imidazole, furan, thiophen, dihydropyridine, diazepine, oxazepine, pyrrolidine, piperidine, hexamethyleneimine, heptamethyleneimine, tetrahydrofuran, tetrahydropyran, 1,4-dioxane, piperazine, homopiperazine, tetrahydrooxazepine, morpholine, thiomorpholine, pyrrole, pyrazole, 1,2,3-triazole, oxazole, oxazolidine, thiazole, thiazolidine, thiadiazolidine, thiadiazinone, isoxazole, imidazoline, imidazolidine, and hexahydropyrimidine. Among them, 5- to 9-membered non-aromatic heterocyclic rings containing one hetero atom or the same or different two hetero atoms (e.g. pyrrolidine, piperidine, hexamethyleneimine, heptamethyleneimine, tetrahydrofuran, tetrahydropyran, 1,4-dioxane, piperazine, homopiperazine, tetrahydrooxazepine, morpholine, thiomorpholine, imidazoline, thiadiazolidine, thiadiazinone, imidazolidine, hexahydropyrimidine etc.) are preferable. In particular, (1) a non-aromatic heterocyclic ring containing one hetero atom selected from a nitrogen atom, an oxygen atom and a sulfur atom, and (2) a non-aromatic heterocyclic ring containing one nitrogen atom and one hetero atom selected from a nitrogen atom, an oxygen atom and a sulfur atom are preferable.

As the "substituent" in the "homocyclic ring or heterocyclic ring which may have a substituent" represented by $B^2$ ring, for example, 1 to 5 substituents selected from (i) a halogen atom (e.g. fluoro, chloro, bromo, iodo etc.), (ii) a nitro group, (iii) a cyano group, (iv) an oxo group, (v) a hydroxy group, (vi) a lower alkyl group (e.g. $C_{1-6}$ alkyl group such as methyl, ethyl, propyl, isopropyl, butyl, isobutyl, tert-butyl, sec-butyl etc.), (vii) a lower alkoxy group (e.g. $C_{1-6}$ alkoxy group such as methoxy, ethoxy, propyloxy, isopropyloxy, butyloxy etc.), (viii) a lower alkylthio group (e.g. $C_{1-6}$ alkylthio group such as methylthio, ethylthio, propylthio etc.), (ix) an amino group, (x) a mono-lower alkylamino group (e.g. mono-$C_{1-6}$ alkylamino group such as methylamino, ethylamino, propylamino etc.), (xi) a di-lower alkylamino group (e.g. di-$C_{1-6}$alkylamino group such as dimethylamino, diethylamino etc.), (xii) a 5- to 7-membered cyclic amino group which may have 1 to 3 hetero atom(s) selected from a nitrogen atom, an oxygen atom and a sulfur atom in addition to carbon atoms and one nitrogen atom (e.g. 1-pyrrolidinyl, piperidino, 1-piperazinyl, morpholino, thiomorpholino etc.), (xiii) a lower alkyl-carbonylamino group (e.g. $C_{1-6}$ alkyl-carbonylamino group such as acetylamino, propionylamino, butyrylamino etc.), (xiv) a lower alkylsulfonylamino group (e.g. $C_{1-6}$ alkylsulfonylamino group such as methylsulfonylamino, ethylsulfonylamino etc.), (xv) a lower alkoxy-carbonyl group (e.g. $C_{1-6}$ alkoxy-carbonyl group such as methoxycarbonyl, ethoxycarbonyl, propoxycarbonyl etc.), (xvi) a carboxy group, (xvii) a lower alkylcarbonyl group (e.g. $C_{1-6}$ alkyl-carbonyl group such as methylcarbonyl, ethylcarbonyl, propylcarbonyl etc.), (xviii) a carbamoyl group, (xix) a mono-lower alkylcarbamoyl group (e.g. mono-$C_{1-6}$ alkyl-carbamoyl group such as methylcarbamoyl, ethylcarbamoyl etc.), (xx) a di-lower alkylcarbamoyl group (e.g. di-$C_{1-6}$ alkyl-carbamoyl group such as dimethylcarbamoyl, diethylcarbamoyl etc.), (xxi) a lower alkylsulfonyl group (e.g. $C_{1-6}$ alkylsulfonyl group such as methylsulfonyl, ethylsulfonyl, propylsulfonyl etc.), (xxii) an aminosulfonyl group, (xxiii) a mono-lower alkylaminosulfonyl group (e.g. mono-$C_{1-6}$ alkyl-aminosulfonyl group such as methylaminosulfonyl, ethylaminosulfonyl etc.), and (xxiv) a di-lower alkylaminosulfonyl group (e.g. di-$C_{1-6}$ alkyl-aminosulfonyl group such as dimethylaminosulfonyl, diethylaminosulfonyl etc.) are used. Inter alia, an oxo group, and a lower alkyl group (e.g. $C_{1-6}$ alkyl group such as methyl, ethyl, propyl, isopropyl, butyl, isobutyl, tert-butyl, sec-butyl etc.) are preferable. An oxo group, a $C_{1-6}$ alkyl group, a $C_{1-6}$ alkyl-carbonylamino group and a $C_{1-6}$ alkylsulfonylamino group are particularly preferred.

When $B^2$ ring has a nitrogen atom in the ring, for example, the $B^2$ ring may have a group represented by the formula:

$>N-R^7$

wherein $R^7$ represents a hydrogen atom, a hydrocarbon group which may have a substituent, an acyl group, or a

heterocyclic group which may have a substituent, in the ring. Further, the $B^2$ ring may have 1 to 3 substituents selected from the aforementioned substituents (i) to (xxiv).

The "hydrocarbon group" in the "hydrocarbon group which may have a substituent" represented by $R^7$ represents a group obtained by removing one hydrogen atom from a hydrocarbon compound, and examples thereof include the following alkyl group, alkenyl group, alkynyl group, cycloalkyl group, aryl group, and aralkyl group, and a combination thereof. Among them, $C_{1-16}$ hydrocarbon groups are preferred.

(1) Alkyl group (e.g. $C_{1-6}$ alkyl group such as methyl, ethyl, propyl, isopropyl, butyl, isobutyl, tert-butyl, sec-butyl, pentyl, hexyl etc.)
(2) Alkenyl group (e.g. $C_{2-6}$ alkenyl group such as vinyl, allyl, isopropenyl, butenyl, isobutenyl, sec-butenyl etc.)
(3) Alkynyl group (e.g. $C_{2-6}$ alkynyl group such as propargyl, ethynyl, butynyl, 1-hexynyl etc.)
(4) Cycloalkyl group (e.g. $C_{3-6}$ cycloalkyl group such as cyclopropyl, cyclobutyl, cyclopentyl, cyclohexyl etc.)
(5) Cross-linked cyclic lower saturated hydrocarbon group (e.g. cross-linked cyclic $C_{8-14}$ saturated hydrocarbon group such as bicyclo[3.2.1]oct-2-yl, bicyclo[3.3.1]non-2-yl, and adamantan-1-yl etc.)
(6) Aryl group (e.g. $C_{6-14}$ aryl group such as phenyl, 1-naphthyl, 2-naphthyl, biphenyl, 2-indenyl, 2-anthryl etc., preferably phenyl group)
(7) Aralkyl group (e.g. $C_{7-16}$ aralkyl group such as phenyl-$C_{1-10}$ alkyl such as benzyl, phenylethyl, phenylpropyl, phenylbutyl, phenylpentyl, phenylhexyl etc.; naphthyl-$C_{1-6}$ alkyl such as $\alpha$-naphthylmethyl; diphenyl-$C_{1-3}$ alkyl such as diphenylmethyl, diphenylethyl etc.)
(8) Aryl-alkenyl group (e.g. $C_{6-14}$ aryl-$C_{2-12}$ alkenyl group such as phenyl-$C_{2-12}$ alkenyl such as styryl, cinnamyl, 4-phenyl-2-butenyl, 4-phenyl-2-butenyl etc.)
(9) Aryl-$C_{2-12}$ alkynyl group (e.g. $C_{6-14}$ aryl-$C_{2-12}$ alkynyl group such as phenyl-$C_{2-12}$ alkynyl such as phenylethynyl, 3-phenyl-2-propynyl, 3-phenyl-1-propynyl etc.)
(10) Cycloalkyl-alkyl group (e.g. $C_{3-7}$ cycloalkyl-$C_{1-6}$ alkyl group such as cyclopropylmethyl, cyclobutylmethyl, cyclopentylmethyl, cyclohexylmethyl, cycloheptylmethyl, cyclopropylethyl, cyclobutylethyl, cyclopentylethyl, cyclohexylethyl, cycloheptylethyl, cyclopropylpropyl, cyclobutylpropyl, cyclopentylpropyl, cyclohexylpropyl, cycloheptylpropyl, cyclopropylbutyl, cyclobutylbutyl, cyclopentylbutyl, cyclohexylbutyl, cycloheptylbutyl, cyclopropylpentyl, cyclobutylpentyl, cyclopentylpentyl, cyclohexylpentyl, cycloheptylpentyl, cyclopropylhexyl, cyclobutylhexyl, cyclopentylhexyl, cyclohexylhexyl etc.)
(11) Aryl-aryl-$C_{1-10}$ alkyl group (e.g. biphenylmethyl, biphenylethyl etc.).

Preferable examples of the "hydrocarbon group" in the "hydrocarbon group which may have a substituent" represented by $R^7$ include a $C_{1-6}$ alkyl group, a $C_{3-6}$ cycloalkyl group, and a $C_{7-16}$ aralkyl group. More preferred is a $C_{7-10}$ aralkyl group (e.g. phenyl-$C_{1-4}$ alkyl such as benzyl, phenylethyl, phenylpropyl etc.).

Examples of the "substituent" in the "hydrocarbon group which may have a substituent" represented by $R^7$ include 1 to 5 (preferably 1 to 3) selected from (i) a halogen atom (e.g. fluoro, chloro, bromo, iodo etc.), (ii) a nitro group, (iii) a cyano group, (iv) an oxo group, (v) a hydroxy group, (vi) an optionally halogenated lower ($C_{1-6}$)alkyl group, (vii) an optionally halogenated lower ($C_{1-6}$)alkoxy group, (viii) an optionally halogenated lower ($C_{1-6}$)alkylthio group, (ix) an amino group, (x) a mono-lower alkylamino group (e.g. mono-$C_{1-6}$ alkylamino group such as methylamino, ethylamino, propylamino etc.), (xi) a di-lower alkylamino group (e.g. di-$C_{1-6}$ alkylamino group such as dimethylamino, diethylamino etc.), (xii) a 5- to 7-membered cyclic amino group which may have 1 to 3 hetero atom(s) selected from a nitrogen atom, an oxygen atom and a sulfur atom in addition to carbon atoms and one nitrogen atom (e.g. 1-pyrrolidinyl, piperidino, 1-piperazinyl, morpholino, thiomorpholino etc.), (xiii) a lower alkyl-carbonylamino group (e.g. $C_{1-6}$ alkyl-carbonylamino group such as acetylamino, propionylamino, butyrylamino etc.), (xiv) a lower alkylsulfonylamino group (e.g. $C_{1-6}$ alkyl-sulfonylamino group such as methylsulfonylamino, ethylsulfonylamino etc.), (xv) a lower alkoxy-carbonyl group (e.g. $C_{1-6}$ alkoxy-carbonyl group such as methoxycarbonyl, ethoxycarbonyl, propoxycarbonyl etc.), (xvi) a carboxy group, (xvii) a lower alkyl-carbonyl group (e.g. $C_{1-6}$ alkyl-carbonyl group such as methylcarbonyl, ethylcarbonyl, propylcarbonyl etc.), (xviii) a carbamoyl group, a thiocarbamoyl group, (xix) a mono-lower alkyl-carbamoyl group (e.g. mono-$C_{1-6}$ alkyl-carbamoyl group such as methylcarbamoyl, ethylcarbamoyl etc.), (xx) a di-lower alkyl-carbamoyl group (e.g. di-$C_{1-6}$ alkyl-carbamoyl group such as dimethylcarbamoyl, diethylcarbamoyl etc.), (xxi) a lower alkylsulfonyl group (e.g. $C_{1-6}$ alkylsulfonyl group such as methylsulfonyl, ethylsulfonyl, propylsulfonyl etc.), (xxii) a lower alkoxy-carbonyl-lower alkyl group (e.g. $C_{1-6}$ alkyl-carbonyl-$C_{1-6}$ alkyl group such as methoxycarbonylmethyl, ethoxycarbonylmethyl, tert-butoxycarbonylmethyl, methoxycarbonylethyl, methoxycarbonylmethyl, methoxycarbonyl(dimethyl)methyl, ethoxycarbonyl(dimethyl)methyl, tert-butoxycarbonyl(dimethyl)methyl etc.), (xxiii) a carboxy-lower alkyl group (e.g. carboxy-$C_{1-6}$ alkyl group such as carboxylmethyl, carboxylethyl, carboxyl(dimethyl)methyl etc.), (xxiv) a heterocyclic group which may have a substituent, (xxv) a $C_{6-14}$ aryl group (e.g. phenyl, naphthyl etc.), (xxvi) a $C_{7-16}$ aralkyl group (e.g. benzyl etc.), (xxvii) a ureido group which may have a substituent (e.g. $C_{1-4}$ alkyl, halogeno$C_{1-4}$ alkyl, $C_{6-10}$ aryl, halogeno$C_{6-10}$ aryl, $C_{1-4}$ alkyl-$C_{6-10}$ aryl, halogeno$C_{1-4}$ alkyl-$C_{6-10}$ aryl, $C_{1-4}$ alkoxy-$C_{6-10}$aryl, benzyl etc.) (e.g. ureido, 3-methylureido, 3-ethylureido, 3-phenylureido, 3-(4-fluorophenyl)ureido, 3-(2-methylphenyl)ureido, 3-(4-methoxyphenyl)ureido, 3-(2,4-difluorophenyl)ureido, 3-[3,5-bis(trifluoromethyl)phenyl]ureido, 3-benzylureido, 3-(1-naphthyl)ureido, 3-(2-biphenylyl)ureido etc.), (xxviii) a thioureido group which may have a substituent (e.g. $C_{1-4}$ alkyl, halogeno$C_{1-4}$ alkyl, $C_{6-10}$ aryl, halogeno$C_{6-10}$ aryl, $C_{1-4}$ alkyl-$C_{6-10}$ aryl, halogeno$C_{1-4}$ alkyl-$C_{6-10}$ aryl, $C_{1-4}$ alkoxy-$C_{6}$-10 aryl, benzyl etc.)(e.g. thioureido, 3-methylthioureido, 3-ethylthioureido, 3-phenylthioureido, 3-(4-fluorophenyl)thioureido, 3-(4-methylphenyl)thioureido, 3-(4-methoxyphenyl) thioureido, 3-(2,4-dichlorophenyl)thioureido, 3-benzylthioureido, 3-(1-naphthyl)thioureido etc.), (xxix) an amidino group which may have a substituent (e.g. 1 to 2 selected from $C_{1-4}$ alkyl, $C_{6-10}$ aryl, nitro-$C_{6-10}$ aryl etc.) (e.g. amidino, $N^1$-methylamidino, $N^1$-ethylamidino, $N^1$-phenylamidino, $N^1$, $N^1$-dimethylamidino, $N^1$,$N^2$-dimethylamidino, $N^1$-methyl-$N^1$-ethylamidino, $N^1$, $N^1$-diethylamidino, $N^1$-methyl-$N^1$-phenylamidino, $N^1$, $N^1$-di (4-nitrophenyl)amidino etc.), (xxx) a guanidino group which may have a substituent (e.g. 1 to 2 selected from $C_{1-4}$ alkyl, $C_{6-10}$ aryl, nitro-$C_{6-10}$ aryl etc.)(e.g. guanidino, 3-methylguanidino, 3,3-dimethylguanidino, 3,3-diethylguanidino etc.), (xxxi) a cyclic aminocarbonyl group which may have a substituent (e.g. $C_{1-4}$ alkyl, halogeno$C_{1-4}$ alkyl, $C_{6-10}$ aryl, halogeno$C_{6-10}$ aryl, $C_{1-4}$ alkyl-$C_{6-10}$ aryl, halogeno$C_{1-4}$ alkyl-$C_{6-10}$ aryl, $C_{1-4}$ alkoxy-$C_{6-10}$ aryl, nitro-$C_{6-10}$ aryl, benzyl, halogenobenzyl, benzoyl, halogenobenzoyl etc.) (e.g. (1-pyrrolidinyl)carbonyl, piperidinocarbonyl, (4-methylpiperidino)carbonyl, (4-phenylpiperidino) carbonyl, (4-benzylpiperidino)carbonyl, (4-benzoylpiperidino)carbonyl, [4-(4-fluorobenzoyl)piperidino]carbonyl, (4-methyl-1-piperazinyl)carbonyl, (4-phenyl-1-piperazinyl)carbonyl, [4-(4-nitrophenyl)-1-piperazinyl]carbonyl, (4-benzyl-1-piperazinyl)carbonyl, morpholinocarbonyl, thiomorpholinocarbonyl etc.), (xxxii) an aminothiocarbonyl group which may have a substituent (e.g. 1 to 2 selected from $C_{1-4}$ alkyl, $C_{6-10}$aryl etc.) (e.g. aminothiocarbonyl, methylaminothiocarbonyl, dimethylaminothiocarbonyl etc.), (xxxiii) an aminosulfonyl group which may have a substituent (e.g. 1 to 2 selected from $C_{1-4}$ alkyl, $C_{6-10}$ aryl etc.) (e.g. aminosulfonyl, methylaminosulfonyl, dimethylaminosulfonyl etc.), (xxxiv) a phenylsulfonylamino group which may have a substituent (e.g. 1 to 2 selected from halogen atom, $C_{1-4}$alkyl, halogeno$C_{1-4}$ alkyl, $C_{1-4}$ alkoxy, nitro, $C_{1-4}$ alkyl-carbonylamino etc.) (e.g. phenylsulfonylamino, (4-methylphenyl)sulfonylamino, (4-chlorophenyl)sulfonylamino, (2,5-dichlorophenyl)sulfonylamino, (4-methoxyphenyl)sulfonylamino, (4-acetylaminophenyl)sulfonylamino, (4-nitrophenyl)phenylsulfonylamino etc.), (xxxv) a sulfo group, (xxxvi) a sulfino group, (xxxvii) a sulfeno group, (xxxviii) a $C_{1-6}$ alkylsulfo group (e.g. methylsulfo, ethylsulfo, propylsulfo etc.), (xxxix) a $C_{1-6}$ alkylsulfino group (e.g. methylsulfino, ethylsulfino, propylsulfino etc.), (xxxx) a $C_{1-6}$ alkylsulfeno group (e.g. methylsulfeno, ethylsulfeno, propylsulfeno etc.), (xxxxi) a phosphono group, (xxxxii) a di-$C_{1-6}$ alkoxyphosphoryl group (e.g. dimethoxyphosphoryl, diethoxyphosphoryl, dipropoxyphosphoryl etc.), (xxxxiii) an aminocarbonyloxy group, (xxxxxi) a mono-lower alkylaminocarbonyloxy group (e.g. mono-$C_{1-6}$ alkylaminocarbonyloxy group such as methylaminocarbonyloxy, ethylaminocarbonyloxy, propylaminocarbonyloxy etc.), (xxxxxii) a di-lower alkylaminocarbonyloxy group (e.g. di-$C_{1-6}$ alkylaminocarbonyloxy group such as dimethylaminocarbonyloxy, diethylaminocarbonyloxy etc.) and (xxxxxiii) a 5- to 7-membered cyclic amino-carbonyloxy group (e.g. 5- to 7-membered cyclic amino-carbonyloxy group which may have 1 to 3 hetero atom(s) selected from a nitrogen atom, an oxygen atom and a sulfur atom in addition to one nitrogen atom (e.g. (1-pyrrolidinyl)carbonyloxy, piperidinocarbonyloxy, (1-piperazinyl)carbonyloxy, morpholinocarbonyloxy, thiomorpholinocarbonyloxy group etc.) etc.).

Among them, preferred are a halogen atom, an optionally halogenated $C_{1-6}$ alkyl group, an optionally halogenated $C_{1-6}$ alkoxy group, a hydroxy group, a nitro group, a cyano group, a carboxy group, a $C_{1-6}$ alkoxy-carbonyl group, a carbamoyl group, an aminothiocarbonyl group, a mono-$C_{1-6}$ alkyl-carbamoyl group, a di-$C_{1-6}$ alkyl-carbamoyl group, an amino group, a mono-$C_{1-6}$ alkylamino group, a di-$C_{1-6}$ alkylamino group, a 5- to 7-membered cyclic amino group, a $C_{1-6}$ alkyl-carbonylamino group, an aminosulfonyl group, a mono-$C_{1-6}$ alkylaminosulfonyl group, a di-$C_{1-6}$ alkylaminosulfonyl group, a phenylsulfonylamino group, and a $C_{1-6}$ alkylsulfonylamino group.

As the "heterocyclic group" in the aforementioned "(xxiv) heterocyclic group which may have a substituent", for example, groups obtained by removing one hydrogen atom from a 5- to 14-membered (monocyclic or di- to tetra-cyclic) heterocyclic ring containing 1 to 6 (preferably 1 to 4) hetero atom(s) selected from a nitrogen atom, an oxygen atom and a sulfur atom are used.

Examples of the monocyclic heterocyclic group include groups obtained by removing one hydrogen atom from monocyclic heterocyclic rings such as pyridine, pyrazine, pyrimidine, imidazole, furan, thiophen, dihydropyridine, diazepine, oxazepine, pyrrolidine, piperidine, hexamethyleneimine, heptamethylemeimine, tetrahydrofuran, piperazine, homopiperazine, tetrahydrooxazepine, morpholine, thiomorpholine, pyrrole, pyrazole, 1,2,3-triazole, oxazole, oxazolidine, thiazole, thiazolidine, isoxazole, imidazoline, triazole, thiadiazole, oxadiazole, oxathiadiazole, triazine, and tetrazole.

As the bicyclic heterocyclic ring, for example, groups obtained by removing one hydrogen atom from bicyclic heterocyclic rings such as indole, dihydroindole, isoindole, dihydroisoindole, benzofuran, dihydrobenzofuran, benzimidazole, benzoxazole, benzisoxazole, benzothiazole, indazole, quinoline, tetrahydroquinoline, isoquinoline, tetrahydroisoquinoline, tetrahydro-1H-1-benzazepine, tetrahydro-1H-2-benzazepine, tetrahydro-1H-3-benzazepine, tetrahydrobenzoxazepine, quinazoline, tetrahydroquinazoline, quinoxaline, tetrahydroquinoxaline, benzodioxane, benzodioxol, benzothiazine, and imidazopyridine are used.

Examples of the tri- or tetra-cyclic heterocyclic group include groups obtained by removing one hydrogen atom from tri- or tetra-cyclic heterocyclic rings such as acridine, tetrahydroacridine, pyrroloquinoline, pyrroloindole, cyclopentindole, and isoindolobenzazepine.

As the "heterocyclic group", groups obtained by removing one hydrogen atom from monocyclic or bicyclic heterocyclic rings are preferred.

Examples of the "substituent" in the "heterocyclic group which may have a substituent" include "substituents" for the "homocyclic ring or heterocyclic ring which may have a substituent" represented by the above-mentioned $B^2$ ring, and the number of substituent(s) is 1 to 5.

Preferable examples of the "hydrocarbon group which may have a substituent" represented by $R^7$ include a $C_{7-16}$ aralkyl group (preferably benzyl etc.) which may have 1 to 5 substituent (s) selected from halogen atom, $C_{1-6}$ alkyl, $C_{1-6}$ alkoxy, nitro, cyano, carbamoyl, mono-$C_{1-6}$ alkyl-carbamoyl, di-$C_{1-6}$ alkyl-carbamoyl, aminosulfonyl, mono-$C_{1-6}$ alkylaminosulfonyl, di-$C_{1-6}$ alkylaminosulfonyl and hydroxy.

Examples of the "acyl group" represented by the above-mentioned $R^7$include acyl groups represented by the formula:

$$-(C=O)-R^8,\ -(C=O)-OR^8,\ -(C=O)-NR^8R^9,\ -SO_2-R^8,\ -SO-R^8,\ -SO_2-\ NR^8R^9,\ -(C=S)-OR^8 \text{ or } -(C=S)NR^8R^9$$

wherein $R^8$ and $R^9$ represent (i) a hydrogen atom, (ii) a hydrocarbon group which may have a substituent or (iii) a heterocyclic group which may have a substituent, respectively, or $R^8$ and $R^9$ may be linked together with an adjacent nitrogen atom to form a nitrogen-containing cyclic group which may have a substituent.

Among them, preferred are acyl groups represented by the formula:

$$-(C=O)-R^8,\ -(C=O)-NR^8R^9,\ -SO_2-R^8 \text{ or } -SO_2-NR^8R^9$$

wherein each symbol is as defined above.

Examples of the "hydrocarbon group which may have a substituent" and the "heterocyclic group which may have a substituent" represented by $R^8$ or $R^9$ include the same "hydrocarbon group which may have a substituent" and "heterocyclic group which may have a substituent" as those represented by the above-mentioned $R^7$.

Examples of the "nitrogen-containing cyclic group which may have a substituent" formed by $R^8$ and $R^9$ include 5- to 9-membered (preferably 5- to 7-membered) nitrogen-containing saturated heterocyclic groups which may have 1 to 3 hetero atom(s) selected from a nitrogen atom, an oxygen atom and a sulfur atom in addition to carbon atoms and one nitrogen atom. More specific examples include groups represented by the formulas:

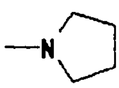, 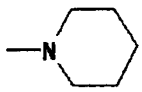, 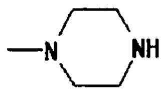, 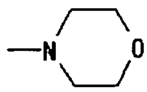,

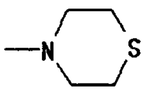, 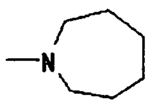, 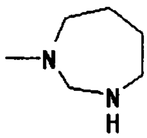, 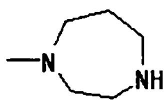

Examples of the "substituent" in the "nitrogen-containing cyclic group which may have a substituent" include the same "substituents" in the "homocyclic ring or heterocyclic ring which may have a substituent" represented by the above-mentioned B ring, and the number of substituent(s) is 1 to 5.

Preferable examples of $R^8$ and $R^9$ include (i) a hydrogen atom, (ii) optionally halogenated $C_{1-6}$ alkyl, (iii) $C_{6-10}$ aryl which may have 1 to 3 substituent(s) selected from $C_{1-6}$ alkyl and $C_{1-6}$ alkoxy, (iii) $C_{7-16}$ aralkyl (e.g. benzyl etc.), and (iv) a 5-or 6-membered heterocyclic group (e.g. pyridyl, thienyl, furyl etc.).

Preferable examples of the "acyl group" represented by the above-mentioned $R^7$ include formyl, optionally halogenated $C_{1-6}$ alkyl-carbonyl (e.g. acetyl, trifluoroacetyl, propionyl etc.), 5- or 6-membered heterocyclic carbonyl (e.g. pyridylcarbonyl, thienylcarbonyl, furylcarbonyl etc.), $C_{6-14}$ aryl-carbonyl (e.g. benzoyl, 1-naphthoyl, 2-naphthoyl etc.), $C_{7-16}$ aralkyl-carbonyl (e.g. phenylacetyl, 3-phenylpropionyl etc.), optionally halogenated $C_{1-6}$ alkylsulfonyl (e.g. methanesulfonyl, trifluoromethanesulfonyl, propylsulfonyl etc.), $C_{6-14}$ arylsulfonyl (e.g. benzenesulfonyl, naphthylsulfonyl etc.), carbamoyl, mono-$C_{1-6}$ alkyl-carbamoyl (e.g. methylcarbamoyl, ethylcarbamoyl etc.), $C_{1-6}$ alkyl-carbamoyl (e.g. dimethylcarbamoyl, diethylcarbamoyl etc.), aminosulfonyl, mono-$C_{1-6}$ alkylaminosulfonyl (e.g. methylaminosulfonyl, ethylaminosulfonyl etc.), and di-$C_{1-6}$ alkylaminosulfonyl (e.g. dimethylaminosulfonyl, diethylaminosulfonyl etc.).

Preferable examples of $R^7$ include hydrogen atom, $C_{1-6}$ alkyl, $C_{1-6}$ alkyl-carbonyl, carbamoyl, mono-$C_{1-6}$ alkyl-carbamoyl, di-$C_{1-6}$ alkyl-carbamoyl, aminosulfonyl, mono-$C_{1-6}$ alkylaminosulfonyl, and di-$C_{1-6}$ alkylaminosulfonyl.

Specific examples of the group represented by the above-mentioned formula:

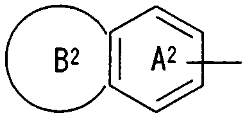

include groups obtained by removing one hydrogen atom from bicyclic condensed benzene rings such as naphthalene; tetrahydronaphthalene; indane; indene; benzo[a]cycloheptene; benzofuran such as 2,3-dihydro-1-benzofuran, 1,3-dihydro-2-benzofuran etc.; chroman; 3,4-dihydro-1H-isochromen; benzoxepine such as 2,3,4,5-tetrahydro-1-benzoxepine, 1,3,4,5-tetrahydro-2-benzoxepine, 1,2,4,5-tetrahydro-3-benzoxepine etc.; benzothiophen such as 2,3-dihydro-1-benzothiophen, 1,3-dihydro-2-benzothiophen etc.; thiochroman; 3,4-dihydro-1H-isothiochromen; benzothiepine such as 2,3,4,5-tetrahydro-1-benzothiepine, 1,3,4,5-tetrahydro-2-benzothiepine, 1,2,4,5-tetrahydro-3-benzothiepine; 3,4-dihydro-2H-1-benzothiopyran; 2,3-dihydro-1H-indole; and 1,2,3,4-tetrahydroquinoline; 2,3-dihydro-1H-isoindole; 1,2,3,4-tetrahydroisoquinoline; benzazepine such as 2,3,4,5-tetrahydro-1H-1-benzazepine, 2,3,4,5-tetrahydro-1H-2-benzazepine, 2,3,4,5-tetrahydro-1H-3-benzazepine; benzazocine such as 1,2,3,4,5,6-hexahydro-1-benzazocine, 1,2,3,4,5,6-hexahydro-2-benzazocine, 1,2,3,4,5,6-hexahydro-3-benzazocine; benzazonine such as 2,3,4,5,6,7-hexahydro-1H-1-benzazonine, 2,3,4,5,6,7-hexahydro-1H-2-benzazonine, 2,3,4,5,6,7-hexahydro-1H-3-benzazonine, and 2,3,4,5,6,7-hexahydro-1H-4-benzazonine; benzoxazole such as 2,3-dihydrobenzoxazole; benzothiazole such as 2,3-dihydrobenzothiazole; benzisothiazole such as 2,3-dihydro-1,2-benzisothiazole, and 1,3-dihydro-2,1-benzisothiazole; benzimidazole such as 2,3-dihydro-1H-benzimidazole; 1,3-dihydro-2,1,3-benzothiadiazole; benzoxazine such as 3,4-dihydro-1H-2,1-benzoxazine, 3,4-dihydro-1H-2,3-benzoxazine, 3,4-dihydro-2H-1,2-benzoxazine, 3,4-dihydro-2H-1,4-benzoxazine, 3,4-dihydro-2H-1,3-benzoxazine, and 3,4-dihydro-2H-3,1-benzoxazine; benzothiazine such as 3,4-dihydro-1H-2,1-benzothiazine, 3,4-dihydro-1H-2,3-benzothiazine, 3,4-dihydro-2H-1,2-benzothiazine, 3,4-dihydro-2H-1,4-benzothiazine, 3,4-dihydro-2H-1,3-benzothiazine, and 3,4-dihydro-2H-3,1-benzothiazine; benzoisothiazine such as 3,4-dihydro-2H-1,2-benzoisothiazine, and 3,4-dihydro-1H-2,1-benzoisothiazine; 3,4-dihydro-1H-2,13-benzothiadiazine; benzodiazine such as 1,2,3,4-tetrahydrocinnoline, 1,2,3,4-tetrahydrophthalazine, 1,2,3,4-tetrahydroquinazoline and 1,2,3,4-tetrahydroquinoxaline; benzoxathiine such as 3,4-dihydro-1,2-benzoxathiine, 3,4-dihydro-2,1-benzoxathiine, 2,3-dihydro-1,4-benzoxathiine, 1,4-dihydro-2,3-benzoxathiine, 4H-1,3-benzoxathiine, and 4H-3,1-benzoxathiine; 1,3-benzodioxol; 1,3-benzodithiol; benzodioxine such as 3,4-dihydro-1,2-benzodioxine, 2,3-dihydro-1,4-benzodioxine, 1,4-dihydro-2,3-benzodioxine, and 4H-1,3-benzodioxine; benzdithiin such as 3,4-dihydro-1,2-benzdithiine, 2,3-dihydro-1,4-benzdithiine, 1,4-dihydro-2,3-benzdithiine, and 4H-1,3-benzdithiine; benzoxazepine such as 2,3,4,5-tetrahydro-1,2-benzoxazepine, 2,3,4,5-tetrahydro-1,3-benzoxazepine, 2, 3, 4, 5-tetrahydro-1,4-benzoxazepine, 2,3,4,5-tetrahydro-1,5-benzoxazepine, 1,3,4,5-tetrahydro-2,1-benzoxazepine, 1,3,4,5-tetrahydro-2,3-benzoxazepine, 1,3,4,5-tetrahydro-2,4-benzoxazepine, 1,2,4,5-tetrahydro-3,1-benzoxazepine, 1,2,4,5-tetrahydro-3,2-benzoxazepine, and 1,2,3,5-tetrahydro-4,1-benzoxazepine; benzothiazepine such as 2,3,4,5-tetrahydro-1,2-benzothiazepine, 2,3,4,5-tetrahydro-1,4-benzothiazepine, 2,3,4,5-tetrahydro-1,5-benzothiazepine, 1,3,4,5-tetrahydro-2,1-benzothiazepine, 1,3,4,5-tetrahydro-2,4-benzothiazepine, 1,2,4,5-tetrahydro-3,1-benzothiazepine, 1,2,4,5-tetrahydro-3,2-benzothiazepine, and 1,2,3,5-tetrahydro-4,1-benzothiazepine; benzodiazepine such as 2,3,4,5-tetrahydro-1H-1,2-benzodiazepine, 2,3,4,5-tetrahydro-1H-1,3-benzodiazepine, 2,3,4,5-tetrahydro-1H-1,4-benzodiazepine, 2,3,4,5-tetrahydro-1H-1,5-benzodiazepine, 2,3,4,5-tetrahydro-1H-2,3-benzodiazepine, and 2,3,4,5-tetrahydro-1H-2,4-benzodiazepine; benzodioxepine such as 4,5-dihydro-1,3-benzodioxepine, 4,5-dihydro-3H-1,2-benzodioxepine, 2,3-dihydro-5H-1,4-benzodioxepine, 3,4-dihydro-2H-1,5-benzodioxepine, 4,5-dihydro-1H-2,3-benzodioxepine, and 1,5-dihydro-2,4-benzodioxepine; benzodithiepine such as 4,5-dihydro-1H-2,3-benzothiepine, 1,5-dihydro-2,4-benzodithiepine, 3,4-dihydro-2H-1,5-benzodithiepine, and 2,3-dihydro-5H-1,4-benzodithiepine; benzoxazocine such as 3,4,5,6-tetrahydro-2H-1,5-benzoxazocine, and 3,4,5,6-tetrahydro-2H-1,6-benzoxazocine; benzothiazocine such as 3,4,5,6-tetrahydro-2H-1,5-benzothiazocine, and 3,4,5,6-tetrahydro-2H-1,6-benzothiazocine; benzodiazocine such as 1,2,3,4,5,6-hexahydro-1,6-benzodiazocine; benzoxathiocine such as 2,3,4,5-tetrahydro-1,6-benzoxathiocine; benzodioxocine such as 2,3,4,5-tetrahydro-1,6-benzodioxocine; benzotrioxepine such as 1, 3, 5-benzotrioxepine, and 5H-1,3,4-benzotrioxepine; benzoxathiazepine such as 3,4-dihydro-1H-5,2,1-benzoxathiazepine, 3,4-dihydro-2H-5,1,2-benzoxathiazepine, 4,5-dihydro-3,1,4-benzoxathiazepine, and 4,5-dihydro-3H-1,2,5-benzoxathiazepine; benzoxadiazepine such as 2,3,4,5-tetrahydro-1,3,4-benzoxadiazepine; benzthiadiazepine such as 2,3,4,5-tetrahydro-1,3,5-benzthiadiazepine; benzotriazepine such as 2,3,4,5-tetrahydro-1H-1,2,5-benzotriazepine; 4,5-dihydro-1,3,2-benzoxathiepine, 4,5-dihydro-1H-2,3-benzoxathiepine, 3,4-dihydro-2H-1,5-benzoxathiepine, 4,5-dihydro-3H-1,2-benzoxathiepine, 4,5-dihydro-3H-2,1-benzoxathiepine, 2,3-dihydro-5H-1,4-benzoxathiepine, and 2,3-dihydro-5H-4,1-benzoxathiepine, inter alia, naphthalene, tetrahydronaphthalene, indane,

indene, 2,3,4,5-tetrahydro-1H-3-benzazepine, 2,3,4,5-tetrahydro-1H-2-benzazepine, 2,3-dihydro-1H-indole, 2,3,4,5-tetrahydro-1,4-benzoxazepine, 2,3-dihydro-1-benzofuran, chroman, 1,3-dihydro-2H-benzimidazole-2-one, and 1,3-dihydro-2,1,3-benzothiadiazole.

Preferable Examples of the case where $B^2$ ring is a heterocyclic ring include the groups represented by the formula:

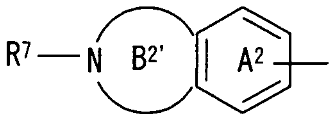

or

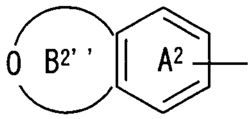

wherein $B^{2'}$ ring represents a 5- to 9-membered nitrogen-containing heterocyclic ring which may have a substituent, $B^{2''}$ ring represents a 5- to 9-membered oxygen-containing heterocyclic ring which may have a substituent, and the other symbols are as defined above.

Examples of the "5- to 9-membered nitrogen-containing heterocyclic ring" in the "5- to 9-membered nitrogen-containing heterocyclic ring which may have a substituent" include 5- to 9-membered nitrogen-containing heterocyclic rings which may have 1 to 3 hetero atom(s) selected from a nitrogen atom, an oxygen atom and a sulfur atom in addition to carbon atoms and one nitrogen atom, and 5- to 9-membered non-aromatic nitrogen-containing heterocyclic rings (e.g. pyrrolidine, piperidine, hexamethyleneimine, heptamethyleneimine, piperazine, homopiperazine, tetrahydrooxazepine, morpholine, thiomorpholine, hexahydropyrimidine, imidazolidine, thiadiazolidine etc.) are preferably used. Examples of the "substituent" include 1 to 3 substituent(s) selected from the same substituents as "substituents" in the "homocyclic ring or heterocyclic ring which may have a substituent" represented by above-mentioned B ring.

Examples of the "5- to 9-membered oxygen-containing heterocyclic ring" in the "5- to 9-membered oxygen-containing heterocyclic ring which may have a substituent" include 5- to 9-membered oxygen-containing heterocyclic rings which may contain 1 to 3 hetero atom(s) selected from an oxygen atom and a sulfur atom in addition to carbon atoms and one oxygen atom, and 5- to 9-membered non-aromatic oxygen-containing heterocyclic rings (e.g. tetrahydrofuran, tetrahydropyran, oxepane etc.) are preferably used. Examples of the "substituent" include 1 to 3 substituent(s) selected from the same substituents as those in the "homocyclic ring or heterocyclic ring which may have a substituent" represented by above-mentioned $B^2$ ring, and preferred are oxo, $C_{1-6}$ alkyl, $C_{1-6}$ alkyl-carbonylamino, and $C_{1-6}$ alkylsulfonylamino.

Among the groups represented by the formula:

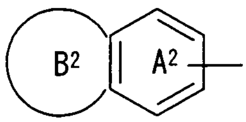

,

more preferable examples include the groups represented by the formula:

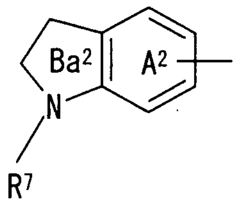

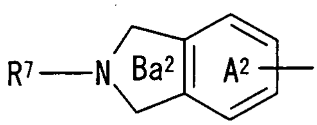

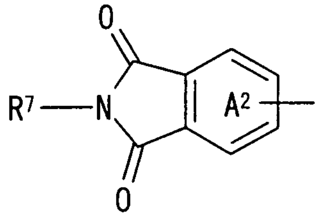

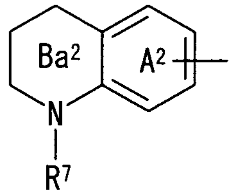

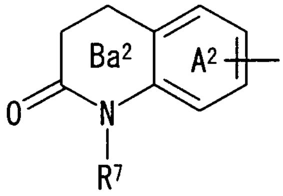

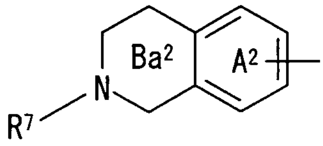

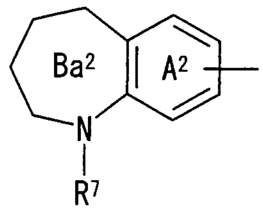

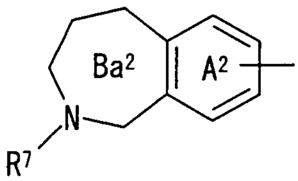

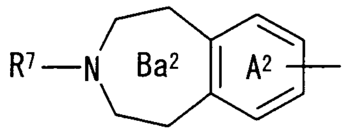

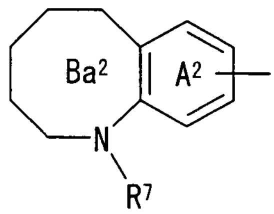

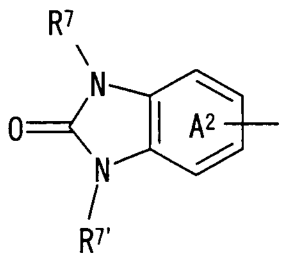

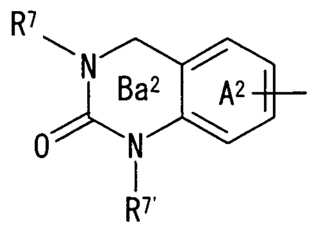

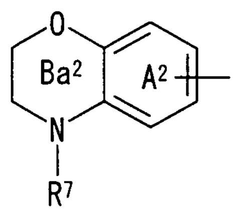

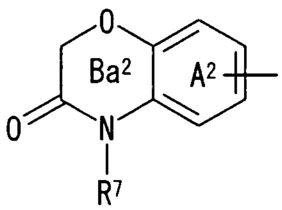

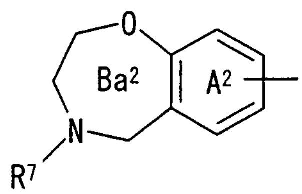

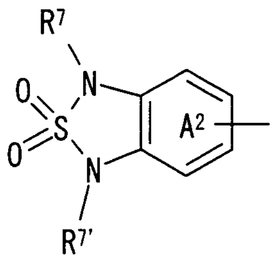 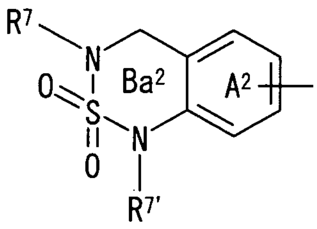 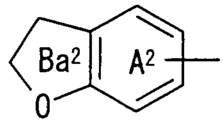 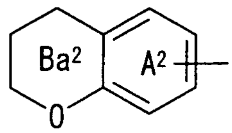

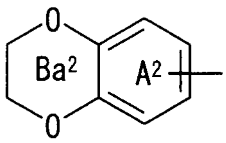

or

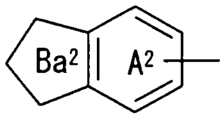

wherein $Ba^2$ ring represents a homocyclic ring or a heterocyclic ring which may have a substituent, $R^{7'}$ is as defined above for $R^7$, and the other symbols are as defined above.

Examples of the "substituent" in the "homocyclic ring or heterocyclic ring which may have a substituent" represented by $Ba^2$ ring include 1 or 2 substituent(s) selected from substituents other than an oxo group among "substituents" in the "homocyclic ring or heterocyclic ring which may have a substituent" represented by above-mentioned $B^2$ ring.

It is preferable that $A^2$ ring is a benzene ring which may have 1 or 2 substituent(s) selected from aminosulfonyl, mono- or di-$C_{1-6}$ alkylaminosulfonyl, carbamoyl and mono- or di-$C_{1-6}$ alkyl-carbamoyl, $Ba^2$ ring may have 1 or 2 substituent (s) selected from $C_{1-6}$ alkyl, $C_{1-6}$ alkyl-carbonylamino and $C_{1-6}$ alkylsulfonylamino, and $R^7$ and $R^{7'}$ represent (1) a $C_{1-6}$ alkyl group or a $C_{7-16}$ aralkyl group, each of which may have 1 or 2 substituent(s) selected from hydroxy and $C_{1-6}$ alkoxy-carbonyl, or (2) formulas: - (C=O) -$R^{8'}$, -(C=O)-$NR^{8'}$ $R^{9'}$ or -$SO_2R^{8'}$ [wherein $R^{8'}$ and $R^{9'}$ represent hydrogen atom, optionally halogenated $C_{1-6}$ alkyl or $C_{6-10}$ aryl], respectively.

Specific examples of the case where the phenyl group in the "optionally condensed phenyl group, and said phenyl group may have a substituent" of the above-mentioned (b) is condensed with a bicyclic homocyclic ring or heterocyclic ring which may have a substituent, or is condensed with two same or different monocyclic homocyclic rings or heterocyclic rings include groups represented by the formula:

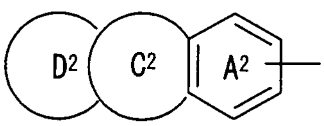 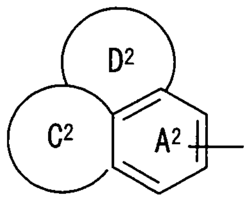 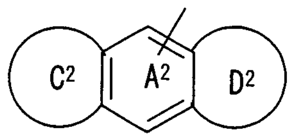

or

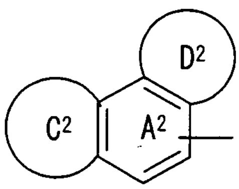

wherein $A^2$ ring is as defined above, and $C^2$ ring and $D^2$ ring represent a homocyclic ring or a heterocyclic ring which may have a substituent.

Examples of the "homocyclic ring" in the "homocyclic ring which may have a substituent" represented by $C^2$

ring or $D^2$ ring include the same homocyclic rings as "homocyclic rings" in the "homocyclic ring which may have a substituent" represented by $B^2$ ring.

Examples of the "heterocyclic ring" in the "heterocyclic ring which may have a substituent" represented by $C^2$ ring or $D^2$ ring include 5- to 9-membered heterocyclic rings which may have 1 to 3 hetero atom(s) selected from a nitrogen atom, an oxygen atom and a sulfur atom (e.g. pyridine, pyrazine, pyrimidine, imidazole, furan, thiophen, dihydropyridine, diazepine, oxazepine, pyrrolidine, piperidine, hexamethyleneimine, heptamethyleneimine, tetrahydrofuran, piperazine, homopiperazine, tetrahydrooxazepine, morpholine, thiomorpholine, hexahydropyrimidine, imidazolidine, thiadiazolidine etc.).

Examples of the "substituent" in the "homocyclic ring or heterocyclic ring optionally having a substituent" represented by $C^2$ ring or $D^2$ ring include the same substituents as "substituents" in the "homocyclic ring or heterocyclic ring which may have a substituent" represented by above-mentioned $B^2$ ring.

Specific examples of the groups represented by the aforementioned formula:

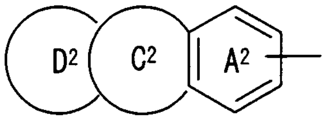

wherein each symbol is as defined above, include groups obtained by removing one hydrogen atom from tricyclic condensed benzene rings such as anthracene, carbazole, 1,2,3,4,4a,9a-hexahydrocarbazole, 9,10-dihydroacridine, 1,2,3,4-tetrahydroacridine, 10, 11-dihydro-5H-dibenz[b,f]azepine, 5,6,7,12-tetrahydrodibenz[b,g]azocine, 6,11-dihydro-5H-dibenz[b,e]azepine, 6,7-dihydro-5H-dibenz[c,e]azepine, 5, 6, 11, 12-tetrahydrodibenz [b, f] azocine, dibenzofuran, 9H-xanthene, 10,11-dihydrodibenz[b,f]oxepine, 6,11-dihydrodibenz[b,e]oxepine, 6,7-dihydro-5H-dibenz[b,g]oxocine, dibenzothiophen, 9H-thioxanthene, 10,11-dihydrodibenzo[b,f]thiepine 6,11-dihydrodibenzo[b,e]thiepine, 6,7-dihydro-5H-dibenzo[b,g]thiocine, 10H-phenothiazine, 10H-phenoxazine, 5,10-dihydrophenazine, 10,11-dibenzo[b,f] [1,4]thiazepine, 10,11-dihydrodibenz[b,f][1,4]oxazepine, 2,3,5,6,11,11a-hexahydro-1H-pyrrolo[2,1-b][3]benzazepine, 10,11-dihydro-5H-dibenzo[b,e][1,4]diazepine, 5,11-dihydrodibenz[b,e][1,4]oxazepine, 5,11-dihydrodibenzo[b,f][1,4]thiazepine, 10,11-dihydro-5H-dibenzo[b,e][1,4]diazepine, and 1,2,3,3a, 8,8a-hexahydropyrrolo[2,3-b]indole.

Examples of the groups represented by the above-mentioned formula:

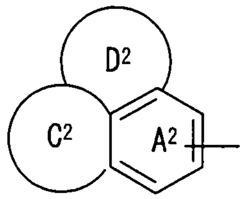

wherein each symbol is as defined above, include groups obtained by removing one hydrogen atom from tricyclic condensed benzene rings such as phenalene, acenaphthylene, 1H,3H-naphtho[1,8-cd][1,2]oxazine, naphtho[1,8-de]-1,3-oxazine, naphtho[1,8-de]-1,2-oxazine, 1,2,2a,3,4,5-hexahydrobenz[cd]indole, 2,3,3a,4,5,6-hexahydro-1H-benzo [de]quinoline, 4H-pyrrolo[3,2,1-ij]quinoline, 1,2,5,6-tetrahydro-4H-pyrrolo[3,2,1-ij]quinoline, 5,6-dihydro-4H-pyrrolo [3,2,1-ij]quinoline, 1H-pyrrolo[3,2,1-ij]quanazoline, 4H-imidazo[4,5,1-ij]quinoline, 2,3,7,8-tetrahydro[1,2,6]thiadiazino [4,3,2-hi]indole, 1,2,6,7-tetrahydro-3H,5H-pyrido[3,2,1-ij]quinazoline, 2,3,8,9-tetrahydro-7H-[1,2,6]thiadiazino[4,3,2-ij] quinoline, 5,6-dihydro-1H,4H-[1,2,5]thiadiazolo[4,3,2-ij]quinoline, 1H,5H-benzo[ij]quinolizine, azepino[3,2,1-hi]indole, 1,2,4,5,6,7-hexahydroazepino[3,2,1-hi]indole, 1H-pyrido[3,2,1-jk][1]benzazepine, 5,6,7,8-tetrahydro-1H-pyrido[3,2,1-jk] [1] benzazepine, 1, 2, 5, 6, 7, 8-hexahydro-1H-pyrido [3,2,1-jk][1]benzazepine, 2,3-dihydro-1H-benz[de]isoqiunoline, 1,2,3,4,4a,5,6,7-octahydronaphtho[1,8-bc]azepine, and 2,3,5,6,7,8-hexahydro-1H-pyrido[3,2,1-jk][1]benzazepine.

Specific examples of the group represented by the above-mentioned formula:

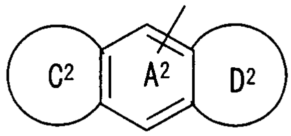

wherein each symbol is as defined above, include groups obtained by removing one hydrogen atom from tricyclic condensed benzene rings such as anthracene, 1,2,3,5,6,7-hexahydrobenzo[1,2-b:4,5-b']dipyrrole, and 1,2,3,5,6,7-hexahydrocyclopenta[f]indole.

Specific examples of the group represented by the above-mentioned formula:

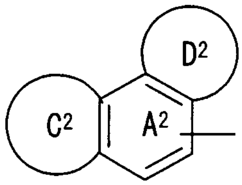

wherein each symbol is as defined above, include groups obtained by removing one hydrogen atom from tricyclic condensed benzene rings such as phenanthrolene, 1,2,3,6,7,8-hexahydrocyclopenta[e]indole, and 2,3,4,7,8,9-hexahydro-1H-cyclopenta[f]quinoline.

Among them, groups represented by the formulas:

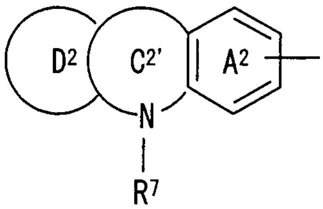 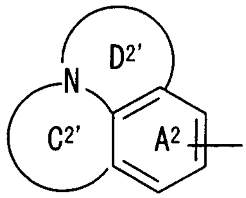

or

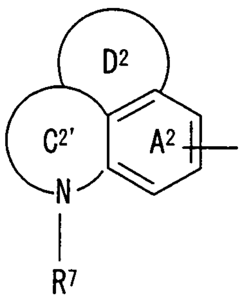

wherein $C^{2'}$ ring and $D^{2'}$ ring represent a 5- to 9- membered nitrogen-containing heterocyclic ring which may have a substituent, respectively, and the other symbols are as defined above, are preferable. Among them, groups represented by the formula:

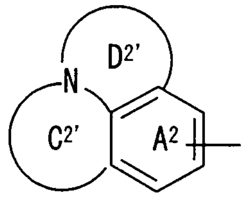

wherein each symbol is as defined above, are further preferred.

Examples of the "5- to 9- membered nitrogen-containing heterocyclic ring which may have a substituent" represented by $C^{2'}$ ring or $D^{2'}$ ring include the same "5- to 9- membered nitrogen-containing heterocyclic ring which may have a substituent" as those represented by $B^{2'}$ ring. As the substituent, an oxo group is preferable.

Inter alia, more preferable examples include groups represented by the formula:

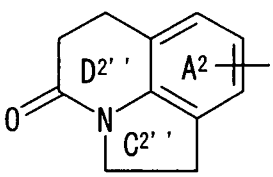
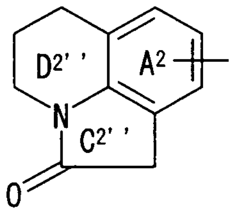
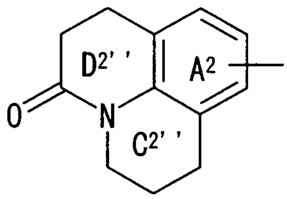
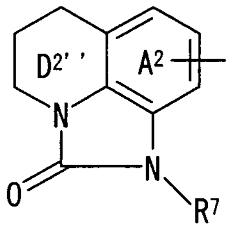

or

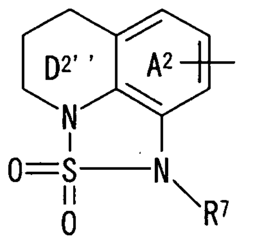

wherein $C^{2''}$ ring and $D^{2''}$ ring represent a nitrogen-containing heterocyclic ring which may have a substituent, and the other symbols are as defined above.

Examples of the "substituent" in the "nitrogen-containing heterocyclic ring which may have a substituent" represented by $C^{2''}$ ring and $D^{2''}$ ring include 1 to 2 substituent(s) selected from "substituents" other than an oxo group in the "homocyclic ring or heterocyclic ring which may have a substituent" represented by $B^2$ ring.

Herein, it is preferable that $A^2$ ring is a benzene ring which may have 1 or 2 substituent(s) selected from aminosulfonyl, mono- or di-$C_{1-6}$ alkylaminosulfonyl, carbamoyl and mono- or di-$C_{1-6}$ alkyl-carbamoyl, C" ring and D" ring may have 1 or 2 substituent(s) selected from $C_{1-6}$ alkyl, $C_{1-6}$ alkyl-carbonylamino and $C_{1-6}$ alkylsulfonylamino, and $R^1$ is (1) a $C_{1-6}$ alkyl group or a $C_{7-16}$ aralkyl group, each of which may have 1 or 2 substituent(s) selected from hydroxy and $C_{1-6}$ alkoxy-carbonyl, or (2) the formulas: -(C=O) -$R^{8'}$, -(C=O)-$NR^{8'}R^{9'}$ or -$SO_2R^{8'}$ [wherein $R^{8'}$ and $R^{9'}$ represent hydrogen atom, optionally halogenated $C_{1-6}$ alkyl or $C_{6-10}$ aryl].

Specific examples of the case where the phenyl group in the "optionally condensed phenyl group, and said phenyl group may have a substituent" of the above-mentioned (c) is condensed with a tricyclic homocyclic ring or heterocyclic ring which may have a substituent include groups represented by the formula:

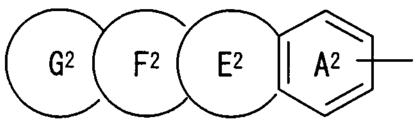

or

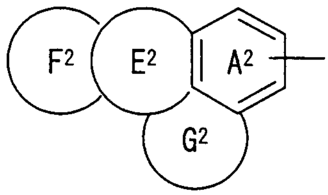

wherein $A^2$ ring is as defined above, and $E^2$ ring, $F^2$ ring and $G^2$ ring represent a homocyclic ring or a heterocyclic ring which may have a substituent.

Examples of the "homocyclic ring or heterocyclic ring which may have a substituent" represented by $E^2$ ring, $F^2$ ring or $G^2$ ring include the same "homocyclic ring or heterocyclic ring which may have a substituent" as those represented by $C^2$ ring or $D^2$ ring.

Among them, preferable examples include:

(i) a group represented by the formula:

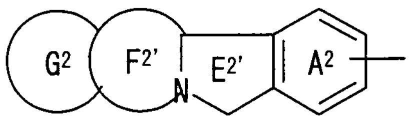

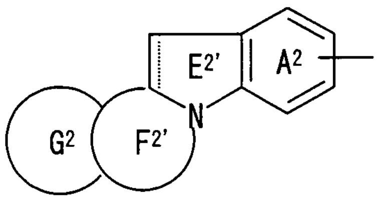

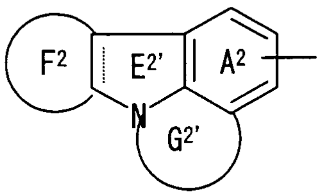

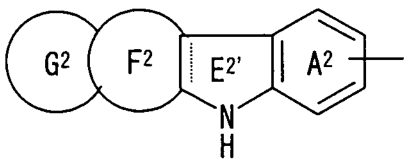

or

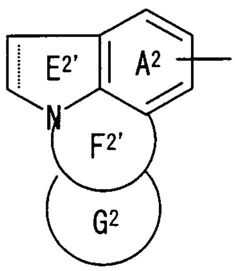

wherein $E^{2'}$ ring, $F^{2'}$ ring and $G^{2'}$ ring represent a 5- to 9-membered nitrogen-containing heterocyclic ring which may have a substituent, --- represents a single bond or a double bond, and the other symbols are as defined above,

(ii) a group obtained by removing one hydrogen atom from rings such as fluoranthene, acephenanthrylene, aceanthrylene, triphenylene, pyrene, chrysene, naphthacene, preyadene, benzo[a]anthracene, indeno[1,2-a]indene, cyclopenta[a]phenanthrene, pyrido[1',2':1,2]imidazo[4,5-b]quinoxaline, 1H-2-oxapyrene, and spiro[piperidine-4.9'-xanthene], and dihydro, tetrahydro, hexahydro, octahydro, decahydro compounds thereof.

Examples of the "5- to 9-membered nitrogen-containing heterocyclic ring which may have a substituent" represented by $E^{2'}$ ring, $F^{2'}$ ring and $G^{2'}$ ring include the same "5- to 9-membered nitrogen-containing heterocyclic rings which may have a substituent" as those represented by $B^{2'}$ ring. As the substituent, an oxo group is preferred.

Specific examples of the group represented by the above-mentioned formula:

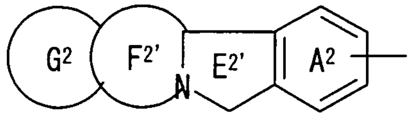

wherein each symbol is as defined above, include groups obtained by removing one hydrogen atom from tetracyclic condensed benzene rings such as 2H-isoindolo[2,1-e]purine, 1H-pyrazolo[4',3':3,4]pyrido[2,1-a]isoindole, 1H-pyrido[2',3':4,5]imidazo[2,1-a]isoindole, 2H,6H-pyrido[1',2':3,4]imidazo[5,1-a]isoindole, 1H-isoindolo[2,1-a]benzimidazole, 1H-pyrido[3',4':4,5]pyrrolo[2,1-a]isoindole, 2H-pyrido[4',3':4,5]pyrrolo[2,1-a]isoindole, 1H-isoindolo[2,1-a]indole, 2H-isoindolo[1,2-a]isoindole, 1H-cyclopenta[4,5]pyrimido[2,1-a]isoindole, 2H,4H-pyrano[4',3':4,5][1,3]oxazino[2,3-a] isoindole, 2H-isoindolo[2,1-a][3,1]benzoxazine, 7H-isoindolo[1,2-b][1,3]benzoxazine, 2H-pyrido[2' ,1' :3,4]pyrazino [2,1-a]isoindole, pyrido[2',3':4,5]pyrimido[2,1-a]isoindole, pyrido[3',2':5,6]pyrimido[2,1-a]isoindole, 1H-pyrido[1',2':3,4] pyrimido[2,1-a]isoindole, isoindolo[2,1-a]quinazoline, isoindolo[2,1-a]quinoxaline, isoindolo[1,2-a]isoquinoline, isoindolo [2,1-b]isoquinoline, isoindolo[2,1-a]quinoline, 6H-oxazino[3',4':3,4][1,4]diazepino[2,1-a]isoindole, azepino[2',1' :3,4] pyrazino[2,1-a]isoindole, 2H,6H-pyrido[2' ,1' :3,4] [1,4]diazepino[2,1-a]isoindole, 1H-isoindolo[1,2-b][1,3,4]benzotriazepine, 2H-isoindolo[2,1-a][1,3,4]benzotriazepine, isoindolo[2,1-d][1,4]benzoxazepine, 1H-isoindolo[2,1-b][2,4]benzodiazepine, 1H-isoindolo[2,1-c][2,3]benzodiazepine, 2H-isoindolo[1,2-a][2,4]benzodiazepine, 2H-isoindolo[2,1-d)[1,4] benzodiazepine, 5H-indolo[2,1-b] [3]benzazepine, 2H-isoindolo[1,2-a][2]benzazepine, 2H-isoindolo[1,2-b)[3]benzazepine, 2H-isoindolo[2,1-b][2]benzazepine, 2H-isoindolo[1,2-b][1,3,4]benzoxadiazocine, isoindolo[2,1-b][1,2,6]benzotriazocine, and 5H-4,8-methano-1H-[1,5]diazacycloundecino[1,11-a]indole.

Specific examples of the group represented by the above-mentioned formula:

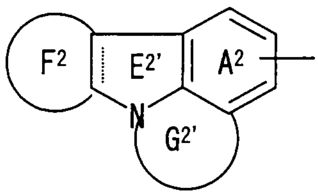

wherein each symbol is as defined above, include groups obtained by removing one hydrogen atom from tetracyclic condensed benzene rings such as 1H,4H-pyrrolo[3',2':4,5]pyrrolo[3,2,1-ij]quinoline, pyrrolo[3,2,1-jk]carbazole, 1H-furo [2',3':4,5]pyrrolo[3,2,1-ij]quinoline, 1H,4H-cyclopenta[4,5]pyrrolo[1,2,3-de]quinoxaline, 1H,4H-cyclopenta[4,5]pyrrolo [3,2,1-ij]quinoline, pyrido[3',4':4,5]pyrrolo[1,2,3-de]benzoxazine, [1,4]oxazino[2,3,4-jk]carbazole, 1H,3H-[1,3]oxazino [5,4,3-jk]carbozole, pyrido[3',4':4,5]pyrrolo[1,2,3-de][1,4]benzothiazine, 4H-pyrrolo[3,2,1-de]phenanthridine, 4H,5H-pyrido[3,2,1-de]phenanthridine, 1H,4H-3a,6a-diazafluoroanthene, 1-oxa-4,6a-diazafluoroanthene, 4-oxa-2,10b-diazafluoroanthene, 1-thia-4,6a-diazafluoroanthene, 1H-pyrazino[3,2,1-jk]carbazole, 1H-indolo[3,2,1-de][1,5]naphthyridine, benzo[b]pyrano[2,3,4-hi]indolizine, 1H,3H-benzo[b]pyrano[3,4,5-hi]indolizine, 1H,4H-pyrano[2',3':4,5]pyrrolo [3,2,1-ij]quinoline, 1H,3H-benzo[b]thiopyrano[3,4,5-hi]indolizine, 1H-pyrido[3,2,1-jk]carbazole, 4H-3-oxa-11b-azacyclohepta[jk]fluorene, 2H-azepino[1',2':1,2]pyrimidino[4,5-b]indole, 1H,4H-cyclohepta[4,5]pyrrolo[1,2,3-de]quinoxaline, 5H-pyrido[3',4':4,5]pyrrolo[1,2,3-ef][1,5]benzoxazepine, 4H-pyrido[3',4':4,5]pyrrolo[3,2,1-jk][4,1]benzothiazepine, 5H-pyrido[3',4':4,5]pyrrolo[1,2,3-ef][1,5]benzothiazepine, 5H-pyrido[4',3':4,5]pyrrolo[1,2,3-ef][1,5]benzothiazepine, [1,2,4]triazepino[6,5,4-jk]carbazole, [1,2,4]triazepino[6,7,1-jk]carbazole, [1,2,5]triazepino[3,4,5-jk]carbazole, 5H-[1,4] oxazepino[2,3,4-jk]carbazole, 5H-[1,4]thiazepino[2,3,4-jk]carbazole, [1,4]diazepino[3,2,1-jk]carbazole, [1,4]diazepino [6,7,1-jk]carbazole, azepino[3,2,1-jk]carbazole, 1H-cycloocta[4,5]pyrrolo[1,2,3-de]quinoxaline, and 1H-cycloocta[4,5] pyrrolo[3,2,1-ij]quinoline.

Specific examples of the group represented by the above-mentioned formula:

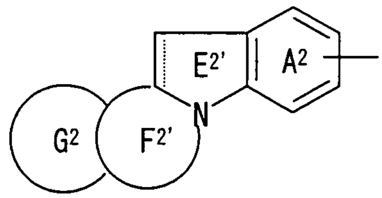

wherein each symbol is as defined above, include groups obtained by removing one hydrogen atom from tetracyclic condensed benzene rings such as 1H-indolo[1,2-a]benzimidazole, 1H-indolo[1,2-b]indazole, pyrrolo[2',1':3,4]pyrazino[1,2-a]indole, 1H,5H-pyrrolo[1',2':4,5]pyrazino[1,2-a]indole, 2H-pyrido[2',3':3,4]pyrrolo[1,2-a]indole, 1H-pyrrolo[2',3':3,4]pyrido[1,2-a]indole, 1H-indolo[1,2-a]indole, 6H-isoindolo[2,1-a]indole, 6H-indolo[1,2-c][1,3]benzoxazine, 1H-indolo[1,2-b] [1,2]benzothiazine, pyrimido[4',5':4,5]pyrimido[1,6-a]indole, pyrazino[2',3':3,4]pyrido[1,2-a]indole, 6H-pyrido[1',2':3,4]pyrimido[1,6-a]indole, indolo[1,2-b]cinnoline, indolo[1,2-a]quinazoline, indolo[1,2-c]quinazoline, indolo[2,1-b]quinazoline, indolo[1,2-a]quinoxaline, indolo[1,2-a] [1,8]naphthyridine, indolo[1,2-b]-2,6-naphthyridine, indolo[1,2-b][2,7]naphthyridine, indolo[1,2-h]-1,7-naphthyridine, indolo[1,2-b]isoquinoline, indolo[2,1-a]isoquinoline, indolo[1,2-a]quinoline, 2H-6H-pyrido[2',1':3,4][1,4]diazepino[1,2-a]indole, 1H-indolo[2,1-c][1,4]benzodiazepine, 2H-indolo[1,2-d][1,4]benzodiazepine, 2H-indolo[2,1-a][2,3]benzodiazepine, 2H-indolo[2,1-b][1,3]benzodiazepine, 1H-indolo[1,2-b][2]benzazepine, 2H-indolo[1,2-a][1]benzazepine, 2H-indolo[2,1-a][2]benzazepine, indolo[1,2-e][1,5]benzodiazocine, and indolo[2,1-b][3]benzazocine.

Specific examples of the group represented by the above-mentioned formula:

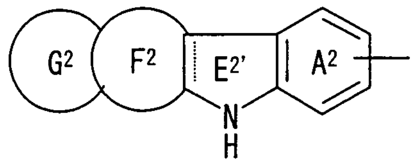

wherein each symbol is as defined above, include groups obtained by removing one hydrogen atom from tetracyclic condensed benzene rings such as 1H-imidazo[1',2':1,2]pyrido[3,4-b]indole, 1H-imidazo[1',2':1,6]pyrido[4,3-b]indole, 1H-imidazo[1',5':1,2]pyrido[3,4-b]indole, 1H-imidazo[1',5':1,6]pyrido[4,3-b]indole, 1H-pyrido[2',1':2,3]imidazo[4,5-b]indole, imidazo[4,5-a]carbazole, imidazo[4,5-c]carbazole, pyrazolo[3,4-c]carbazole, 2H-pyrazino[1',2':1,5]pyrrolo[2,3-b]indole, 1H-pyrrolo[1',2':1,2]pyrimido[4,5-b]indole, 1H-indolizino[6,7-b]indole, 1H-indolizino[8,7-b]indole, indolo[2,3-b]indole, indolo[3,2-b]indole, pyrrolo[2,3-a]carbazole, pyrrolo[2,3-b]carbazole, pyrrolo[2,3-c]carbazole, pyrrolo[3,2-a]carbazole, pyrrolo[3,2-b]carbazole, pyrrolo[3,2-c]carbazole, pyrrolo[3,4-a]carbazole, pyrrolo[3,4-b]carbazole, pyrrolo[3,4-c]carbazole, 1H-pyrido[3',4':4,5]furo[3,2-b]indole, 1H-furo[3,4-a]carbazole, 1H-furo[3,4-b]carbazole, 1H-furo[3,4-c]carbazole, 2H-furo[2,3-a]carbazole, 2H-furo[2,3-c]carbazole, 2H-furo[3,2-a]carbazole, 2H-furo[3,2-c]carbazole, 1H-pyrido[3',4':4,5]thieno[2,3-b]indole, thieno[3',2':5,6]thiopyrano[4,3-b]indole, thieno[3',4':5,6]thiopyrano[4,3-b]indole, 1H-[1]benzothieno[2,3-b]indole, 1H-[1]benzothieno[3,2-b]indole, 1H-thieno[3,4-a]carbazole, 2H-thieno[2,3-b]carbazole, 2H-thieno[3,2-a]carbazole, 2H-thieno[3,2-b]carbazole, cyclopenta[4,5]pyrrolo[2,3-f]quinoxaline, cyclopenta[5,6]pyrido[2,3-b]indole, pyrido[2',3':3,4]cyclopenta[1,2-b]indole, pyrido[2',3':4,5]cyclopenta[1,2-b]indole, pyrido[3',4':3,4]cyclopenta[1,2-b]indole, pyrido[3',4':4,5]cyclopenta[1,2-b]indole, pyrido[4',3':4,5]cyclopenta[1,2-b]indole, 1H-cyclopenta[5,6]pyrano[2,3-b]indole, 1H-cyclopenta[5,6]thiopyrano[4,3-b]indole, cyclopenta[a]carbazole, cyclopenta[c]carbazole, indeno[1,2-b]indole, indeno[2,1-b]indole, [1,2,4]triazino[4',3':1,2]pyrido[3,4-b]indole, 1,3,5-triazino[1',2':1,1]pyrido[3,4-b]indole, 1H-[1,4]oxazino[4',3':1,2]pyrido[3,4-b]indole, 1H-[1,4]oxazino[4',3':1,6]pyrido[3,4-b]indole, 4H-[1,3]oxazino[3',4':1,2]pyrido[3,4-b]indole, indolo[3,2-b][1,4]benzoxazine, 1,3-oxazino[6,5-b]carbazole, 2H-pyrimido[2',1':2,3][1,3]thiazino[5,6-b]indole, 2H-[1,3]thiazino[3',2':1,2]pyrido[3,4-b]indole, 4H-[1,3]thiazino[3',4':1,2]pyrido[3,4-b]indole, indolo[2,3-b][1,4]benzothiazine, indolo[3,2-b][1,4]benzothiazine, indolo[3,2-c][2,1]benzothiazine, 1,4-thiazino[2,3-a]carbazole, [1,4]thiazino[2,3-b]carbazole, [1,4]thiazino[2,3-c]carbazole, 1,4-thiazino[3,2-b]carbazole, 1,4-thiazino[3,2-c]carbazole, 1H-indolo[2,3-g]pteridine, 1H-indolo[3,2-g]pteridine, pyrazino[1',2':1,2]pyrido[3,4-b]indole, pyrazino[1',2':1,2]pyrido[4,3-b]indole, 1H-pyrido[2',3':5,6]pyrazino[2,3-b]indole, 1H-pyrido[3',2':5,6]pyrazino[2,3-b]indole, 1H-pyrido[3',4':5,6]pyrazino[2,3-b]indole, pyrido[1',2':1,2]pyrimido[4,5-b]indole, pyrido[1',2':1,2]pyrimido[5,4-b]indole, pyrido[2',1':2,3]pyrimido[4,5-b]indole, pyrimido[1',2':1,2]pyrido[3,4-b]indole, pyrimido[1',2':1,6]pyrido[3,4-b]indole, pyrimido[5',4':5,6]pyrano[2,3-b]indole, pyridazino [4' , 5' : 5, 6] thiopyrano [4, 5-b] indole, 1H-indolo[3,2-c]cinnoline, 1H-indolo[2,3-b]quinoxaline, 1H-pyrazino[2,3-a]carbazole, 1H-pyrazino[2,3-b]carbazole, 1H-pyrazino[2,3-c]carbazole, 1H-pyridazino[3,4-c]carbazole, 1H-pyridazino[4,5-b]carbazole, 1H-pyrimido[4,5-a]carbazole, 1H-pyrimido[4,5-c]carbazole, 1H-pyrimido[5,4-a]carbazole, 1H-pyrimido[5,4-b]carbazole, 1H-pyrimido[5,4-c]carbazole, 7H-1,4-dioxino[2',3':5,6][1,2]dioxino[3,4-b]indole, 6H-[1,4]benzodioxino[2,3-b]indole, 6H-[1,4]benzodithiino[2,3-b]indole, 1H-indolo[2,3-b]-1,5-naphthyridine, 1H-indolo[2,3-b][1,6]naphthyridine, 1H-indolo[2,3-b][1,8]naphthyridine, 1H-indolo[2,3-c]-1,5-naphthyridine, 1H-indolo[2,3-c][1,6]naphthyridine, 1H-indolo[2,3-c][1,7]naphthyridine, 1H-indolo[2,3-c][1,8]naphthyridine, 1H-indolo[3,2-b]-1,5-naphthyridine, 1H-indolo[3,2-b][1,7]naphthyridine, 1H-indolo[3,2-b][1,8]naphthyridine, 1H-indolo[3,2-c][1,8]naphthyridine, indolo[2,3-a]quinolizine, indolo[2,3-b]quinolizine, indolo[3,2-a]quinolizine, indolo[3,2-b]quinolizine, pyrano[4',3':5,6]pyrido[3,4-b]indole, pyrido[4',3':4,5]pyrano[3,2-b]indole, pyrido[4',3':5,6]pyrano[2,3-b]indole, pyrido[4',3':5,6]pyrano[3,4-b]indole, 1H-indolo[2,3-c]isoquinoline, 1H-indolo[3,2-c]isoquinoline, 1H-indolo[2,3-c]quinoline, 1H-indolo[3,2-c]quinoline, 1H-pyrido[2,3-a]carbazole, 1H-pyrido[2,3-b]carbazole, 1H-pyrido[2,3-c]carbazole, 1H-pyrido[3,2-a]carbazole, 1H-pyrido[3,2-b]carbazole, 1H-pyrido[3,2-c]carbazole, 1H-pyrido[3,4-a]carbazole, 1H-pyrido[3,4-b]carbazole, 1H-pyrido[3,4-c]carbazole, 1H-pyrido[4,3-a]carbazole, 1H-pyrido[4,3-b]carbazole, 1H-pyrido[4,3-c]carbazole, 1H-quindoline, 1H-quininedoline, 1H-pyrano[3',4':5,6]pyrano[4,3-b]indole, [1]benzopyrano[2,3-b]indole, [1]benzopyrano[3,2-b]indole, [1]benzopyrano[3,4-b] indole, [1] benzopyrano [4, 3-b] indole, [2] benzopyrano [4, 3-b]indole, pyrano[2,3-a]carbazole, pyrano[2,3-b]carbazole, pyrano[2,3-c]carbazole, pyrano [3,2-a] carbazole, pyrano[3,2-c]carbazole, pyrano[3,4-a]carbazole, 1H-phosphinolino[4,3-b]indole, [1]benzothiopyrano[2,3-b]indole, [1]benzothiopyrano[3,2-b]indole, [1]benzothiopyrano[3,4-b]indole, [1]benzothiopyrano[4,3-b]indole, [2]benzothiopyrano[4,3-b]indole, 1H-benzo[a]carbazole, 1H-benzo[b]carbazole, 1H-benzo[c]carbazole, [1,6,2]oxathiazepino[2',3':1:2]pyrido[3,4-b]indole, 1H-azepino[1',2':1,2]pyrido[3,4-b]indole, 1H-pyrido[1',2':1,2]azepino[4,5-b]indole, 2H-pyrido[1',2':1,2]azepino,[3,4-b]indole, 1H-pyrido[3',2':5,6]oxepino[3,2-b]indole, 1H-pyrido[4',3':5,6]oxepino[3,2-b]indole, 2H-pyrido[2',3':5,6]oxepino[2,3-b]indole, 2H-pyrido[2',3':5,6]oxepino[3,2-b]indole, 2H-pyrido[3',4':5,6]oxepino[3,2-b]indole, pyrido[2',3':4,5]cyclohepta[1,2-b]indole, pyrido[3',2':3,4]cyclohepta[1,2-b]indole, pyrido[3',4':4,5]cyclohepta[1,2-b]indole, pyrido[3',4':5,6]cyclohepta[1,2-b]indole, 2H-pyrano[3',2':2,3]azepino[4,5-b]indole, 1H-indolo[3,2-b][1,5]benzoxazepine, 1H-indolo[3,2-d][1,2]benzoxazepine, 1H-indolo[2,3-c][1,5]benzothiazepine, [1,4]diazepino[2.3-a]carbazole, indolo[2,3-b][1,5]benzodiazepine, indolo[2,3-d][1,3]benzodiazepine, indolo[3,2-b][1,4]benzodiazepine, indolo[3,2-b][1,5]benzodiazepine, indolo[3,2-d][1,3]benzodiazepine, indolo [3, 2-d] [2, 3] benzodiazepine, indolo[2,3-a][3]benzazepine, indolo[2,3-c][1]benzazepine, indolo[2,3-d][1]benzazepine, indolo[2,3-d][2]benzazepine, indolo[3,2-b][1]benzazepine, indolo[3,2-c][1]benzazepine, indolo[3,2-d][1]benzazepine, 1H-indolo[2,1-b][3]benzazepine, 1H-[1]benzoxepino[5,4-b]indole, 1H-[2]benzoxepino[4,3-b]indole, 1H-[1]benzothiepino[4,5-b]indole, 1H-[1]benzothiepino[5,4-b]indole, benzo[3,4]cyclohepta[1,2-b]indole, benzo[4,5]cyclohepta[1,2-b]indole, benzo[5,6]cyclohepta[1,2-b]indole, benzo[6,7]cyclohepta[1,2-b]indole, cyclohepta[b]carbazole, 4H-[1,5]oxazocino[5',4':1,6]pyrido[3,4-b]indole, azocino[1',2':1,2]pyrido[3,4-b]indole, 2,6-methano-2H-azecino[4,3-b]indole, 3,7-methano-3H-azecino[5,4-b]indole, pyrido[1',2':1,8]azocino[5,4-b]indole, pyrido[4',3':6,7]oxocino[2,3-b]indole, pyrido[4',3':6,7]oxocino[4,3-b]indole, 1,5-methano-1H-azecino[3,4-b]indole, 2,6-methano-1H-azecino[5,4-b]indole, 1H-pyrido[3',4':5,6]cycloocta[1,2-b]indole, 1,4-ethanooxocino[3,4-b]indole, pyrano[3',4':5,6]cycloocta[1,2-b]indole. 1H-indolo[2,3-c][1,2,5,6]benzotetrazocine, 1H-indolo[2,3-c][1,6]benzodiazocine, 6,13b-methano-13bH-azecino[5,4-b]indole, oxocino[3,2-a]carbazole, 1H-benzo[g]cycloocta[b]indole, 6,3-(iminomethano]-2H-1,4-thiazonino[9,8-b]indole, 1H,3H-[1,4]oxazonino[4',3':1,2]pyrido[3,4-b]indole, 2H-3,6-ethanoazonino[5,4-b]indole, 2H-3,7-methanoazacycloundecino[5,4-b]indole, 1H-6,12b-ethanoazonino[5,4-b]indole, indolo[3,2-e] [2]benzazonine, 5,9-methanoazacycloundecino[5,4-b]indole, 3,6-ethano-3H-azecino[5,4-b]indole, 3,7-methano-3H-azacycloundecino[5,4-b]indole, pyrano[4',3':8,9]azecino[5,4-b]indole, 1H-indolo[2,3-c][1,7]benzodiazecine, 1H-indolo[3,2-e][2]benzazecine, benzo[e]pyrrolo[3,2-b]indole, benzo[e]pyrrolo[3,2-g]indole, benzo[e]pyrrolo[3,2,1-hi]indole, benzo[e]pyrrolo[3,4-b]indole, benzo[g]pyrrolo[3,4-b]indole, 1H-benzo[f]pyrrolo[1,2-a]indole, 1H-benzo[g]pyrrolo[1,2-a]indole, 2H-benzo[e]pyrrolo[1,2-a]indole, 1H-benzo[f]pyrrolo[2,1-a]isoindole, 1H-benzo[g]pyrrolo[2,1-a]isoindole, 2H-benzo[e]pyrrolo[2,1-a]isoindole, isoindolo[6,7,1-cde]indole, spiro[cyclohexane-1,5'-[5H]pyrrolo[2,1-a]isoindole], isoindolo[7,1,2-hij]quinoline, 7,11-methanoazocino[1,2-a]indole, 7,11-methanoazocino[2,1-a]isoindole, dibenz[cd,f]indole, dibenz[cd,g]indole, dibenz[d,f]indole, 1H-dibenz[e,g]indole, 1H-dibenz[e,g]isoindole, naphtho[1,2,3-cd]indole, naphtho[1,8-ef]indole, naphtho[1,8-fg]indole, naphtho[3,2,1-cd]indole, 1H-naphtho[1,2-e]indole, 1H-naphtho[1,2-f]indole, 1H-naphtho[1,2-g]indole, 1H-naphtho[2,1-e]indole, 1H-naphtho[2,3-e]indole, 1H-naphtho[1,2-f]isoindole, 1H-naphtho[2,3-e]isoindole, spiro[1H-carbazole-1,1'-cyclohexane], spiro[2H-carbazole-2,1'-cyclohexane], spiro[3H-carbazole-3,1'-cyclohexane], cyclohepta[4,5]pyrrolo[3,2-f]quinoline, cyclohepta[4,5]pyrrolo[3,2-h]quinoline, azepino[4,5-b]benz[e]indole, 1H-azepino[1,2-a]benz[f]indole, 1H-azepino[2,1-a]benz[f]isoindole, benzo[e]cyclohepta[b]indole, and benzo[g]cyclohepta[b]indole.

Specific example of the group represented by the above-mentioned formula:

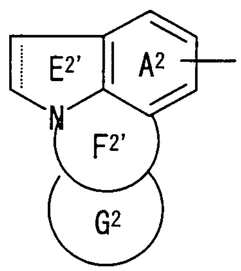

wherein each symbol is as defined above, include groups obtained by removing one hydrogen atom from tetracyclic condensed benzene rings such as 1H-dipyrrolo[2,3-b:3',2',l'-hi]indole, spiro[cyclopentane-1,2'(1'H)-pyrrolo[3,2,1-hi]indole], spiro[imidazolidine-4,1'(2'H)-[4H]pyrrolo[3,2,1-ij]quinoline], pyrido[2,3-b]pyrrolo[3,2,1-hi]indole, pyrido[4,3-b]pyrrolo[3,2,1-hi]indole, benzo[de]pyrrolo[3,2,1-ij]quinoline, 3H-pyrrolo[3,2,1-de]acridine, 1H-pyrrolo[3,2,1-de]phenanthrydine, spiro[cyclohexane-1,6'-[6H]pyrrolo[3,2,1-ij]quinoline], 4,9-methanopyrrolo[3,2,1-1m] [1] benzoazocine, spiro [cycloheptane-1, 6' - [6H]pyrrolo[3,2,1-ij]quinoline], 1H-pyrano[3,4-d]pyrrolo[3,2,1-jk][1]benzazepine, 3H-benzo[b]pyrrolo [3,2,1-jk][4,1]benzoxazepine, 7H-indolo[1,7-ab][4,1]benzoxazepine, benzo[b]pyrrolo[3,2,1-jk][1,4]benzodiazepine, indolo[1,7-ab][1,4]benzodiazepine, indolo[1,7-ab][1]benzazepine, indolo[7,1-ab][3]benzazepine, 1H-cyclohepta[d][3,2,1-jk] [1]benzazepine, spiro[azepino [3,2,1-hi]indole-7(4H), 1'-cycloheptane], 4H-5,11-methanopyrrolo[3,2,1-no][1]benzazacycloundecine, and spiro[azepino[3,2,1-hi]indole-7(4H),1'-cyclooctane].

Among them, further preferable is a group represented by the formula:

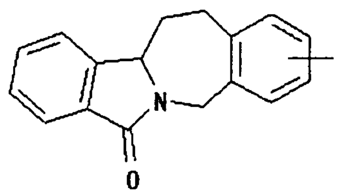

In addition, when the "aromatic ring group" in the "optionally condensed 5- or 6-membered aromatic ring group" represented by $Ar^2$ is, for example, "an aromatic heterocyclic group", examples of fusion of the "aromatic heterocyclic group" include:

(d) the case where the aromatic heterocyclic group is condensed with a monocyclic aromatic ring which may have a substituent, and

(e) the case where the aromatic heterocyclic group is condensed with a di- to tri-cyclic aromatic ring which may have a substituent, or condensed with two same or different monocyclic aromatic rings.

Specific examples include 1-, 2- or 3- indolyl; 1-, 2-or 3-isoindolyl; 2- or 3-benzofuranyl; 2- or 3-benzothiofuranyl; 1- or 3-benzimidazolyl; 2-benzoxazolyl; 2-benzothiazolyl; 1,2-benzisothiazol-3-yl; 1,2-benzisoxazol-3-yl, 2-, 3- or 4-quinolyl; 1-, 3- or 4-isoquinolyl; 2- or 3-quinoxalinyl; 1- or 4-phthalazinyl; naphthyridinyl such as 1,8-naphthyridin-2-yl, and 1,5-naphthyridin-3-yl; 2- or 4-quinazolinyl; 3- or 4-cinnolinyl; 9-acridinyl; 2-, 6- or 8-purinyl; 2-, 4-, 6- or 8-pteridinyl.

Preferable examples of $Ar^2$ include groups represented by the formula:

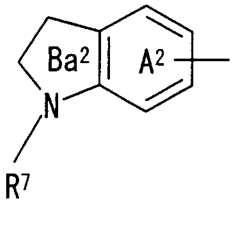
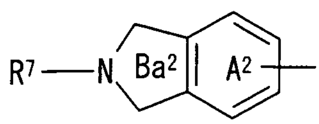
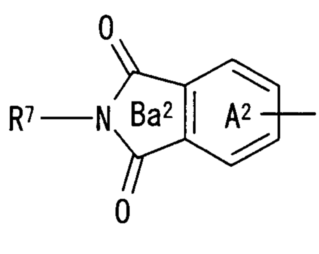

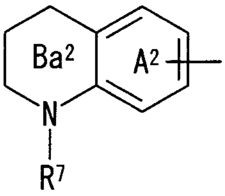
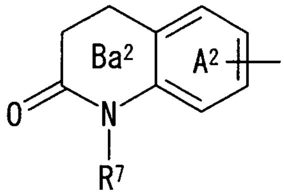
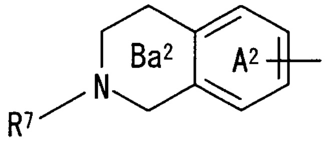

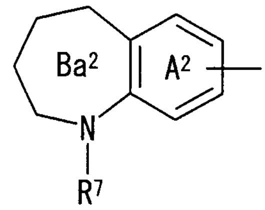
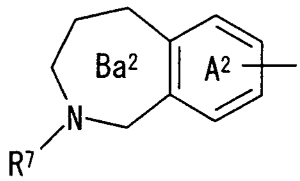
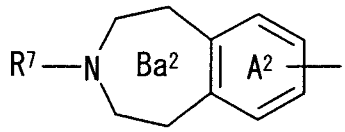

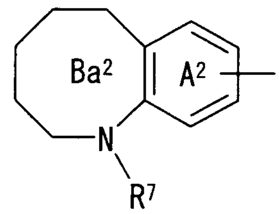
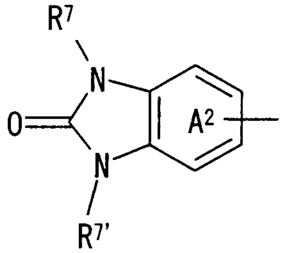
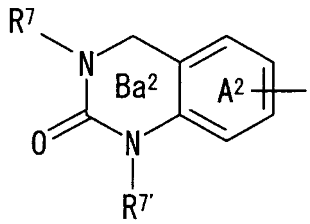

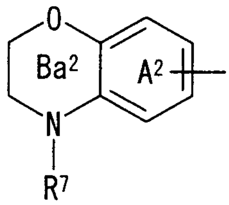
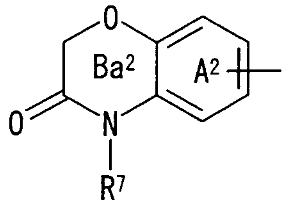
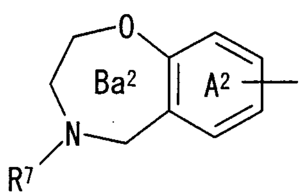

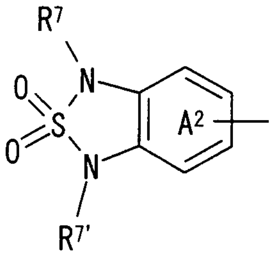
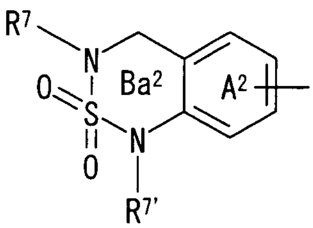
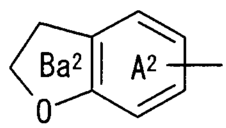
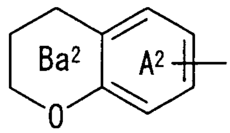

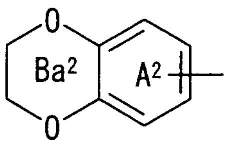 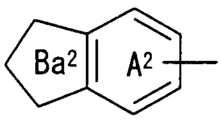 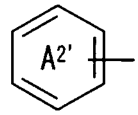 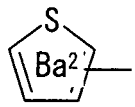 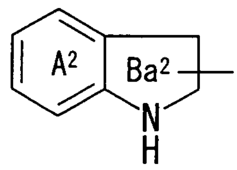

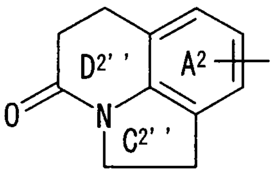 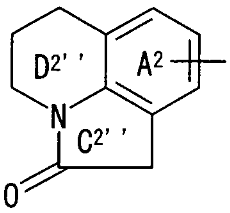 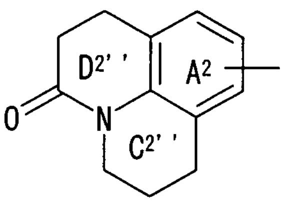 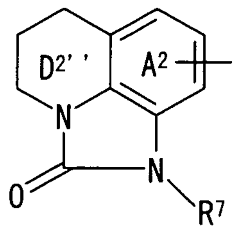

or

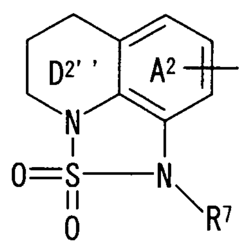

wherein $A^{2'}$ ring is as defined for $A^2$ ring, and the other symbols are as defined above.

Herein, as $A^2$ ring and $A^{2'}$ ring, a benzene ring which may have 1 to 4 substituent(s) selected from (i) halogen (fluoro etc.), (ii) $C_{1-6}$ alkoxy (methoxy etc.), (iii) halogeno$C_{1-6}$ alkoxy (trifluoromethoxy)etc.), (iv) amino, (v) (mono or di) $C_{1-6}$ alkylamino (methylamino, ethylamino, dimethylamino, diethylamino etc.), (vi) 1-pyrrolidinyl, (vii) piperidino, (viii) 1-piperazinyl, (ix) N-methyl-1-piperazinyl, (x) N-acetyl-1-piperazinyl (xi) morpholino, (xii) hexamethyleneimino, (xiii) imidazolyl, (xiv) $C_{1-6}$ alkyl (propyl etc.) which may be substituted with carboxy optionally esterified with $C_{1-6}$ alkyl (methyl etc.), (xv) lower alkyl-carbonylamino (acetylamino etc.), (xvi) lower alkylsulfonylamino (methylsulfonylamino etc.), (xvii) aminosulfonyl, (xviii) (mono or di) $C_{1-6}$ alkylaminosulfonyl, (xix) 5- to 7-membered cyclic amino-sulfonyl ((1-pyrrolidinyl) sulfonyl, piperidinosulfonyl, (1-piperazinyl)sulfonyl, morpholinosulfonyl etc.), (xx) carbamoyl, (xxi) (mono or di) $C_{1-6}$ alkyl-carbamoyl, (xxi) 5-to 7-membered cyclic amino-carbonyl ((1-pyrrolidinyl)carbonyl, piperidinocarbonyl, (1-piperazinyl) carbonyl, morpholinocarbonyl etc.), and (xxii) cyano is preferred. More preferably, A ring is a benzene ring which may have 1 or 2 substituent(s) selected from aminosulfonyl, mono- or di-$C_{1-6}$ alkylaminosulfonyl, carbamoyl and mono- or di-$C_{1-6}$ alkyl-carbamoyl, and A' ring is a benzene ring which has 1 or 2 substituent(s) selected from aminosulfonyl, mono- or di-$C_{1-6}$ alkylaminosulfonyl, $C_{1-6}$ alkyl-carbonylamino and $C_{1-6}$ alkylsulfonylamino, and further may have 1 to 4 substituent(s) (e.g. $C_{1-6}$ alkoxy etc.).

In addition, it is preferable that $Ba^2$ ring, $C^{2''}$ ring and $D^{2''}$ ring may have 1 or 2 substituent(s) selected from $C_{1-6}$ alkyl, $C_{1-6}$ alkyl-carbonylamino and $C_{1-6}$ alkylsulfonylamino, and $R^1$ and $R^{1'}$ are (1) a hydrogen atom, (2) a $C_{1-6}$ alkyl group or a $C_{7-16}$ aralkyl group, each of which may have 1 or 2 substituent(s) selected from hydroxy and $C_{1-6}$ alkoxy-carbonyl, or (3) a group represented by the formulas: -(C=O)-$R^{8'}$, -(C=O)-$NR^{8'}$-$R^{9'}$ or -$SO_2R^{8'}$ [wherein $R^{8'}$ and $R^{9'}$ represent hydrogen atom, optionally halogenated $C_{1-6}$ alkyl or $C_{6-10}$aryl, respectively], respectively.

The "spacer having a main chain of 1 to 10 of atoms" in the "spacer having a main chain of 1 to 10 of atoms which may have a substituent" represented by L means an interval in which 1 to 10 atom(s) of a main chain are linked. Herein, the "atom number of main chain" is counted so that the number of the atoms of a main chain becomes the smallest. For example, an atom number of 1,2-cyclopentylene is counted as 2, and an atom number of 1,3-cyclopentylene is counted as 3.

Examples of the "spacer having a main chain of 1 to 10 of atoms which may have a substituent" include a combination of 1 to 5, preferably 1 to 3 divalent group(s) selected from -O-, -S-, -CO-, -SO-, -$SO_2$-, -$NR^{10}$- ($R^{10}$ represents hydrogen atom, optionally halogenated $C_{1-10}$ alkyl, optionally halogenated $C_{1-6}$ alkyl-carbonyl, optionally halogenated

$C_{1-6}$ alkylsulfonyl), a divalent $C_{1-10}$ non-cyclic hydrocarbon group which may have a substituent, a divalent $C_{3-9}$ cyclic hydrocarbon group which may have a substituent and a divalent heterocyclic group which may have a substituent.

Examples of the "substituent" in the "spacer having a main chain of 1 to 10 of atoms which may have a substituent", that is, the "substituent" in the "divalent $C_{1-10}$ non-cyclic hydrocarbon group which may have a substituent", the "divalent $C_{3-9}$ cyclic hydrocarbon group which may have a substituent" and the "divalent heterocyclic group which may have a substituent" include 1 to 5, preferably 1 to 3 substituent(s) selected from halogen atom (e.g. fluorine, chlorine, bromine, iodine etc.), oxo, $C_{1-3}$ al kylenedioxy (e.g. methylenedioxy, ethylenedioxy etc.), nitro, cyano, optionally halogenated $C_{1-6}$ alkoxy, optionally halogenated $C_{1-6}$ alkylthio, hydroxy, amino, mono- or di-$C_{1-6}$ alkylamino, formyl, carboxy, carbamoyl, thiocarbamoyl, optionally halogenated $C_{1-6}$ alkyl-carbonyl, $C_{1-6}$ alkoxy-carbonyl, mono- or di-$C_{1-6}$ alkyl-carbamoyl, optionally halogenated $C_{1-6}$ alkylsulfonyl, formylamino, optionally halogenated $C_{1-6}$alkyl-carboxamide, $C_{1-6}$ alkoxy-carboxamide, $C_{1-6}$ alkylsulfonylamino, $C_{1-6}$ alkyl-carbonyloxy, $C_{1-6}$ alkoxy-carbonyloxy, mono-, di-$C_{1-6}$ alkyl-carbamoyloxy and phenyl. Among them, halogen atom such as fluorine, oxo, hydroxy, and phenyl are preferred.

Examples of the "divalent $C_{1-10}$ non-cyclic hydrocarbon group" in the "divalent $C_{1-10}$ non-cyclic hydrocarbon group which may have a substituent" include $C_{1-10}$ alkylene, $C_{2-10}$ alkenylene, and $C_{2-10}$ alkynylene.
Examples of the "$C_{1-10}$ alkylene" include $-CH_2-$, $-(CH_2)_2-$, $-(CH_2)_3-$, $-(CH_2)_4-$, $-(CH_2)_5-$, $-(CH_2)_6-$, $-(CH_2)_7-$, $-(CH_2)_8-$, $-(CH_2)_9-$, $-(CH_2)_{10}-$, $-(CH_2)_3CH(CH_3)-(CH_2)_4CH(CH_3)-$, $-(CH_3)CHCH_2-$, $-(CH_3)CH(CH_2)_2-$, and $-(CH(CH_3))_2-$.

Examples of the "$C_{2-10}$ alkenylene" include $-CH=CH-$, $-CH_2-CH=CH-$, $-C(CH_3)_2-CH=CH-$, $-CH_2-CH=CH-CH_2-$, $-CH_2-CH_2-CH=CH-$, $-CH=CH-CH=CH-$, and $-CH=CH-CH_2-CH_2-CH_2-$.

Examples of the "$C_{2-10}$ alkynylene" include $-C\equiv C-$, $-CH_2C\equiv C-$, $-CH_2-C\equiv C-CH_2-CH2-$.

Examples of the "divalent $C_{3-9}$ cyclic hydrocarbon group" in the "divalent $C_{3-9}$ cyclic hydrocarbon group which may have a substituent" include $C_{3-9}$ cycloalkylene, $C_{3-9}$ cycloalkenylene, and $C_{6-14}$ arylene (phenylene etc.).

Examples of the "$C_{3-9}$ cycloalkylene" include:

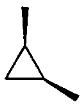 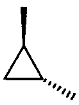 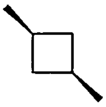 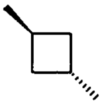 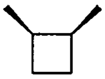 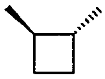

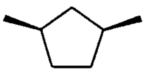 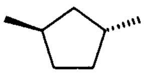 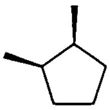 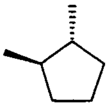 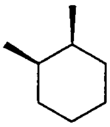 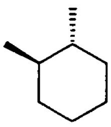

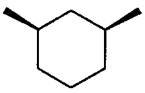 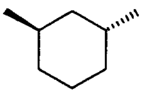 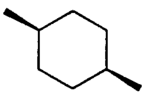 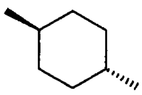 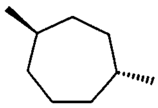

and the like.

Examples of the "$C_{3-9}$ cycloalkenylene" include:

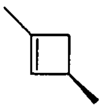 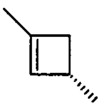 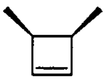 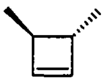 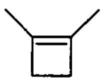 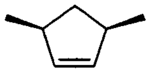

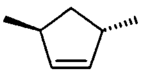 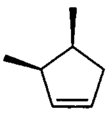 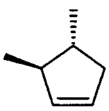 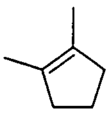 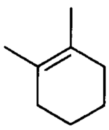 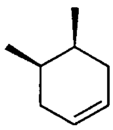

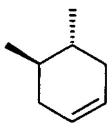
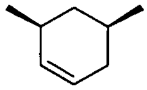
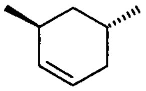
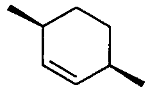
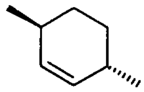
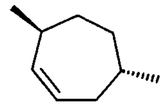

and the like.

Examples of the "divalent heterocyclic group" in the "divalent heterocyclic group which may have a substituent" include groups obtained by removing two hydrogen atoms from 4- to 14-membered (preferably 5- to 9-membered) aromatic or non-aromatic heterocyclic rings containing 1 to 4 hetero atom(s) selected from a nitrogen atom, an oxygen atom and a sulfur atom. Examples of such the aromatic or non-aromatic heterocyclic ring include the same rings as those exemplified for the "heterocyclic ring which may have a substituent" represented by the above-mentioned B ring.

As L, $C_{1-10}$ alkylene which may have a substituent is preferred, inter alia, a $C_{2-6}$ alkylene group which may have 1 to 4 substituent(s) selected from halogen atom, hydroxy, oxo and phenyl (e.g. $-(CH_2)_2-$, $-(CH_2)_3-$, $-(CH_2)_4-$, $-(CH_2)_5-$, $-(CH_2)_6-$, $-CHFCH_2-$, $-CHF(CH_2)_2-$, $-CHF(CH_2)_3-$, $-CHF(CH_2)_4-$, - $CF_2CH_2-$, $-CF_2(CH_2)_2-$, $-CF_2(CH_2)_3-$, $-CF_2(CH_2)_4$, $-(CH_2)_3CH(CH3)-$, $-(CH_2)_4CH(CH_3)-$, $-(CH_2)_3CH(CF_3)-$, $-(CH_2)_4CH(CF_3)-$, - $(CH_3)CHCH_2-$, $-(CH_3)CH(CH_2)_2-$, $-(CH(CH_3))_2-$, $-CH_2CH(OH)-$, - $CH_2CO-$, $-(Ph)CHCH_2-$ etc.) is preferable and, further, $C_{3-7}$ cycloalkylene or a combination of $C_{3-7}$ cycloalkylene and $C_{1-4}$ alkylene (e.g.

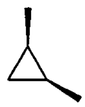
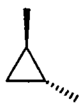
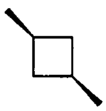
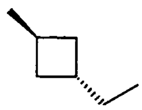
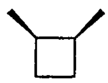
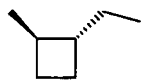

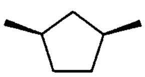
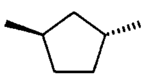
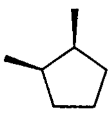
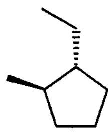
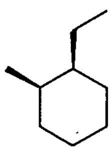
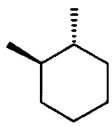

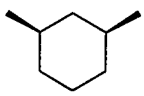
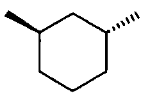
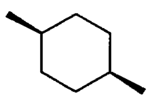
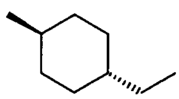
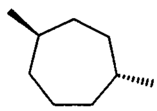

) is also preferable.

Examples of the ring formed between L and $Ar^2$ include 1-oxoindan-2-yl, 1-oxo-1,2,3,4-tetrahydronaphthalen-2-yl, 5-oxo-6,7,8,9-tetrahydro-5H-benzo[a]cyclohepten-6-yl, 2,5-dioxo-1,2,3,5,6,7-hexahydroindeno[5,6-d]imidazol-6-yl, 2,5-dioxo-2,3,5,6,7,8-hexahydro-1H-naphtho[2,3-d]imidazol-6-yl, 2,5-dioxo-1,2,3,5,6,7,8,9-octahydrocyclohepta[f]benzimidazol-6-yl, 2,8-dioxo-2,3,4,6,7,8-hexahydro-1H-cyclopenta[g]quinazolin-7-yl, 2,9-dioxo-1,2,3,4,6,7,8,9-octahydrobenzo[g]quinazolin-8-yl, 2 , 10-dioxo-2, 3, 4, 6,7, 8, 9, 10-octahydro-1H-cyclohepta[g]qunazolin-9-yl, 2,2-dioxido-5-oxo-3,5,6,7-tetrahydro-1H-indeno [5,6-c] [1,2,5] thiadiazol-6-yl, 2,2-dioxido-5-oxo-1,3,5,6,7,8-hexahydronaphto[2,3-c][1,2,5]thiadiazol-6-yl, 2,2-dioxido-5-oxo-3,5,6,7,8.9-hexahydro-1H-cyclohepta[f][2,1,3]benzothiadiazol-6-yl, 2,2-dioxido-8-oxo-1,3,4,6,7,8-hexahydroindeno[5,6-c][1,2,6]thiadiazin-7-yl, 2,2-dioxide-9-oxo-3,4,6,7,8,9-hexahydro-1H-naphtho[2,3-c][1,2,6]thiadiazin-8-yl, and 2,2-dioxide-10-oxo-1,3,4,6,7,8,9,10-octahydrocyclohepta[g][2,1,3]benzothiadiazin-9-yl.

Preferable example includes a ring represented by the formula:

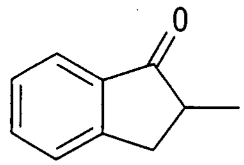
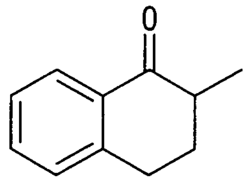
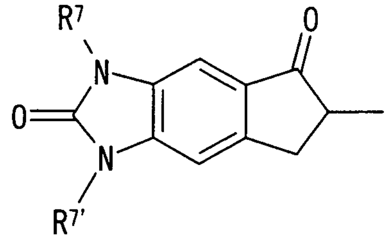

or

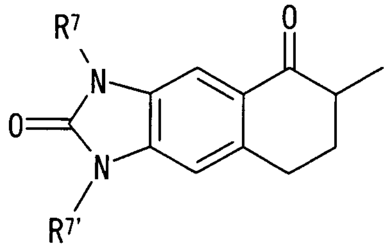

wherein the symbols are as defined above.

Examples of the "amino group which may have a substituent" represented by $Y^2$ include a group represented by the formula:

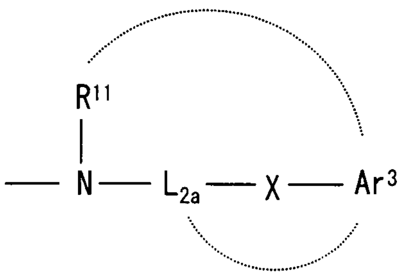

wherein $R^{11}$ represents a hydrogen atom or a hydrocarbon group which may have a substituent, $L_{2a}$ represents a $C_{1-4}$ alkylene group which may have a substituent, X represents a bond, an oxygen atom or a nitrogen atom, and $Ar^3$ represents an aromatic ring group which may have a substituent, or $Ar^3$ and $R^{11}$, or $Ar^3$ and $L_{2a}$ may be linked together to form a ring.

Examples of the "hydrocarbon group which may have a substituent" represented by $R^{11}$ include the same groups as "hydrocarbon groups which may have a substituent" represented by $R^7$

Examples of the "substituent" in the "hydrocarbon group which may have a substituent" represented by $R^{11}$ include the same groups as "substituents" in the "hydrocarbon group which may have a substituent" represented by $R^7$, and the number of substituents(s) is 1 to 3.

Examples of the "$C_{1-4}$ alkylene group which may have a substituent" represented by $L_{2a}$ include groups having the number of carbon chains of 1 to 4 among "$C_{1-10}$ alkylene groups" exemplified for the "spacer having a main chain of 1 to 10 atoms which may have a substituent" represented by L.

Examples of the "substituent" in the "$C_{1-4}$ alkylene group which may have a substituent" represented by $L_{2a}$ include the same groups as "substituents" in the "spacer having a main chain of 1 to 10 atoms which may have a substituent" represented by L.

Examples of the "hydrocarbon group which may have a substituent", the "acyl group" and the "heterocyclic group which may have a substituent" represented by $R^{1a}$ in the "a bond, an oxygen atom or $NR^{1a}$ ($R^{1a}$ represents a hydrogen atom, a hydrocarbon group which may have a substituent, an acyl group or a heterocyclic group which may have a substituent)" represented by X include the same groups as those in $R^7$.

Examples of the "aromatic ring group" in the "aromatic ring group which may have a substituent" represented by $Ar^3$ include the same groups as "optionally condensed 5- or 6-membered aromatic ring groups" represented by $Ar^2$. Examples of the "substituent" in the "aromatic ring group which may have a substituent" represented by $Ar^3$ include the

same groups as "substituents" in the "represents an optionally condensed 5- or 6-membered aromatic ring group, and said aromatic ring group may have a substituent" represented by $Ar^2$, and the number of substituent(s) is 1 to 5.

Examples of the ring formed by binding $Ar^3$ and $R^{11}$ together include rings represented by the formula:

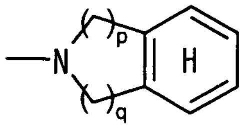

wherein p and q represent an integer of 1 to 3, respectively, and H ring represents a benzene ring which may have 1 to 3 substituent(s) selected from halogen, hydroxy, optionally halogenated $C_{1-6}$alkyl and optionally halogenated $C_{1-6}$ alkoxy.

Examples of the ring formed by binding $Ar^3$ and $L_{2a}$ include rings represented by the formula:

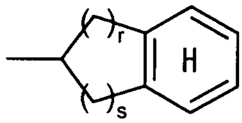

wherein r represents an integer of 0 to 2, s represents an integer of 1 to 3, and r+s is an integer of 2 to 5, and H ring is as defined above.

Examples of the "nitrogen-containing saturated heterocyclic group" in the "nitrogen-containing saturated heterocyclic group which may have a substituent" represented by $Y^2$ include 5- to 9- membered (preferably 5-to 7-membered) nitrogen-containing saturated heterocyclic groups which may contain 1 to 3 hetero atom(s) selected from a nitrogen atom, an oxygen atom and a sulfur atom in addition to carbon atoms and one nitrogen atom. Specific examples include groups represented by the formula:

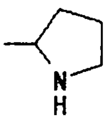 , 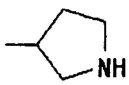 , 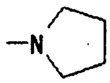 , 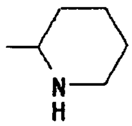 , 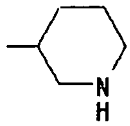 ,

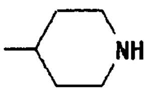 , 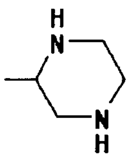 , 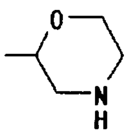 , 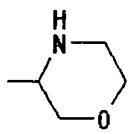 , 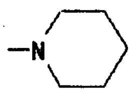 ,

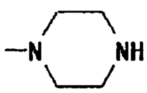 , 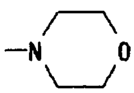 , 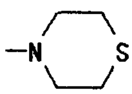 , 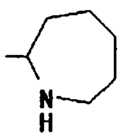 , 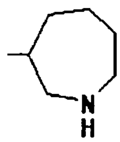 ,

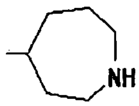 , 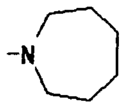 or 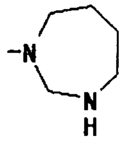

Among them, preferred is a 6-membered cyclic group.

Further preferable is a group represented by the formula:

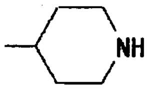

Examples of the "substituent" in the "nitrogen-saturated heterocyclic group which may have a substituent" include the same groups as "substituents" in the "heterocyclic ring which may have a substituent" represented by above-mentioned $B^2$ ring, and the number of substituent(s) is 1 to 5. In addition, nitrogen in the "nitrogen-containing saturated heterocyclic group" of the "nitrogen-containing saturated heterocyclic group which may have a substituent" may have the same group as a group represented by the formula:

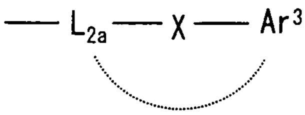

wherein each symbol is as defined above.

Preferable examples of $Y^2$ include groups represented by the formula:

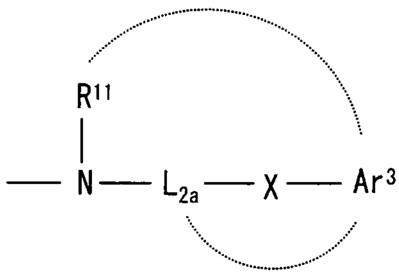

or

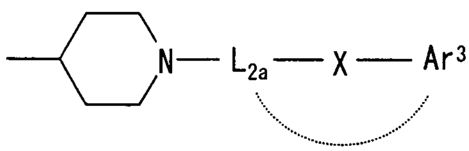

wherein each symbol is as defined above.

As $R^{11}$, (i) a hydrogen atom, (ii) a $C_{1-4}$alkyl group which may have 1 to 3 substituent(s) selected from halogen atom (preferably fluoro etc.) and hydroxy, or (iii) a $C_{7-16}$ aralkyl (benzyl etc.) is preferable, and a hydrogen atom or a $C_{1-4}$ alkyl group is more preferable.

As $L_{2a}$, a $C_{2-4}$alkylene group which may have 1 to 4 substituent(s) selected from halogen atom, hydroxy, oxo and phenyl (e.g. $-(CH_2)_2-$, $-(CH_2)_3-$, $-(CH_2)_4-$, $-CHFCH_2-$, $-CHF(CH_2)_2-$, $-CHF(CH_2)_3$, $-CF_2CH_2-$, $-CF_2(CH_2)_2-$, $-CF_2(CH_2)_3-$, $-(CH_3)CHCH_2-$, $-(CH_3)CH(CH_2)_2-$, $-(CH(CH_3))_2-$, $-CH_2CH(OH)-$, $-CH_2CO-$, $-(Ph)CHCH_2-$ etc.) is preferable,

a $C_{2-3}$ alkylene group which may have hydroxy, oxo or phenyl (e.g. $-(CH_2)_2-$, $-(CH_2)_3-$, $-(CH_3)CHCH_2-$, $-(Ph)CHCH_2-$, $-CH_2CH(OH)-$, $-CH_2CO-$etc.) is more preferable, and an ethylene group is most preferable.

As X, a bond, an oxygen atom or NH is preferable, and a bond is more preferable.

Preferable examples of $Ar^3$ include:

(1) (i) a $C_{6-10}$ aryl group (phenyl etc.) or (ii) a 5- or 6-membered aromatic heterocyclic group containing 1 to 4 hetero atom(s) selected from a nitrogen atom, an oxygen atom and a sulfur atom (optionally condensed with a benzene ring) (e.g. thienyl, furyl, pyrrolyl, imidazolyl, pyrazolyl, thiazolyl, isothiazolyl, oxazolyl, isooxazolyl, pyridyl, pyrazinyl, pyrimidinyl, pyridazinyl, oxadiazolyl, thiadiazolyl, furazanyl, indolyl, isoindolyl, benzofuranyl, quinolyl, isoquinolyl, naphthyridinyl, quinazolinyl, cinnolinyl, acridinyl etc.), each of which may have 1 to 5 (preferably 1 to 3) substituent (s) selected from halogen (fluoro, chloro etc.), $C_{1-6}$ alkyl (methyl, ethyl etc.), halogeno$C_{1-6}$ alkyl (trifluoromethyl etc.), hydroxy, $C_{1-6}$ alkoxy (methoxy, ethoxy etc.), halogeno$C_{1-6}$ alkoxy (trifluoromethoxy, trifluoroethoxy etc.), nitro, amino, cyano, carbamoyl, carbamoyl which may be substituted with $C_{1-6}$ alkyl or amino which may be substituted with formyl (NHCHO, $NHCONH_2$, NHCONHMe etc.), $C_{1-3}$alkylenedioxy (methylenedioxy etc.), aminocarbonyloxy group which may be substituted with $C_{1-6}$ alkyl (aminocarbonyloxy, methylaminocarbonyloxy, ethylaminocarbonyloxy, dimethylaminocarbonyloxy, diethylaminocarbonyloxy etc.), 5-to 7-membered cyclic amino-carbonyloxy ((1-pyrrolidinyl)carbonyloxy, piperidinocarbonyloxy etc.), aminosulfonyl, mono-$C_{1-6}$ alkylaminosulfonyl and di-$C_{1-6}$ alkylaminosulfonyl,

(2) the case where $Ar_2$ and R are linked together to form a ring represented by the formula:

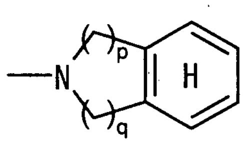

wherein each symbol is as defined above, or

(3) the case where $Ar^3$ and $L_{2a}$ are linked together to form a ring represented by the formula:

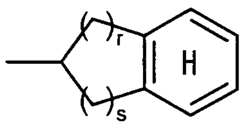

wherein each symbol is as defined above.

More preferable examples of $Ar^3$ include (i) a $C_{6-10}$ aryl group (phenyl etc.) or (ii) a 5- or 6-membered aromatic heterocyclic group (optionally condensed with a benzene ring) containing 1 to 4 hetero atom(s) selected from a nitrogen atom, an oxygen atom and a sulfur atom (thienyl, indolyl etc.), each of which may have 1 to 3 substituent(s) selected from halogen, nitro, hydroxy, optionally halogenated $C_{1-6}$ alkyl, optionally halogenated $C_{1-6}$ alkoxy and aminosulfonyl.

More preferable $Y^2$ is a group represented by the formula:

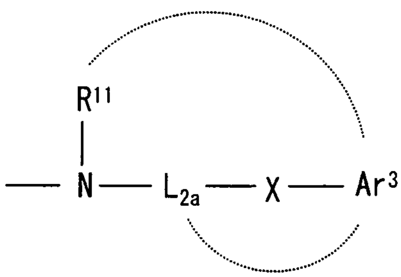

wherein each symbol is as defined above.

Preferably, the compound (II) is a compound wherein $Ar^2$ is a group represented by the formulas:

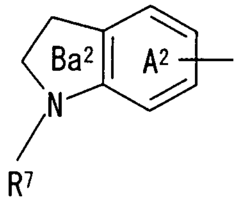

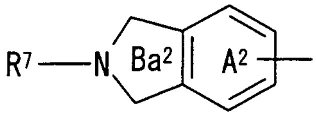

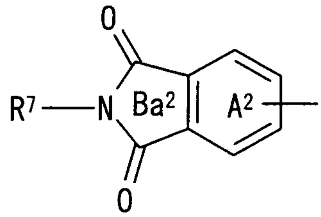

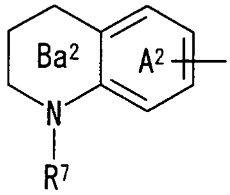

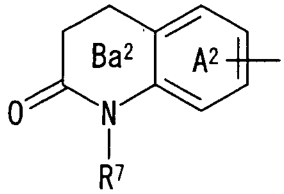

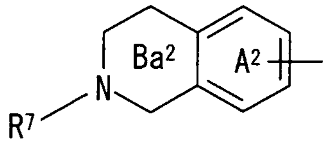

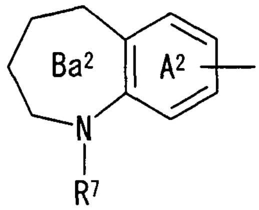

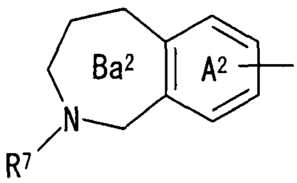

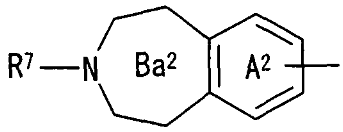

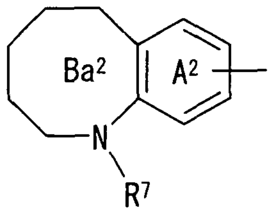

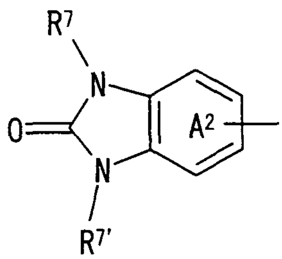

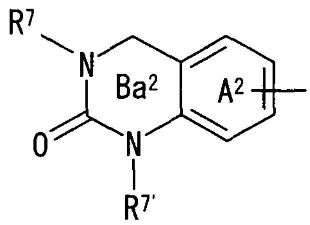

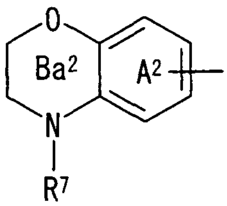

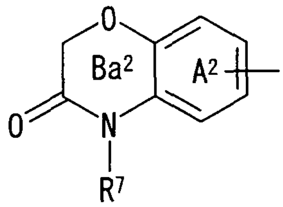

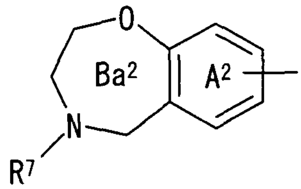

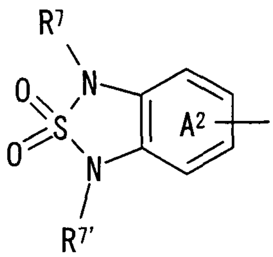
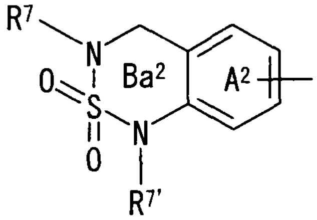
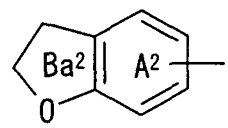
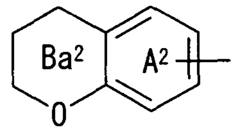

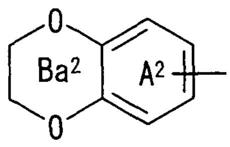
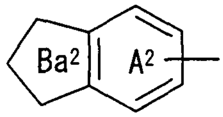
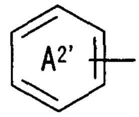
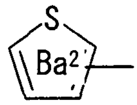
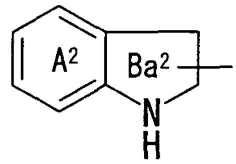

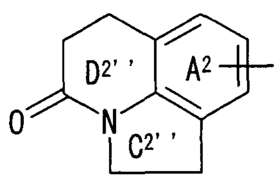
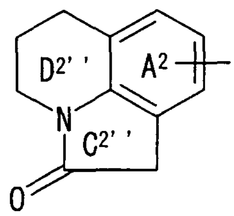
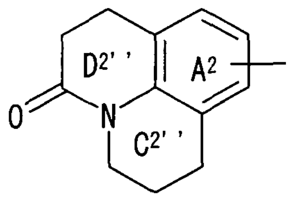
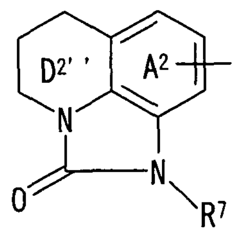

or

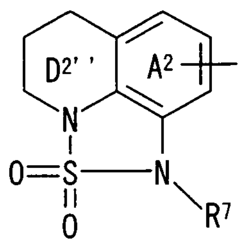

wherein the symbols are as defined above, ($A^2$ ring is preferably a benzene ring which may have 1 or 2 substituent(s) selected from aminosulfonyl, mono- or di-$C_{1-6}$ alkylaminosulfonyl, carbamoyl and mono- or di-$C_{1-6}$ alkyl-carbamoyl, $A^{2'}$ ring is preferably a benzene ring which has 1 or 2 substituent(s) selected from aminosulfonyl, mono-or di-$C_{1-6}$ alkylaminosulfonyl, $C_{1-6}$ alkyl-carbonylamino and $C_{1-6}$ alkylsulfonylamino, and further may have 1 to 4 substituent(s) (e.g. $C_{1-6}$alkoxy etc.), $Ba^2$ ring, $C^{2''}$ ring and $D^{2''}$ ring may preferably have 1 or 2 substituent(s) selected from $C_{1-6}$ alkyl, $C_{1-6}$ alkyl-carbonylamino and $C_{1-6}$ alkylsulfonylamino, respectively, and $R^7$ and $R^{7'}$ preferably are (1) a hydrogen atom, (2) a $C_{1-6}$ alkyl group or a $C_{7-16}$ aralkyl group (benzyl etc.), each of which may have 1 or 2 substituent(s) selected from hydroxy and $C_{1-6}$ alkoxy-carbonyl, or (3) a group represented by the formula: - (C=O)-$R^{8'}$, -(C=O)-$NR^{8'}R^{9'}$ or -$SO_2R^{8'}$ [wherein $R^{8'}$ and $R^{9'}$ represent a hydrogen atom, optionally halogenated $C_{1-6}$ alkyl or $C_{6-10}$ aryl, respectively], respectively);

L is (1) a $C_{2-6}$ alkylene group which may have 1 to 4 substituent(s) selected from halogen atom, hydroxy, oxo and phenyl (e.g. $-(CH_2)_2-$, $-(CH_2)_3-$, $-(CH_2)_4-$, $-(CH_2)_5-$, $-(CH_2)_6-$, $-CHFCH_2-$, $-CHF(CH_2)_2-$, $-CHF(CH_2)_3-$, $-CHF(CH_2)_4-$, $-CF_2CH_2-$, $- CF_2(CH_2)_2-$, $-CF_2(CH_2)_3-$, $-CF_2(CH_2)_4-$, $-(CH_2)_3CH(CH_3)-$, $- (CH_2)_4CH(CH_3)-$, $-(CH_2)_3CH(CF_3)-$, $-(CH_2)_4CH(CF_3)-$, $- (CH_3) CHCH_2-$, $- (CH_3) CH(CH_2)_2-$ , $- (CH (CH_3))_2 -$, $-CH_2CH (OH) -$ , $-CH_2CO-$, $- (Ph) CHCH_2-$ etc.), or (2) $C_{3-7}$ cycloalkylene or a combination of $C_{3-7}$ cycloalkylene and $C_{1-4}$ alkylene (e.g.

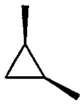 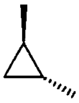 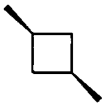 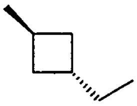 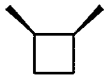 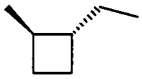

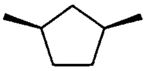 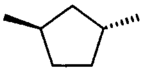 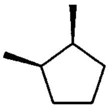 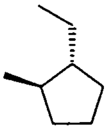 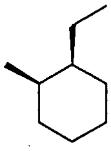 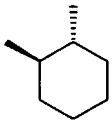

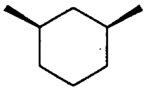 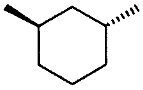 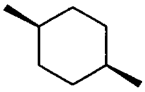 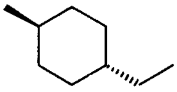 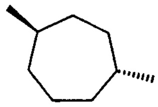

etc.);

$Y^2$ is a group represented by the formulas:

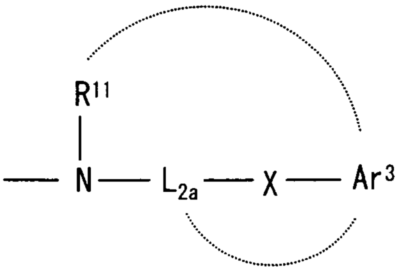

or

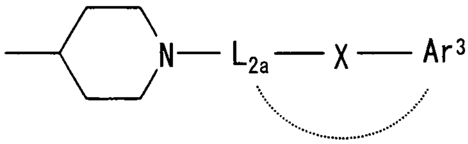

wherein each symbol is as defined above.

$R^{11}$ is (i) a hydrogen atom, (ii) a $C_{1-4}$ alkyl group which may have 1 to 3 substituent(s) selected from a halogen atom (e.g. fluoro etc.) and hydroxy, or (iii) $C_{7-16}$ aralkyl (benzyl etc.);

$L_{2a}$ is a $C_{2-4}$ alkylene group which may have 1 to 4 substituent(s) selected from halogen atom, hydroxy, oxo and phenyl (e.g. $-(CH_2)_2-$, $-(CH_2)_3-$, $-(CH_2)_4-$, $-CHFCH_2-$, $-CHF(CH_2)_2-$, $-CHF(CH_2)_3-$, $-CF_2CH_2-$, $-CF_2(CH_2)_2-$, $-CF_2(CH_2)_3-$, $-(CH_3)CHCH_2-$, $-(CH_3)CH(CH_2)_2-$, $-(CH(CH_3))_2-$, $-CH_2CH(OH)-$, $-CH_2CO-$, $-(Ph)CHCH_2-$ etc.);

X is a bond, an oxygen atom or NH;

$Ar^3$ is (1) (i) a $C_{6-10}$ aryl group (phenyl etc.) or (ii) a 5- or 6-membered aromatic heterocyclic group (optionally condensed with a benzene ring) containing 1 to 4 hetero atom(s) selected from a nitrogen atom, an oxygen atom and a sulfur atom (e.g. thienyl, furyl, pyrrolyl, imidazolyl, pyrazolyl, thiazolyl, isothiazolyl, oxazolyl, isooxazolyl, pyridyl, pyrazinyl, pyrimidinyl, pyridazinyl, oxadiazolyl, thiadiazolyl, furazanyl, indolyl, isoindolyl, benzofuranyl, quinolyl, isoquinolyl, naphthyridinyl, quinazolinyl, cinnolinyl, acridinyl etc.), each of which may have 1 to 5 (preferably 1 to 3) substituent(s) selected from halogen (fluoro, chloro etc.), $C_{1-6}$ alkyl (methyl, ethyl etc.), halogeno$C_{1-6}$ alkyl (trifluoromethyl etc.), hydroxy, $C_{1-6}$ alkoxy (methoxy, ethoxy etc,), halogeno$C_{1-6}$ alkoxy (trifluoromethoxy, trifluoroethoxy etc.), nitro, amino, cyano, carbamoyl, carbamoyl which may be substituted with $C_{1-6}$alkyl or amino which may be substituted with formyl (NHCHO, $NHCONH_2$, NHCONHMe etc.), $C_{1-3}$ alkylenedioxy (methylenedioxy etc.), an aminocarbonyloxy group which may be substituted with

$C_{1-6}$ alkyl (aminocarbonyloxy, methylaminocarbonyloxy, ethylaminocarbonyloxy, dimethylaminocarbonyloxy, diethylaminocarbonyloxy etc.), 5-to 7-membered cyclic amino-carbonyloxy ((1-pyrrolidinyl)carbonyloxy, piperidinocarbonyloxy etc.), aminosulfonyl, mono-$C_{1-6}$ alkylaminosulfonyl and di-$C_{1-6}$ alkylaminosulfonyl, (2) the case where $Ar^3$ and $R^{11}$ are linked together to form a ring represented by the formula:

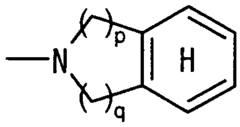

wherein each symbol is as defined above, or (3) the case where $Ar^3$ and $L_{2a}$ are linked together to form a ring represented by the formula:

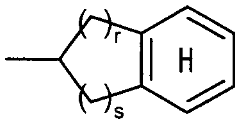

wherein each symbol is as defined above.

In addition, among the compound (II), a compound represented by the formula:

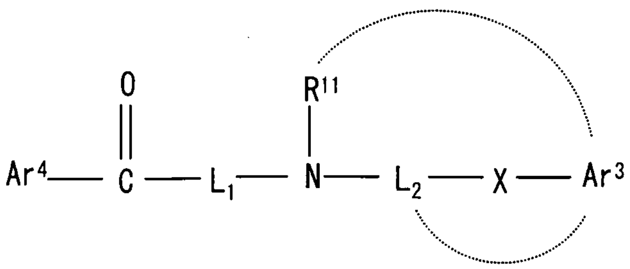 (IIa)

wherein $Ar^4$ represents a bicyclic to tetracyclic condensed benzene ring group which may have a substituent, $L_1$ represents a $C_{4-6}$ alkylene group which may have a substituent, and the other symbols are as defined above, (hereinafter, sometimes, abbreviated as the compound (IIa)) or a salt thereof is a novel compound.

The "bicyclic to tetracyclic condensed benzene ring group" in the "bicyclic to tetracyclic condensed benzene ring which may have a substituent" represented by $Ar^4$ include the same groups as those exemplified with respect to the condensed benzene ring group exemplified as an example of condensation of the "phenyl group" in the case that the "5- or 6-membered aromatic ring group" in the "optionally condensed 5- or 6-membered aromatic ring group" represented by $Ar^2$ is "phenyl group".

The "substituent" in the "bicyclic to tetracyclic condensed benzene ring group which may have a substituent" represented by $Ar^4$ include the same groups as those exemplified with respect to the "substituent" in the "represents optionally condensed 5- or 6-membered aromatic ring group, and said aromatic ring group may have a substituent" represented by $Ar^2$.

Preferable examples of $Ar^2$ include groups represented by the formulas:

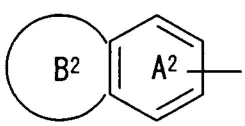 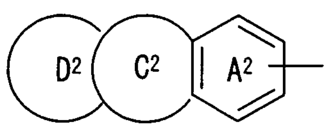 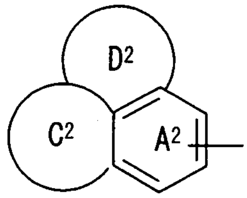

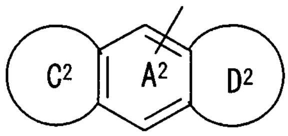
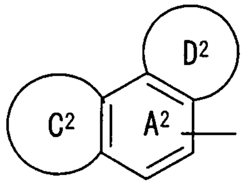
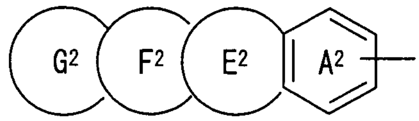

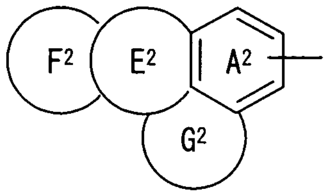

wherein the symbols are as defined above.

More preferable examples of $Ar^4$ include groups represented by the formula:

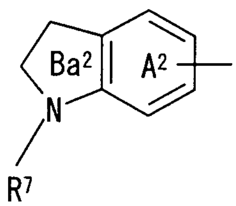
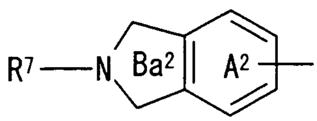
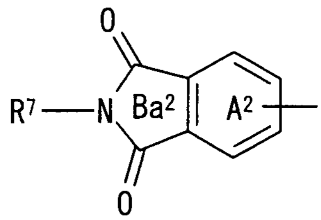

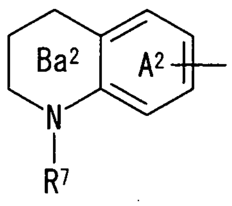
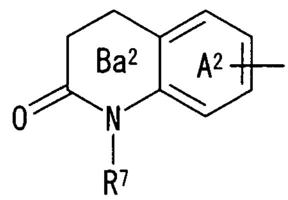
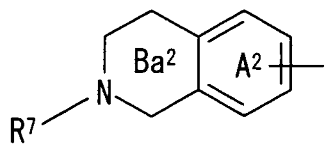

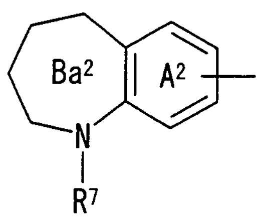
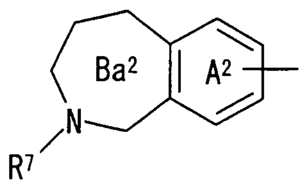
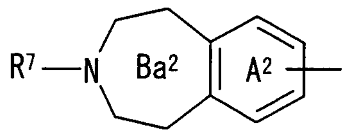

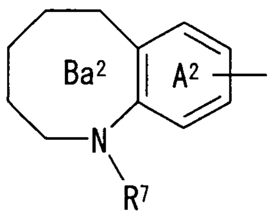
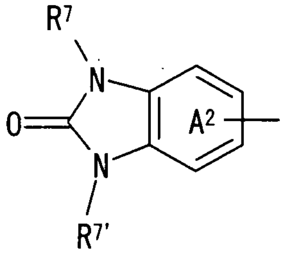
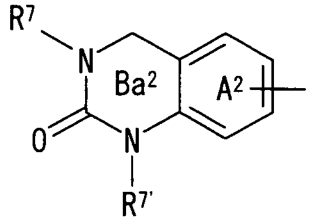

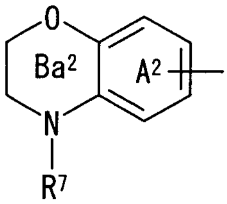
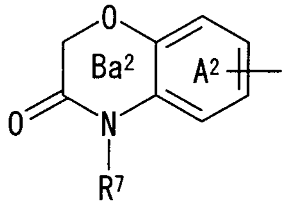
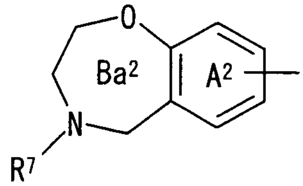

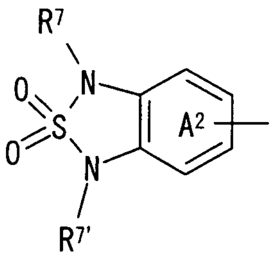
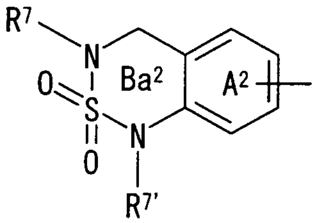
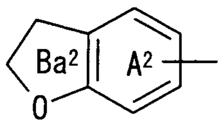
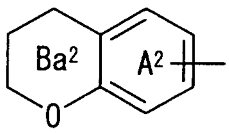

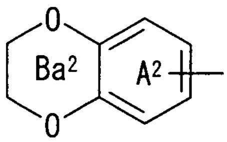
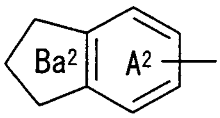
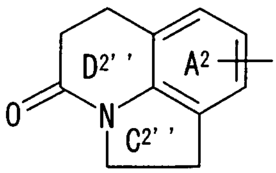
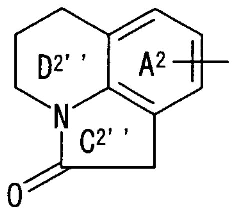

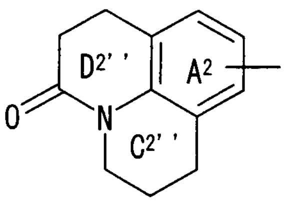
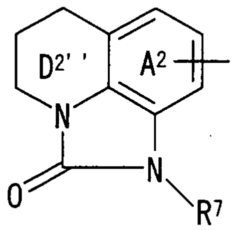
or
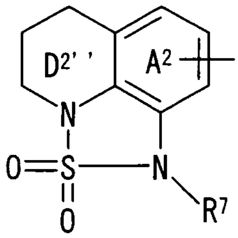

wherein the symbols are as defined above.

Herein, as $A^2$ ring, a benzene ring which may have 1 to 2 substituent(s) selected from (i) halogen (fluoro etc.), (ii) $C_{1-6}$ alkoxy (methoxy etc.), (iii) halogeno$C_{1-6}$ alkoxy (trifluoromethoxy etc.), (iv) amino, (v) (mono or di)$C_{1-6}$ alkylamino (methylamino, ethylamino, dimethylamino, diethylamino etc.), (vi) 1-pyrrolidinyl, (vii) piperidino, (viii) 1-piperazinyl, (ix) N-methyl-1-piperazinyl, (x) N-acetyl-1-piperadinyl, (xi) morpholino, (xii) hexamethyleneimino, (xiii) imidazolyl, (xiv) $C_{1-6}$

alkyl (propyl etc.) which may be substituted with carboxy optionally esterified with $C_{1-6}$ alkyl (methyl etc.), (xv) lower alkyl-carbonylamino (acetylamino etc.), (xvi) lower alkylsulfonylamino (methylsulfonylamino etc.), (xvii) aminosulfonyl, (xviii) (mono or di) $C_{1-6}$ alkylaminosulfonyl, (xix) 5- to 7-membered cyclic amino-sulfonyl ((1-pyrrolidinyl)sulfonyl, piperidinosulfonyl, (1-piperazinyl)sulfonyl, morpholinosulfonyl etc.), (xx) carbamoyl, (xxi) (mono or di) $C_{1-6}$ alkylcarbamoyl, (xxi) 5-to 7-membered cyclic amino-carbonyl ((1-pyrrolidinyl)carbonyl, piperidinocarbonyl, (1-piperazinyl)carbonyl, morpholinocarbonyl etc.) and (xxii) cyano is preferred. More preferably, A ring is a benzene ring which may have 1 or 2 substituent(s) selected from aminosulfonyl, mono-or di-$C_{1-6}$ alkylaminosulfonyl, carbamoyl and mono-or di-$C_{1-6}$ alkyl-carbamoyl.

In addition, it is preferable that $Ba^2$ ring, $C^{2"}$ ring and $D^{2"}$ ring may have 1 or 2 substituent(s) selected from $C_{1-6}$ alkyl, $C_{1-6}$ alkyl-carbonylamino and $C_{1-6}$ alkylsulfonylamino, respectively, and $R^7$ and $R^{7'}$ are (1) a hydrogen atom, (2) a $C_{1-6}$ alkyl group or a $C_{7-16}$ aralkyl group, each of which may have 1 or 2 substituent(s) selected from hydroxy and $C_{1-6}$ alkoxy-carbonyl, or (3) a group represented by the formula: $-(C{=}O)-R^{8'}$, $-(C{=}O)-NR^{8'}R^{9'}$ or $-SO_2R^{8'}$ [wherein $R^{8'}$ and $R^{9'}$ represent a hydrogen atom, optionally halogenated $C_{1-6}$ alkyl or $C_{6-10}$ aryl, respectively) .

Examples of the "$C_{4-6}$ alkylene group which may have a substituent" represented by $L_1$ include those having carbon chain number of 4 to 6 among the "$C_{1-10}$ alkylene group" exemplified with respect to the "spacer having a main chain of 1 to 10 atoms which may have a substituent" represented by L.

The "substituent" in the "$C_{4-6}$ alkylene group which may have a substituent" represented by $L_1$ include the same groups as those exemplified with respect to the "substituent" in the "spacer having a main chain of 1 to 10 atoms which may have a substituent" represented by L. Preferable examples include 1 to 4 substituent(s) selected from halogen atom (e.g. fluorine, chlorine, bromine, iodine etc.), nitro, cyano, optionally halogenated $C_{1-6}$alkoxy, and hydroxy.

Preferable examples of $L_1$ include $-(CH_2)_4-$, $-(CH_2)_5-$, $-(CH_2)_6-$, $-CHF(CH_2)_3-$, $-CHF(CH_2)_4-$, $-CF_2(CH_2)_3-$, $-CF_2(CH_2)_4-$, $-(CH_2)_3CH(CH_3)-$, $-(CH_2)_4CH(CH_3)-$, $-(CH_2)_3CH(CF_3)-$, and $-(CH_2)_4CH(CF_3)-$,

The compound (IIa) is preferably a compound wherein $Ar^4$ is a group represented by the formula:

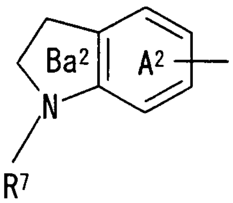

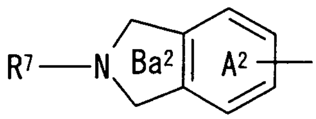

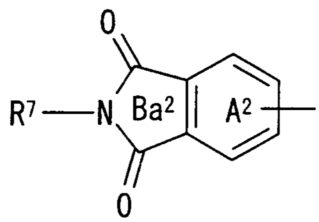

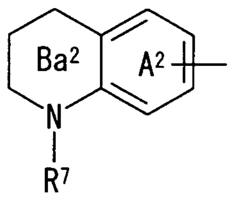

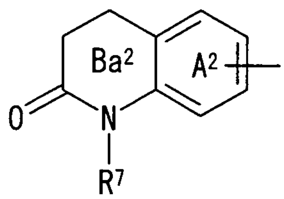

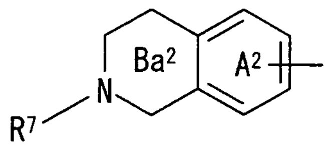

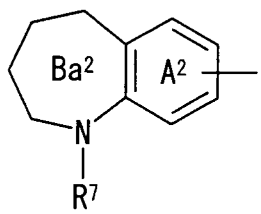

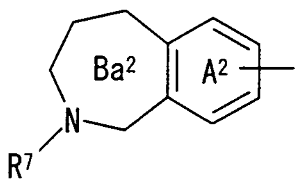

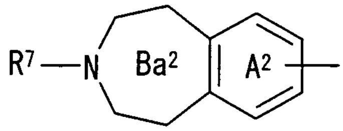

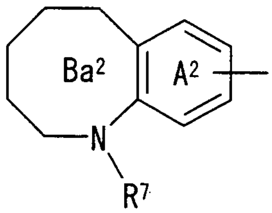
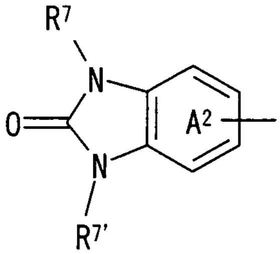
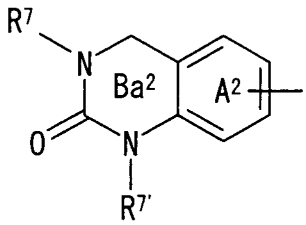

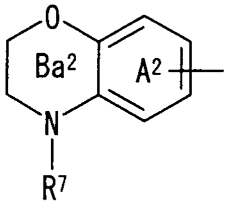
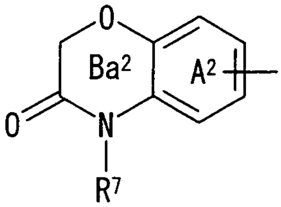
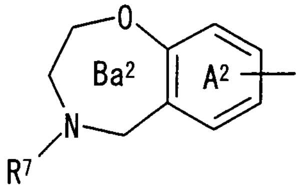

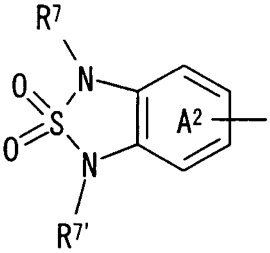
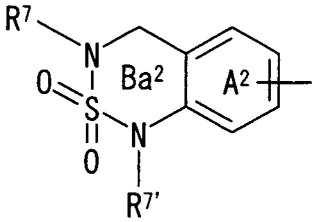
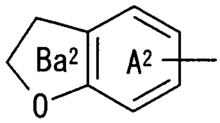
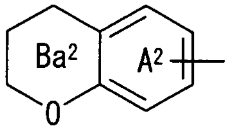

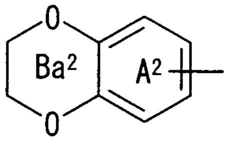
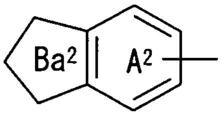
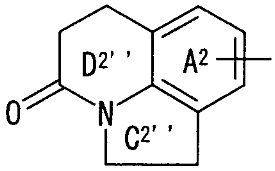
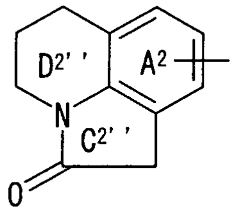

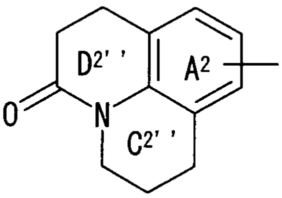
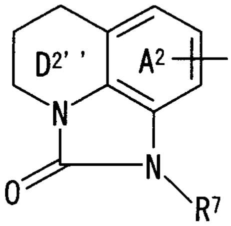
or
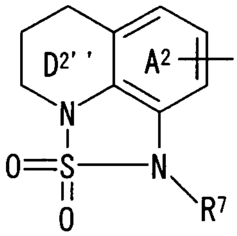

wherein the symbols are as defined above, (more preferably a group represented by:

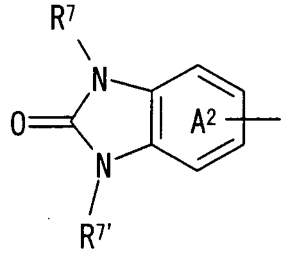 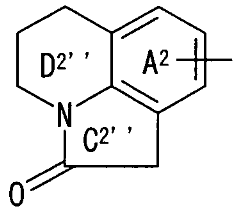 or 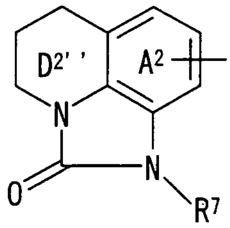

wherein the symbols are as defined above) ($A^2$ ring is preferably a benzene ring which may have 1 or 2 substituent(s) selected from aminosulfonyl, mono- or di-$C_{1-6}$ alkylaminosulfonyl, carbamoyl and mono- or di-$C_{1-6}$alkyl-carbamoyl, $Ba^2$ ring, $C^{2''}$ ring and $D^{2''}$ ring may preferably have 1 or 2 substituent (s) selected from $C_{1-6}$ alkyl, $C_{1-6}$ alkyl-carbonylamino and $C_{1-6}$ alkylsulfonylamino, respectively, and $R^1$ and $R^{1'}$ are preferably (1) a hydrogen atom, (2) a $C_{1-6}$ alkyl group or a $C_{7-16}$ aralkyl group (benzyl etc.), each of which may have 1 or 2 substituent(s) selected from hydroxy and $C_{1-6}$ alkoxy-carbonyl, or (3) a group represented by the formula: -(C=O)-R8', -(C=O)-$NR^{8'}R^{9'}$ or -$SO_2R^{8'}$ [wherein $R^{8'}$ and $R^{9'}$ represent hydrogen atom, optionally halogenated $C_{1-6}$ alkyl or $C_{6-10}$ aryl, respectively], respectively);

$L_1$ is a $C_{4-5}$ alkylene group which may have 1 or 2 halogen atom(s) such as $-(CH_2)_4-$, $-(CH_2)_5-$, $-CHF(CH_2)_3-$, - CHF $(CH_2)_4-$, $-CF_2(CH_2)_3-$, $-CF_2(CH_2)_4-$, $-(CH_2)_3CH(CH_3)-$, and - $(CH_2)_4CH(CH_3)-$ (preferably unsubstituted $C_{4-5}$alkylene group);

$R^{11}$ is (i) a hydrogen atom, (ii) a $C_{1-4}$alkyl group which may have 1 to 3 substituent(s) selected from halogen atom (preferably fluoro etc.) and hydroxy, or (iii) $C_{7-16}$ aralkyl (benzyl etc.) (more preferably, hydrogen atom or $C_{1-4}$ alkyl group);

$L_2$ is a $C_{2-4}$alkylene group which may have 1 to 4 substituent(s) selected from halogen atom, hydroxy, oxo and phenyl (e.g. $-(CH_2)_2-$, $-(CH_2)_3-$, $-(CH_2)_4-$, $-CHFCH_2-$, CHF $(CH_2)_2-$, -CHF $(CH_2)_3-$, $-CF_2CH_2-$, $-CF_2(CH_2)_2-$, $-CF_2$ $(CH_2)_3-$, - $(CH_3)$ $CHCH_2-$, $-(CH_3)CH(CH_2)_2-$, $-(CH(CH_3))_2-$, $-CH_2CH$ (OH) -, - $CH_2CO-$, $-(Ph)CHCH_2-$ etc.) (more preferably, $C_{2-3}$alkylene group which may have hydroxy, oxo or phenyl);

X is a bond, an oxygen atom or NH (more preferably, a bond);

$Ar^3$ is (1) (i) a $C_6$-$_{10}$ aryl group (phenyl etc.) or (ii) a 5- or 6-membered aromatic heterocyclic group (optionally condensed with a benzene ring) containing 1 to 4 hetero atom(s) selected from a nitrogen atom, an oxygen atom and a sulfur atom (e.g. thienyl, furyl, pyrrolyl, imidazolyl, pyrazolyl, thiazolyl, isothiazolyl, oxazolyl, isooxazolyl, pyridyl, pyrazinyl, pyrimidinyl, pyridazinyl, oxadiazolyl, thiadiazolyl, furazanyl, indolyl, isoindolyl, benzofuranyl, quinolyl, isoquinolyl, naphthrydinyl, quinazolinyl, cinnolinyl, acridinyl etc.), each of which may have 1 to 5 (preferably 1 to 3) substituents selected from halogen (fluoro, chloro etc.), $C_{1-6}$alkyl (methyl, ethyl etc.), halogeno$C_{1-6}$alkyl (trifluoromethyl etc.), hydroxy, $C_{1-6}$ alkoxy (methoxy, ethoxy etc.), halogeno$C_{1-6}$ alkoxy (trifluoromethoxy, trifuloroethoxy etc.), nitro, amino, cyano, carbamoyl, carbamoyl which may be substituted with $C_{1-6}$ alkyl or amino which may be substituted with formyl (NHCHO, $NHCONH_2$, NHCONHMe etc.), $C_{1-3}$ alkylenedioxy (methylenedioxy etc.), an aminocarbonyloxy group which may be substituted with $C_{1-6}$ alkyl (aminocarbonyloxy, methylaminocarbonyloxy, ethylaminocarbonyloxy, dimethylaminocarbonyloxy, dimethylaminocarbonyloxy etc.), 5- to 7-membered cyclic amino-carbonyloxy ((1-pyrrolidinyl)carbonyloxy, piperidinocarbonyloxy etc.), aminosulfonyl, mono-$C_{1-6}$ alkylaminosulfonyl and di-$C_{1-6}$ alkylaminosulfonyl, (2) the case where $Ar^3$ and $R^{11}$ are linked together to form a ring represented by the formula:

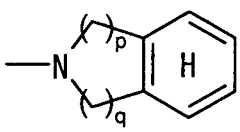

wherein each symbol is as defined above, or (3) the case where $Ar^3$ and $L_2$ are linked together to form a ring represented by the formula:

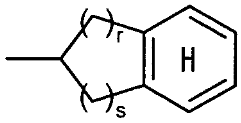

wherein each symbol is as defined above ($Ar^3$ is more preferably (i) a $C_{6-10}$aryl group (phenyl etc.) or (ii) a 5-or 6-membered aromatic heterocyclic group (optionally condensed with a benzene ring) containing 1 to 4 hetero atom(s) selected from a nitrogen atom, an oxygen atom and a sulfur atom (thienyl, indolyl etc.), each of which may have 1 to 3 substituent(s) selected from halogen, nitro, hydroxy, optionally halogenated $C_{1-6}$ alkyl, optionally halogenated $C_{1-6}$ alkoxy and aminosulfonyl).

In addition, among the compound (II), a compound represented by the formula:

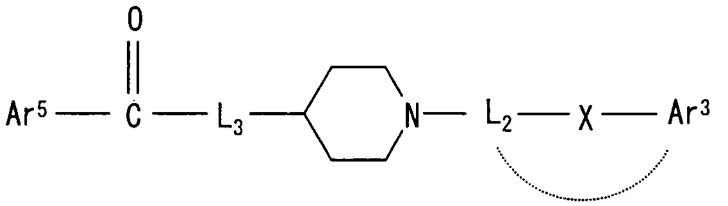

(IIb)

wherein $Ar^5$ represents a benzimidazole ring group, a quinazoline ring group, a 1,4-benzoxazine ring group or a tricyclic to tetracyclic condensed benzene ring group, each of which may have a substituent, $L_3$ represents a $C_{2-4}$ alkylene group which may have a substituent, and the other symbols are as defined above (hereinafter, sometimes, abbreviated as the compound (IIb)) or a salt thereof is a novel compound.

The "tricyclic to tetracyclic condensed benzene ring group" in the "tricyclic to tetracyclic condensed benzene ring group which may have a substituent" represented by $Ar^5$ include the same groups as those exemplified with respect to the condensed benzene ring group exemplified as an example of fusing of the "phenyl group" in the case that the "5- or 6-membered aromatic ring group" in the "optionally condensed 5- or 6-membered aromatic ring group" represented by Ar is the "phenyl group".

The "substituent" in the "a benzimidazole ring group, a quinazoline ring group, a 1,4-benzoxazine ring group or a tricyclic to tetracyclic condensed benzene ring group, each of which may have a substituent" represented by $Ar^5$ include the same groups as those exemplified with respect to the "substituent" in the "represents an optionally condensed 5-or 6-membered aromatic ring group, and said aromatic ring group may have a substituent" represented by $Ar^2$.

Examples of the "$C_{2-4}$ alkylene group which may have a substituent" represented by $L_3$ include those having carbon chain number of 2 to 4 among the "$C_{1-10}$ alkylene group" exemplified with respect to the "spacer having a main chain of 1 to 10 atoms which may have a substituent" represented by L.

The "substituent" in the "$C_{2-4}$ alkylene group which may have a substituent" represented by $L_3$ include the same groups as those exemplified with respect to the "substituent" in the "spacer having a main chain of 1 to 10 atoms which may have a substituent" represented by L. Preferable examples include 1 to 4 substituent(s) selected from halogen atom (e.g. fluorine, chlorine, bromine, iodine etc.), nitro, cyano, optionally halogenated $C_{1-6}$ alkoxy, and hydroxy.

Preferable examples of $L_3$ include a $C_{2-4}$ alkylene group which may have 1 or 2 halogen atom(s), such as $-(CH_2)_2-$, $-(CH_2)_3-$, $-(CH_2)_4-$, $-CHFCH_2-$, $-CHF(CH_2)_2-$, $-CF_2CH_2-$, and $-CF_2(CH_2)_2-$.

The compound (IIb) is preferably a compound wherein $Ar^5$ is a group represented by the formulas:

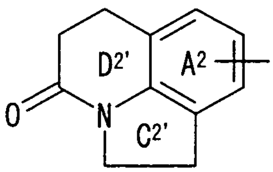

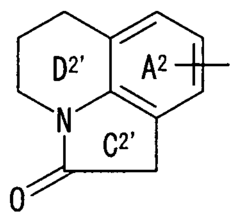

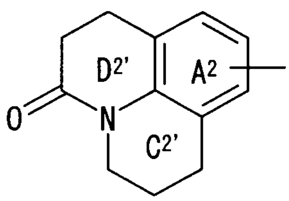

wherein the symbols are as defined above (among the groups, it is preferable that $A^2$ ring is unsubstituted, and $C^{2'}$ ring

and $D^{2'}$ ring have no substituent other than an oxo group in the formulas);
$L_3$ is a $C_{2-3}$alkylene group (more preferably, ethylene group) which may have 1 or 2 halogen atom(s), such as - $(CH_2)_2$-, -$(CH_2)_3$-, -$CHFCH_2$-, -$CHF(CH_2)_2$-, -$CF_2CH_2$- and - $CF_2(CH_2)_2$-;
$L_2$ is a $C_{2-4}$ alkylene group which may have 1 to 4 substituent(s) selected from halogen atom, hydroxy, oxo and phenyl, such as - $(CH_2)_2$-, - $(CH_2)_3$-, -$CF_2CH_2$-, -$CF_2(CH_2)_2$-, $(CH_3)CHCH_2$-, -$(CH_3)CH(CH_2)_2$-, -$CH_2CH(OH)$-, -$CH_2CO$-, and-$(Ph)CHCH_2$- (more preferably, $C_{2-3}$ alkylene group which may have hydroxy, oxo or phenyl);
X is a bond, an oxygen atom or NH (more preferably, bond or oxygen atom);
$Ar^3$ is (1) (i) a $C_{6-10}$ aryl group (phenyl etc.) or (ii) a 5- or 6-membered aromatic heterocyclic group (optionally condensed with a benzene ring) containing 1 to 4 hetero atom(s) selected from a nitrogen atom, an oxygen atom and a sulfur atom (e.g. thienyl, furyl, pyrrolyl, imidazolyl, pyrazolyl, thiazolyl, isothiazolyl, oxazolyl, isooxazolyl, pyridyl, pyrazinyl, pyrimidinyl, pyridazinyl, oxadiazolyl, thiadiazolyl, furazanyl, indolyl, isoindolyl, benzofuranyl, quinolyl, isoquinolyl, naphthyridinyl, quinazolinyl, cinnolinyl, acridinyl etc.), each of which may have 1 to 5 (preferably 1 to 3) substituent(s) selected from halogen (fluoro, chloro etc.), $C_{1-6}$ alkyl (methyl, ethyl etc.), halogeno$C_{1-6}$ alkyl (trifluoromethyl etc.), hydroxy, $C_{1-6}$ alkoxy (methoxy, ethoxy etc.), halogeno$C_{1-6}$ alkoxy (trifluoromethoxy, trifluoroethoxy etc.), nitro, amino, cyano, carbamoyl, carbamoyl which may be substituted with $C_{1-6}$alkyl or amino which may be substituted with formyl (NHCHO, $NHCONH_2$, NHCONHMe etc.), $C_{1-3}$ alkylenedioxy (methylenedioxy etc.), an aminocarbonyloxy group which may be substituted with $C_{1-6}$ alkyl (aminocarbonyloxy, methylaminocarbonyloxy, ethylaminocarbonyloxy, dimethylaminocarbonyloxy, diethylaminocarbonyloxy etc.), 5-to 7-membered cyclic amino-carbonyloxy ((1-pyrrolidinyl)carbonyloxy, piperidinocarbonyloxy etc.), aminosulfonyl, mono-$C_{1-6}$ alkylaminosulfonyl and di-$C_{1-6}$ alkylaminosulfonyl (more preferably, (i) a $C_{6-10}$ aryl group (phenyl etc.) or (ii) a 5-or 6-membered aromatic heterocyclic group (optionally condensed with a benzene ring) containing 1 to 4 hetero atom(s) selected from a nitrogen atom, an oxygen atom and a sulfur atom (thienyl, indolyl etc.), each of which may have 1 to 3 substituent(s) selected from halogen, nitro, hydroxy, optionally halogenated $C_{1-6}$ alkyl, optionally halogenated $C_{1-6}$ alkoxy and aminosulfonyl, or (2) $Ar^3$ and $L_2$ are linked together to form a ring represented by the formula:

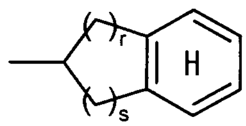

wherein each symbol is as defined above.
In addition, among the compound (II), a compound represented by the formula:

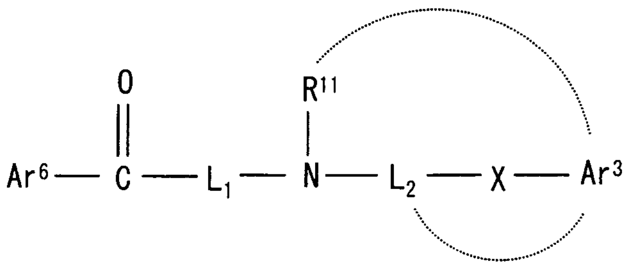 (IIc)

wherein $Ar^6$ represents a benzene ring group which has 1 or 2 substituent(s) selected from aminosulfonyl, mono-or di-$C_{1-6}$ alkylaminosulfonyl, $C_{1-6}$ alkyl-carbonylamino and $C_{1-6}$ alkylsulfonylamino, and further may have 1 to 4 substituent (s), and the other symbols are as defined above (hereinafter, sometimes, abbreviated as the compound (IIc)) or a salt thereof is a novel compound.
The "substituent" in the "benzene ring group further may have 1 to 4 substituent(s)" represented by $Ar^6$ include the same groups as those exemplified with respect to the "substituent" in the "represents an optionally condensed 5-or 6-membered aromatic ring group, and said aromatic ring group may have a substituent" represented by $Ar^2$ (provided that aminosulfonyl, mono-or di-$C_{1-6}$ alkylaminosulfonyl, $C_{1-6}$ alkyl-carbonylamino and $C_{1-6}$ alkylsulfonylamino are excluded).
The compound (IIc) is preferably a compound wherein $Ar^6$ is a benzene ring group which has 1 or 2 (more preferably 1) substituent(s) selected from aminosulfonyl, mono- or di-$C_{1-6}$ alkylaminosulfonyl, $C_{1-6}$ alkyl-carbonylamino and $C_{1-6}$alkylsulfonylamino, and further may have 1 or 2 (more preferably 1) $C_{1-4}$ alkoxy;
$L_1$ is a $C_{4-5}$alkylene group which may have 1 or 2 halogen atom(s) (preferably, unsubstituted $C_{4-5}$ alkylene group), such

as $-(CH_2)_4-$, $-(CH_2)_5-$, $-CHF(CH_2)_3-$, $-CHF(CH_2)_4-$, $-CF_2(CH_2)_3-$, $-CF_2(CH_2)_4-$, $-(CH_2)_3CH(CH_3)-$, and $-(CH_2)_4CH(CH_3)-$; $R^{11}$ is (i) a hydrogen atom, (ii) a $C_{1-4}$alkyl group which may have 1 to 3 substituent(s) selected from halogen atom (preferably fluoro etc.) and hydroxy, or (iii) a $C_{7-16}$ aralkyl (benzyl etc.) (more preferably, hydrogen atom or $C_{1-4}$ alkyl group);

$L_2$ is a $C_{2-4}$ alkylene group which may have 1 to 4 substituent(s) selected from halogen atom, hydroxy, oxo, and phenyl (e.g. $-(CH2)_2-$, $-(CH2)_3-$, $-(CH_2)_4-$, $-CHFCH_2-$, $-CHF(CH_2)_2-$, $-CHF(CH_2)_3-$, $-CF_2CH_2-$, $-CF_2(CH_2)_2-$, $-CF_2(CH_2)_3-$, $-(CH_3)CHCH_2-$, $-(CH_3)CH(CH_2)_2-$, $-(CH(CH_3))_2-$, $-CH_2CH(OH)-$, $-CH_2C0-$, $-(Ph)CHCH_2-$ etc.) (more preferably, $C_{2-3}$ alkylene group which may have hydroxy or oxo);

X is a bond, an oxygen atom or NH (more preferably a bond);

$Ar^3$ is (1) (i) a $C_{6-10}$ aryl group (phenyl etc.) or (ii) a 5- or 6- membered aromatic heterocyclic group (optionally condensed with a benzene ring) containing 1 to 4 hetero atom(s) selected from a nitrogen atom, an oxygen atom and a sulfur atom (e.g. thienyl, furyl, pyrrolyl, imidazolyl, pyrazolyl, thiazolyl, isothiazolyl, oxazolyl, isooxazolyl, pyridyl, pyrazinyl, pyrimidinyl, pyridazinyl, oxadiazolyl, thiadiazolyl, furazanyl, indolyl, isoindolyl, benzofuranyl, quinolyl, isoqiunolyl, naphthyridinyl, quinazolinyl, cinnolinyl, acridinyl etc.), each of which may have 1 to 5 (preferably 1 to 3) substituent(s) selected from halogen (fluoro, chloro etc.), $C_{1-6}$ alkyl (methyl, ethyl etc.), halogeno$C_{1-6}$ alkyl (trifluoromethyl etc.), hydroxy, $C_{1-6}$ alkoxy (methoxy, ethoxy etc.), halogeno$C_{1-6}$alkoxy (trifluoromethoxy, trifluoroethoxy etc.), nitro, amino, cyano, carbamoyl, carbamoyl which may be substituted with $C_{1-6}$ alkyl or amino which may be substituted with formyl (NHCHO, $NHCONH_2$, NHCONHMe etc.), $C_{1-3}$ alkylenedioxy (methylenedioxy etc.), an aminocarbonyloxy group which may be substituted with $C_{1-6}$ alkyl (aminocarbonyloxy, methylaminocarbonyloxy, ethylaminocarbonyloxy, dimethylaminocarbonyloxy, diethylaminocarbonyloxy etc.), 5- to 7-membered cyclic amino-carbonyloxy ((1-pyrrolidinyl)carbonyloxy, piperidinocarbonyloxy etc.), aminosulfonyl, mono-$C_{1-6}$ alkylaminosulfonyl and di-$C_{1-6}$ alkylaminosulfonyl, (2) the case where $Ar^3$ and $R^{11}$ are linked together to form a ring represented by the formula:

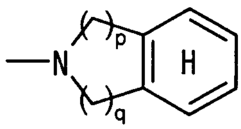

wherein each symbol is as defined above, or (3) the case where $Ar^3$ and $L_2$ are linked together to form a ring represented by the formula:

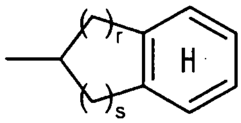

wherein each symbol is as defined above ($Ar^2$ is more preferably (i) a $C_{6-10}$ aryl group (phenyl etc.) or (ii) a 5-or 6-membered aromatic heterocyclic group (optionally condensed with a benzene ring) containing 1 to 4 hetero atom(s) selected from a nitrogen atom, an oxygen atom and a sulfur atom (thienyl, indolyl etc.), each of which may have 1 to 3 substituent(s) selected from halogen, nitro, hydroxy, optionally halogenated $C_{1-6}$ alkyl, optionally halogenated $C_{1-6}$ alkoxy and aminosulfonyl).

In addition, among the compound (II), a compound represented by the formula:

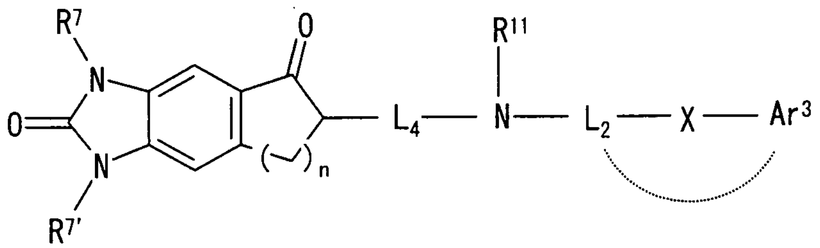
(IId)

wherein n represents an integer of 1 or 2, $L_4$ represents a $C_{3-5}$ alkylene group which may have a substituent, and the

other symbols are as defined above (hereinafter, sometimes, abbreviated as the compound (IId)) or a salt thereof is a novel compound.

Examples of the "$C_{3-5}$ alkylene group which may have a substituent" represented by $L_4$ include those having a carbon chain number of 3 to 5 among the "$C_{1-10}$ alkylene group" exemplified with respect to the "spacer having a main chain of 1 to 10 atoms which may have a substituent" represented by L.

The "substituent" in the "$C_{3-5}$ alkylene group which may have a substituent" represented by $L_4$ include the same groups as those exemplified with respect to the "substituent" in the "spacer having a main chain of 1 to 10 atoms which may have a substituent" represented by L. Preferable examples include 1 to 4 substituent(s) selected from halogen atom (e.g. fluorine, chlorine, bromine, iodine etc.), nitro, cyano, optionally halogenated $C_{1-6}$ alkoxy, and hydroxy.

The compound (IId) is preferably a compound wherein $R^7$ and $R^{7'}$ are a hydrogen atom or an optionally halogenated $C_{1-6}$ alkyl group (methyl, ethyl, trifluoromethyl etc.);

$L_4$ is a $C_{3-5}$ alkylene group (more preferably $C_{3-4}$ alkylene group) which may have 1 or 2 halogen atom(s), such as $-(CH_2)_3-$, $-(CH_2)_4-$, $-(CH_2)_5-$, $-CHF(CH_2)_2-$, $-CHF(CH_2)_3-$, - $CF_2(CH_2)_2-$, $-CF_2(CH_2)_3-$, $-(CH_2)_2CH(CH_3)-$, and$-(CH_2)_3CH(CH_3)-$);

$R^{11}$ is (i) a hydrogen atom, (ii) a $C_{1-4}$ alkyl group which may have 1 to 3 substituent(s) selected from halogen atom (preferably fluoro etc.) and hydroxy, or (iii) $C_{7-16}$ aralkyl (benzyl etc.) (more preferably a hydrogen atom or a $C_{1-4}$ alkyl group) ;

$L_2$ is a $C_{2-4}$ alkylene group which may have 1 to 4 substituent(s) selected from halogen atom, hydroxy, oxo, and phenyl (e.g. $-(CH_2)_2-$, $-(CH_2)_3-$, $-(CH_2)_4-$, $-CHFCH_2-$, - $CHF(CH_2)_2-$, $-CHF(CH_2)_3-$, $-CF_2CH_2-$, $-CF_2(CH_2)_2-$, $-CF_2(CH_2)_3-$, - $(CH_3)CHCH_2-$, $-(CH_3)CH(CH_2)_2-$, $-(CH(CH_3))_2-$, $-CH_2CH(OH)-$, - $CH_2CO-$, $-(Ph)CHCH_2-$ etc.) (more preferably $C_{2-3}$ alkylene group which may have hydroxy or oxo);

X is a bond, an oxygen atom or NH (more preferably a bond);

$Ar^3$ is (1) (i) a $C_{6-10}$ aryl group (phenyl etc.) or (ii) a 5- or 6-membered aromatic heterocyclic group (optionally condensed with a benzene ring) containing 1 to 4 hetero atom(s) selected from a nitrogen atom, an oxygen atom and a sulfur atom (e.g. thienyl, furyl, pyrrolyl, imidazolyl, pyrazolyl, thiazolyl, isothiazolyl, oxazolyl, isoxazolyl, pyridinyl, pyrazinyl, pyrimidinyl, pyridazinyl, oxadiazolyl, thiadiazolyl, furazanyl, indolyl, isoindolyl, benzofuranyl, quinolyl, isoquinolyl, naphthyridinyl, quinazolinyl, cinnolinyl, acridinyl etc.), each of which may have 1 to 5 (preferably 1 to 3) substituent(s) selected from halogen (fluoro, chloro etc.), $C_{1-6}$ alkyl (methyl, ethyl etc.), halogeno$C_{1-6}$ alkyl (trifluoromethyl etc.), hydroxy, $C_{1-6}$ alkoxy (methoxy, ethoxy etc.), halogeno$C_{1-6}$ alkoxy (trifluoromethoxy, trifluoroethoxy etc.), nitro, amino, cyano, carbamoyl, carbamoyl which may be substituted with $C_{1-6}$ alkyl or amino which may be substituted with formyl (NHCHO, $NHCONH_2$, NHCONHMe etc.), $C_{1-3}$ alkylenedioxy (methylenedioxy etc.), an aminocarbonyloxy group which may be substituted with $C_{1-6}$ alkyl (aminocarbonyloxy, methylaminocarbonyloxy, ethylaminocarbonyloxy, dimethylaminocarbonyloxy, diethylaminocarbonyloxy etc.), 5-to 7-membered cyclic amino-carbonyloxy ((1-pyrrolidinyl)carbonyloxy, piperidinocarbonyloxy etc.), aminosulfonyl, mono-$C_{1-6}$ alkylaminosulfonyl and di-$C_{1-6}$ alkylaminosulfonyl, (2) the case where $Ar^3$ and $R^{11}$ are linked together to form a ring represented by the formula:

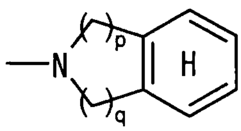

wherein each symbol is as defined above, or (3) the case where $Ar^3$ and $L_2$ are linked together to form a ring represented by the formula:

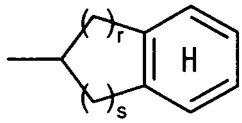

wherein each symbol is as defined above ($Ar^3$ is more preferably (i) a $C_{6-10}$ aryl group (phenyl etc.) or (ii) a 5-or 6-membered aromatic heterocyclic group (optionally condensed with a benzene ring) containing 1 to 4 hetero atom(s) selected from a nitrogen atom, an oxygen atom and a sulfur atom (thienyl, indolyl etc.), each of which may have 1 to 3 substituent(s) selected from halogen, nitro, hydroxy, optionally halogenated $C_{1-6}$ alkyl, optionally halogenated $C_{1-6}$ alkoxy and aminosulfonyl).

Specifically, as the compound (II), (IIa), (IIb), (IIc) or (IId), the compounds described in Reference Examples 1 to 242 and Examples 1 to 414 of WO03/57254 are used.

When the compound (II), (IIa), (IIb), (IIc) or (IId) (hereinafter, referred to as the compound B) is a salt, examples of such salt include salts with an inorganic base, an ammonium salt, salts with an organic base, salts with an inorganic acid, salts with an organic acid, an salts with a basic or acidic amino acid.

Preferable examples of the salt with an inorganic base include alkali metal salts such as a sodium salt and a potassium salt; alkaline earth metal salts such as a calcium salt, a magnesium salt and a barium salt; an aluminum salt.

Preferable examples of the salt with an organic base include salts with trimethylamine, triethylamine, pyridine, picoline, ethanolamine, diethanolamine, triethanolamine, bicyclohexylamine, and N,N-dibenzylethylenediamine.

Preferable examples of the salt with an inorganic acid include salts with hydrochloric acid, hydrobromic acid, nitric acid, sulfuric acid and phosphoric acid.

Preferable examples of the salt with an organic acid include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, and p-toluenesulfonic acid.

Preferable examples of the salt with a basic amino acid include salts with arginine, lysine and ornithine, and preferable examples of the salt with an acidic amino acid include aspartic acid and glutamic acid.

Among these salts, pharmaceutically acceptable salts are preferred. For example, when Compound B has an acidic functional group, it may be an inorganic salt such as an alkali metal salt (e.g. sodium salt, potassium salt etc.), and an alkaline earth metal salt (e.g. calcium salt, magnesium salt, barium salt etc.), or an ammonium salt. When Compound B has a basic functional group, it may be an inorganic salt such as hydrochloride, sulfate, phosphate and hydrobromide; or an organic salt such as acetate, maleate, fumarate, succinate, methanesulfonate, p-toluenesulfonate, citrate, and tartarate.

The compound B may be an anhydride or a hydrate. In the case of a hydrate, Compound B may have 0.1 to 5 of water molecules.

Further, the compound B may be labeled with an isotope element (e.g. $^{3}H$, $^{14}C$, $^{35}S$ etc.).

When the compound B contains an optical isomer, a steric isomer, a positional isomer or a rotational isomer, these are also included in the compound of the present invention, and each of the isomers may be obtained as a single compound by a synthetic method or separation method known per se. For example, when the compound B has an optical isomer, an optical isomer resolved from the compound is included in the compound B.

The optical isomer can be prepared by a method known per se. Specifically, an optically active isomer can be obtained by using an optically active compound in the synthesis, or optically resolving the final mixture of racemic isomers with a conventional method.

As an optical resolution method, a method known per se, for example, a fractional recrystallization method, a chiral column method and a diastereomer method which will be described in detail below, is used.

1) Fractional recrystallization method

A method of obtaining a free optical isomer by forming a salt of a racemic compound and an optically active compound (e.g. (+)-mandelic acid, (-)-mandelic acid, (+)-tartaric acid, (-)-tartaric acid, (+)-1-phenethylamine, (-)-1-phenethylamine, cinchonine, (-)-cinchonidine, brucine etc), separating this by a fractional recrystallization method and, if necessary, via a neutralizing step.

2) A chiral column method

A method for separating a racemic compound or a salt thereof by applying to an optical isomer separating column (chiral column). For example, in the case of liquid chromatography, optical isomers are separated by adding a mixture of optical isomers to a chiral column such as ENANTIO-OVM (manufactured by Toso) or CHIRAL series manufactured by Daicel, and developing this with water, various buffers (e.g. phosphate buffer), or an organic solvent (e.g. ethanol, methanol, isopropanol, acetonitrile, trifluoroacetic acid, diethylamine) alone or a mixture thereof. In addition, in the case of gas chromatography, optical isomers are separated by using a chiral column such as CP-Chirasil-DeXCB (manufactured by GL Science).

3) A diastereomer method

A method of obtaining an optical isomer by chemically reacting a mixture of racemic compounds with an optically active reagent to give a mixture of diastereomers, separating single substance with usual separation means (e.g. fractional recrystallization, chromatography method etc.) and separating off an optically active reagent moiety by chemical treatment such as hydrolysis reaction. For example, when Compound B has hydroxy or primary or secondary amino in a molecule,

a diastereomer of ester or amido can be obtained by subjecting the compound and an optically active organic acid (e.g. MTPA [α-methoxy-α-(trifluoromethyl)phenylacetic acid], (-)-menthoxyacetic acid, etc.) to a condensation reaction. On the other hand, when Compound B has a carboxylic acid group, a diastereomer of amido or ester is obtained by subjecting the compound and an optically active amine or alcohol reagent to a condensation reaction. The separated diastereomer is converted into an optical isomer of the original compound by subjecting to acid hydrolysis or base hydrolysis reaction.

A prodrug of the compound B refers to a compound which is converted into the compound B by a reaction with an enzyme or gastric acid under a physiological condition in vivo, that is, a compound which is changed to the compound B by enzymatic oxidation, reduction or hydrolysis, and a compound which is changed into the compound B by hydrolysis with gastric acid. Examples of a prodrug of the compound B include a compound in which an amino group of the compound B is acylated, alkylated or phosphorylated [e.g. compound in which an amino group of the compound B is eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, or tert-butylated]; a compound in which a hydroxy group of the compound B is acylated, alkylated, phosphorylated or converted into borate (e.g. compound in which a hydroxy group of the compound B is acetylated, palmitoylated, propanoylated, pivaloylated, succinylated, fumarylated, alanylated, or dimethylaminomethylcarbonylated); a compound in which a carboxyl group of the compound B is esterified or amidated (e.g. compound in which a carboxyl group of the compound B is ethyl esterified, phenyl esterified, carboxymethyl esterified, dimethylaminomethyl esterified, pivaloyloxymethyl esterified, ethoxycarbonyloxyethyl esterified, phthalidyl esterified, (5-methyl-2-oxo-1,3-dioxolen-4-yl)methyl esterified, cyclohexyloxycarbonylethyl esterified, or methylamidated). These compounds can be prepared from the compound B with a method known per se.

A prodrug of the compound B may be a compound which is converted into the compound B under physiological conditions as described in "Development of Drugs", Volume 7, Molecular Design, Hirokawa Shoten, 1990; pages 163-198.

The compound of the formula (I) or its salt can be produced by known methods or their modification. Specifically, the desired compounds can be produced according to

(1) the methods described in JP 3-173867 A (EP-A-0378207), JP 64-79151 A (EP-A-0296560), etc., when the "optionally condensed phenyl which may be substituted by a substituent or substituents" represented by Ar does not form a condensed ring;
(2) the methods described in JP 5-140149 A (EP-A-0487071), JP 6-166676 A (EP-A-0560235), JP 6-206875 A (EP-A-0567090), JP 2-169569 A (USP 4895814), etc., when the "optionally condensed phenyl which may be substituted by a substituent or substituents" represented by Ar is condensed with an optionally substituted monocyclic heterocyclic ring;
(3) the methods described in JP 7-206854 A (EP-A 0607864), etc., when the "optionally condensed phenyl which may be substituted by a substituent or substituents" represented by Ar is condensed with an optionally substituted bicyclic heterocycle or with two identical or different monocyclic heterocycle (provided that at least one of two is a monocyclic heterocycle); and
(4) the methods described in JP 7-309835 A (EP-A 0655451), etc., when the "optionally condensed phenyl which may be substituted by a substituent or substituents" represented by Ar is condensed with an optionally substituted tricyclic heterocycle.

2) Compounds of the formula:

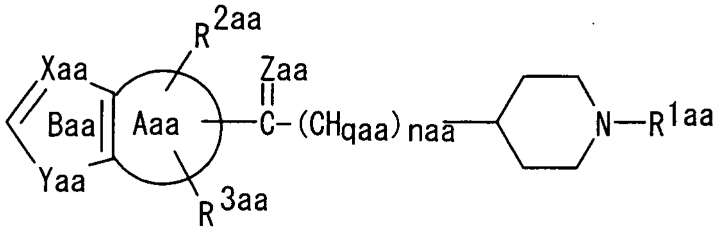

wherein one of the side chain containing C=Zaa, $R^{2aa}$ and $R^{3aa}$ is attached to the carbon atom indicated by an asterisk * on the ring Baa; the ring Aaa is benzo, thieno, pyrido, pyrazino, pyrimido, furano, seleno, pyrrolo, thiazolo or imidazolo; $R^{1aa}$ is phenyl, phenyl-$C_{1-6}$ alkyl, cinnamyl or heteroarylmethyl (where the heteroaryl includes imidazolo, thiazolo, thieno, pyrido or isoxazolo), and the phenyl and heteroaryl may be substituted by 1 or 2 substituents selected from $C_{1-6}$ alkyl, $C_{1-6}$ alkoxy and halogen; $R^{2aa}$ and $R^{3aa}$ each represents independently a hydrogen atom, $C_{1-6}$ alkoxy, $C_{1-6}$ alkyl optionally

substituted by 1 to 3 fluorine atoms, benzyloxy, hydroxy, phenyl, benzyl, halogen, nitro, Cyano, $COOR^{4aa}$, $CONHR^{4aa}$, $NR^{4aa}R^{5aa}$, $NR^{4aa}COR^{5aa}$ or $SOpaaCH_2Ph$ (where paa is 0, 1 or 2), or $R^{2aa}$ and $R^{3aa}$ taken with the adjacent carbon atoms may form a 5- or 6-membered ring (carbon, nitrogen and oxygen atoms constitutes the ring), for example, methylenedioxy, ethylenedioxy or lactam ring; $R^{4aa}$ and $R^{5aa}$ each represents independently hydrogen or $C_{1-6}$ alkyl, or $R^{4aa}$ and $R^{5aa}$ in $NR^{4aa}R^{5aa}$ taken with the adjacent nitrogen atom may form a 4- to 8-membered ring containing at least one nitrogen atom (the other atoms constituting the ring are carbon, oxygen and nitrogen); in addition, $R^{4aa}$ and $R^{5aa}$ in $NR^{4aa}$ $COR^{5aa}$ taken with the adjacent nitrogen atom and carbon atom may form a 4- to 8-membered lactam ring; Xaa is nitrogen or CH, and Yaa is oxygen, sulfur or $NR^{6aa}$; $R^{6aa}$ is hydrogen, $C_{1-6}$ alkyl, CO-$C_{1-6}$ alkyl or $SO_2$-phenyl (where the phenyl may be substituted by 1 to 5 substituents independently selected from $C_{1-4}$ alkyl); naa is an integer of 1 to 4; qaa each is independently 1 or 2; Zaa is oxygen or sulfur; or salts thereof. Such compounds are exemplified by 1-(2-methyl-1H-benzimidazol-5-yl)-3-[1-(phenylmethyl)-4-piperidinyl]-1-propanone, 1-(6-methylbenzo[b]thien-2-yl)-3-[1-(phenylmethyl)-4-piperidinyl]-1-propanone, 1-(6-methyl-indol-2-yl)-3-[1-(phenylmethyl)-4-piperidinyl]-1-propanone, and the like.

The above-mentioned compounds or salts thereof can be produced according to the process as described in WO 93/07140 or its modification.

3) Compounds of the formula:

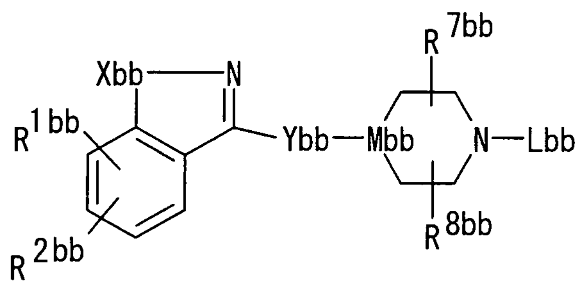

wherein $R^{1bb}$ and $R^{2bb}$ each is hydrogen, $C_{1-6}$ alkoxy, benzyloxy, phenoxy, hydroxy, phenyl, benzyl, halogen, nitro, cyano, group of the formula: $COR^{5bb}$, $-COOR^{5bb}$, $-CONHR^{5bb}$, $-NR^{5bb}R^{6bb}$ or $-NR^{5bb}COR^{6bb}$ (wherein $R^{5bb}$ and $R^{6bb}$ each is i) hydrogen atom, ii) $C_{1-6}$ alkyl, iii) phenyl or benzyl which may be substituted by 1 or 2 substituents selected from halogen, $C_{1-4}$ alkyl, trifluoromethyl, $C_{1-4}$ alkoxy, cyano, nitro and hydroxy; or $R^{5bb}$ and $R^{6bb}$ in $-NR^{5bb}R^{6bb}$ taken together may form a 4- to 8-membered nitrogen-containing ring; $R^{5bb}$ and $R^{6bb}$ in $-NR^{5bb}COR^{6bb}$ taken together may form a 4- to 8-membered lactam ring), $C_{1-6}$ alkyl optionally substituted by 1 to 3 fluorine atoms, group of the formula: $SO_{pbb}CH_2$-phenyl or $SO_{pbb}C_{1-6}$ alkyl (wherein pbb is 0, 1 or 2), pyridylmethyloxy, thienylmethyloxy, 2-oxazolyl, 2-thiazolyl or benzenesulfonamido (said phenoxy, benzyloxy, phenyl, benzyl, benzenesulfonamido, pyridylmethyloxy, thienylmethyloxy, 2-oxazolyl, and 2-thiazolyl may be substituted by 1 or 2 substituents selected from halogen, $C_{1-6}$ alkyl, trifluoromethyl, $C_{1-6}$ alkoxy, cyano, nitro and hydroxy); or $R^{1bb}$ and $R^{2bb}$, when they are attached to the adjacent carbon atoms and when Xbb is oxygen, sulfur or $NR^{4bb}$ ($R^{4bb}$ is hydrogen or $C_{1-4}$ alkyl), taken with the attached carbon atoms may form a group of the formula:

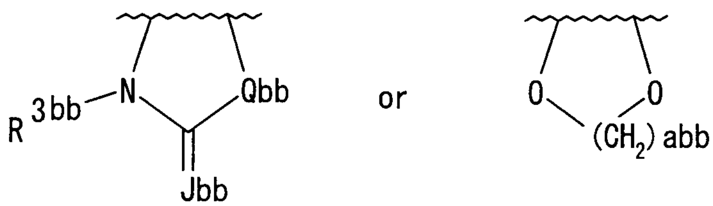

wherein Jbb is oxygen, sulfur or $NR^{4bb}$; abb is 1 or 2; $R^{3bb}$ is hydrogen or $C_{1-6}$ alkyl; Qbb is oxygen, sulfur, NH, $CHCH_3$, $C(CH_3)_2$, -CH=CH- or $(CH_2)_{1bb}$; and 1bb is an integer of 1 to 3;

Xbb is oxygen, sulfur, -CH=CH-, -CH=N-, -NH=CH-, -N=N- or $NR^{4bb}$ ($R^{4bb}$ has the same significance as mentioned above); Ybb is - $(CH_2)_{mbb}$-, -CH=CH $(CH_2)_{nbb}$- , $-NR^{4bb}$ $(CH_2)_{mbb}$- or - $O(CH_2)_{mbb}$- ($R^{4bb}$ has the same significance as mentioned above; nbb is an integer of 0 to 3; mbb is an integer of 1 to 3);

Mbb is -CH- or nitrogen;
Lbb is i) phenyl or phenyl-$C_{1-6}$ alkyl which may be substituted by 1 to 3 substituents selected from halogen, $C_{1-6}$ alkyl, $C_{1-6}$ alkoxy, $C_{1-6}$ alkoxy-carbonyl and $C_{1-6}$ alkyl-carbonyl, ii) cinnamyl, iii) pyridylmethyl, or iv) group of the formula:

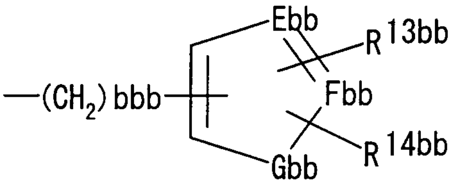

wherein bbb is an integer of 1 to 4; $R^{13bb}$ and $R^{14bb}$ each is hydrogen, $C_{1-4}$ alkyl, halogen or phenyl; Ebb and Fbb each is -CH- or nitrogen; Gbb is oxygen, sulfur or $NR^{4bb}$ ($R^{4bb}$ has the same significance as mentioned above); provided that when both of Ebb and Fbb are nitrogen, then one of $R^{13bb}$ and $R^{14bb}$ is absent;
$R^{7bb}$ and $R^{8bb}$ each is hydrogen, $C_{1-6}$ alkyl, $C_{1-6}$ alkoxy-carbonyl, $C_{1-6}$ alkyl-carbonyl, or $C_{1-6}$ alkoxy; provided that said $C_{1-6}$ alkoxy is not attached to the carbon atom adjacent to the nitrogen, or salts thereof. Such compounds are exemplified by 3-[2-[1-(phenylmethyl)-4-piperidinyl]ethyl]-5,6,8-trihydro-7H-isoxazolo[4,5-g]quinolin-7-one, 6,8-dihydro-3-[2-[1-(phenylmethyl)-4-piperidinyl]ethyl]-7H-pyrrolo[5,4-g]-1,2-benzisoxazol-7-one, 5,7-dihydro-3-[2-[1-(phenylmethyl)-4-piperidyl]ethyl]-6H-pyrrolo[5,4-f]-1,2-benzisoxazol-6-one, and the like.

The above-mentioned compounds or salts thereof can be produced according to the process described in JP 6-500794 A (WO 92/17475) or its modification.

4) Compounds of the formula:

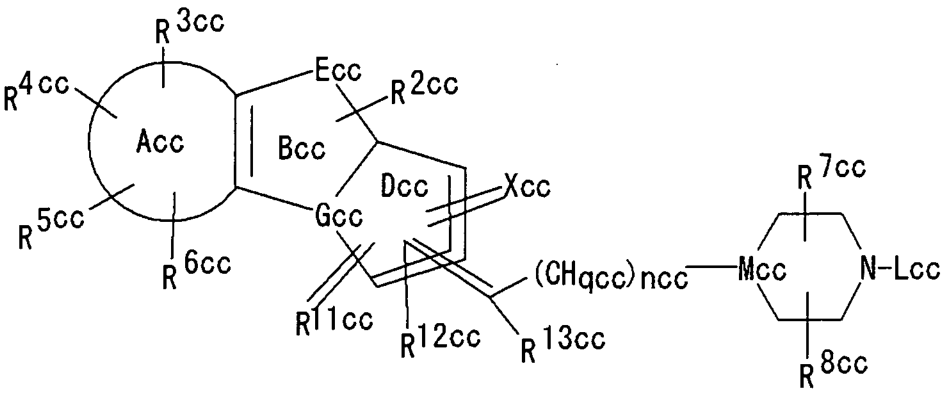

wherein the ring Acc is benzo, thieno, pyrido, pirazino, pyrimido, furano, seleno or pyrrolo;
$R^{2cc}$ is hydrogen, $C_{1-4}$ alkyl, benzyl, fluoro or cyano;
$R^{3cc}$, $R^{4cc}$, $R^{5cc}$ and $R^{6cc}$ each is hydrogen, $C_{1-6}$ alkoxy, benzyloxy, phenoxy, hydroxy, phenyl, benzyl, halogen, nitro, cyano, -$COOR^{9cc}$, -$CONHR^{9cc}$,-$NR^{9cc}R^{10cc}$, -$NR^{9cc}COR^{10cc}$, or $C_{1-6}$ alkyl which may be substituted by 1 to 3 fluorine atoms; $SO_{pcc}CH_2$-phenyl (pcc is 0, 1 or 2), pyridylmethyloxy or thienylmethyloxy (said phenoxy, benzyloxy, phenyl, pyridylmethyloxy and thienylmethyloxy may be substituted by 1 or 2 substituents selected from halogen, $C_{1-4}$ alkyl, trifluoromethyl, $C_{1-4}$ alkoxy, cyano, nitro and hydroxy); or two of $R^{3cc}$, $R^{4cc}$, $R^{5cc}$ and $R^{6cc}$, taken with the adjacent carbon atoms, may form a saturated 5- or 6-membered ring (e.g., methylenedioxy, ethylenedioxy or lactam ring) in which each atom is carbon, nitrogen or oxygen in addition to the adjacent carbon atoms;
$R^{9cc}$ and $R^{10cc}$ each is hydrogen or $C_{1-6}$ alkyl; or
$R^{9cc}$ and $R^{10cc}$ in $NR^{9cc}R^{10cc}$ taken together may form a 4- to 8-membered cyclic amino in which one of the ring-constituting atoms is nitrogen and the others are carbon; or
$R^{9cc}$ and $R^{10cc}$ in $NR^{9cc}COR^{10cc}$ taken together may form a 4- to 8-membered lactam ring;
Gcc is carbon or nitrogen;
Ecc is carbon, nitrogen, oxygen, sulfur, sulfoxide or sulfone;

--- is a single bond or double bond;
the carbon located at any of the 1-, 2- or 3-position adjacent to a carbonyl group on the ring DCC may be replaced by an appropriate nitrogen (to form a lactam ring as said carbon is located at the 1-, 2- or 3-position on the ring DCC);
Xcc is O, S, $NOR^{1cc}$, hydrogen or $C_{1-6}$ alkyl (provided that a double bond is formed between Xcc and the ring Dcc, only when the atom on the ring Dcc to which Xcc is attached is carbon, and Xcc is O, S or $NOR^{1cc}$);
$R^{1cc}$ is hydrogen or $C_{1-6}$ alkyl;
qcc is 1 or 2;
when the ring Dcc is a lactam, ncc is an integer of 1 to 3, and when the ring Dcc is not lactam, ncc is 0 or an integer of 1 to 3;
Mcc is carbon or nitrogen;
Lcc is phenyl, phenyl-$C_{1-6}$ alkyl, cinnamyl or pyridylmethyl (said phenyl and phenyl-$C_{1-6}$ alkyl may be substituted by 1 to 3 substituents selected from $C_{1-6}$ alkyl, $C_{1-6}$ alkoxy, $C_{1-6}$ alkoxy- carbonyl, $C_{1-6}$ alkyl-carbonyl and halogen);
$R^{11cc}$ is hydrogen, halogen, hydroxy, $C_{1-4}$ alkyl, $C_{1-4}$ alkoxy or oxygen;
$R^{12cc}$ and $R^{13cc}$ each is hydrogen, fluoro, hydroxy, acetoxy, O-mesylate, O-tosylate, $C_{1-4}$ alkyl or $C_{1-4}$ alkoxy; or when both of $R^{12cc}$ and $R^{13cc}$ are attached to carbon atoms, they taken with the atoms to which they are attached may form a 3- to 5-membered ring in which the constituting atoms are carbon or oxygen;
$R^{7cc}$ and $R^{7cc}$ each is hydrogen, $C_{1-6}$ alkyl or $C_{1-6}$ alkoxy (said $C_{1-6}$ alkoxy is not bound to the carbon adjacent to the nitrogen, $C_{1-6}$ alkoxy-carbonyl and $C_{1-6}$ alkyl-carbonyl); or $R^{8cc}$ and $R^{12cc}$, taken with the atoms to which they are attached, may form a 4- to 7-membered saturated carbocycle (one of the above-mentioned carbon atoms may be replaced by oxygen, nitrogen or sulfur);
provided that (a) when Ecc is carbon, nitrogen, oxygen, sulfur, sulfoxido or sulfone, Gcc is carbon; (b) when Gcc is nitrogen, Ecc is carbon or nitrogen; (c) when both of Ecc and Gcc are nitrogen, and when Gcc is carbon and Ecc is oxygen, sulfur, sulfoxido or sulfone, $R^{2cc}$ is absent; (d) the atoms located at the 1-, 2- and 3-positions on the ring Dcc each is not bound through more than one double bond; (e) when $R^{11cc}$ is oxygen, it is bound to the ring Dcc through a double bond, and when $R^{11cc}$ is other than oxygen, it is bound to the ring Dcc by a single bond; (f) when Xcc and $R^{11cc}$ both are oxygen and respectively bound to the carbon at the 1- and 3-positions or at the 3- and 1-positions on the ring Dcc, the carbon at the 2-position on the ring Dcc is replaced by nitrogen; and (g) Xcc is bound to the ring Dcc at the adjacent position at which a hydrocarbon group containing a group of the formula:

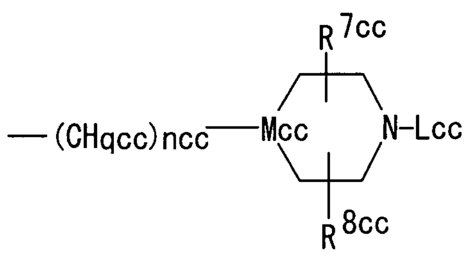

is attached, or salts thereof. Such compounds are exemplified by 2,3-dihydro-2-[[1-(phenylmethyl)-4-piperidinyl]methylene]-1H-pyrrolo[1,2-a]indol-1-one, 1,2,3,4-tetrahydro-4-methyl-2-[[1-(phenylmethyl)-4-piperidinyl]methylene]-cyclopent[b]-indol-3-one, 2,3-dihydro-2-[[1-(phenylmethyl)-4-piperidin-yl]methyl]-1H-pyrrolo[1,2-a]benzimidazol-1-one, 1,2,3,4-tetrahydro-6-methyl-2-[[1-(phenylmethyl)-4-piperidinyl]-ethyl]pyrrolo[3,4-b]indol-3-one, and the like.

The above-mentioned compounds or salts thereof can be produced according to the process described in JP 4-234845 A (EP-A 441517) or its modification.

5) Compounds of the formula:

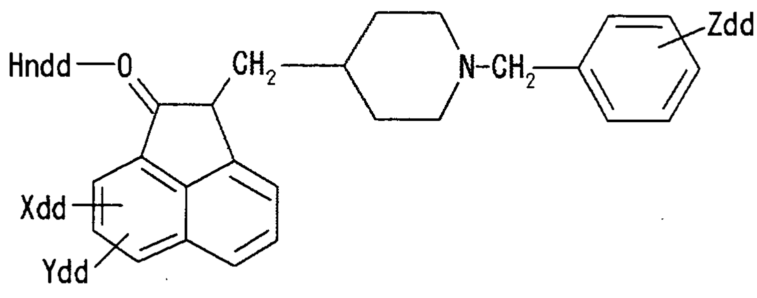

wherein Xdd is hydrogen, lower alkyl, lower alkoxy, hydroxy or nitro; Ydd is hydrogen or lower alkoxy; or Xdd and Ydd taken together form a group of $-OCH_2O-$ (in this case each position of Xdd and Ydd attached on the benzene ring has to be adjacent each other); Zdd is hydrogen, lower alkyl, lower alkoxy, hydroxy, halogen or nitro; ndd is 0 or 1; or salts thereof. Such compounds are exemplified by 2-[(N-benzylpiperidin-4-yl)methyl]-2a,3,4,5-tetrahydro-1(2H)-acenaphthylen-1-one, 2-[[N-(3-fluorobenzyl)piperidin-4-yl)methyl]-2a,3,4,5-tetrahydro-1(2H)-acenaphthylen-1-one, and the like.

The above-mentioned compounds or salts thereof can be produced according to the process described in JP 6-116237 A (EP-A 517221, USP 5,106,856) or its modification.

6) Compounds of the formula:

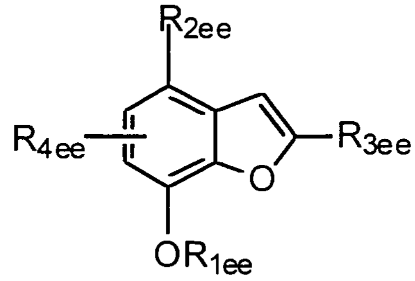

wherein $R_{1ee}$ is hydrogen, lower alkyl, aryl lower alkyl, $CONHR_{11ee}$ or $CONR_{6ee}R_{7ee}$; $R_{2ee}$ is hydrogen, cyano, $CH_2NR_{8ee}R_{9ee}$, $CONHR_{5ee}$ or $CONR_{6ee}R_{7ee}$; $R_{3ee}$ is a group of the formula:

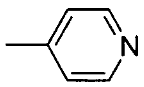 , 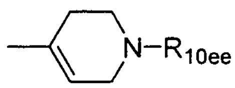 or 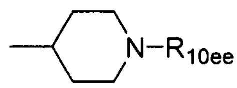

(where $R_{10ee}$ is hydrogen, lower alkyl, aryl lower alkyl, $CONHR_{5ee}$, $CONR_{6ee}R_{7ee}$, acyl, acyloxy lower alkyl or acyloxy-aryl lower alkyl); $R_{4ee}$ is hydrogen, halogen, lower alkyl or lower alkoxy; $R_{5ee}$ is hydrogen, lower alkyl or aryl lower alkyl; $R_{6ee}$ is lower alkyl or aryl lower alkyl; $R_{7ee}$ is lower alkyl or aryl lower alkyl; $R_{8ee}$ is hydrogen, lower alkyl, aryl lower alkyl or acyl; $R_{9ee}$ is hydrogen, lower alkyl or aryl lower alkyl; $R_{11ee}$ is lower alkyl, aryl or aryl lower alkyl; provided that when $R_{1ee}$ is hydrogen or lower alkyl, $R_{2ee}$ is not hydrogen;
or salts thereof. Such compounds are exemplified by 1-methyl-4-(4-cyano-7-methoxy-2-benzofuranyl)piperidine, 1-methyl-4-(4-N,N-diethylamido-7-methoxy-2-benzofuranyl)-piperidine, 1-methyl-4-(4-N,N-diethylaminomethyl-7-methoxy-2-benzofuranyl)piperidine, and the like.

The above-mentioned compounds or salts thereof can be produced according to the process described in JP 7-109275 A or its modification.

7) Compounds of the formula:

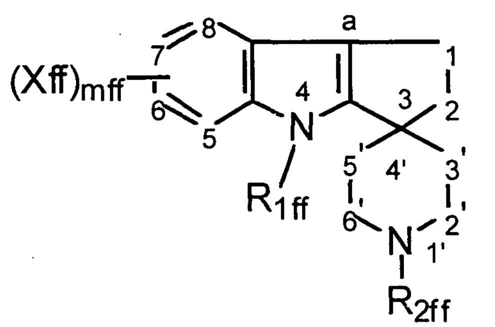

wherein Xff is hydrogen, halogen, lower alkoxy, lower alkyl, hydroxy or trifluoromethyl; mff is 1 or 2; $R_{1ff}$ is hydrogen or lower alkyl; $R_{2ff}$ is hydrogen, a group of the formula:

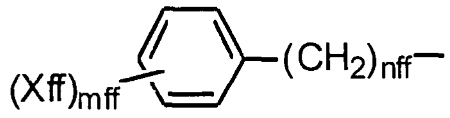

(wherein nff is 1 or 2; Xff and mff have the same significance as mentioned above), a group of the formula:

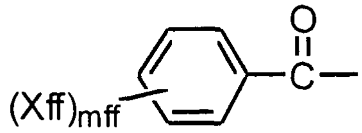

(wherein Xff and mff have the same significance as mentioned above), or a group of the formula:

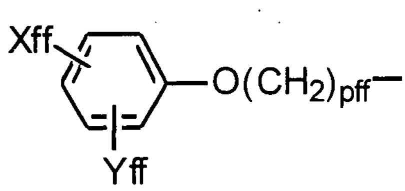

(wherein Xff has the same significance as mentioned above; Yff is hydrogen or a group of the formula: $COR_{4ff}$ (where $R_{4ff}$ is hydrogen or lower alkyl); pff is 2 or 3);
or salts thereof. Such compounds are exemplified by 1,4-dihydro-7-methoxy-4-methyl-1'-phenylmethylspiro[cyclopent-[b]indole-3(2H),4'-piperidine], 1,4-dihydro-4-methyl-1'-(4-methoxyphenyl)methylspiro[cyclopent[b]indole-3(2H),4'-piperidine], and the like.

The above-mentioned compounds or salts thereof can be produced according to the process described in WO 97/37992 or its modification.

8) Compounds of the formula:

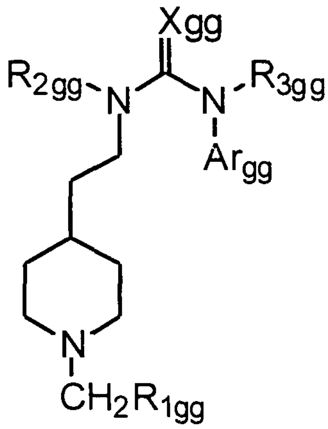

wherein $R_{1gg}$ is $C_{5-7}$ cycloalkyl, phenyl, or phenyl substituted by $C_{1-4}$ alkyl, $C_{1-4}$ alkoxy, nitro or halogen; $R_{2gg}$ and $R_{3gg}$ each is independently hydrogen or $C_{1-4}$ alkyl; Xgg is sulfur, oxygen, $CH-NO_2$ or $N-R_{5gg}$ (where $R_{5gg}$ is hydrogen, hydroxy, $C_{1-4}$ alkoxy, $C_{1-4}$ alkyl, cyano or $C_{1-4}$ alkylsulfonyl; Argg means a pyridyl or phenyl which may be substituted by 1 or more of substituents selected from halogen, $C_{1-4}$ alkyl, $C_{1-4}$ alkoxy, $C_{1-4}$ acyl, cyano, nitro, trifluoromethyl and trifluoromethoxy; or salts thereof. Such compounds are exemplified by N-phenyl- N'-[2-(1-benzyl-4-piperidyl)ethyl]-1,1-diamino-2-nitro-ethylene, 1-(2-pyridyl)-3-[2-(1-benzyl-4-piperidyl)ethyl]-thiourea, 1-phenyl-2-hydroxy-3-[2-(1-benzyl-9-piperidyl)-ethyl]guanidine, and the like.

The above-mentioned compounds or salts thereof can be produced according to the process described in JP 5-148228 A (EP-A 516520) or its modification.

9) Compounds of the formula:

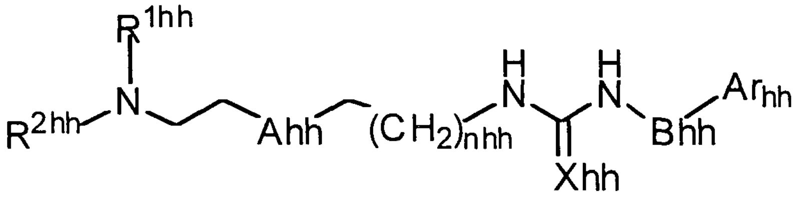

wherein $R^{1hh}$ is $C_{1-4}$ alkyl; $R^{2hh}$ is $C_{5-7}$ cycloalkyl, $C_{5-7}$ cycloalkyl-methyl, benzyl, or benzyl substituted by $C_{1-4}$ alkyl, $C_{1-4}$ alkoxy, halogen or nitro; Ahh is oxygen or methylene; Bhh is a direct bonding, methylene or carbonyl; Arhh is pyridyl, a group of the following formula:

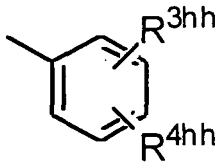

(wherein $R^{3hh}$ and $R^{4hh}$ each means independently hydrogen, halogen, nitro, $C_{1-4}$ alkyl, $C_{1-4}$ alkoxy, phenyl or trifluoromethoxy),
oxofluorenyl of the following formula:

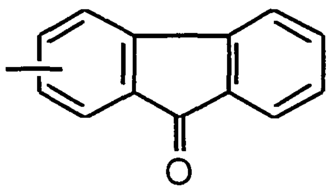

dioxoanthracenyl of the following formula:

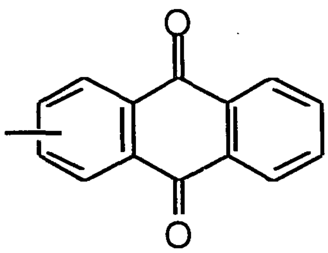

or naphthyl; nhh means 1 or 2; Xhh means oxygen or sulfur; or salts thereof. Such compounds are exemplified by 1-[2-[2-(N-benzyl-N-methylamino)ethoxy]ethyl]-3-(3-nitrobenzoyl)thiourea, 1-[2-[2-(N-benzyl-N-methylamino)ethoxy]ethyl]-3-(9-oxo-2-fluorenoyl)thiourea, and the like.

The above-mentioned compounds or salts thereof can be produced according to the process as described in JP 5-194359 A (EP-A 526313) or its modification.

10) Compounds of the formula:

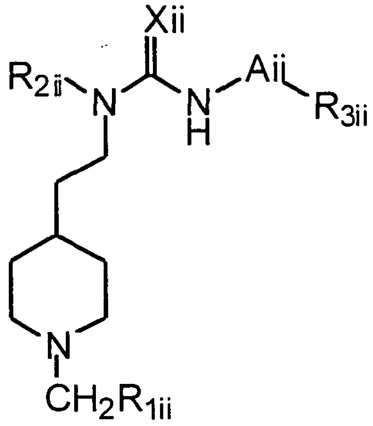

wherein $R_{1ii}$ is $C_{5-7}$ cycloalkyl, phenyl, or phenyl substituted by $C_{1-4}$ alkyl, $C_{1-4}$ alkoxy or halogen; $R_{2ii}$ is hydrogen or $C_{1-4}$ alkyl; Xii is oxygen or sulfur; Aii is methylene, carbonyl or sulfonyl; $R_{3ii}$ is (1) a group of the formula:

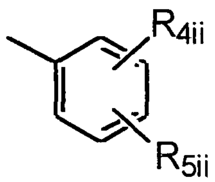

(wherein $R_{4ii}$ and $R_{5ii}$ each is independently hydrogen, halogen, nitro, $C_{1-4}$ alkyl, $C_{1-4}$ alkoxy, $C_{1-4}$ acyl, benzoyl, $C_{1-4}$ alkylsulfonyl or trifluoromethoxy, or $R_{4ii}$ and $R_{5ii}$ taken together may form methylenedioxy); (2) a group of the formula:

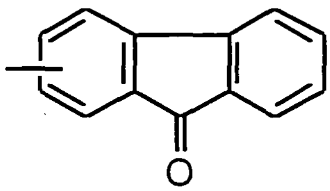

or (3) a group of the formula:

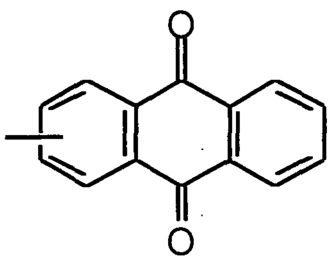

provided that when Xii is oxygen, Aii is a group other than methylene; or salts thereof. Such compounds are exemplified by 1-(3-nitrobenzoyl)-3-[2-(1-benzyl-4-piperidyl)ethyl]thiourea, 1-(9,10-dioxo-2-anthracenoyl)-3-[2-(1-benzyl-4-piperidyl)-ethyl]thiourea, and the like.

The above-mentioned compounds or salts thereof can be produced according to the process as described in JP 6-507387 A (WO 92/14710) or its modification.

11) Compounds of the formula:

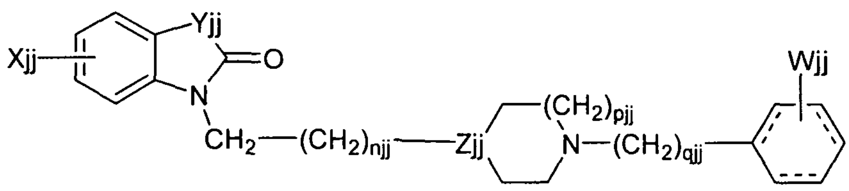

wherein njj is 1, 2 or 3; pjj is 1 or 2; qjj is 1 or 2; Xjj is independently a hydrogen atom, lower alkyl, aryl, aryloxy, CN, lower alkoxy, halogen, hydroxy, nitro, trifluoromethyl, alkylsulfonamido, NHCORjj (where Rjj is lower alkyl or aryl), $NR_{1jj}R_{2jj}$ (where $R_{1jj}$ and $R_{2jj}$ each is independently hydrogen or lower alkyl, or they taken together may form a ring), $CO_2R_{jj}$ (where $R_{jj}$ is lower alkyl), or in some cases one or more of substituents selected from further lower alkyl-substituted cycloalkyl, cycloalkenyl and bicycloalkyl; Yjj is CO or $CR_{3jj}R_{4jj}$ (where $R_{3jj}$ and $R_{4jj}$ each is independently a hydrogen atom, lower alkyl or lower alkoxy, or they taken together form a cyclic acetal); Zjj is N or CH; and the group of the formula:

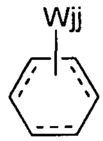

is in some cases a substituted phenyl or cyclohexyl (where Wjj is independently one or more of substituents selected from hydrogen atom, lower alkyl, lower alkoxy and halogen); (provided that the following compounds are excluded: compounds in which njj=1, pjj=1, qjj=1, Xjj=H, Yjj=CO, Zjj=N, and the group of the formula:

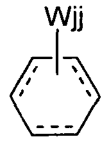

is an unsubstituted phenyl; and compound in which njj=2, pjj=1, qjj=1, Xjj=H, Yjj=CO, Zjj=N, and the group of the formula:

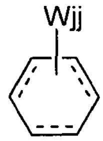

is 4-chlorophenyl); or stereoisomers, optical isomers or racemates thereof, or their salts. Such compounds are exemplified by 5-cyclohexyl-1,3-dihydro-1-[2-[1-(phenylmethyl)-4-piperidinyl]ethyl]-2H-indol-2-one, and the like.

The above-mentioned compounds or salts thereof can be produced according to the process described in JP 7-502272 A (WO 93/12085) or its modification.

12) Compounds of the formula:

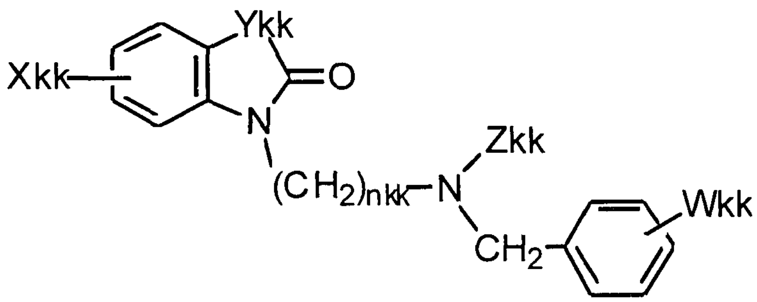

wherein nkk is 3, 4, 5, 6 or 7; Xkk is independently a hydrogen atom, lower alkyl, aryl, lower alkoxy, halogen, trifluoromethyl, nitro, -NHCOR$_{kk}$ (where R$_{kk}$ is lower alkyl or aryl), -NR$_{1kk}$R$_{2kk}$ (where R$_{1kk}$ and R$_{2kk}$ each is independently hydrogen or lower alkyl, or they taken together form a ring), or in some cases one or more of substituents selected from further lower alkyl-substituted cycloalkyl, cycloalkenyl and bicycloalkyl; Ykk is CO or CR$_{3kk}$R$_{4kk}$ (where R$_{3kk}$ and R$_{4kk}$ each is independently a hydrogen atom, lower alkyl or lower alkoxy, or they taken together form a cyclic acetal); Zkk is lower alkyl; and Wkk is one or more of substituents selected from hydrogen atom, lower alkyl, lower alkoxy and halogen; or stereoisomers, optical isomers or racemates thereof, or their salts. Such compounds are exemplified by 5-cyclohexyl-1,3-dihydro-1-[5-(N-ethyl-N-phenylmethylamino)pentyl]-2H-indol-2-one, 5-cyclohexyl-1-[5-(N-ethyl-N-phenylmethylamino)-pentyl]-1H-indole-2,3-dione, and the like.

The above-mentioned compounds or salts thereof can be produced according to the process described in JP 8-511515 A (WO 94/29272) or its modification.

13) Compounds of the formula (III):

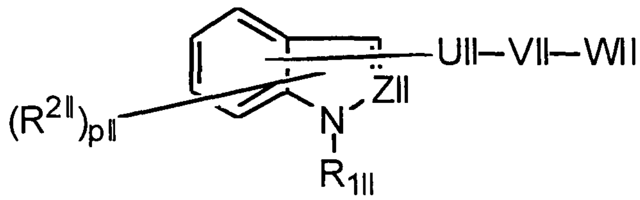

(III)

wherein R$_{111}$ and R$^{211}$ each is hydrogen, a group selected from the following substituent group All, or aryl, aralkyl, aralkyloxycarbonyl, arylamino, arylamino- alkyl, heterocyclic group, heterocyclic alkyl or heterocyclic aminoalkyl which may respectively be substituted by 1 to 3 (same or different) substituents selected from the following substituent group All; pll is an integer of 1 to 3; Ull is a group of the formula: -CO- or -CH(OR$_{311}$)- (where R$_{311}$ is hydrogen or hydroxy-protecting group); Vll is a group of the formula: -(CH=CH)mll- (CH$_2$)nll- (where mll is an integer of 0 to 2; nll is an integer of 0 to 7; provided that mll and nll are not 0 concurrently); W11 is a nitrogen-containing heterocyclic group which has an attaching point with V11 on the endocyclic nitrogen atom, a group of the formula (211) :

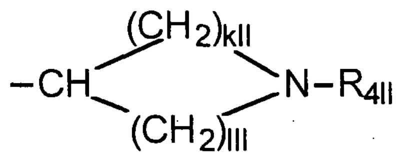

(2ll)

(wherein kll and 111 are the same or different representing 1 to 4; R$_{411}$ has the same significance as in R$_{511}$ and R$_{611}$ as mentioned below), or, in the above-mentioned general formula (211), when the cyclic alkylene forms 5- or 6-membered ring, a group in which said ethylene of the 5- or 6-membered ring is condensed with 1 or 2 benzene rings, or a group of the formula: -NR$_{511}$R$_{611}$ (where R$_{511}$ and R$_{611}$ each is hydrogen, a group selected from the following substituent group All, or an aryl, arylcarbonyl, aralkyl, heterocyclic or heterocyclic alkyl which may be substituted by 1 to 3 substituents selected from the following substituent group All);

The substituent group All: Lower alkyl, cycloalkyl, aryl, heterocyclic group, aralkyl, halogen, amino, lower alkylamino, arylamino, amino lower alkyl, lower alkylaminoalkyl, lower alkynylaminoalkyl, nitro, cyano, sulfonyl, lower alkylsulfonyl, halogenoalkylsulfonyl, lower alkanoyl, arylcarbonyl, arylalkanoyl, lower alkoxy, lower alkoxycarbonyl, halogeno-lower

alkyl, N-lower alkynyl, N-cyanoamino, N-lower alkynyl and N-methylaminomethyl; or salts thereof. Such compounds are exemplified by 1-methyl- 3-[3-(1-benzyl-4-piperidyl)propionyl]indole, 1-methyl-3-[3-[1-(3-fluorobenzyl)-4-piperidyl] propionyl]-5-fluoroindole, 1-methyl-3-[3-[1-(2-chlorobenzyl)-4-piperidyl]propionyl]-indazole, and the like.

The above-mentioned compounds or salts thereof can be produced according to the process as described in JP 6-41070 A (EP-A 562832) or its modification.

14) Compounds of the formula:

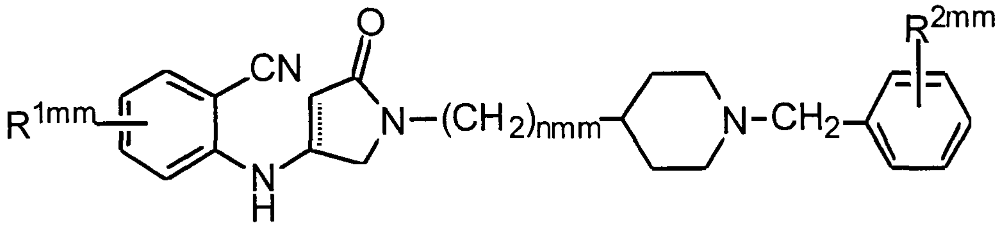

wherein $R^{1mm}$ is hydrogen, halogen, alkyl, alkoxy or alkylthio; $R^{2mm}$ is hydrogen, halogen, alkyl or alkoxy; nmm is an integer of 0 - 7; the broken line indicates the optional presence of a double bond, or salts thereof. Such compounds are exemplified by N-[1-[4-(1-benzylpiperidyl)ethyl]-2-oxo-3-pyrrolin-4-yl]-2-aminobenzonitrile, N-[1-[4-(1-benzylpiperidyl) propyl]-2-oxo-3-pyrrolin-4-yl]-2-aminobenzonitrile, and the like.

The above-mentioned compounds or salts thereof can be produced according to the process described in JP 5-9188 A or its modification.

15) Compounds of the formula:

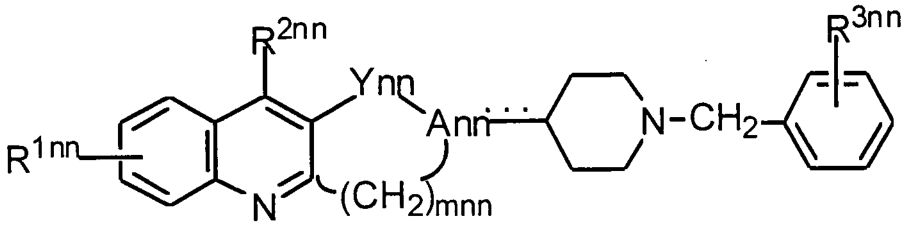

wherein >Ann $\cdots$ represents >N-$(CH_2)$nnn-, >C=, >C=CH$(CH_2)$nnn- or >CH $(CH_2)$ nnn- (where nnn is an integer of 0 - 7); Ynn is >C=O or >CHOH; $R^{1nn}$ is hydrogen, halogen, alkyl, alkoxy or alkylthio; $R^{2nn}$ is hydrogen, halogen, hydroxy, alkyl, alkoxy, optionally substituted phenyl, phenoxy, alkanoyl or optionally substituted amino; $R^{3nn}$ is hydrogen, halogen, alkyl or alkoxy; mnn is an integer of 1 - 3; or salts thereof. Such compounds are exemplified by 9-amino- 2-[4-(1-benzylpiperidyl)ethyl]-2,3-dihydropyrrolo[3,4-b]-quinolin-1-one, 9-amino-2-[2-(1-benzylpiperidin-4-yl)ethyl]- 1,2,3,4-tetrahydroacridin-1-one, 9-methoxy-2-[4-(1-benzyl-piperidyl)ethyl]-2,3-dihydropyrrolo[3,4-b]quinoline-1-one, and the like.

The above-mentioned compounds or salts thereof can be produced according to the process described in JP 5-279355 A (EP-A 481429) or its modification.

16) Compounds of the formula:

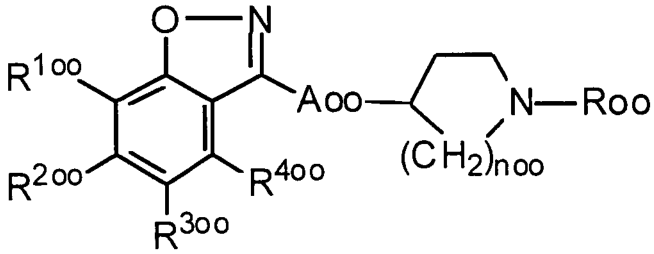

wherein $R_{oo}$ is hydrogen, alkyl, alkenyl, cycloalkylalkyl, phenylalkyl, naphthylalkyl, cycloalkylalkenyl, phenylalkenyl or naphthylalkenyl; $R^{1oo}$, $R^{2oo}$, $R^{3oo}$ and $R^{400}$ are the same or different each representing hydrogen atom, halogen, alkyl,

phenyl, phenyl- alkyl, alkoxy, heteroaryl, heteroarylalkyl, phenylalkoxy, phenoxy, heteroarylalkoxy, heteroaryloxy, acyl, acyloxy, hydroxy, nitro, cyano, - $NHCOR^{5oo}$, $-S(O)_{moo}R^{5oo}$, $-NHSO_2R^{5oo}$, $-CONR^{6oo}R^{7oo}$, $-NR^{6oo}R^{7oo}$, - $OCONR^{6oo}R^{7oo}$, $-OCSNR^{6oo}R^{7oo}$ $-SO_2NR^{6oo}R^{7oo}$ or $-COOR^{8oo}$; or $R^{1oo}$ $R^{2oo}$, $R^{3oo}$ and $R^{4oo}$ are taken together, when they are adjacent each other, to form an optionally substituted $-O(CH_2)$poo-, -O $(CH_2)$ qooO-, -O $(CH_2)$ rooN $(R^{9oo})$ -, -O $(CH_2)$ soo-CON $(R^{9oo})$ -, - $N(R^{9oo})$CO-CH=CH- or a group forming benzene ring or heteroaromatic ring (where $R^{5oo}$ is alkyl, phenyl or phenylalkyl; $R^{6oo}$ and $R^{7oo}$ are the same or different each representing a hydrogen atom, alkyl, phenyl or phenylalkyl, or they taken with the adjacent nitrogen atom may form a heterocycle; $R^{8oo}$ is alkyl, phenyl or phenylalkyl; $R^{900}$ is hydrogen, alkyl, phenylalkyl or acyl; moo is 0, 1 or 2; poo, qoo, roo and soo are the same or different representing 1, 2 or 3); Aoo is a straight or branched chain alkylene; noo is 1, 2 or 3; in the above-mentioned definition, the alkyl, alkenyl, alkoxy, phenyl, phenoxy, cycloalkylalkyl, phenylalkyl, naphthylalkyl, cycloalkylalkenyl, phenylalkenyl, naphthylalkenyl, phenylalkoxy, heteroaryl,

heteroaryloxy, heteroarylalkyl, heteroarylalkoxy, benzene ring and heteroaromatic ring may be substituted by 1 to 3 substituents selected from halogen, alkyl, alkoxy, acyl, acyloxy, hydroxy, nitro, cyano, $-NHCOR^{5oo}$, $-S(O)_{moo}R^{5oo}$, - $NHSO_2R^{5oo}$, $-CONR^{6oo}R^{7oo}$, $-NR^{6oo}R^{7oo}$, $-OCONR^{6oo}R^{7oo}$, $-OCSNR^{6oo}R^{7oo}$, - $SO_2NR^{6oo}R^{7oo}$ or $-COOR^{8oo}$; (where $R^{5oo}$, $R^{6oo}$ , $R^{7oo}$ , $R^{8oo}$ and moo have the same significance as mentioned above); or salts thereof. Such compounds are exemplified by 3-[2-(1-benzyl-4-piperidyl)ethyl]-6,7-dimethoxy-1,2-benzisoxazole, 3-[2-(1-benzyl-4-piperidyl)ethyl]-6-(N-methyl-acetamino)-1,2-benzisoxazole, and the like.

The above-mentioned compounds or salts thereof may be produced according to the process described in JP 5-320160 A (WO 93/04063) or its modification.

17) Compounds of the formula:

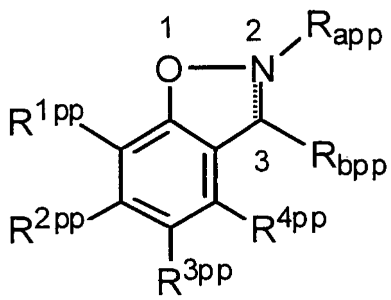

wherein when the bonding between the 2- and 3-positions is a single bond, $R_{app}$ represents a group of the formula:

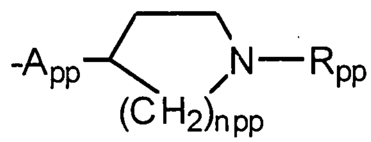

(wherein Rpp is hydrogen, alkyl, alkenyl, cycloalkyl- alkyl, cycloalkylalkenyl, phenylalkyl, phenylalkenyl, naphthylalkyl or naphthylalkenyl; App is straight or branched chain alkylene; npp is 1, 2 or 3), and $R_{bpp}$ is oxygen; when the bonding between the 2- and 3-positions is a double bond, then $R_{app}$ is absent, $R_{bpp}$ represents a group of the formula:

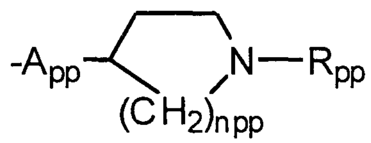

(wherein each symbol has the same significance as mentioned above) or a group of the formula:

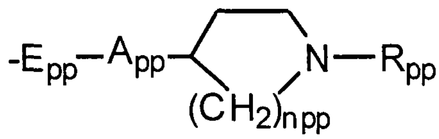

(wherein Epp is oxygen or sulfur, and the other symbols have the same significance as mentioned above); $R^{1pp}$, $R^{2pp}$, $R^{3pp}$ and $R^{4pp}$ are the same or different each representing hydrogen, halogen, alkyl, alkoxy, phenyl, phenylalkyl, phenylalkoxy, phenoxy, heteroaryl, heteroarylalkyl, heteroarylalkoxy, heteroaryloxy, acyl, acyloxy, hydroxy, nitro, cyano, $-NHCOR^{5pp}$, $-S(O)_{mpp}R^{5pp}$, $-NHSO_2R^{5pp}$, $-CONR^{6pp}R^{7pp}$, $-NR^{6pp}R^{7pp}$, $-OCSNR^{6pp}R^{7pp}$, $-SO_2NR^{6pp}R^{7pp}$ or $-COOR^{8pp}$ (where $R^{5pp}$ is alkyl, phenyl or phenylalkyl; $R^{6pp}$ and $R^{7pp}$ are the same or different each representing hydrogen, alkyl, phenyl or phenylalkyl, or they taken together with the adjacent nitrogen atom form a heterocycle; $R^{8pp}$ is hydrogen, alkyl, phenyl or phenylalkyl; mpp is 0, 1 or 2; in the above-mentioned definition, the alkyl, alkenyl, alkoxy, phenyl, phenylalkyl, phenylalkenyl, phenylalkoxy, phenoxy, cycloalkylalkyl, cycloalkylalkenyl, naphthylalkyl, naphthylalkenyl, heteroaryl, heteroarylalkyl, heteroarylalkoxy and heteroaryloxy may be substituted by 1 to 3 substituents selected from halogen, alkyl, alkoxy, acyl, acyloxy, hydroxy, nitro, cyano, $-NHCOR^{5pp}$, $-S(O)mppR^{5pp}$, $-NHSO_2R^{5pp}$, $-CONR^{6pp}R^{7pp}$, $-NR^{6pp}R^{7pp}$, $-OCONR^{6pp}R^{7pp}$, $-OCSNR^{6pp}R^{7pp}$, $-SO_2NR^{6pp}R^{7pp}$ or $-COOR^{8pp}$; (where $R^{5pp}$, $R^{6pp}$, $R^{7pp}$, $R^{8pp}$ and mpp have the same significance as mentioned above); or salts thereof. Such compounds are exemplified by 3-[2-(1-benzyl-4-piperidyl)ethyl]-6,7-dimethoxy-1,2-benzisox-azole, 6-benzoylamino-2-[3-(1-benzyl-4-piperidyl)propyl]-1,2-benzisoxazol-3(2H)-one, 6-benzoylamino-2-[2-(1-benzyl-4-piperidyl)ethyl]-1,2-benzisoxazol-3(2H)-one, and the like.

The above-mentioned compounds or salts thereof can be produced according to the process described in JP 6-41125 A (WO 93/04063) or its modification.

18) Compounds of the formula:

Mqq-Wqq-Yqq-Aqq-Qqq

wherein Mqq is a group of formula:

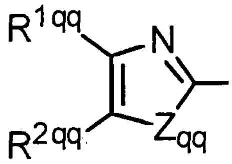

(wherein $R^{1qq}$ is hydrogen, lower alkyl, optionally substituted heterocyclic group or optionally substituted aryl, and $R^{2qq}$ is hydrogen, lower alkyl, optionally substituted heterocyclic group or optionally substituted aryl, or $R^{1qq}$ and $R^{2qq}$ taken each other form a group of the formula:

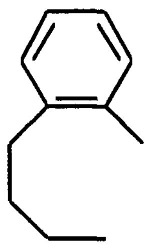

Zqq is S or O), a group of the formula:

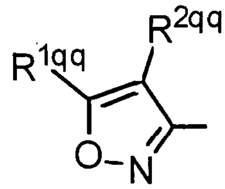

(wherein $R^{1qq}$ and $R^{2qq}$ have the same significance as mentioned above), or a group of the formula:

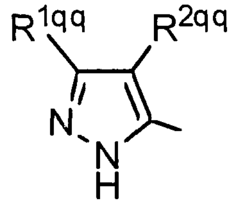

(wherein $R^{1qq}$ and $R^{2qq}$ have the same significance as mentioned above); Wqq is a bonding, lower alkylene or lower alkenylene; Yqq is lower alkylene, -NH-, -CO-, a group of the formula: -CONR$^{3qq}$- (where $R^{3qq}$ is hydrogen or lower alkylene) or a group of the formula: -CHR$^{7qq}$- (where $R^{7qq}$ is hydroxy or protected hydroxy); Aqq is a bonding or lower alkylene; Qqq is a group of the formula: -NR$^{8qq}$R$^{9qq}$ (where $R^{8qq}$ is lower alkyl; $R^{9qq}$ is aryl (lower) alkyl) or a group of the formula:

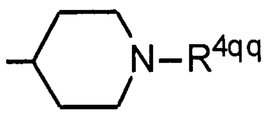

(wherein $R^{4qq}$ is lower alkyl or optionally substituted aryl(lower)alkyl); or salts thereof. Such compounds are exemplified by 4-(pyridin-3-yl)-5-methyl-2-[[2-(1-benzylpiperidin-4-yl)-ethyl]carbamoyl]thiazole, 2-[[2-(1-benzylpiperidin-4-yl)ethyl] carbamoyl]-4-(4-chlorophenyl)-5-methyloxazole, 5-[[2-(1-benzylpiperidin-4-yl)ethyl]carbamoyl]-3-(4-nitrophenyl)pyrazole, and the like.

The above-mentioned compounds or salts thereof can be produced according to the process described in JP 5-345772 A or its modification.

19) Compounds of the formula:

$R_{1rr}$-Qrr-Zrr-Xrr-Arr-Mrr

wherein $R_{1rr}$ is lower alkyl, optionally substituted heterocyclic group, optionally substituted aryl, optionally substituted aryl(lower)alkyl or aryl(lower)alkenyl; Qrr is oxadiazolediyl; Zrr is a bonding or vinyl; Xrr is a bonding, a group of the formula: -CONR$_{4rr}$- (where $R_{4rr}$ is hydrogen or lower alkyl), a group of the formula: -CHR$_{8rr}$- (where $R_{8rr}$ is hydroxy or protected hydroxy), -CO- or -NHCO-; Arr is a bonding, lower alkylene or lower alkenylene; Mrr is a heterocyclic group which may be substituted by a substituent selected from lower alkyl, imino-protecting group and optionally substituted aryl(lower)alkyl and which contains at least one nitrogen atom; or salts thereof. Such compounds are exemplified by 5-(quinuclidin-3-yl)-3-[[2-(1-benzylpiperidin-4-yl)ethyl]-carbamoyl]-1,2,4-oxadiazole, 3-[[2-(1-benzylpiperidin-4-yl)ethyl] carbamoyl]-5-(4-nitrophenyl)-1,2,4-oxadiazole, and the like.

The above-mentioned compounds or salts thereof can be produced according to the process described in JP 7-502529 A (WO 93/13083) or its modification.

20) Compounds of the formula:

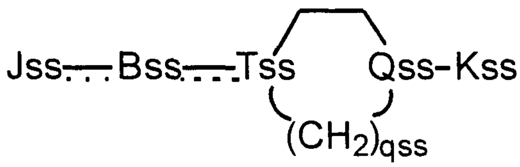

wherein Jss is (a) the following substituted or unsubstituted group: (1) phenyl, (2) pyridyl, (3) pyrazyl, (4) quinolyl, (5) cyclohexyl, (6) quinoxalyl, or (7) furyl, (b) a monovalent or divalent group selected from the following group, of which the phenyl moiety may be substituted: (1) indanyl, (2) indanonyl, (3) indenyl, (4) indenonyl, (5) indanedionyl, (6) tetralonyl, (7) benzsuberonyl, (8) indanolyl, or (9) a group of the formula:

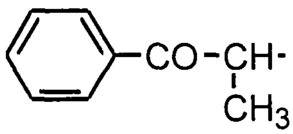

(c) a monovalent group derived from a cyclic amide compound, (d) lower alkyl, or
(e) a group of the formula $R_{1ss}$-CH=CH- (where $R_{1ss}$ is hydrogen or lower alkoxycarbonyl);
Bss is a group of the formula: $-(CHR_{2ss})nss-$, a group of the formula: $-CO-(CHR_{2ss})nss-$, a group of the formula: $-NR_{3ss}-(CHR_{2ss})nss-$ (where $R_{3ss}$ is hydrogen, lower alkyl, acyl, lower alkylsulfonyl, optionally substituted phenyl or benzyl), a group of the formula: $-CO-NR_{4ss}-$ $(CHR_{2ss})$ nss-(where $R_{4ss}$ is hydrogen, lower alkyl or phenyl), a group of the formula: $-CH=CH-(CHR_{2ss})nss-$, a group of the formula: - $O-COO-(CHR_{2ss})nss-$, a group of the formula: $-O-CO-NH-(CHR_{2ss})$ nss-, a group of the formula: $-NH-CO-(CHR_{2ss})nss-$, a group of the formula: $-CH_2-CO-NH-(CHR_{2ss})nss-$, a group of the formula: - $(CH_2)_2-CO-NH-(CHR_{2ss})nss-$, a group of the formula: $-C(OH)H-(CHR_{2ss})nss-$ (in the above formulae, nss indicates 0 or an integer of 1 - 10; $R_{2ss}$ means hydrogen or methyl when the alkylene of the formula $-(CHR_{2ss})nss-$ has no substituent or it has 1 or more of methyl), a group of the formula: =(CH-CH=CH)bss- (where bss is an integer of 1 - 3), a group of the formula: $=CH-(CH_2)css-$ (where css is 0 or an integer of 1 - 9), a group of the formula: =(CH-CH) dss= (where dss is 0 or an integer of 1 - 5), a group of the formula: $-CO-CH=CH-$ $CH_2-$, a group of the formula: $-CO-CH_2-C(OH)H-CH_2-$, a group of the formula: $-C(CH_3)H-CO-NH-CH_2-$, a group of the formula: -CH=CH- $CO-NH-(CH_2)_2-$, a group of the formula: -NH-, a group of the formula: -O-, a group of the formula: -S-, dialkylaminoalkyl- carbonyl group or lower alkoxycarbonyl;
Tss is nitrogen or carbon atom;
Qss is nitrogen, carbon or a group of the formula >N→O;
Kss is hydrogen, substituted or unsubstituted phenyl, arylalkyl of which the phenyl moiety may be substituted, cinnamyl of which the phenyl moiety may be substituted, lower alkyl, pyridylmethyl, cycloalkylalkyl, admantanemethyl, furylmethyl, cycloalkyl, lower alkoxy-carbonyl or acyl;
qss is an integer of 1 - 3;
--- indicates a single bond or double bond; or salts thereof. Such compounds are exemplified by 1-benzyl-4-[(5,6-dimethoxy-1-indanon)-2-yl]methylpiperidine, N-[4'-(1'-benzylpiperidyl)ethyl]-2-quinoxalinecarboxylic amide, 4-[4'-(N-benzyl) piperidyl]-p-methoxybutyrophenone, 1-[4'-(1'-benzylpiperidin)ethyl]-1,2,3,4-tetrahydro-5H-1-benzazepin-2-one, and the like.

The above-mentioned compounds or salts thereof can be produced according to the process described in JP 64-79151 A (USP 4,895,841) or its modification.

21) Compounds of the formula:

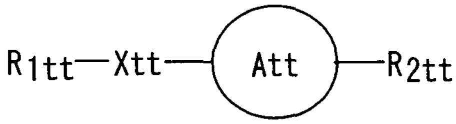

wherein $R_{1tt}$ is a monovalent group derived from a compound selected from optionally substituted benzene, pyridine, pyrazine, indole, anthraquinone, quinoline, optionally substituted phthalimide, homophthalimide, pyridinecarboxylic imide, pyridine-N-oxide, pyrazinecarboxylic imide, naphthalenedicarboxylic imide, optionally substituted quinazolidinedione, 1,8-naphthalimide, bicyclo[2.2.2]oct-5-ene-2,3-dicarboxylic imide and pyromellic imide; Xtt is a group of the formula:

$-(CH_2)$mtt- (where mtt is an integer of 0 - 7), a group of the formula: $-O(CH_2)$ntt-, a group of the formula: $-S(CH_2)$ntt-, a group of the formula: $-NH(CH_2)$ntt-, a group of the formula: $-SO_2NH(CH_2)$ntt a group of the formula: -NHCO $(CH_2)$ ntt-, a group of the formula: $-NH(CH_2)$ntt-CO-, a group of the formula: $-COO(CH_2)$ntt-, a group of the formula: $-CH_2NH$ $(CH_2)$ ntt-, a group of $-CONR_{3tt}-(CH_2)$ntt- (in the definition of Xtt, all of ntt in the formulae indicate an integer of 1 - 7; $R_{3tt}$ is lower alkyl or benzyl group), a group of the formula: $-O-CH_2CH_2CH(CH_3)-$, a group of the formula: $-O-CH(CH_3)CH_2CH_2-$, a group of the formula: $-O-CH_2CH_2CH=$, a group of the formula: $-O-CH_2CH(OH)CH_2-$; the ring Att represents a group of the formula:

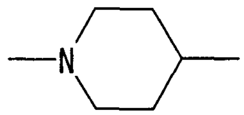

a group of the formula:

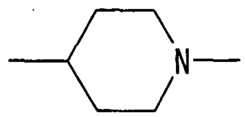

a group of the formula:

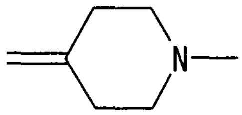

a group of the formula:

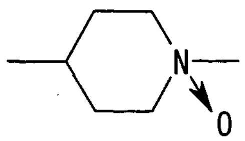

$R_{2tt}$ is hydrogen, lower alkyl, optionally substituted benzyl, optionally substituted benzoyl, pyridyl, 2-hydroxyethyl, pyridylmethyl, or a group of the formula:

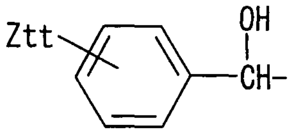

(wherein Ztt means halogen); or salts thereof. Such compounds are exemplified by N-methyl- N-[2-(1'-benzylpiperidin-4'-yl)ethyl]-4-benzylsulfonylbenz-amide, N-[2-(N'-benzylpiperidin-4'-yl)ethyl]-4-nitrophthal-imide, N-[2-(N'-benzylpiperidin-4'-yl)ethyl]-1,8-naphthal-imide, and the like.

The above-mentioned compounds or salts thereof can be produced according to the process described in JP 62-234065 A (EP-A 229391) or its modification.

22) Compounds of the formula:

$R^{1uu}$- $(CH_2)$ nuu-Zuu

wherein $R^{1uu}$ is an optionally substituted group derived from cyclic amide compounds; nuu is 0 or an integer of 1 - 10; Zuu is (1) a group of the formula:

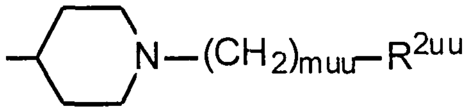

(wherein $R^{2uu}$ is optionally substituted aryl, cycloalkyl or heterocyclic group; muu is an integer of 1 - 6) or (2) a group of the formula:

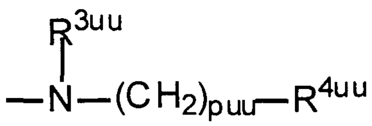

(wherein $R^{3uu}$ is hydrogen or lower alkyl; $R^{4uu}$ is optionally substituted aryl, cycloalkyl or heterocyclic group; puu is an integer of 1 - 6); provided that the following cases are excluded: when the optionally substituted cyclic amide compound is quinazolidinone or quinazolidinedione in the definition of $R^{1uu}$, and when $R^{2uu}$ and $R^{4uu}$ are aryl in the definition of Zuu; or salts thereof. Such compounds are exemplified by 3-[2-(1-benzyl-4-piperidyl)ethyl]-5-methoxy-2H-3,4-dihydro-1,3-benzoxazin-2-one, 3-[2-[1-(4-pyridylmethyl)-4-piperidyl]-ethyl]-2H-3,4-dihydro-1,3-benzoxazin-2-one, 3-[2-[1-(1,3-dioxolan-2-ylmethyl)-4-piperidyl]ethyl]-5-methoxy-1,2,3,4-tetrahydroquinazoline-2,4-dione, 3-[2-(1-benzyl-4-piperidyl)ethyl]-6-methoxy-2H-3,4-dihydro-1,3-benzoxadine-2,4-dione, and the like.

The above-mentioned compounds or salts thereof can be produced according to the process described in JP 4-235161 A (EP-A 468187) or its modification.

23) An optically active indanone derivative of the formula:

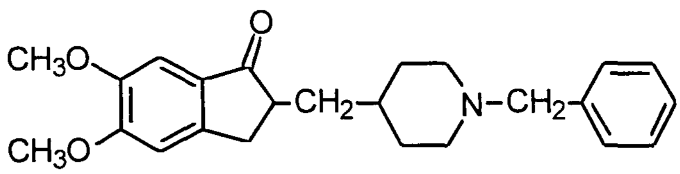

or salts thereof.

The above-mentioned compound or salts thereof can be produced according to the process described in JP 4-21670 A or its modification.

24) Compounds of the formula:

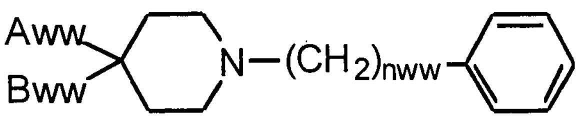

wherein nww is 0 or an integer of 1 or 2; Aww is a group of the formula:

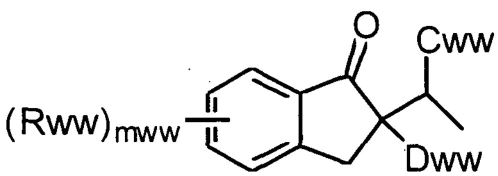

(wherein Cww is hydrogen or hydroxy; Dww is hydrogen or lower hydroxyalkyl; Rww is the same or different representing a group selected from hydrogen atom, lower alkyl and lower alkoxy; mww is 0 or an integer of 1 - 4) or a group of the formula:

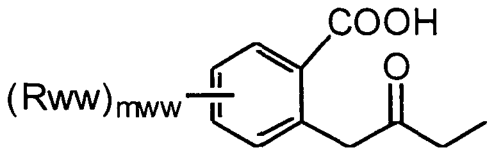

(wherein each symbol has the same significance as mentioned above); Bww is hydrogen or hydroxy; or alternatively, Aww and Bww taken together form a double bond to form a group of the formula:

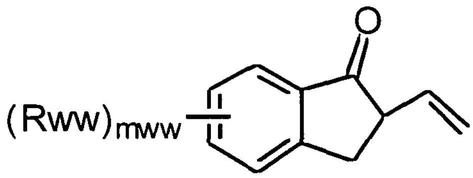

(wherein each symbol has the same significance as mentioned above); or salts thereof. Such compounds are exemplified by 1-benzyl-4- (5,6-dimethoxy-1-indanon-2-yl)hydroxymethylpiperidine, 1-benzyl-4-(5,6-dimethoxy-2-hydroxymethyl-1-indanon-2-yl)-methylpiperidine, 1-benzyl-4-[3-(4,5-dimethoxy-2-carboxy-phenyl)-2-oxo]propylpiperidine, and the like.

The above-mentioned compounds or salts thereof can be produced according to the process described in JP 9-268176 A or its modification.

25) Compounds of the formula:

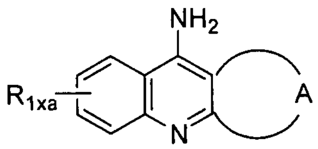

wherein $R_{1xa}$ is hydrogen, halogen, hydroxy, lower alkoxy, lower alkyl or mono(or di or tri)halo(lower)alkyl; the group of the formula:

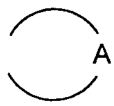

represents a moiety of the formula:

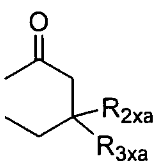 , 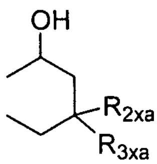 , 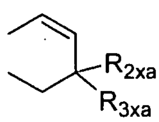 ,

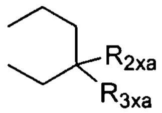,
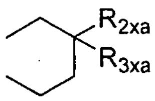
or
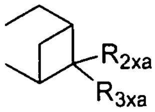

(wherein $R_{2xa}$ and $R_{3xa}$ each is lower alkyl); or salts thereof. Such compounds are exemplified by 9-amino-6-chloro-3,3-dimethyl-1,2,3,4-tetrahydroacridine, and the like.

The above-mentioned compounds or salts thereof can be produced according to the process described in JP 2-167267 A or its modification.

26) Aminoazaacridine derivatives of the formula:

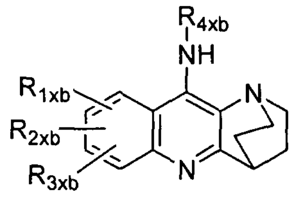

wherein $R_{1xb}$, $R_{2xb}$ and $R_{3xb}$ each is hydrogen, halogen, trifluoromethyl, lower alkyl, lower cycloalkyl, lower alkoxy, lower alkoxymethyl, lower alkylthio, nitro, amino, lower alkanoylamino, lower alkylamino, hydroxy, phenyl or phenyl substituted by halogen, lower alkyl or lower alkoxy; $R_{4xb}$ is hydrogen, lower alkyl, aralkyl, diaralkyl, or a group of the formula: $R_{5xb}$-CO- ($R_{5xb}$ is lower alkyl, lower cycloalkyl, aralkyl, phenyl or phenyl substituted by halogen, lower alkyl or lower alkoxy); or salts thereof. Such compounds are exemplified by 9-amino- 8-fluoro-1,2,3,4-tetrahydro-1,4-ethano-1-azaacridine, and the like.

The above-mentioned compounds or salts thereof can be produced according to the process described in JP 63-166881 A or its modification.

27) Compounds of the formula:

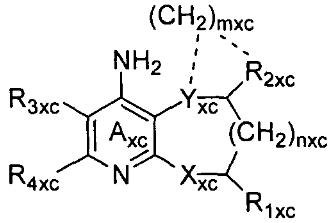

wherein $R_{1xc}$ is hydrogen or lower alkyl; $R_{2xc}$ is independently hydrogen or lower alkyl, or it taken with $R_{6xc}$ forms a cyclic alkylene chain; $R_{3xc}$ and $R_{4xc}$ each is independently hydrogen, or they taken together with the ring $A_{xc}$ form a quinoline ring or tetrahydroquinoline ring; $X_{xc}$ is oxygen, sulfur or N-$R_{5xc}$, and $R_{5xc}$ is hydrogen or lower alkyl; $Y_{xc}$ is oxygen or N-$R_{6xc}$, and $R_{6xc}$ is independently hydrogen or lower alkyl, or it taken with $R_{2xc}$ forms a cyclic alkylene; nxc is 0 or 1; mxc is an integer of 0 - 4; or salts thereof. Such compounds are exemplified by 4'-amino- quinolino[2,3-b]-4-methyl-5,6-dihydro-1,4-oxazine, 4'-amino- 5',6',7',8'-tetrahydroquinolino[2,3-b]-4-methyl-5,6-dihydro-1,4-oxazine, and the like.

The above-mentioned compounds or salts thereof can be produced according to the process described in JP 2-96580 A or its modification.

28) Compounds of the formula:

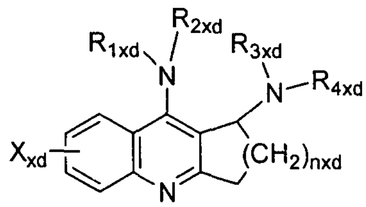

wherein nxd is 1, 2 or 3, and Xxd is hydrogen, lower alkyl, lower alkoxy, halogen, hydroxy, nitro or trifluoromethyl; $R_{1xd}$ and $R_{2xd}$ each is independently hydrogen, lower alkyl or aryl lower alkyl, but they cannot be aryl lower alkyl concurrently; $R_{3xd}$ and $R_{4xd}$ each is independently hydrogen, lower alkyl, aryl lower alkyl, formyl or lower alkylcarbonyl, or the group $-NR_{3xd}R_{4xd}$ represents the following group:

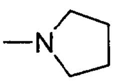 , 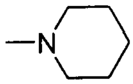 , 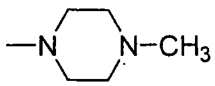 , 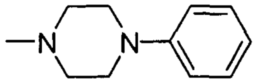

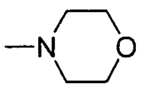 , 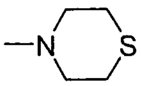 or 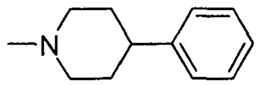

as a whole; or stereoisomers thereof or their salts. Such compounds are exemplified by 1-(1-piperidinyl)-1,2,3,4-tetrahydro-9-acridinamine, N-1-ethyl-1,2,3,4-tetrahydro-1,9-acridine-diamine, and the like.

The above-mentioned compounds or salts thereof can be produced according to the process described in JP 3-153667 A or its modification.

29) Compounds of the formula:

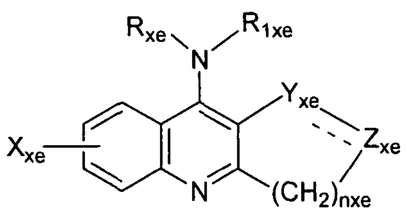

wherein nxe is 1, 2 or 3; $X_{xe}$ is hydrogen, $C_1$-$C_6$ alkyl, $C_1$-$C_6$ alkoxy, halogen, hydroxy, nitro, trifluoromethyl, $NHCOR_{2xc}$ (where $R_{2xc}$ is $C_1$-$C_6$ alkyl) or $NR_{3xc}R_{4xc}$ (where $R_{3xc}$ and $R_{4xc}$ are independently hydrogen or $C_1$-$C_6$ alkyl); $R_{xc}$ is hydrogen or $C_1$-$C_6$ alkyl; $R_{1xc}$ is hydrogen, $C_1$-$C_6$ alkyl, di-$C_1$-$C_6$ alkylamino-$C_1$-$C_6$ alkyl, aryl-$C_1$-$C_6$ alkyl, diaryl-$C_1$-$C_6$ alkyl, furyl-$C_1$-$C_6$ alkyl, thienyl-$C_1$-$C_6$ alkyl, oxygen-bridged aryl-$C_1$-$C_6$ alkyl, oxygen-bridged diaryl-$C_1$-$C_6$ alkyl, oxygen-bridged furyl-$C_1$-$C_6$ alkyl, or oxygen-bridged thienyl-$C_1$-$C_6$ alkyl; $Y_{xe}$ is C=O or $CR_{5xc}OH$ (where $R_{5xc}$ is hydrogen or $C_1$-$C_6$ alkyl) ; and $Z_{xe}$ is $CH_2$ or $C=CR_{6xc}R_{7xc}$ (where $R_{6xc}$ and $R_{7xc}$ are independently hydrogen or $C_1$-$C_6$ alkyl), or $Y_{xe}$ and $Z_{xe}$ taken together form $CR_{5xc}$=CH (where $CR_{5xc}$ and CH respectively correspond to $Y_{xe}$ and $Z_{xe}$); or optical antipodes thereof or their salts. Such compounds are exemplified by 9-amino-3,4-dihydroacridin-1(2H)-one, 9-amino-1,2,3,4-tetrahydroacridin-1-ol, and the like.

The above-mentioned compounds or salts thereof can be produced according to the process described in JP 61-148154 A or JP 5-41141 B or its modification.

30) Compounds of the formula:

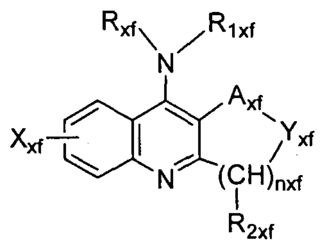

wherein nxf is 1 - 4; $R_{xf}$ is hydrogen, lower alkyl or lower alkylcarbonyl; $R_{1xf}$ is hydrogen, lower alkyl, lower alkyl-carbonyl, aryl, di (lower) alkylamino (lower) alkyl, aryl lower alkyl, diaryl lower alkyl, oxygen-bridged aryl lower alkyl, or oxygen-bridged diaryl lower alkyl; Axf is a direct bonding or $(CHR_{3xf})$mxf; mxf is 1 - 3; Xxf is hydrogen, lower alkyl, cycloalkyl, lower alkoxy, halogen, hydroxy, nitro, trifluoromethyl, formyl, lower alkylcarbonyl, arylcarbonyl, -SH, lower alkyl- thio, $-NHCOR_{4xf}$ or $NR_{5xf}R_{6xf}$; in the above formulae, $R_{4xf}$ is hydrogen or lower alkyl; $R_{5xf}$ and $R_{6xf}$ each is independently hydrogen, lower alkyl or cycloalkyl; Yxf is O, S or $NR_{7xf}$; each $R_{2xf}$, each $R_{3xf}$ and $R_{7xf}$ are independently hydrogen or lower alkyl, or two of them concurrently form a methylene or ethylene group which constitutes a moiety of a ring comprising at least 5 atoms; provided that when Axf is $CH_2$, Yxf is $NCH_3$, $(CHR_{2xf})$nxf is $CH_2CH_2$, Xxf is H, $CH_3$, Cl, Br or $NO_2$, and $R_{xf}$ is H, then $R_{1xf}$ is neither H, methyl, ethyl, propyl, butyl nor benzyl; when Axf is $-CH_2-$ or CHR'-, Yxf is NH or NR', and $(CHR_{2xf})$ nxf is $-CH_2CH_2-$ or $CH_2CHR'$ -, then the group $-NR_{xf}R_{1xf}$ is neither $-NH_2$, $-NHC_6H_5$ nor di(lower) alkylamino (lower) alkylamino, and each R' is independently lower alkyl; when Axf is $CH_2$, Yxf is NH or NR', and $(CHR_{2xf})$ nxf is $-(CH_2)_3-$ or $CHR'CH_2CH_2-$, then the group $-NR_{xf}R_{1xf}$ is not $-NH_2$; when Axf is $-CH_2CH_2-$, Yxf is NH or NR', and $(CHR_{2xf})$ nxf is $-CH_2CH_2-$ or $CHR'CH_2-$, then the group $-NR_{xf}R_{1xf}$ is not $-NH_2$; or optical or geometrical isomers thereof or their salts. Such compounds are exemplified by 9-amino-2,3-dihydrothieno[3,2-b]quinoline, 10-amino-3,4-dihydro-1H-thiopyrano[4,3-b]-quinoline, and the like.

The above-mentioned compounds or salts thereof can be produced according to the process described in JP 63-284175 A or its modification.

31) Compounds of the formula:

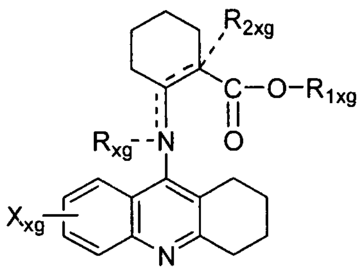

wherein Xxg is hydrogen, lower alkyl, lower alkoxy or halogen; $R_{xg}$ is, when it is present, hydrogen, lower alkyl or aryl lower alkyl; $R_{1xg}$ is hydrogen, lower alkyl or aryl lower alkyl; and $R_{2xg}$ is, when it is present, hydrogen or lower alkyl; or salts thereof. Such compounds are exemplified by 2-(1,2,3,4- tetrahydro-9-acridinimino)cyclohexanecarboxylic acid, ethyl 2-(1,2,3,4-tetrahydro-9-acridinimino)cyclohexanecarboxylate, and the like.

The above-mentioned compounds or salts thereof can be produced according to the process described in JP 3-95161 A or its modification.

32) Compounds of the formula:

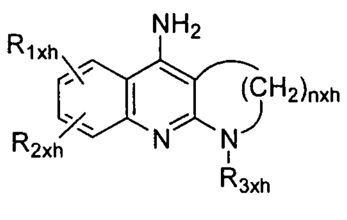

wherein $R_{1xh}$ and $R_{2xh}$ each is hydrogen, halogen, lower alkyl, trifluoromethyl, hydroxy, lower alkoxy, lower alkanoyloxy,

nitro, amino or lower alkanoylamino; $R_{3xh}$ is hydrogen, alkyl of 1 - 15 carbon atoms, cycloalkyl, aralkyl of 7 - 15 carbon atoms optionally substituted by halogen, lower alkyl or lower alkoxy, alkanoyl of 2 - 15 carbon atoms, or benzoyl which may be substituted by halogen, lower alkyl, lower alkoxy, nitro, hydroxy or amino; nxh is an integer of 2 - 5; or salts thereof. Such compounds are exemplified by 6-amino-1-benzyl-2,3,4,5-tetrahydro-1H-azepino[2,3-b]quinoline, 5-amino-6-fluoro-1,2,3,4-tetrahydrobenzo[d][1,8]naphthyridine, and the like.

The above-mentioned compounds or salts thereof can be produced according to the process described in JP 3-220189 A or its modification.

33) 4-Amino-5,6,7,8-tetrahydrothieno[2,3-b]quinoline derivatives of the formula:

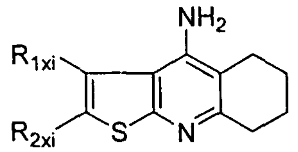

wherein $R_{1xi}$ and $R_{2xi}$ each is hydrogen or straight or branched chain alkyl of 1 - 4 carbon atoms, provided that they are not hydrogen concurrently; or salts thereof. Such compounds are exemplified by 4-amino- 2,3-dimethyl-5,6,7,8-tetrahydrothieno[2,3-b]quinoline, and the like.

The above-mentioned compounds or salts thereof can be produced according to the process described in JP 4-134083 A or its modification.

34) 4-Amino-2,3-cycloalkenopyridine and 4-aminoquinoline derivatives of the formula:

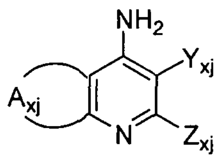

wherein Axj represents alkylene of the formula $-(CH_2)$nxj-(where nxj is an integer of 3 - 5), which is bound to two adjacent carbon atoms on the adjacent pyridine nucleus to form a cycloalkenone group or which is associated with two adjacent carbon atoms on the adjacent pyridine nucleus to form a benzene ring; and (i) when Axj forms a cycloalkenone group, then Yxj represents hydrogen, halogen, $C_{1-6}$ lower alkyl or amino, and Zxj represents hydrogen, hydroxy, halogen, amino, a group of the formula $-NR_{1xj}R_{2xj}$ ($R_{1xj}$ and $R_{2xj}$ are the same or different representing lower alkyl or benzyl), pyrrolidyl, piperidyl, piperazyl, N-substituted piperazyl, pyridyl, or a group of the formula:

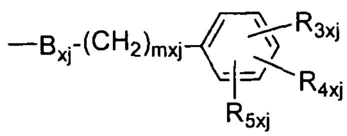

(wherein B is oxygen or sulfur; mxj is an integer of 0 - 2; $R_{3xj}$, $R_{4xj}$ and $R_{5xj}$ are the same or different representing hydrogen, halogen, trifluoromethyl, hydroxy, lower alkoxy, straight or branched ($C_1$-$C_6$) lower alkyl, amino, or acylamino), or Zxj represents pyridylthio; and (ii) when Axj forms a benzene ring, then Yxj represents hydrogen or $C_1$-$C_6$ lower alkyl, and Zxj represents a group of the formula $-CONR_{6xj}R_{7xj}$ (where $R_{6xj}$ and $R_{7xj}$ each is hydrogen or $C_1$-$C_6$ lower alkyl, or alternatively $R_{6xj}$ and $R_{7xj}$ are taken together to form a $C_3$-$C_6$ cycloalkyl), or Zxj represents a group of the formula:

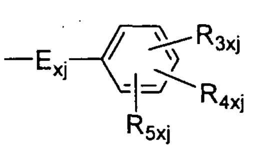

wherein Exj is $C_2$-$C_6$ alkylene or a group of the formula - (CH=CH)pxj- (where pxj is 1 or 2), and $R_{3xj}$, $R_{4xj}$ and $R_{5xj}$ have the same significance as mentioned above; or salts thereof. Such compounds are exemplified by 4-amino- 2-(N-methylcarbamoyl)quinoline, and the like.

The above-mentioned compounds or salts thereof can be produced according to the process described in JP 4-66571 A or its modification.

35) Polycyclic aminopyridine compounds of the formula:

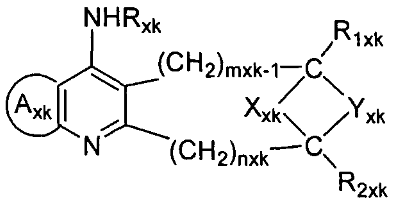

wherein $R_{xk}$ is hydrogen, alkyl, aralkyl or acyl; $R_{1xk}$ and $R_{2xk}$ each is independently hydrogen, alkyl, aralkyl, alkoxy, alkoxy-carbonyl, amino or amino substituted by 1 or 2 of alkyl, aralkyl or acyl; mxk and nxk each is 1, 2 or 3; Xxk and Yxk each is independently a bonding between two carbon atoms, oxygen or sulfur, a group N-$R_{3xk}$ (where the group $R_{3xk}$ and $R_{xk}$ have the same significance as mentioned above), or an alkylene or alkenylene crosslink which contains 1 - 5 carbon atoms and may contain 1 or more of the substituent $R_{4xk}$ (where $R_{4xk}$ is independently hydrogen, straight or branched chain lower alkyl of 1 - 4 carbon atoms, alkenyl or alkylidene, phenyl or phenyl which is substituted by 1 or more of lower alkyl of 1 - 4 carbon atoms, lower alkoxy of 1 - 4 carbon atoms or halogen, aralkyl, lower alkoxy of 1 - 4 carbon atoms, or hydroxy); and when Yxk is alkenylene, the latter can be condensed with a saturated or unsaturated carbocyclic or heterocyclic ring, and the above-mentioned ring may be substituted by 1 or more of groups of $R_{5xk}$ ($R_{5xk}$ is hydrogen, lower alkyl or lower alkoxy of 1 - 4 carbon atoms, or halogen); and the group of formula:

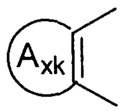

represents a moiety of the formula:

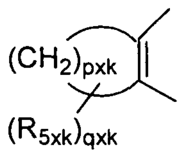

or

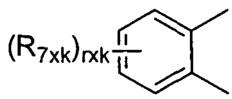

;pxk, qxk and rxk each is 1 or more; and $R_{6xk}$ or $R_{7xk}$ may be independently hydrogen, halogen, lower alkoxy or lower alkyl; or salts thereof. Such compounds are exemplified by (+)-12-amino-6,7,10,11-tetrahydro-9-ethyl-7,11-methanocycloocta-[b]quinoline, (+)-12-amino-6,7,10,11-tetrahydro-9-methyl-7,11-methanocycloocta[b]quinoline, and the like.

The above-mentioned compounds or salts thereof can be produced according to the process described in JP 11-500144 A or its modification.

36) Compounds of the formula:

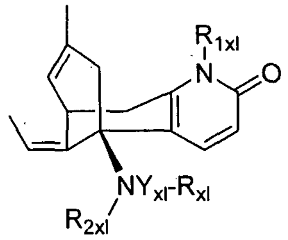

wherein $Y_{x1}$ is -C=O or $-R_{2x1}$; Y is =CH; $R_{x1}$ is $C_1$-$C_5$ lower alkyl, a group of the formulae:

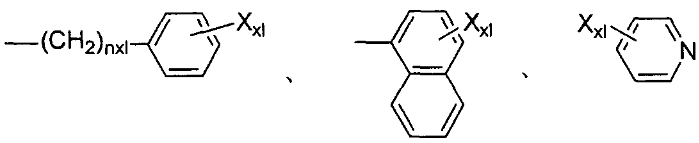

(where nxl = 0 or 1; Xxl is hydrogen, $C_1$-$C_5$ lower alkyl, $C_1$-$C_5$ lower alkoxy, nitro, halogen, carboxy, alkoxycarbonyl, hydroxymethyl, hydroxy, bis-$C_1$-$C_5$ lower alkyl-substituted amino), $-(CH_2)_{mxl}COOZxl$ (where mxl = 0 - 5; Zxl is hydrogen or $C_1$-$C_5$ lower alkyl), -CH=CH-Gxl (where Gxl is phenyl, furanyl, carboxy, or alkoxycarbonyl), and dihydro- or tetrahydro-pyridyl substituted by $C_1$-$C_5$ lower alkyl at the nitrogen atom; $R_{1x1}$ is hydrogen, $C_1$-$C_5$ lower alkyl, pyridoyl and $C_1$-$C_5$ lower alkoxy-substituted benzoyl; $R_{2x1}$ is hydrogen or $C_1$-$C_5$ lower alkyl; or salts thereof. Such compounds are exemplified by one of the formula:

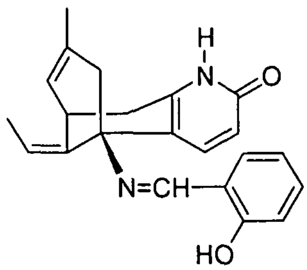

and the like.

The above-mentioned compounds or salts thereof can be produced according to the process described in JP 10-511651 A or its modification.

37) Compounds of the formula:

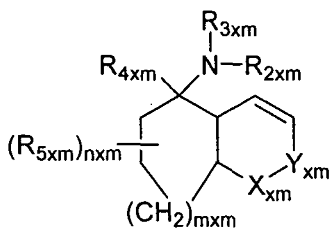

wherein Xxm-Yxm is a group of the formula:

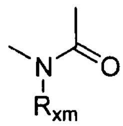

(wherein $R_{xm}$ is hydrogen, lower alkyl, lower alkenyl, lower alkynyl or aryl lower alkyl) or a group of the formula:

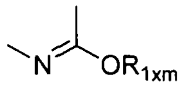

(wherein $R_{1xm}$ is hydrogen, lower alkyl or aryl lower alkyl); $R_{2xm}$ and $R_{3xm}$ each is independently hydrogen, lower alkyl, aryl lower alkyl, diaryl lower alkyl, lower cycloalkenyl lower alkyl, lower alkoxy, aryl lower alkoxy or lower alkanoyl, or $R_{2xm}$ and $R_{3xm}$ taken with the attached nitrogen atom form a group of the formula:

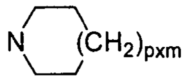

(wherein pxm is 0 or 1) or a group of the formula:

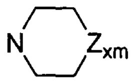

(wherein Zxm is O, S or a group of the formula $NR_{6xm}$ ($R_{6xm}$ is hydrogen, lower alkyl or aryl lower alkyl)); $R_{4xm}$ is hydrogen, lower alkyl or aryl lower alkyl; $R_{5xm}$ is hydrogen, lower alkyl or aryl lower alkyl; mxm is 0, 1 or 2; and nxm is 1 or 2; or geometrical and optical isomers thereof or their salts. Such compounds are exemplified by N-(1,2,5,6,7,8-hexahydro-5-methyl-2-oxo-5-quinolinyl)acetamide, 5-[[2-(3,4-dichlorophenyl)ethyl]amino]-5,6,7,8-tetrahydro-1-methyl-2 (1H)-quinoline, and the like.

The above-mentioned compounds or salts thereof can be produced according to the process described in JP 4-290872 A or its modification.

38) Compounds of the formula:

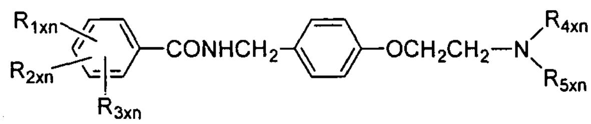

wherein $R_{1xn}$, $R_{2xn}$ and $R_{3xn}$ each is hydrogen, lower alkyl, lower alkoxy, hydroxy, halogen, nitro, cyano, amino optionally substituted by lower alkyl, or sulfamoyl optionally substituted by lower alkyl, or $R_{1xn}$ and $R_{2xn}$ taken together form methylenedioxy; $R_{4xn}$ and $R_{5xn}$ each is lower alkyl or cycloalkyl of 3 to 6 carbon atoms, or they taken together with the attached nitrogen atom may form 1-pyrrolidinyl, 1-piperidinyl, 1-piperazinyl or 4-morpholinyl, each of which may be substituted by lower alkyl; or salts thereof. Such compounds are exemplified by N-[4-[2-(dimethylamino)ethoxy]benzyl]-2-ethoxybenzamide, 4-amino-N-[4-[2-(dimethylamino)ethoxy]benzyl]-2-methoxy-5-sulfamoyl-benzamide, and the like.

The above-mentioned compounds or salts thereof can be produced according to the process described in JP 2-231421 A or its modification.

39) Compounds of the formula:

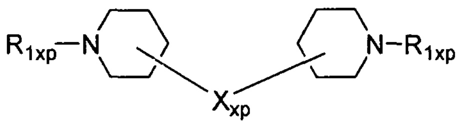

wherein Xxp is straight or branched chain alkylene of 1 - 10 carbon atoms or a group of the formula:

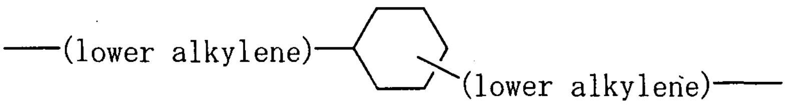

or of the formula:

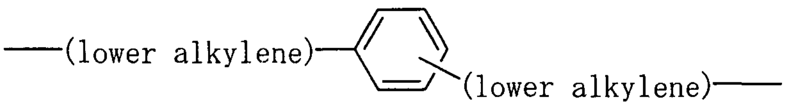

$R_{1xp}$ is Arxp-$CHR_{2xp}$ (where Arxp is unsubstituted phenyl or phenyl substituted by halogen, trifluoromethyl, lower alkyl or lower alkoxy; $R_{2xp}$ is hydrogen or lower alkyl), cinnamyl of which the phenyl moiety is unsubstituted or substituted by halogen, lower alkyl or lower alkoxy, a cycloalkylmethyl, or methyl substituted by heterocyclic aromatic group; and when one linkage of X to the two piperidine rings is placed at the 2-position, the other is at the 2'-position, and when one is at the 3-position, the other is at the 3'-position, and when one is at the 4-position, the other is at the 4'-position; or salts thereof. Such compounds are exemplified by 1,6-di-(1-benzyl-4-piperidyl)hexane, 1,5-di-(1-benzyl-4-piperidyl)pentane, and the like.

The above-mentioned compounds or salts thereof can be produced according to the process described in JP 4-18071 A or its modification.

40) Compounds of the formula:

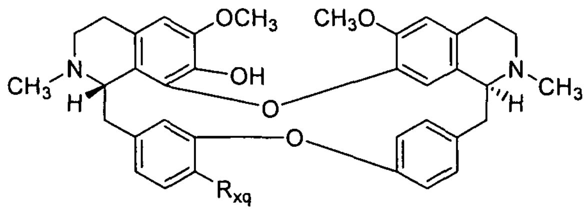

wherein Rxq is hydroxy or methoxy, or salts thereof.

The above-mentioned compounds or salts thereof can be produced according to the process described in JP 4-159225 A or its modification.

41) 9-Amino-1,2,3,4-tetrahydroacridine represented by the following formula or salts thereof.

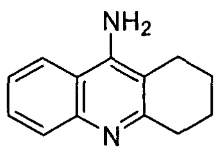

The above-mentioned compound or salts thereof can be produced according to the process described in JP 4-346975 A or its modification.

42) Compounds of the formula:

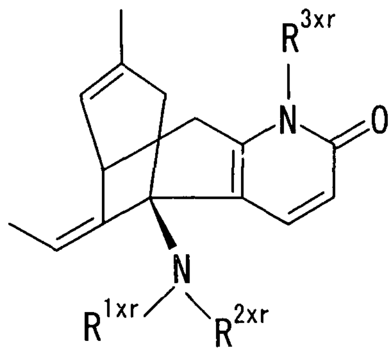

wherein $R^{1xr}$, $R^{2xr}$ and $R^{3xr}$ each is hydrogen or lower alkyl; or salts thereof.

Huperzine A represented by the following formula or salts thereof.

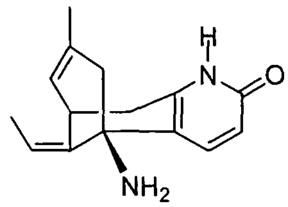

The above-mentioned compounds or salts thereof can be produced according to the process described in USP 5,177,082, J. Am. Chem. Soc., 1991, 113, p. 4695-4696, or J. Am. Chem. Soc., 1989, 111, p. 4116-4117, or its modification, or obtained by extraction and isolation from a Chinese herb, Qian ceng ta (Lycopodium serratum (Huperizia serrata) Thunb).

43) Galanthamine or galanthamine derivatives represented by the following structural formula.

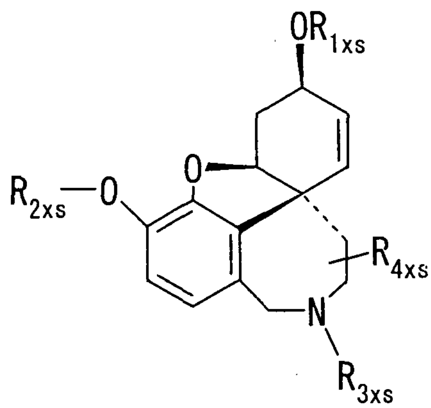

In the above formula, $R_{1xs}$ and $R_{2xs}$ are the same or different, each representing hydrogen or acyl such as lower alkanoyl, for example, acetyl, or a straight or branched alkyl, for example, methyl, ethyl, propyl, isopropyl, and the like.

$R_{3xs}$ is straight or branched alkyl, alkenyl or alkaryl, and these groups may be replaced optionally by halogen, cycloalkyl, hydroxy, alkoxy, nitro, amino, aminoalkyl, acylamino, heteroaryl, heteroaryl-alkyl, aroyl, aroylalkyl, or cyano.

$R_{4xs}$ means hydrogen or halogen attached to at least one of carbon atoms that constitute the tetra-cyclic skeletal structure; provided that when $R_4$ is placed at the adjacent position to the nitrogen atom, $R_4$ is preferably different from halogen, as well as from, for example, hydrohalides such as hydrobromide, hydrochloride, etc., methyl sulfate or methiodide.

Such a compound is exemplified by galanthamine represented by the following formula or salts thereof.

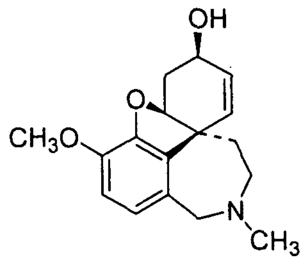

The above-mentioned compounds or salts thereof can be produced according to the process described in JP 6-507617 A, Heterocycles, 1977, 8, p. 277-282, or J. Chem. Soc. (C), 1971, p. 1043-1047, or its modification process, or obtained by extraction and isolation from a Liliaceae plant such as Galanthus nivalis or Galanthus waronowii.

44) Substituted amines of the formula:

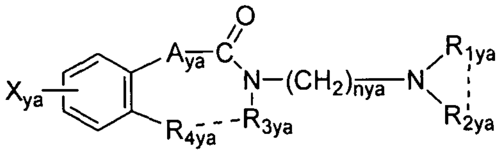

wherein $R_{1ya}$ and $R_{2ya}$ each is independently hydrogen or optionally substituted hydrocarbon residue, or they taken with the adjacent nitrogen atom form a heterocyclic group; as for $R_{3ya}$ and $R_{4ya}$, $R_{3ya}$ represents hydrogen or an optionally substituted hydrocarbon residue or acyl and $R_{4ya}$ represents hydrogen, or $R_{3ya}$ and $R_{4ya}$ taken together may form $-(CH_2)_{mya}$- CO-, $-CO-(CH_2)_{mya}$- or $(CH_2)_{mya+1}$- (where mya is 0, 1 or 2); $A_{ya}$ represents $-(CH_2)_{lya}$- (lya is 0, 1 or 2) or -CH=CH-; $X_{ya}$ indicates 1 or more of substituents; nya is an integer of 4 to 7; or salts thereof.

The above-mentioned compounds or salts thereof can be produced according to the process described in JP 2-91052 A or its modification.

45) Aminoketone derivatives of the formula:

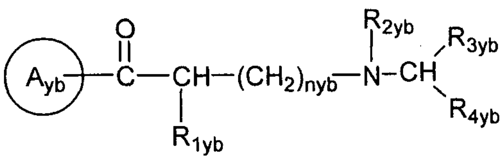

wherein the ring $A_{yb}$ is a 5- to 8-membered cyclic group which may be substituted and may contain 1 or 2 ring-constituting heteroatoms of O, S and N; $R_{1yb}$ is hydrogen or optionally substituted hydrocarbon residue; $R_{2yb}$ is hydrogen or lower alkyl; $R_{3yb}$ is an optionally substituted aromatic group; $R_{4yb}$ is hydrogen or lower alkyl or optionally substituted aromatic group; and nyb is an integer of 2 - 7; or salts thereof.

The above-mentioned compounds or salts thereof can be produced according to the process described in JP 3-95143 A or its modification.

46) Aralkylamine derivatives of the formula:

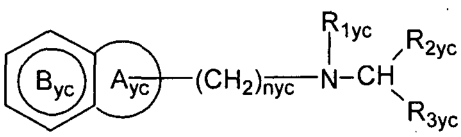

wherein $R_{1yc}$ is hydrogen or lower alkyl; $R_{2yc}$ is an optionally substituted aromatic group; $R_{3yc}$ is hydrogen or lower alkyl or optionally substituted aromatic group; nyc is an integer of 0 - 7; the ring $A_{yc}$ is a 5- to 8-membered cyclic group which may be substituted and may contain 1 or 2 ring-constituting heteroatoms of O and S; the ring $B_{yc}$ is an optionally substituted benzene ring; or salts thereof.

The above-mentioned compounds or salts thereof can be produced according to the process described in JP 3-141244 A or its modification.

47) Aminonaphthalene compounds of the formula:

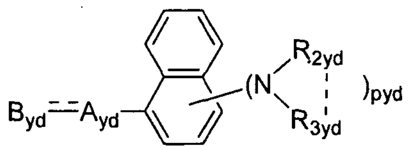

wherein $B_{yd}$ is an optionally substituted saturated or unsaturated 5- to 7-membered aza-heterocyclic group; $A_{yd}$ is a bonding or hydrocarbon residue, or bivalent or trivalent aliphatic hydrocarbon residue optionally substituted by oxo, hydroxyimino or hydroxy; --- indicates a single bond or double bond (provided that when $A_{yd}$ is a bonding, thenis a single bond), $R_{2yd}$ and $R_{3yd}$ each is independently hydrogen or optionally substituted hydrocarbon residue, or they taken with the adjacent nitrogen atom may form a cyclic amino; pyd is 1 or 2; or salts thereof.

The above-mentioned compounds or salts thereof can be produced according to the process described in JP 3-223251 A or its modification.

48) Condensed heterocyclic carboxylic acid derivatives of the formula:

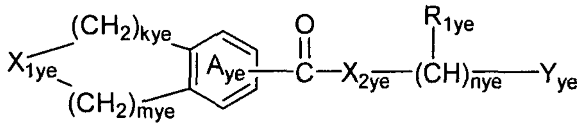

wherein $X_{1ye}$ is $R_{4ye}$-N ($R_{4ye}$ is hydrogen, optionally substituted hydrocarbon group or optionally substituted acyl), oxygen or sulfur; $X_{2ye}$ is $R_{5ye}$-N ($R_{5ye}$ is hydrogen, optionally substituted hydrocarbon group or optionally substituted acyl) or oxygen; the ring $A_{ye}$ is a benzene ring which may be substituted by an additional substituent; $R_{1ye}$ is hydrogen or optionally substituted hydrocarbon group; each of $R_{1ye}$ may be different according to repetition of nye; $Y_{ye}$ is optionally substituted amino or optionally substituted nitrogen-containing saturated heterocyclic group; nye is an integer of 1 to 10; kye is an integer of 0 to 3; and mye is an integer of 1 to 8; or salts thereof.

The above-mentioned compounds or salts thereof can be produced according to the process described in JP 5-239024 A or its modification.

49) Unsaturated carboxylic amide derivatives of the formula:

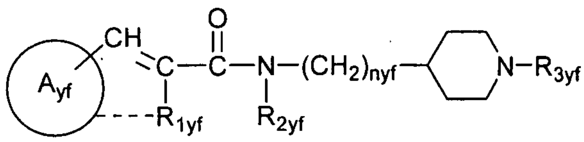

wherein the ring $A_{yf}$ is an optionally substituted aromatic ring; $R_{1yf}$ is hydrogen or optionally substituted hydrocarbon residue, or it is taken with the adjacent group of -CH=C-and the two carbon atoms constituting the ring $A_{yr}$ to form an optionally substituted carbocycle; $R_{2yf}$ is hydrogen, or optionally substituted hydrocarbon residue or acyl; $R_{3yf}$ is an optionally substituted hydrocarbon residue; and nyf is an integer of 2 to 6; or salts thereof.

The above-mentioned compounds or salts thereof can be produced according to the process described in JP 2-138255 A or its modification.

Then, the production process of the compound B will be explained. The compound (II) is produced according to the production of the compounds (IIa), (IIb), (IIc) and (IId) shown hereinafter.

The compounds (IIa), (IIc) and (IId) are produced by, for example, the methods of "process A" and "process B" hereinafter, and the compound (IIb) is produced by, for example, the method of "process C" hereinafter. When an

alkylation reaction, a hydrolysis reaction, an amination reaction, an esterification reaction, an amidation reaction, an esterification reaction, an etherification reaction, an oxidation reaction, a reduction reaction, a reductive amination reaction and the like are carried out in "process A" to "process C", these reactions are carried out according to known methods. These reactions include methods described in "ORGANIC FUNCTIONAL GROUP PREPARATIONS" the second edition, published by ACADEMIC PRESS INC. in 1989; "Comprehensive Organic Transformation" published by VCH Publishers Inc. in 1989, and the like.

Further, in the processes described hereinafter, the compounds (III), (III'), (III"), (IV), (V), (VI), (VII), (VII'), (VII"), (VIII), (IX), (X), (XI), (XII), (XIII), (XIV), (XV), (XVI), (XVII), (XVIII), (XIX), (XX), (XXI) and (XXII) may form salts, respectively. As the "salt", for example, the "salt" of the aforementioned "when the compound B is a salt" can be applied.

[Process A] The process for preparing the compound (IIa), (IIc) or (IId) by a coupling reaction of the compound (III), (III') or (III") with the compound (IV).

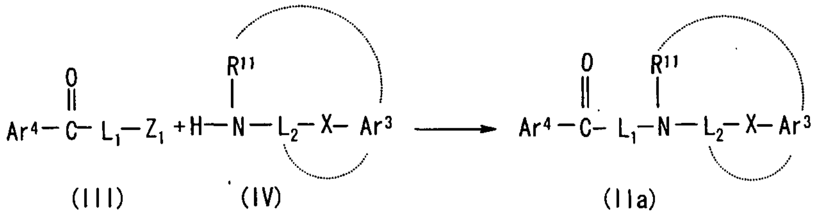

(III) (IV) (IIa)

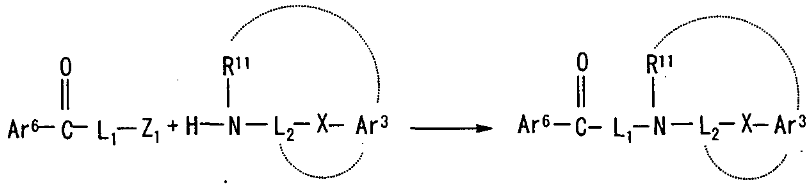

(III') (IV) (IIc)

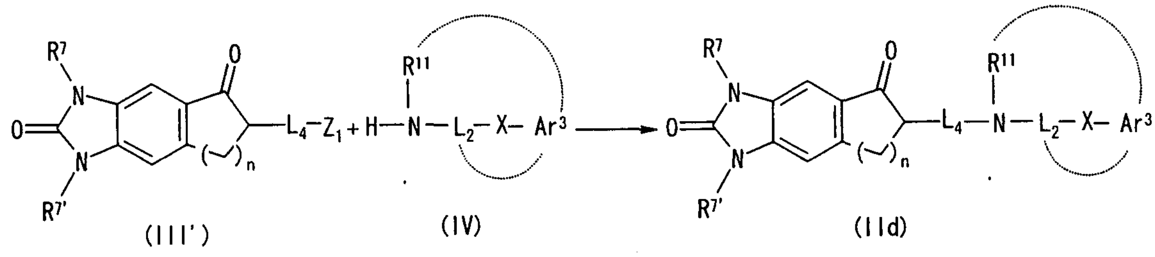

(III') (IV) (IId)

wherein $Z_1$ represents a leaving group, and the other symbols are as defined above.

As the "leaving group" represented by $Z_1$, for example, halogen atom (e.g. chloro, bromo, iodo), $C_{1-6}$ alkylsulfonyloxy (e.g. methanesulfonyloxy, ethanesulfonyloxy, trifluoromethanesulfonyloxy), and $C_{6-10}$ arylsulfonyloxy (e.g. benzenesulfonyloxy, p-toluenesulfonyloxy) are used. In particular, halogen atom (e.g. chloro, bromo etc.), and methanesulfonyloxy are preferable.

This coupling reaction can be carried out without a solvent, or by dissolving or suspending the compounds in a suitable solvent such as a hydrocarbon solvent, an alcohol solvent, an ether solvent, a halogenated hydrocarbon solvent, an aromatic solvent, a nitrile solvent, an amide solvent, a ketone solvent, a sulfoxide solvent, carboxylic acid solvent, or water. These solvents may be used by mixing two or more at an appropriate ratio. Preferably, no solvent, or an alcohol solvent such as ethanol, an aromatic solvent such as toluene, or an amide solvent such as dimethylformamide is used.

This coupling reaction can be carried out by adding a suitable base. Alternatively, the base can be used as a solvent.

Examples of the "base" include:

1) strong basis such as hydride of an alkali metal or an alkaline earth metal (e.g. lithium hydride, sodium hydride,

potassium hydride, calcium hydride etc.), amides of an alkali metal or an alkaline earth metal (e.g. lithium amide, sodium amide, lithium diisopropylamide, lithium bicyclohexylamide, lithium hexamethyldisilazide, sodium hexamethyldisilazide, potassium hexamethyldisilazide etc.), and lower alkoxide of an alkali metal or an alkaline earth metal (e.g. sodium methoxide, sodium ethoxide, potassium tert-butoxide etc.);
2) inorganic bases such as hydroxide of an alkali metal or an alkaline earth metal (e.g. sodium hydroxide, potassium hydroxide, lithium hydroxide, barium hydroxide etc.), carbonate of an alkali metal or an alkaline earth metal (e.g. sodium carbonate, potassium carbonate, cesium carbonate etc.), and bicarbonate of an alkali metal or an alkaline earth metal (e.g. sodium bicarbonate, potassium bicarbonate); and
3) organic bases such as amines such as triethylamine, diisopropylethylamine, and N-methylmorpholine; amidines such as DBU (1,8-diazabicyclo[5.4.0]undec-7-ene), and DBN (1,5-diazabicyclo[4,3,0]non-5-ene); basic heterocyclic compounds such as pyridine, dimethylaminopyridine, imidazole, and 2,6-lutidine.

As the "base", alkali metal salts such as potassium carbonate, and amines such as triethylamine and diisopropylethylamine are preferable.
Upon this coupling reaction, a hydrogen atom of the compound (IV) may be substituted with a metal atom, for example, an alkali metal such as lithium and sodium in advance.
This coupling reaction can be carried out at -100°C to 300°C, preferably 0°C to 150°C. The reaction time is 1 minute to 1 day.
This coupling reaction can be carried out at an arbitrary ratio of the compound (III), (III') or (III") and the compound (IV), and either of them may be used as a solvent.
The compound (IV) can be prepared by a method known per se or a similar method thereto. For example, the compound (IV) can be prepared by the method described in Comprehensive Organic Transformation VCH Publishers Inc., 1989, more specifically, the method described in J. Org. Chem.24,1106(1959), Bull. Chem. Soc. Jpn.63,1252(1990), Synth. Commun.14, 1099(1984), Tetrahedron 49, 1807(1993), J. Heterociclic Chem. 28, 1587(1991), J.Org. Chem. 60, 7086(1995) or a similar method thereto.
The compound (III) or (III') can be prepared, for example, by a method such as Friedel-Crafts reaction shown below.

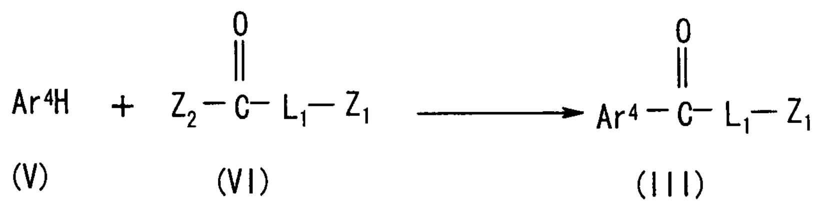

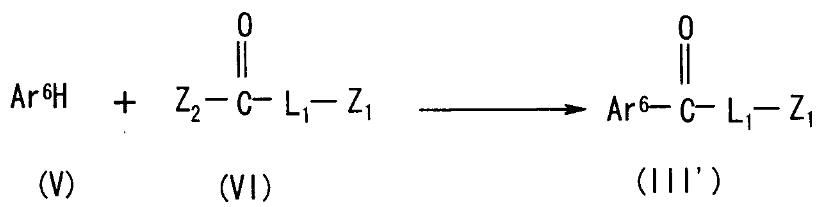

wherein $Z_2$ represents a leaving group, and the other symbols are as defined above.
As the "leaving group" represented by $Z_2$, the same leaving groups as those for the above-mentioned $Z_1$ can be applied. Preferred is a halogen atom (e.g. chloro, bromo etc.) or a hydroxy group.
This reaction can be preferably carried out by adding an acid catalyst, but the reaction can be carried out without adding an acid catalyst. As the acid catalyst used in the reaction, mineral acids such as sulfuric acid, phosphoric anhydride, and polyphosphoric acid, and Lewis acids such as aluminum chloride, tin tetrachloride, titanium tetrachloride, boron trifuloride, triethylaluminum, diethylaluminum chloride, and zinc chloride can be used. Preferable examples include polyphosphoric acid, aluminum chloride, diethylaluminum chloride, and zinc chloride. An acid catalyst can be used at an arbitrary equivalent, usually, 0.1 equivalent to 10 equivalent relative to the compound (V) or the compound (VI). Occasionally, an acid catalyst can be used as a solvent.
This reaction can be carried out without a solvent, or by dissolving or suspending reactants in a suitable solvent such as a hydrocarbon solvent, an ether solvent a halogenated hydrocarbon solvent, a nitrated hydrocarbon solvent,

an aromatic solvent, a nitrile solvent, an amide solvent, a ketone solvent, a sulfoxide solvent, and a carboxylic acid solvent. These may be used by mixing two or more at an appropriate ratio. Preferably, for example, no solvent, or a halogenated hydrocarbon solvent such as dichloromethane and 1,2-dichlroethane, a nitrated hydrocarbon solvent such as nitromethane, an aromatic solvent such as nitrobenzene, and carbon disulfide are used.

This reaction can be carried out at -100°C to 300°C and, usually, 0°C to 150°C is preferable. The reaction time is, for example, 1 minute to 3 days.

This reaction can be carried out at an arbitrary ratio of the compound (V) and the compound (VI), and either of them can be also used as a solvent.

The compound (V) can be prepared by a method known per se or a similar method thereto. The compound (V) can be prepared by the method, for example, described in Synthesis 10, 862 (1984), J. Chem. Soc. 1518 (1964), Synthesis 851 (1984), and JP 9-124605 A or a similar method thereto.

The compound (VI) can be prepared by a method known per se or a similar method thereto. The compound (VI) can be prepared by the method, for example, described in Org. Syn. Coll. Vol.1, 12 (1941), Helv. Chem. Acta 42, 1653 (1959) or a similar method thereto.

The compound (III) or (III') can be prepared by a method other than the aforementioned Friedel-Crafts reaction. Examples of the "method other than the Friedel-Crafts reaction" include a method using an organic metal reagent such as a method using an arylmagnesium reagent described in Tetrahedron Lett. 27, 929(1986), J. Am. Chem. Soc. 70, 426 (1948), Synlett 3, 225 (1996), a method using an organic zinc reagent described in Tetrahedron 46, 6061(1990). Alternatively, for example, the compound (III) or (III') can be prepared using a method for synthesis from an active methylene derivative described in JP 3-95143 A.

The compound (III") can be prepared, for example, by a method of introducing a substituent V into the compound (XIII) to obtain the compound (XIV), coupling the compound (XIV) and the compound (XV), and removing the substituent V as shown below:

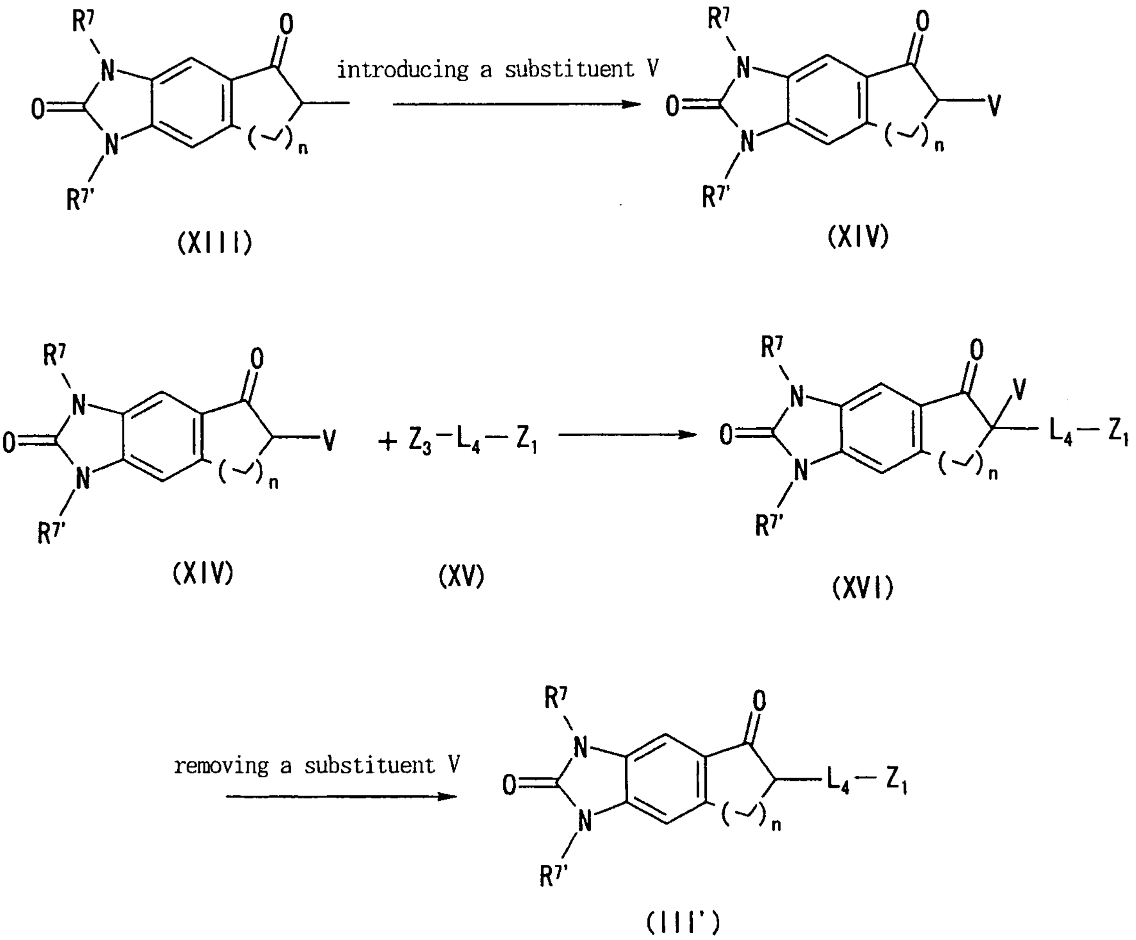


(XIII) (XIV)

(XIV) (XV) (XVI)

(III')

wherein substituent V represents an electron withdrawing group, $Z_3$ represents a leaving group, and the other symbols

are as defined above.

As the "electron withdrawing group" represented by the substituent V, for example, a $C_{1-8}$ alkoxycarbonyl group (e.g. methoxycarbonyl, tert-butoxycarbonyl group etc.), a $C_{7-16}$ aralkyloxycarbonyl group (e.g. benzyloxycarbonyl group etc.), a carboxyl group, and a cyano group are used. Preferable is a $C_{1-4}$ alkoxycarbonyl group (e.g. methoxycarbonyl, tert-butoxycarbonyl group etc.).

As the "leaving group" represented by $Z_3$, the same leaving groups as those for the above-mentioned $Z_1$ can be applied. Preferred is a halogen atom (e.g. chloro, bromo etc.), a p-toluenesulfonyloxy group or a methanesulfonyloxy group.

The reaction of "introducing a substituent V" can be carried out without solvent, or in a suitable solvent. As the "solvent", the same solvents as "solvents" described for the above-mentioned Process A can be used. For example, no solvent, or an ether solvent such as tetrahydrofuran, an aromatic solvent such as toluene, and an amide solvent such as dimethylformamide are preferred.

As a precursor of the substituent V in this reaction of "introducing a substituent V", for example, carbonic acid ester (dimethyl carbonate etc.), halocarbonic acid ester (methyl chlorocarbonate) or carbon dioxide is used.

Alternatively, this reaction of "introducing a substituent V" may be carried out by adding an appropriate base. In addition, the base may be used as a solvent. As the "base", the same bases as "bases" described for the above-mentioned Process A can be used. For example, sodium hydride, sodium amide, and lithium diisopropylamide are preferred.

This reaction of "introducing a substituent V" can be carried out at -100°C to 300°C, and 0°C to 150°C is preferred. The reaction time is, for example, 1 minute to 1 day.

The coupling reaction of the compound (XIV) and the compound (XV) can be carried out without solvent or in a suitable solvent. As the "solvent", the same solvents as "solvents" described for the above-mentioned Process A can be used. For example, no solvent, or an ether solvent such as tetrahydrofuran, an aromatic solvent such as toluene, and an amide solvent such as dimethylformamide are preferred.

Alternatively, this "coupling reaction of the compound (XIV) and the compound (XV)" can be carried out by adding an appropriate base. In addition, the base may be used as a solvent. As the "base", the same bases as "bases" described for the above-mentioned Process A can be used. For example, sodium hydride, sodium amide, and lithium diisopropyl amide are preferable.

This "coupling reaction of the compound (XIV) and the compound (XV)" can be carried out at -100°C to 300°C, and 0°C to 150°C is preferred. The reaction time is, for example, 1 minute to 1 day.

The reaction of "removing a substituent V" can be carried out without solvent or in a suitable solvent. As the "solvent", the same solvents as "solvents" described for the above-mentioned Process A can be used. For example, no solvent, or an ether solvent such as diglyme, an aromatic solvent such as xylene, a sulfoxide solvent such as dimethyl sulfoxide, and water are preferred.

Alternatively, this reaction of "removing a substituent V" may be carried out by adding an appropriate acid or salt. In addition, the acid may be used also as a solvent. As the "acid", a mineral acid such as hydrochloric acid and sulfuric acid, and p-toluenesulfonic acid are preferred. As the "salt", for example, sodium chloride is used.

This reaction of "removing a substituent" is preferably carried out by heating. Preferably, the reaction is carried out at room temperature to 250°C. The reaction time is, for example, 1 minute to 1 day.

The compound (XIII) can be prepared according to the method, for example, described in J. Org. Chem. 36, 2480 (1971). That is, for example, as shown hereinafter, the compound (XIII) can be prepared by a method of preparing the compound (XIX) by Friedel-Crafts reaction of the compound (XVII) and the compound (XVIII), converting the compound (XIX) into the compound (XX) by reduction reaction, followed by halogenation to obtain the compound (XXI), and carrying out intarmolecular Friedel-Crafts reaction. Alternatively, the compound (XIII) can be also prepared by intramolecular Friedel-Crafts reaction of the compound (XX).

Alternatively, the compound (XIII) can be prepared, for example, according to the method of Org. Syn. Coll. Vol.4, 898(1963). That is, for example, the compound (XIII) can be prepared by tandem-type intramolecular Friedel-Crafts reaction of the compound (XVII) and the compound (XXII) as shown below:

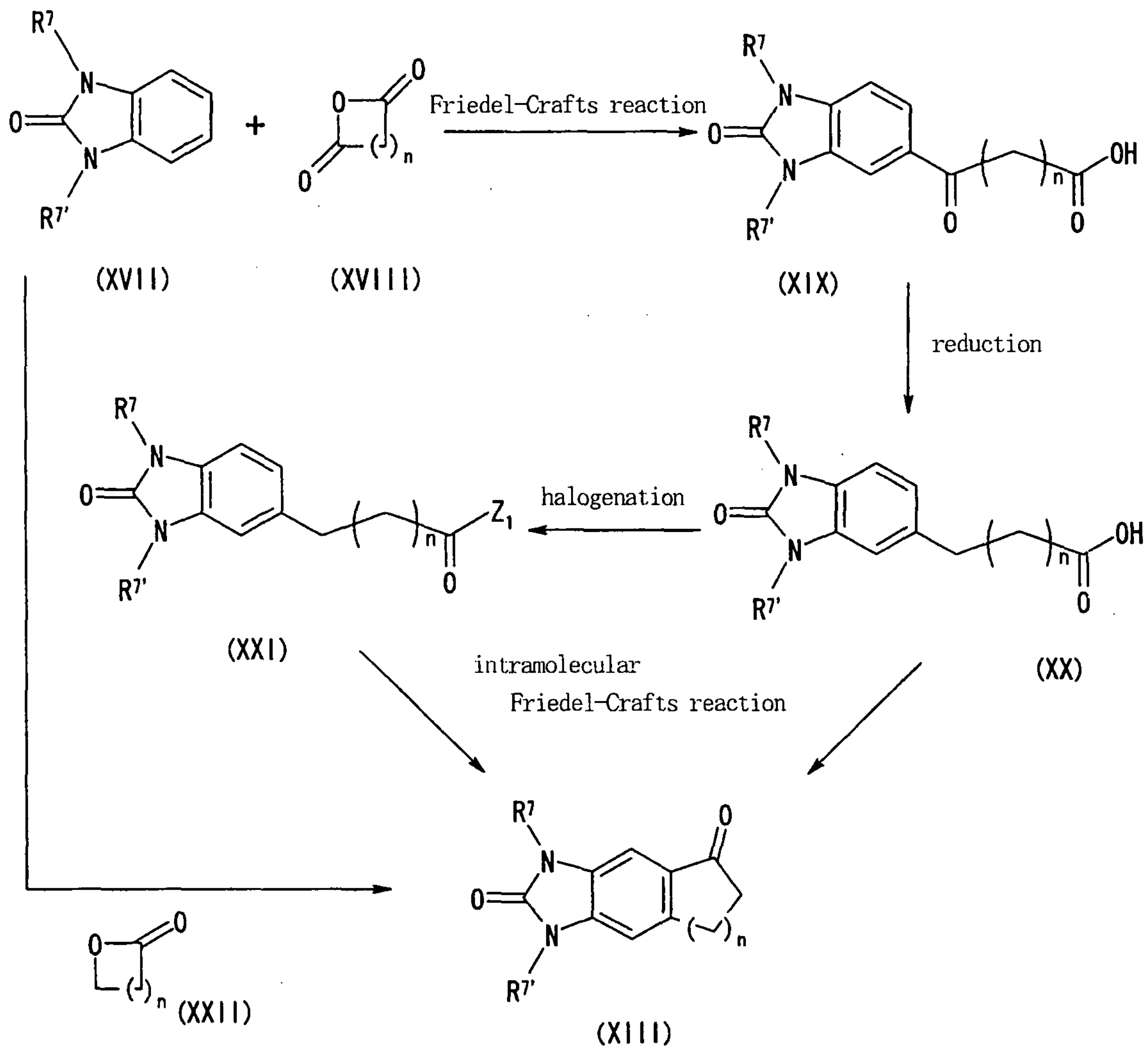


tandem-type intramolecular Friedel-Crafts reaction

wherein the symbols as defined above.

The "Friedel-Crafts reaction of the compound (XVII) and the compound (XVIII)" can be carried out by a method according to the aforementioned "Friedel-Crafts reaction of the compound (V) and the compound (XI)". In the "reduction" reaction of the compound (XVIII) to the compound (XX), catalytic reduction using, for example, a palladium catalyst, Clemmensen reduction, for example, described in Org. React. 22, 401 (1975), and Wolff-Kishner reduction, for example, described in, Org. React. 4,378 (1948) can be used.

The "halogenation" reaction of the compound (XX) to the compound (XXI) is carried out by using a reagent used for halogenation such as thionyl chloride, oxalyl chloride, and chlorine. Alternatively, the halogenation reagent may be used as a solvent.

The "intramolecular Friedel-Crafts reaction" from the compound (XX) to the compound (XIII) can be carried out by a method according to the aforementioned "Friedel-Crafts reaction of the compound (V) and the compound (XI)". As Lewis acid, polyphosphoric acid is preferable.

The "intramolecular Friedel-Crafts reaction" from the compound (XXI) to the compound (XIII) can be carried out by a method according to the aforementioned "Friedel-Crafts reaction of the compound (V) and the compound (XI)".

The preparation of the compound (XIII) by the "tandem-type intramolecular Friedel-Crafts reaction" of the compound (XVII) and the compound (XXII) can be carried out by a method according to the aforementioned "Friedel-Crafts reaction of the compound (V) and the compound (XI)".

[Process B] The process for preparing the compound (IIa), (IIc) or (IId) by a coupling reaction of the compound (VII) or (VII') and the compound (VIII).

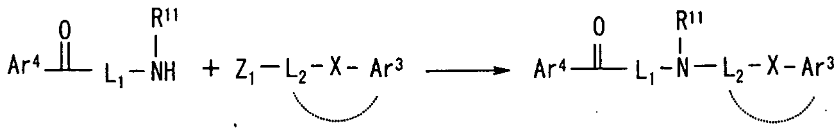

(VII) (VIII) (IIa)

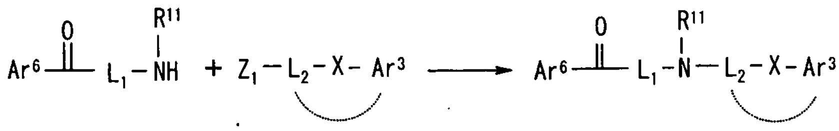

(VII') (VIII) (IIc)

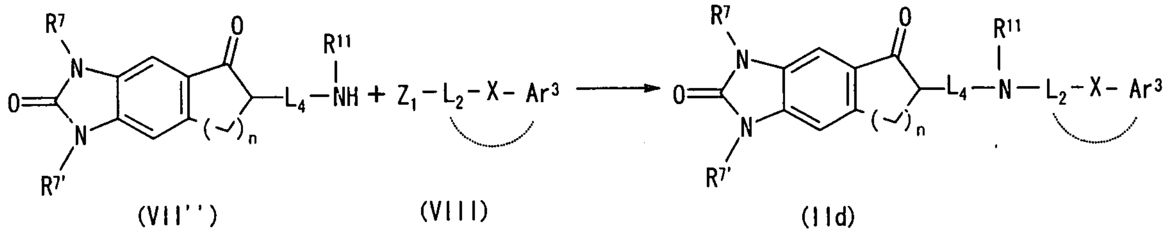

(VII'') (VIII) (IId)

wherein the symbols are as defined above.

This coupling reaction can be carried out without a solvent, or in an appropriate solvent. As the "solvent", the same solvents as "solvents" described for the above-mentioned Process A can be used. For example, no solvent, or an alcohol solvent such as ethanol, an aromatic solvent such as toluene, and an amide solvent such as dimethylformamide are preferable.

Alternatively, this coupling reaction may be carried out by adding an appropriate base. In addition, the base may be also used as a solvent. As the "base" the same bases as "bases" described for the above-mentioned Process A can be used.

Upon the present coupling reaction, a hydrogen atom of the compound (VII) or (VII') may be substituted with a metal atom, for example, an alkali metal such as lithium and sodium in advance.

This coupling reaction may be carried out at -100°C to 300°C, and 0°C to 150°C is preferred. The reaction time is, for example, 1 minute to 1 day.

This coupling reaction can be carried out at an arbitrary ratio of the compound (VII) or (VII') and the compound (VIII), and either of them may be used also as a solvent.

The compound (VIII) can be prepared by a method known per se or a similar method thereto.

The compound (VII), (VII') or (VII") can be prepared, for example, by a coupling reaction of the aforementioned the compound (III) or (III') and the compound (IX) as shown below.

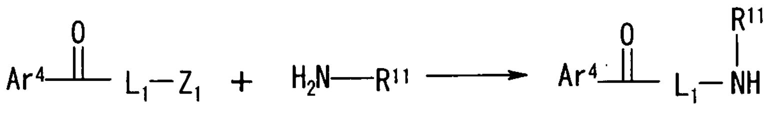

(III) (IX) (VII)

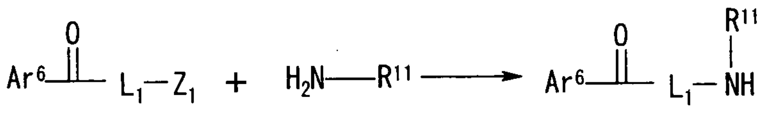

(III') (IX) (VII')

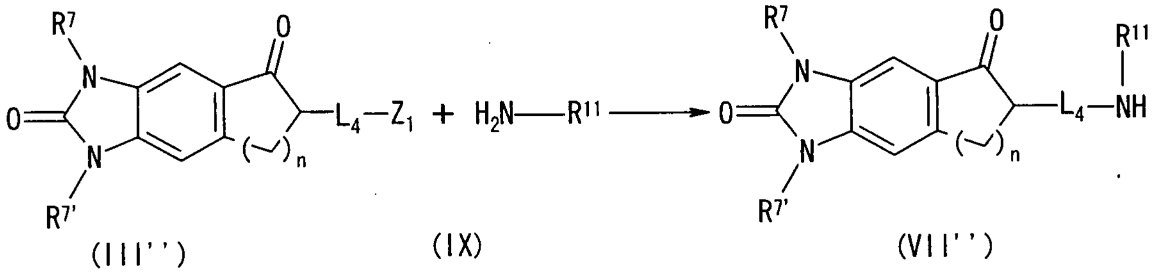

(III'') (IX) (VII'')

wherein the symbols are as defined above.

This coupling reaction can be carried out without solvent or in an appropriate solvent. As the "solvent", the same solvents as "solvents" described for the above-mentioned Process A can be used. For example, no solvent, or an alcohol solvent such as ethanol, an aromatic solvent such as toluene, and an amide solvent such as dimethylformamide are preferable.

Alternatively, this coupling reaction may be carried out by adding an appropriate base. In addition, the base may be used also as a solvent. As the "base", the same bases as "bases" described for the above-mentioned Process A can be used.

Upon this coupling reaction, a hydrogen atom of the compound (IX) may be substituted with a metal atom, for example, an alkali metal such as lithium and sodium in advance.

The present coupling reaction can be carried out at - 100°C to 300°C, and 0°C to 150°C is preferred. The reaction time is, for example, 1 minute to 1 day.

This coupling reaction can be carried out at an arbitrary ratio of the Compound (III) or (III') and the compound (IX), and either of them may be also used as a solvent.

The compound (IX) can be prepared by a method known per se or a similar method thereto. For example, the compound (IX) can be prepared by the method described in Comprehensive Organic Transformation VCH Publishers Inc., 1989, specifically, a reductive amination reaction described in Organic Reactions (Org. Rxs.) 14, 52 (1965), Synthesis 30(1972), a reduction reaction of nitriles described in Org. Rxs. 6, 469(1951), Chem. Pharm. Bull. 32, 873(1984), a reduction reaction of azide described in J. Med. Chem. 12, 658(1969), J. Am. Chem. Soc. 73, 5865(1951), and a Gabriel synthesis method described in Org. Syn. Coll. Vol.2, 83(1943), J. Am. Chem. Soc. 72, 2786(1950).

[Process C] The process for preparing the compound (IIb) by a coupling reaction of the compound (X) and the compound (VIII).

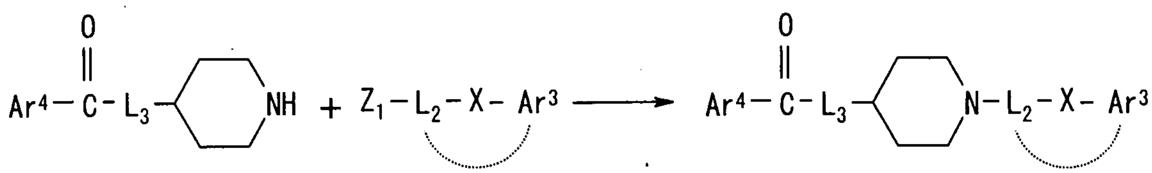

(X) (VIII) (IIb)

wherein the symbols are as defined above.

This coupling reaction can be carried out according to the aforementioned Process A. Specifically, the present reaction can be carried out without solvent, or in an appropriate solvent. As the "solvent", the same solvents as "solvents" described for the above-mentioned Process A can be used. For example, no solvent, or an alcohol solvent such as ethanol, an aromatic solvent such as toluene, a nitrile solvent such as acetonitrile, and an amide solvent such as

dimethylformamide are preferable.

Alternatively, this coupling reaction may be carried out by adding an appropriate base. In addition, the base may be also used as a solvent. As the "base", the same bases as "bases" described for the above-mentioned Process A can be used. Potassium carbonate, triethylamide, and diisopropylethylamine are preferred.

Upon this coupling reaction, a hydrogen atom of the compound (X) may be substituted with a metal atom, for example, an alkali metal such as lithium and sodium in advance.

This coupling reaction can be carried out at -100°C to 300°C, and 0°C to 150°C is preferred. The reaction time is, for example, 1 minute to 1 day.

This coupling reaction can be carried out at an arbitrary ratio of the compound (X) and the compound (VIII), and further either of them may be also used as a solvent.

The compound (X) can be prepared, for example, by a method such as preparing the compound (XII) by Friedel-Crafts reaction of the compound (V) and the compound (XI), followed by subjecting to a deprotection reaction as shown below.

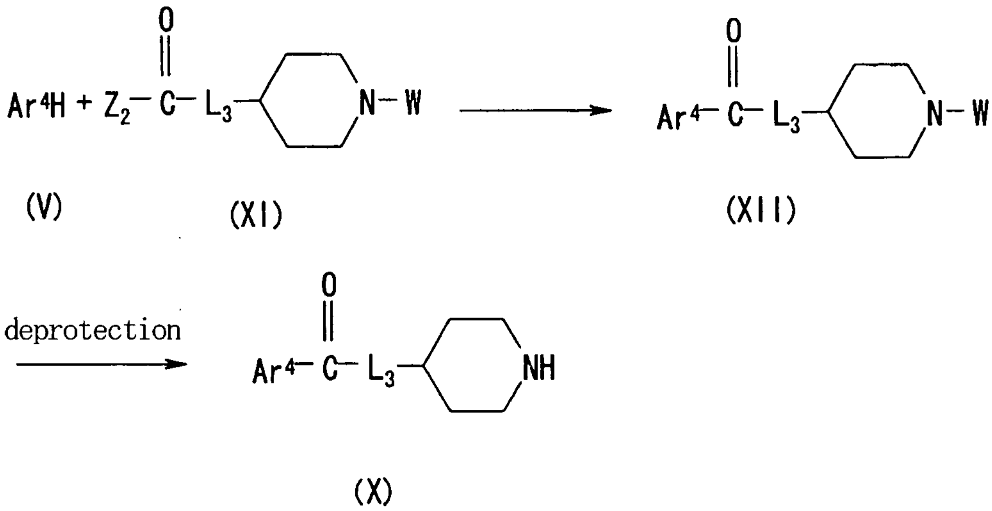


(V) (XI) (XII) (X)

wherein W represents a protecting group for amine, and the other symbols are as defined above.

As the protecting group W for amine, for example, protecting groups described in Protective Groups in Organic Synthesis, Third Edition, Wiley-Interscience (1999) can be used. Specifically, examples thereof include acyl groups such as formyl, $C_{1-6}$ alkyl-carbonyl which may have a substituent(e.g. acetyl, ethylcarbonyl, trifluoroacetyl, chloroacetyl etc.), benzoyl, $C_{1-6}$ alkyl-oxycarbonyl (e.g. methoxycarbonyl, ethoxycarbonyl, t-butoxycarbonyl etc.), phenyloxycarbonyl (e.g. phenoxycarbonyl etc.), and $C_{7-15}$ aralkyloxy-carbonyl (e.g. benzyloxycarbonyl, fluorenyloxycarbonyl etc.), and hydrocarbon groups such as trityl and phthaloyl. Inter alia, acetyl, trifluoroacetyl and benzyloxycarbonyl are preferred.

$Z_2$ is preferably a halogen atom (e.g. chloro, bromo etc.) or a hydroxy group.

The Friedel Crafts reaction of the compound (V) and the compound (XI) can be preferably carried out by adding an acid catalyst, and may be carried out without adding an acid catalyst. As the acid catalyst used in the reaction, the same acid catalysts as those used in preparation of the above-mentioned compound (III) are used. Preferable examples include polyphosphoric acid, aluminum chloride, diethylaluminum chloride, and zinc chloride. The acid catalyst can be used at an arbitrary equivalent, usually at 0.1 equivalent to 10 equivalents relative to the compound (V) or the compound (XI). Occasionally, the acid catalyst may be also used as a solvent.

As the solvent, same solvents as those used in preparation of the above-mentioned compound (III) can be applied. Preferable examples include no solvent, or a halogenated hydrocarbon solvent such as dichloromethane, and 1,2-dichloroethane, a nitrated hydrocarbon solvent such as nitromethane, an aromatic solvent such as nitrobenzene, and carbon disulfide.

This reaction can be carried out at -100°C to 300°C and, usually, 0°C to 150°C is preferred. The reaction time is, for example, 1 minute to 3 days.

Deprotection of the compound (XII) can be carried out according to the method described in the aforementioned Protective Groups in Organic Synthesis, Third Edition, Wiley-Interscience (1999). Specifically, deprotection is carried out by acid treatment, alkali hydrolysis, or catalytic reduction reaction.

The compound (XI) can be prepared by a method known per se or a similar method thereto. For example, the compound (XI) can be prepared by the method described in JP 5-140149 A, Chem. Pharm. Bull., 34, 3747 (1986), Chem.

Pharm. Bull., 41, 529 (1993), and EP-A-0,378,207 or a similar method thereto.

When the compounds (IIa) to (IId) prepared by the aforementioned [Process A] to [Process C] are primary or secondary amine, those compounds can be isolated and purified after derived to other derivatives, if necessary. Preferable examples of "other derivatives" include compounds in which the primary or secondary amine is protected with a general amine protecting group. Examples of the "general amine protecting group" include the protecting groups described in Protective Groups in Organic Synthesis, Third Edition, Wiley-Interscience (1999). Specifically, examples include an acyl group such as formyl, $C_{1-6}$ alkyl-carbonyl which may have a substituent (e.g. acetyl, ethylcarbonyl etc.), benzoyl, $C_{1-6}$ alkyl-oxycarbonyl (e.g. methoxycarbonyl, ethoxycarbonyl, t-butoxycarbonyl etc.), phenyloxycarbonyl (e.g. phenoxycarbonyl etc.), and $C_{7-15}$ aralkyloxy-carbonyl (e.g. benzyloxycarbonyl, fluorenyloxycarbonyl etc.), and hydrocarbon groups such as trityl and phthaloyl. Preferably, an acetyl group, a benzoyl group, a t-butoxycarbonyl group, a benzyloxycarbonyl group, and a benzyl group are used. Purified "other derivatives" can be derived to the original primary or secondary amine or a salt thereof by a deprotection reaction suitable for each of them. The "deprotection reaction" can be carried out according to the method, for example, described in the aforementioned Protective Groups in Organic Synthesis, Third Edition, Wiley-Interscience (1999). Specifically, the deprotecting reaction is carried out by acid treatment, alkaline hydrolysis, or catalytic reducing reaction.

As the "salt" in the "primary or secondary amine or a salt thereof", the "salt" in the aforementioned "when the compound B is a salt" can be applied.

The compounds (IIa) to (IId) can be prepared by processes other than the aforementioned processes. For example, they may be prepared by reductive amination reaction described in J. Am. Chem. Soc. 2897 (1971), Synthesis 135 (1976), Tetrahedron Lett. 5595 (1990), an addition reaction of amine to epoxide described in Synth. Commun. 177 (1973), Tetrahedron Lett. 4661 (1990), Michael addition reaction of amine to a conjugated double bond described in Org. Rxs. 79 (1949), Organic Synthesis Coll. Vol. 1, 196 (1941), a reduction reaction of amide described in Synthesis 752 (1978), Org. Rxs. 303 (1942), or a Mannich reaction described in Org. Rxs. 469 (1941), Angew. Chem. 265 (1956).

As described above, the compounds A and B of the present invention have no limitation for their molecular structure as long as they are compounds having the AChE inhibitory activity without BuChE inhibitory activity as defined herein, and, for example, a compound having a carbamate structure in its molecule is also included. Examples of the carbamate compound having the AChE inhibitory activity without BuChE inhibitory activity include:

50) phenserine or its derivative represented by the formula:

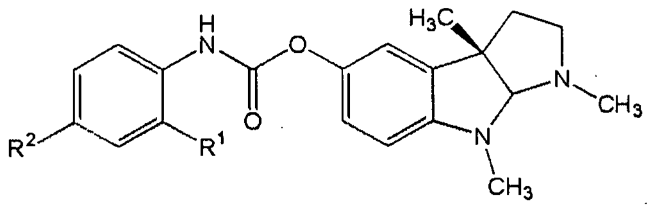

wherein $R^1$ represents hydrogen or $C_{1-3}$ alkyl (for example, methyl, ethyl, propyl, isopropyl) and $R^2$ represents hydrogen (provided that when $R^1$ is methyl, $R^2$ is hydrogen or methyl)], or a salt thereof.

The above-mentioned compound or its salt can be produced by the method described in Qian-sheng Yu et al., J. Med. Chem. 44, p4062-4071 (2001) or its modification.

Since the compound of the present invention has characteristic of having AChE inhibitory activity without BuChE inhibitory activity, it exhibits an enhancing effect of contraction of the detrusor muscle at urination, while it does not increase the basic tension of the detrusor muscle at the collection of urine and does not lower bladder compliance. Then, it can prevent such a serious side effect as the inhibition of the collection function of urine as that of conventional non-selective choline esterase inhibitors such as distigmine and neostigigmine. Thus, the compound of the present invention can be used as a safer and more effective preventive or remedy for lower urinary tract diseases, preferably a preventive or remedy for urinary disturbance, in particular, a preventive or remedy for difficulty of urination.

The compound of the present invention has low toxicity and can be used as a preventive or remedy for lower urinary tract diseases, preferably a preventive or remedy for urinary disturbance for mammals (e.g., human being, mouse, rat, rabbit, cat, dog, cow, horse, pig, monkey, and the like) as it is or by mixing it with a pharmacologically acceptable carrier to prepare a pharmaceutical composition (hereinafter, referred to as the pharmaceutical composition of the present invention).

Herein, as the pharmacologically acceptable carrier, various organic or inorganic carrier substances which are conventionally used as raw materials for preparations are used, and they are compounded as an excipient, a lubricant, a binder and a disintegrant in solid preparations; a solvent, a solubilizing aid, a suspending agent, an isotonizing agent, a buffer and a soothing agent in liquid preparations, etc. Further, if necessary, preparation additives such as an antiseptic,

an antioxidant, a colorant and a sweetening agent can be used.

Preferred examples of the excipient include lactose, white sugar, D-mannitol, D-sorbitol, starch, α-starch, dextrin, crystalline cellulose, low substitution degree hydroxypropylcellulose, carboxymethylcellulose sodium, gum arabic, dextrin, plulan, light anhydrous silicic acid, synthetic aluminum silicate, magnesium metaaluminosilicate and the like.

Preferred examples of the lubricant include magnesium stearate, calcium stearate, talc, colloidal silica and the like.

Preferred examples of the binder include α-starch, sucrose, gelatin, gum arabic, methylcellulose, carboxymethylcellulose, carboxymethylcellulose sodium, crystalline cellulose, white sugar, D-mannitol, trehalose, dextrin, plulan, hydroxypropylcellulose, hydroxypropylmethylcellulose, polyvinyl pyrrolidone and the like.

Preferred examples of the disintegrant include lactose, white sugar, starch, carboxymethylcellulose, carboxymethylcellulose calcium, cross carmellose sodium, carboxymethylstarch sodium, soft anhydrous silicic acid, low substitution degree hydroxypropylcellulose and the like.

Preferred example of the solvent include water for injection, saline, Ringer's solution, alcohol, propylene glycol, ethylene glycol, sesame oil, corn oil, olive oil, cottonseed oil and the like.

Preferred examples of the solubilizing aid include polyethylene glycol, propylene glycol, D-mannitol, trehalose, benzyl benzoate, ethanol, trisaminomethane, cholesterol, triethanolamine, sodium carbonate, sodium succinate, sodium salicylate, sodium acetate and the like.

Preferred examples of the suspending agent include surfactants such as stearyltriethanolamine, sodium laurylsulfate, laurylaminopropionic acid, lecithin, benzalkonium chloride, benzetonium chloride and glycerin monostearate; hydrophilic synthetic polymers, for example, such as a polyvinyl alcohol, polyvinyl pyrrolidone, carboxymethylcellulose sodium, methylcellulose, hydroxymethylcellulose hydroxyethylcellulose and hydroxypropylcellulose; polysorbates, polyoxyethylene hardened ricinus and the like.

Preferred examples of the isotonizing agent include sodium chloride, glycerin, D-mannitol, D-sorbitol, glucose and the like.

Preferred examples of the buffer include buffers such as a salt of phosphoric acid, a salt of acetic acid, a salt of carbonic acid and a salt of succinic acid; and the like.

Preferred examples of the soothing agent include benzyl alcohol and the like.

Preferred examples of the antiseptic include para-oxybenzoic acid esters, chlorobutanol, benzyl alcohol, phenethyl alcohol, dehydroacetic acid, sorbic acid and the like.

Preferred examples of the antioxidant include sulfite, a salt of ascorbic acid and the like.

Preferred examples of the colorant include dyes for food such as a water-soluble tar dye for food (dyes for food such ac Reds No. 2 and No. 3 for food, Yellows No. 4 and No. 5 for food, and Blues No. 1 and No. 2 for food), water-insoluble lake dyes (aluminum salts of the above-mentioned water-soluble tar dye for food), natural dyes (for example, β-carotin, chlorophyll, colcothar and the like), etc.

Preferred examples of the sweetening agent include saccharine sodium, dipotassium glycyrrhizinate, aspartame, stevia sweetener and the like.

The dosage form of the pharmaceutical composition includes, for example, that for oral administration such as tablets, capsules (including soft capsules and microcapsules), granules, powders, syrups, emulsions and suspensions; that for parenteral administration such as injectable preparations (for example, those for subcutaneous, intravenous, intramuscular, and intraperitoneal administration and the like), preparations for external application (for example, those for transnasal and percutaneous administration, ointments and the like), suppositories (for example, those for rectal and transvaginal administration and the like), pellets and drops. Each of them can be safely administrated by oral or parenteral administration.

The pharmaceutical composition can be produced by a conventional method in the pharmaceutical field (for example, the method described in Japanese Pharmacopoeia and the like). The specific production method of preparations is described in details below.

For example, a preparation for oral administration can be produced by adding an excipient (for example, lactose, white sugar, D-mannitol, starch or the like), a disintegrant (for example, carboxymethylcellulose calcium or the like), a binder (for example, α-starch, gum arabic, carboxymethylcellulose, hydroxypropylcellulose, polyvinyl pyrrolidone or the like) or a lubricant (for example, talc, magnesium stearate, polyethylene glycol 6000 or the like) and the like, to an effective ingredient, and then, if necessary, coating the mixture by a known method using a coating base for the purpose of masking a taste and/or providing enteric property or sustainability.

Examples of the coating base include a sugar coating base, a water-soluble film coating base, an enteric film coating base, a sustained release coating base and the like.

As the sugar coating base, white sugar is used, and further, 1 or more materials selected from talc, precipitated calcium carbonate, gelatin, gum arabic, plulan and carnauba wax may be used in combination.

Examples of the water-soluble film coating base include cellulose polymers such as hydroxypropylcellulose, hydroxypropylmethylcellulose, hydroxyethylcellulose and methylhydroxyethylcellulose; synthetic polymers such as polyvinylacetal diethylamino acetate, aminoalkyl methacrylate copolymer-E [Eudragit E (trade name), Rohm Pharma Co.] and polyvinyl pyrrolidone; polysaccharides such as plulan.

Examples of the enteric film coating base include cellulose polymers such as hydroxypropylcellulose phthalate, hydroxypropylmethylcellulose acetate succinate, carboxymethylethylcellulose and acetic acid cellulose phthalate; acrylic acid-base polymers such as methacrylic acid copolymer-L [Eudragit L (trade name), Rohm Pharma Co.], methacrylic acid copolymer-LD [Eudragit L-30D55 (trade name), Rohm Pharma Co.] and methacrylic acid copolymer-S [Eudragit S (trade name), Rohm Pharma Co.], natural products such as shellac, etc.

Examples of the sustained release film coating base include cellulose polymers such as ethylcellulose; acrylic acid-base polymers such as aminoalkyl methacrylate copolymer-RS [Eudragit RS (trade name), Rohm Pharma Co.] and suspension of ethyl acrylate-methyl methacrylate copolymer [Eudragit NE (trade name), Rohm Pharma Co.], etc.

Two or more of the above-mentioned coating bases may used by mixing them at an appropriate proportion. Further, for example, light screening agents such as titanium oxide and ferric oxide may be used at the time of coating.

An injectable preparation is produced by dissolving, suspending or emulsifying an effective ingredient together with a dispersant (for example, polysorbate 80, polyoxyethylene hardened ricinus 60 or the like), polyethylene glycol, carboxymethylcellulose, sodium arginate or the like), a preservative (for example, methylparaben, propylparaben, benzyl alcohol, chlorobutanol, phenol or the like) and a isotonizing agent (for example, sodium chloride, glycerin, D-mannitol, D-sorbitol, glucose or the like) in a water-soluble solvent (for example, distilled water, saline, Ringer's solution and the like) or an oil soluble solvent (for example, plant oils such as olive oil, sesame oil, cotton oil and cone oil, propylene glycol and the like). At this time, additives such as a dissolution aid (for example, sodium salicylate, sodium acetate and the like), a stabilizer (for example, human serum albumin and the like) and a soothing agent (for example, benzyl alcohol and the like) may be used, if necessary.

The pharmaceutical composition of the present invention can be used as a preventive or remedy for urinary disturbance and in particular, a preventive or remedy for difficulty of urination which is caused by, for example, the following 1) to 7): 1) prostatauxe, 2) bladder neck obstruction, 3) neurogenic bladder, 4) diabetes, 5) operation, 6) low tonic bladder, and 7) Sjogren's syndrome (dry eye, dry mouth, vaginal dry and the like).

More specifically, it can be used as a preventive or remedy for difficulty of urination caused by low tonic bladder due to prostatauxe, low tonic bladder due to diabetes, low tonic bladder due to diabetic neuropathy, idiopathic low tonic bladder (including low tonic bladder due to the aging), low tonic bladder due to multiple sclerosis, low tonic bladder due to Parkinson's disease, low tonic bladder due to spiral cord damage, postoperative low tonic bladder, low tonic bladder due to brain obliteration, neurogenic bladder due to diabetes, neurogenic bladder due to diabetic neuropathy, neurogenic bladder due to multiple sclerosis, neurogenic bladder due to Parkinson's disease, neurogenic bladder due to spiral cord damage, neurogenic bladder due to brain obliteration and the like.

The compound of the present invention can be used in combination with a drug for treating a disease causing urinary disturbance (for example, difficulty of urination), or a drug which is administered for treating another disease, but causes urinary disturbance (for example, difficulty of urination) by itself.

Examples of the "drug treating a disease causing urinary disturbance" includes a remedy for prostatauxe, a remedy for prostate cancer, a remedy for bladder neck sclerosis, a remedy for chronic cystitis, a remedy for constipation, a remedy for colorectal cancer, a remedy for uterine cancer, a remedy for diabetes, a remedy for cerebrovascular diseases, a remedy for spinal cord damage, a remedy for spinal cord tumor, a remedy for multiple sclerosis, a remedy for dementia including Alzheimer's disease, a remedy for Parkinson's disease, a remedy for progressive supranuclear palsy, a remedy for Guillan-Barre syndrome, a remedy for acute pan autonomaic nerve abnormality, a remedy for olivepontocerebeller atrophy, a remedy for cervical spondylosis and the like.

Examples of the remedy for prostatauxe include Allylestrenol, Chlonmadinone acetate, Gestonorone caproate, Nomegestrol, Mepartricin, Finasteride, PA-109, THE-320 and the like. Further, a remedy for urinary disturbance accompanied with prostatauxe include α-reductase inhibitors such as YM-31758, YM-32906, KF-20405, MK-0434, Finasteride and CS-891.

Examples of the remedy for prostate cancer include Isofamide, Estramustine phosphate sodium, Cyproterone, Chlormadinone acetate, Flutamide, Cisplatin, Lonidamine, Peplomycin, Leuprorelin, Finasteride, Triptorelin-DDS, Buserelin, Goserelin-DDS, Fenretinide, Bicalutamide, Vinorelbine, Nilutamide, Leuprolide-DDS, Deslorelin, Cetrorelix, Ranpirnase, Leuprorelin-DDS, Satraplatin, Prinomastat, Exisulind, Buserelin-DDS, Abarelix-DDS and the like.

Examples of the remedy for bladder neck sclerosis include α-blocking agents such as an α1-blocking agent. Examples of the α-blocking agent include Tamsulosin, Prazosin, Tetrazosin, Doxazosin, Urapidil, Indoramin, Alfuzosin, Dapiprazole, Naftopidil, Ro 70-0004, KMD-3213, GYKI-16084, JTH-601, Z-350, Rec-15-2739, SK&F-86466, Bunazosin, BMY-15037, Buflomedil, Neldazosin, Moxisylyte, SL-890591, LY-23352, ABT-980, AIO-8507-L, L-783308, L-780945, SL-910893, GI-231818, SK&F-106686, RWJ-38063, Selodosin, Fiduxosin and the like.

Examples of the remedy for chronic cystitis of the bladder include Flavoxate hydrochloride and the like.

Examples of the remedy for constipation include Sennoside A/B, Phenovalin, and the like.

Examples of the remedy for colorectal cancer include Chromomycin A3, Fluorouracil, Tegafur, Krestin and the

like.

Examples of the remedy for uterine cancer include Chromomycin A3, Fluorouracil, Bleomycin hydrochloride, Medroxyprogesterone acetate and the like.

Examples of the remedy for diabetes include an insulin resistance improver, an insulin secretagogue, Biguanide agent, insulin, an α-glucosidase inhibitor, a β3-adrenalin receptor agonist and the like.

Examples of the insulin resistance improver include Pioglitazone or its salt (preferably hydrochloride), Troglitazone, Rosiglitazone or its salt, (preferably maleate), JTT-501, GI-262570, MCC-555, YM-440, DRF-2593, BM-13-1258, KRP-297, CS-011 and the like.

Examples of the insulin secretagogue include a sulfonyl urea agent and the like.

Specific examples of the sulfonyl urea agent include Tolbutamid, Chloropropamide, Tolazamide, acetohexamide, Glyclopyramide, and its ammonium salt, Glibendamide, Gliclazide, Glimepiride and the like. In addition to the above drugs, examples of the insulin secreagogue include Repaglinide, Nateglinide, KAD-1229, JTT-608 and the like.

Examples of the Biguanide agent include Metformin, Buformin and the like.

Examples of insulin include animal insulin extracted from the bovine or porcine pancreas; semi synthetic human insulin enzymatically synthesized from insulin extracted from the porcine pancreas; synthetic human insulin genetically engineered using *E. coli* and yeast, and the like. As the insulin, zinc insulin containing 0.45 to 0.9 (w/w)% of zinc; zinc protamine insulin produced from zinc chloride, protamine sulfate, and insulin and the like are also used. Further, insulin may be a fragment or a derivative thereof (for example, INS-1 and the like).

Examples of the α-glucosidase inhibitor include Acarbose, Voglibose, Miglitol, Emiglitate and the like.

Examples of the β3-adrenalin receptor agonist include AJ-9677, BMS-196085, SB-226552, SR-58611-A, CP-114271, L-755507 and the like.

In addition to the above, examples of the remedy for diabetes also include Ergoset, Pramlintide, Leptin, BAY-27-9955 and the like.

Examples of the remedy for cerebrovascular diseases include Nicaraven, Bencyclane fumarate, Eurnamonine, Flunarizine, Nilvadipine, Ibudilast, Argatroban, Nizofenone, Naftidrofuryl, Nicergoline, Nimodipine, Papaveroline, Alteplase, Viquidil hydrochloride, Moxisylyte, Pentoxifylline, Dihydroergotoxine mesylate, Lemildipine, Cyclandelate, Xanthinol nicotinate, Febarbamate, Cinnarizine, Memantine, Ifenprodil, Meclofenoxate hydrochloride, Ebselen, Clopidogrel, Nebracetam, Edaravone, Clinprost-DDS, Vatanidipine, Ancrod, Dipyridamole, and the like.

Examples of the remedy for spinal cord damage include Methylprednisolone, Dural graft matrix and the like.

Examples of the remedy for spinal cord tumor include Nimustine hydrochloride and the like.

Examples of the remedy for multiple sclerosis include Interferon-β-1b and the like.

Examples of the remedy for dementia including Alzheimer's disease include Antiracism, Arginine pyroglutamate, Nefiracetam, Nimodipine, Piracetam, Propentfylline, Vinpocetine, Indeloxazine, Vitamin E, Cinepazide, Memantine, Lisuride hydrogen malate, Pramiracetam, Zuclopenthixol, Protirelin, EGB-761, Acetyl-L-carnitine, Phosphatidylserine, Nebracetam, Taltireline, Choline alphoscerate, Ipidacrine, Talsaclidine, Cerebrolysin, Rofecoxib, ST-618, T-588, Tacrine, Physostigmine-DDS, Huperzine A, Donepezil, Rivastigmine, Metrifonate, TAK-147 and the like.

Examples of the remedy for Parkinson's disease include Talipexole, Amantadine, Pergolide, Bromocriptine, Selegiline, Mazaticol hydrochlolide, Memantine, Lisuride hydrogen malate, Trihexyphenidyl, Piroheptin hydrochlolide, Terguride, Ropinirole, Ganglioside-GM1, Droxidopa, Riluzole, Gabergoline, Entacapone, Rasagiline, Pramipexole, L-dopa-methylester, Tolcapone, Remacemide, Dihydroergocryptine, Carbidopa, Selegiline-DDS, Apomorphine, Apomorphine-DDS, Etilevodopa, Levodopa and the like.

Examples of the remedy for progressive supranuclear palsy include L-dopa, carbidopa, bromocriptine, pergolide, Lisuride, amitriptyline and the like.

Examples of the remedy for Guillan-Barre syndrome include TRH preparations such as steroid agent, protireline and the like.

Examples of the remedy for acute pan autonomic nerve abnormality include a steroid agent, L-threo-DOPS, dihydroergotamine, amezinium and the like.

Examples of the remedy for olivepontocerebeller atrophy include a TRH preparation, a steroid agent, midodorine, amezinium and the like.

Examples of the remedy for cervical spondylosis include anti-inflammatory analgesic drug and the like.

Examples of the "drug which is administered for treating another disease, but causes urinary disturbance by itself" include an analgesic drug (morphine, tramadol hydrochloride and the like), a centrally-acting skeletal muscle relaxant (baclofen and the like), a butyrophenone antipsychotic (haloperidol and the like), a remedy for overactive bladder (a remedy for frequent urine and incontinence of urine) (muscarine antagonists (anticholinergic agents) such as Oxybutynin hydrochloride, Propiverin hydrochloride, Tolterodine, Darifenacin, YM-905/YM-537, temiverin (NS-21), KRP-197, trospium, and the like; smooth muscle relaxants such as Flavoxate hydrochloride and the like; muscle relaxants such as NC-1800 and the like; Beta 2 agonists such as Clenbutol and the like; potassium channel opening agents such as ZD-0947, NS-8, KW-7158, WAY-151616 and the like; PGE2 antagonists such as ONO-8711 and the like; vanilloid

receptor agonists such as rezinipheratoxin, capsaicin and the like; tachykinin agonists such as TAK-637, SR-48968 (saredutant), SB-223412 (talnerant) and the like; deltaopioid agonist and the like), an antispasm drug (butylscopolamine bromide, tropium bromide, tiquizium bromide, timepidium bromide, propantheline bromide and the like), a remedy for digestive tract ulcer (Colantyl, Mesafirin, Cimetidine and the like), a remedy for Parkinson' disease (trihexylphenyzyl hydrochloride, piperidene, Mazaticol hydrochloride, Revodopa and the like), an antihistamine agent (diphenehydrazine, chlorphenylamine maleate, homochlorocyclizine hydrochloride, and the like), a tricyclic antidepressant agent (Imipramine hydrochloride, Amitriptyline hydrochloride, Clomipramine hydrochloride, Amoxapine, desipramine hydrochloride, and the like), a phenothiazine antipsychotic (Chlorpromazine, Properiazine, Levomepromazine, Thioridazine and the like), a benzodiazepine tranquilizer and sleeping sedative (Diazepam, Chlordiazepoxide, clothiazepam, Estazolam and the like), antiarrhythmic (disopyramide, and the like), a vasodilator (hydralazine hydrochloride and the like), a cerebral peripheral circulation improver (Pentoxyphylline and the like), a bronchodilator (Theophylline, Ephedrine hydrochloride, methylephedrine hydrochloride and the like), an β-adrenalin blocking agent (Propranolol hydrochloride and the like), a cold preparation (Danrich and the like), a peripheral skeletal muscle relaxant (Dantrolene sodium and the like), an antitubercular agent (isoniazid and the like), etc.

Among these combinations, preferable combination is that of crystals and 8-[3-[1-[(3-fluorophenyl)methyl]-4-piperidinyl]-1-oxopropyl]-1,2,5,6-tetrahydro-4H-pyrrolo[3,2,1-ij]quinolin-4-on or its salt with a α-blocking agent such as Tramsulosin, Prazosin, or the like.

When the compound of the present invention is used in combination with the above-described drug for treating a disease causing urinary disturbance or drug causing urinary disturbance, for example, (1) they may be formulated into a single preparation, if necessary, together with a pharmaceutically acceptable excipient according to a known pharmaceutical production process, (2) they may be formulated into separate preparations, if necessary, using pharmaceutically acceptable excipients, respectively, and the resulting preparations are used in combination simultaneously or at time interval(s), or (3) they may be formulated into respective separate preparation, if necessary, together with excipients according to a known to obtain a set of preparations (kit, etc.). In case of (2), the frequency of administration of respective preparations may be different from each other as long as the purpose of the present invention is achieved. The amounts of the effective ingredients in such preparations may be those within a range of effective amounts of the respective effective ingredients or within a pharmaceutically and pharmacologically acceptable range. Specifically, the amount is usually about 0.01 to about 100% by weight.

The dose of the preventive or remedy for urinary disturbance of the present invention varies depending on a particular administration subject, administration route, disease and the like, but for example, as the remedy for difficulty of urination, a single dose of an effective ingredient for an adult (approximately 60 kg of body weight) is about 0.005 to 100 mg, preferably about 0.05 to 30 mg, and more preferably about 0.2 to 10 mg as a preparation for oral administration. These can be administrated once, or divided into several times per a day.

When the drugs are used in combination, administration shall be based on the minimum recommended clinical dose of respective drugs and the dose can be appropriately selected depending on a particular administration subject, the age and body weight of the administration subject, symptom, administration time, administration method, dosage form, combination of drugs and the like. The dose regimen for a specific subject is determined in accordance with the age, body weight, general health conditions, gender, food, administration time, administration method, excretion rate, combination of drugs and degree of disease conditions of the subject to be treated, with considering those or other factors.

The daily dosage of a combination of the compound of the present invention and at least one compound selected from remedies for various diseases or its salt is typically within a range between about 1/50 of the minimum recommended clinical dose or more and below the maximum recommended level in case of administration of respective compounds alone.

The present invention also provides a rapid, simple and easy method for screening a substance exhibiting preventing or treating activity of urinary disturbance without inhibition of the collection function of urine.

The screening method of the present invention is characterized in measuring and comparing the AChE inhibitory activity and the BuChE inhibitory activity of a test compound. The measurement method of the AChE inhibitory activity and the BuChE inhibitory activity is not specifically limited, and any known method can be used. For example, a thiocholine method (Ellman method) and the like which are used in Example 1 described hereinafter are preferably mentioned. The 50% inhibitory concentration ($IC_{50}$) of respective enzymes is calculated based on the inhibitory activity (% of control) of the test compound at various concentrations, and a ratio of the $IC_{50}$ value of BuChE to the $IC_{50}$ value of AChE is determined. As a result, for example, a compound in which the fore-mentioned $IC_{50}$ ratio is about 20 or more, preferably about 100 or more, more preferably about 1000 or more, and most preferably about 10000 or more has the AChE inhibitory activity and has not the BuChE inhibitory activity, namely, it is selected as the candidate of a preventive or remedy for urinary disturbance which does not inhibit the collection function of urine. The preventive or treatment activity for urinary disturbance of these selected compounds can be examined by measuring various parameters at urination and the collection of urine by, for example, pressure/flow study using test animals (for example, refer to "The Journal of Urology", 1955, Vol.154, p.580: American Journal of Physiology 1955, Vol.269, p.98; Neurourology and Urodynamics,

1996, Vol.15, p.513) which is shown in Example 3 hereinafter.

Further, the compound of the present invention can be used alone, or in combination with another preventive or remedy (anticholinergic agent and the like) for overactive bladder and the like, for a prevention and treatment for urinary disturbance without being accompanied with dry mouth, for example, overactive bladder (for example, frequent urination, frequent urination at night, incontinence of urine and the like). The compound of the present invention can suppress a side effect, for example, dry mouth which is induced by administration of another drug, for example, a preventive or remedy for urinary disturbance (for example, anticholinergic agents such as Oxybutynin hydrochloride, Properibeline hydrochloride, Tolterodine, Daliphenacin, YM-905/YM-537, temiverine (NS-21), KRP-197, trospium, and the like).

Preferred examples of the screening method of a drug include the selection of a compound not influencing the collection function of urine by measuring the above-mentioned inhibitory activity of AChE and BuChE, the observation of antagonistic activity for sialoschesis induced by an anticholinergic agent described in Example 5. Further, for example, the influence on a main pharmacological effect (overactive bladder suppressing activity) of an anticholinergic agent by simultaneous administration of a selected compound can be examined by measuring the volume of the bladder by the method described in Example 6 and the like.

In the screening method, butyrylcholine esterase 50% inhibitory concentration for acetylcholine esterase 50% inhibitory concentration can be used as the basis of selection of a compound having acetylcholine esterase inhibitory activity with substantially free from butyrylcholine esterase inhibitory activity which does not influence the collection function of urine. The above-mentioned compound, for a prevention and treatment for urinary disturbance without being accompanied with dry mouth, for example, overactive bladder (for example, frequent urination, frequent urination at night, incontinence of urine and the like), 50% inhibitory concentration is about 20 or more, preferably about 100 or more, more preferably about 1000 or more, and most preferably about 10000 or more is effective for use in order to suppress side effects, for example, dry mouth which is induced by administration of another drug, for example, a preventive or remedy for urinary disturbance (for example, anticholinergic agents such as oxybutynin hydrochloride, Propiverine hydrochloride, Tolterodine, Daliphenacine, YM-905/YM-537, Temiverine (NS-21), KRP-197, trospium, and the like).

When the compound of the present invention and an anticholinergic agent which is used as a preventive or remedy for overactive bladder are used in combination, the ratio used can be changed by balance of the induction of a main pharmacological effect (overactive bladder suppressing action) and a side effect (dry mouth) of an anticholinergic agent in a patient which is an administration subject. Namely, when the dry mouth is serious, the dose of the compound of the present invention is high, and when the dry mouth is not so serious, it is low to the contrary. The specific ratio used of the compound of the present invention and another preventive or remedy for overactive bladder is 1 : 0.0001 to 10000, preferably 1 : 0.001 to 1000 and more preferably 1 : 0.01 to 100.

When the compound of the present invention is used for a preventive or remedy for urinary disturbance without being accompanied with dry mouth, or a preventive or remedy for a side effect induced by administration of another drug, the dose of the compound of the present invention varies depending on a particular administration subject, administration route, diseases and the like, but for example, the single dose of an effective ingredient for an adult (approximately 60 kg in body weight) is about 0.005 to 100 mg, preferably about 0.05 to 30 mg, and more preferably about 0.2 to 10 mg as a preparation for oral administration. These can be administrated once, or divided into several times per a day.

In case of using the compound of the present invention and another preventive or remedy for overactive bladder in combination, administration shall be based on the minimum recommended clinical doses of respective drugs and the dose can be suitably selected depending on a particular administration subject, the age and body weight of the administration subject, symptom, administration time, administration method, dosage form, combination of drugs and the like. The dose regimen for a specific subject is determined in accordance with the age, body weight, general health conditions, gender, food, administration time, administration method, excretion rate, combination of drugs and degree of disease conditions of the subject to be treated, with considering those or other factors.

The daily dosage of a combination of the compound of the present invention and at least one compound selected from remedies for various diseases or its salt is typically within a range between about 1/50 of the minimum recommended clinical dose or more and below the maximum recommended level in case of administration of respective compounds alone.

By combination of the compound of the present invention with another preventive or remedy for overactive bladder (hereinafter, abbreviated as the combined drug), superior effects such as

(1) the dose can be reduced in comparison with administrating the compound of the present invention or the combined drug alone,

(2) the drug to be used in combination with the compound of the present invention can be selected in accordance with the conditions of a subject (mild case, serious case and the like),

(3) the term for treatment can be set longer by selecting the compound of the present invention and the combined drug whose action mechanism is different,

(4) the effect of treatment can be sustained by selecting the compound of the present invention and the combined drug whose action mechanism is different,

(5) a synergistic effect is obtained by using the compound of the present invention in combination with the combined drug,

(6) dry mouth induced by administration of the combined drug is mitigated by using the compound of the present invention in combination with the combined drug, and the like.

Hereinafter, the combination use of the compound (I) of the present invention and the combined drug is referred to as "the combined agent of the present invention".

The administration timing of the compound of the present invention and the combined drug is not specifically limited at the time of using the combined agent of the present invention, and the compound of the present invention or its pharmaceutical composition and the combined drug or its pharmaceutical composition may be simultaneously administrated for an administration subject, or may be administrated at different time. The dose of the combined drug can be determined according to a dose clinically used, and can be appropriately selected depending a particular administration subject, administration route, disease, combination and the like.

The dosage form of the combined agent of the present invention is not specifically limited, and the compound of the present invention and the combined drug may be combined at the time of administration. Examples of such administration include (1) the administration of a single preparation prepared by simultaneously formulating the compound of the present invention and the combined drug, (2) the simultaneous administration of two different preparations prepared by separately formulating the compound of the present invention and the combined drug via the same administration route, (3) the administration of two different preparations prepared by separately formulating the compound of the present invention and the combined drug via the same administration route at time interval(s), (4) the simultaneous administration of two different preparations prepared by separately formulating the compound of the present invention and the combined drug, via different administration routes, (5) the administration of two different preparations prepared by separately formulating the compound of the present invention and the combined drug via different administration routes at time interval(s) (for example, administration in the order of the compound of the present invention and the combined drug, or vice versa), and the like.

The combined agent of the present invention has low toxicity and can be orally or parenterally administered (for example, topical, rectal, intravenous administration and the like) as pharmaceutical preparations, for example, tablets (including sugar-coated tablets and film coated tablets), powders, granules, capsules (including soft capsules), liquid agents, injectable preparations, suppositories and slow-releasing agents prepared by compounding the compound of the present invention and/or the above-mentioned combined drug with pharmacologically acceptable carriers according to a known method. The injectable preparations can be administrated intravenously, intramuscularly, subcutaneously or in organs, or directly to foci.

The pharmacologically acceptable carriers which may be used for production of the combined agent of the present invention include various organic and inorganic substances which are conventionally used as preparation materials, and examples thereof include an excipient, a lubricant, a binder and a disintegrant in a solid preparation, or a solvent, a solubilizing aid, a suspending agent, an isotonizing agent, a buffer and a soothing agent in liquid preparations, etc. Further, if necessary, usual preparation additives such as an antiseptic, an antioxidant, a colorant, a sweetening agent, an adsorbing agent and a wetting agent can be also used.

Examples of the excipient include lactose, white sugar, D-mannitol, starch, corn starch, crystalline cellulose, soft anhydrous silicic acid and the like.

Examples of the lubricant include magnesium stearate, calcium stearate, talc, colloidal silica and the like.

Examples of the binder include crystalline cellulose, white sugar, D-mannitol, dextrin, hydroxypropylcellulose, hydroxypropylmethylcellulose, polyvinyl pyrrolidone, starch, sucrose, gelatin, methylcellulose, carboxymethylcellulose sodium, and the like.

Examples of the disintegrant include starch, carboxymethylcellulose, carboxydimethylcellulose calcium, carboxymethylstarch sodium, L-hydroxypropylcellulose and the like.

Examples of the solvent include water for injection, an alcohol, propylene glycol, macrogol, sesame oil, corn oil, olive oil and the like.

Examples of the solubilizing aid include polyethylene glycol, propylene glycol, D-mannitol, benzyl benzoate, ethanol, trisaminomethane, cholesterol, triethanolamine, sodium carbonate, sodium succinate and the like.

Examples of the suspending agent include surfactants such as stearyltriethanolamine, sodium laurylsulfate, laurylaminopropionic acid, lecithin, benzalkonium chloride, benzetonium chloride and glycerin monostearate; hydrophilic synthetic polymers, for example, such as a polyvinyl alcohol, polyvinyl pyrrolidone, carboxymethylcellulose sodium, methylcellulose, hydroxymethylcellulose, hydroxyethylcellulose, hydroxypropylcellulose and the like.

Examples of the isotonizing agent include glucose, D-sorbitol, sodium chloride, glycerin, D-mannitol and the like.

Examples of the buffer include buffers of a phosphate, an acetate, a carbonate, a succinate and the like.

Examples of the soothing agent include benzyl alcohol and the like.

Examples of the antiseptic include para-oxybenzoic acid esters, chlorobutanol, benzyl alcohol, phenethyl alcohol, dehydroacetic acid, sorbic acid and the like.

Examples of the antioxidant include a bisulfate, an ascorbate, $\alpha$-tocopherol and the like.

The compounding ratio of the compound of the present invention and the combined drug in the combined agent of the present invention can be appropriately selected according to a particular administration subject, administration route, disease and the like.

For example, the content of the compound of the present invention in the combined agent of the present invention varies depending on the dosage form, but is usually about 0.01 to 100% by weight, preferably about 0.1 to 50% by weight, more preferably about 0.5 to 20% by weight based on the total preparation.

The content of the combined drug in the combined agent of the present invention varies depending on the dosage form, but is usually about 0.01 to 100% by weight, preferably about 0.1 to 50% by weight, more preferably about 0.5 to 20% by weight based on the total preparation.

The content of additives such as carriers in the combined agent of the present invention varies depending on the dosage form, but is usually about 1 to 99.99% by weight, preferably about 10 to 99% based on the total preparation and.

Further, when the compound of the present invention and the combined drug are separately formulated, respectively, the similar content may be used.

These preparations can be produced by a per se known method usually employed in the production steps.

For example, the compound of the present invention or the combined drug can be formulated into a water-soluble injectable preparation together with a dispersant (for example, Tween 80 (manufactured by Atlas Powder Co., USA), HCO 60 (manufactured by Nikko Chemicals Co., Ltd.), polyethylene glycol, carboxymethylcellulose, sodium arginate, hydroxypropylmethylcellulose, dextrin and the like), a stabilizer (for example, ascorbic acid, sodium pyrosulfite and the like), a surfactant (for example, Polysolvate 80, macrogol and the like), a dissolving agent (for example, glycerin, ethanol and the like), a buffer (for example, phosphoric acid and its alkali metal salt, succinic acid and its alkali metal salt and the like), a tonicity agent (for example, sodium chloride, potassium chloride, mannitol, sorbitol, glucose and the like), a pH adjuster (for example, hydrochloric acid, sodium hydroxide and the like), a preservative (for example, ethyl para-oxybenzoate, benzoic acid, methylparaben, propylene paraben, benzyl alcohol and the like), a solubilizing agent (for example, concentrated glycerin, meglumine and the like), a solubilizing aid (for example, propyleneglycol, white sugar and the like), a soothing agent (for example, glucose, benzyl alcohol and the like), or as an oily injectable preparation by being dissolved, suspended or emulsified in vegetable oil such as olive oil, sesame oil, cottonseed oil and corn oil, and a solubilizing agent such as propyleneglycol to prepare an injectable preparation.

For producing a preparation for oral administration, for example, the preparation for oral administration can be produced by adding, to the compound of the present invention and the combined drug, an excipient (for example, lactose, white sugar, starch and the like), a disintegrant (for example, starch, calcium carbonate and the like), a binder (for example, starch, gum arabic, carboxymethylcellulose, polyvinyl pyrrolidone, hydroxypropylcellulose and the like) or a lubricant (for example, talc, magnesium stearate, polyethylene glycol 6000 and the like) and the like, compression-molding the mixture, and then, if necessary, coating the mixture by a known method using a coating base for the purpose of masking taste, enteric property or sustainability. As the coating agent, there can be used hydroxypropylmethylcellulose, ethylcellulose, hydroxymethylcellulose, hydroxypropylcellulose, polyoxyethylenglycol, Tween 80, Pluronic F68, cellulose acetate phthalate, hydroxypropylmethylcellulose phthalate hydroxypropylmethylcellulose succinate, Eudragit (manufactured by Rohm Pharma Co., Germany, a methacrylic acid-acrylic acid copolymer) and a dye (for example, colcothar, titanium oxide and the like), and the like. The preparation for oral administration may be a rapid-releasing preparation or a sustained-release preparation.

For example, in order to obtain a suppository, the compound of the present invention and the combined drug can be prepared in the form of an oily or aqueous solid, semisolid or liquid suppository according to a per se known method. Examples of an oily base used for the above-mentioned composition include glycerides of higher aliphatic fatty acids [for example, cacao butter, witteb sols (manufactured by Dynamite Nobel AG, Germany) and the like], middle chain aliphatic fatty acids [for example, Migriols (manufactured by Dynamite Nobel AG, Germany) and the like], or vegetable oil (for example, sesame oil, soy bean oil, cotton oil and the like), and the like. Further, examples of an aqueous base include polyethylene glycols and propylene glycol, and examples of an aqueous gel base include natural gum, a cellulose derivative, a vinyl polymer, an acrylic acid and the like.

The above-mentioned sustained-release preparation includes a sustained-release microcapsule preparation and the like.

A per se known method can be employed for preparing the sustained-release microcapsule preparation, but for example, preferably, the preparation is molded into the sustained-release preparation of [2] as shown hereinafter and administered.

It is preferable that the compound of the present invention is molded for the preparation for oral administration such as solid preparations (for example, powders, granules, tablets, capsules and the like), or molded for preparations

for rectal administration such as a suppository. In particular, the preparation for oral administration is preferable.
The combined drug can be prepared in the above-mentioned dosage form in accordance with a particular kind of drugs.
Hereinafter, [1] the injectable preparation of the compound of the present invention or the combined drug and its production, [2] the sustained-release preparation or rapid-release preparation of the compound of the present invention or the combined drug and its production, and [3] the sublingual tablet, buccal or intraoral rapid disintegrating preparation of the compound of the present invention or the combined drug and its production will be specifically illustrated.

[1] Injectable preparation and its production

An injectable preparation obtained by dissolving the compound of the present invention or the combined drug in water is preferable. The injectable preparation may contain benzoate and/or salicylate.
The injectable preparation is obtained by dissolving both of the compound of the present invention or the combined drug and, if necessary, benzoate and/or salicylate in water.
Examples of the benzoate and/or salicylate include a salt of an alkali metal such as sodium, potassium and the like, a salt of an alkaline earth metal such as magnesium, calcium and the like, an ammonium salt, a meglumine salt, organic acid salts such as Tromethamol and the like.
The concentration of the compound of the present invention or the combined drug in the injectable preparation is about 0.5 to 50% by w/v, preferably about 3 to 20% by w/v. Further, the concentration of benzoate and/or salicylate is about 0.5 to 50% by w/v, preferably about 3 to 20% by w/v.
Further, in the injectable preparation, there can be suitably compounded, for example, a stabilizer (for example, ascorbic acid, sodium pyrosulfite and the like), a surfactant (for example, Polysolvate 80, macrogol and the like), a dissolving agent (for example, glycerin, ethanol and the like), a buffer (for example, phosphoric acid and its alkali metal salt, succinic acid and its alkali metal salt and the like), a tonicity agent (for example, sodium chloride, potassium chloride, and the like), a dispersant (for example, hydroxypropylmethylcellulose, dextrin and the like), a pH adjuster (for example, hydrochloric acid, sodium hydroxide and the like), a preservative (for example, ethyl para-oxybenzoate, benzoic acid and the like), a solubilizing agent (for example, concentrated glycerin, meglumine and the like), solubilizing aid (for example, propyleneglycol, white sugar and the like), a soothing agent (for example, glucose, benzyl alcohol and the like), and the like. These additives are compounded at a proportion by which they are generally used for an injectable preparation.
Preferably, the injectable preparation is adjusted at pH of 2 to 12, preferably 2.5 to 8.0 by addition of a pH adjuster.
The injectable preparation is obtained by dissolving the compound of the present invention or the combined drug and, if necessary, benzoate and/or salicylate, and further, if necessary, the above-mentioned additives in water. Dissolution thereof can be carried out in any order, and can be appropriately carried out in the same manner as the production of a conventional injectable preparation.
The aqueous solution for injection is preferably warmed and, as with a conventional injectable preparation, for example, sterilization by filtration, heat sterilization under high pressure can be carried out to obtain the injectable preparation.
Preferably, the aqueous solution for injection is sterilized by heating under high pressure, for example, under conditions of 100°C to 121°C for 5 minutes to 30 minutes.
Antibiotic properties may be imparted to the preparation so that it can be used as a preparation which is divided for administration many times.

[2] Sustained-release preparation or rapid-release preparation and its production

The sustained-release preparation obtained by coating nuclei containing the compound of the present invention or the combined drug, with a coating agent such as a water-insoluble substance or a swelling polymer is preferable. For example, the sustained-releasing preparation for oral administration which is administrated once a day is preferable.
Examples of the water-insoluble substance used for the coating agent include cellulose ethers such as ethylcellulose, butylcellulose and the like; cellulose esters such as cellulose acetate, cellulose propionate and the like; polyvinyl esters such as polyvinyl acetate, polyvinyl butylate and the like; acrylic acid polymers such as a acrylic acid-methacrylic acid copolymer, methylmethacrylic copolymer ethoxyethylmethacrylate-cinnamoethylmethacrylate-aminoalkylmethacrylate copolymer, polyacrylic acid, polymethacrylic acid, a methacrylic acid alkyl amide copolymer, a poly(methyl methacrylate), a polymethacrylate, a polymethacryl amide, an aminoalkyl acrylate copolymer, poly(methacrylic acid anhydride), a glycidyl methacrylate copolymer, in particular, Eudragit (Rohm Pharma Co.) such as Eudragit RS-100, RL-100, RS-30D, RL-30D, RL-PO, RS-PO (ethyl acrylate-methyl methacrylate-chlorotrimethylammoniumethyl methacrylate copolymer), Eudragit NE-30D (methyl methacrylate-ethyl acrylate copolymer); hardened oil such as hardened ricinus (for example, LOVELY WAX (Freund Industries Co., Ltd.) and the like), waxes such as carnauba wax, fatty acid glycerin

ester and paraffin, polyglycerin fatty acid ester and the like.

The swelling polymer is preferably a polymer which has an acidic dissociation group and shows swelling depending on pH, and a polymer having an acidic dissociation group whose swelling is little in an acidic region such as the stomach and whose swelling is enlarged at a neutral region such as the small bowel and large bowel.

Examples of the polymer which has an acidic dissociation group and shows swelling depending on pH include crosslinking type polyacrylic acid polymers such as Carbomer 934P, 940, 941, 974P, 980, 1324 and the like, Polycarbophil, Calcium Polycarbophil (either of then is manufactured by BF Goodrich Co., Ltd.), HIGHBISWAKO 103, 104, 105, 304 (any of them is manufactured by Wako Chemical Industries, Ltd.).

The coating agent used for the sustained-release preparation may further contain a hydrophilic substance.

Examples of the hydrophilic substance include polysaccharides such as plulan, dextrin and an alkali metal salt of arginic acid which may optionally have a sulfuric acid group; polysaccharides such as hydroxypropylcellulose, hydroxypropylmethylcellulose and sodium carboxymethylcellulose which has a hydroxyalkyl group or a carboxyalkyl group; methylcellulose, a polyvinyl pyrrolidone, a polyvinyl alcohol, a polyethylene glycol and the like.

The content of the water-insoluble substance in the coating agent of the sustained-release preparation is about 30 to 90% (w/w), preferably about 35 to about 80% (w/w), more preferably about 40 to 75% (w/w), and the content of the swelling polymer is about 3 to 30% (w/w), preferably about 3 to about 15% (w/w). The coating agent may further contain the hydrophilic substance and in that case, the content of the hydrophilic substance in the coating agent is about 50% (w/w) or less, preferably about 5 to about 40% (w/w), more preferably about 5 to 35% (w/w). The above-mentioned % (w/w) indicates % by weight for the coating agent composition in which a solvent (for example, water, lower alcohols such as methanol and ethanol, and the like) is excluded from the coating solution.

As exemplified below, the sustained-release preparation is produced by preparing nuclei containing a drug, and then coating the nuclei obtained with a coating solution in which the water-insoluble substance and the swelling polymer were dissolved by heating, or dissolved or dispersed in a solvent.

I. Preparation of nuclei containing drug

The form of the nuclei containing a drug which is coated with a coating agent (hereinafter, sometimes, merely referred to as the nuclei) is not specifically limited, but is formed preferably into particles such as granules or fine particles.

When the nuclei are granules or fine particles, their mean particle diameter is preferably about 150 to 2000 μm, more preferably about 500 to 1400 μm.

The nuclei can be prepared by a conventional production method. For example, a drug is mixed with an appropriate excipient, a binder, disintegrant, a lubricant, a stabilizing agent and the like, followed by subjecting to a wet extrusion granulation method, a fluidized bed granulation method and the like to prepare the nuclei.

The content of the drug in the nuclei is about 0.5 to 95% (w/w); preferably about 5.0 to about 80% (w/w), more preferably about 30 to about 70% (w/w).

Examples of the excipient contained in the nuclei include sugars such as lactose, white sugar, mannitol and glucose, starch, crystalline cellulose, calcium phosphate, corn starch and the like. Among these, crystalline cellulose and corn starch are preferable.

Examples of the binder include a polyvinyl alcohol, hydroxypropylcellulose, polyethylene glycol, polyvinyl pyrrolidone, Pluronic F68, gum Arabica, gelatin, starch and the like. Examples of the disintegrant include carboxydimethylcellulose calcium (ECG 505), Crosscaromerose sodium (Ac-Di-Sol), a crosslinking polyvinyl pyrrolidone (Crosspovidone), low substituted hydroxypropylcellulose (L-HPC) and the like. Among these, hydroxypropylcellulose, polyvinyl pyrrolidone and low substituted hydroxypropylcellulose are preferable. Examples of the lubricant and coagulation preventive agent include talc, magnesium stearate and its inorganic salts, and as a lubricating agent, polyethylene glycol and the like are used. Examples of the stabilizer include acids such as tartaric acid, citric acid, succinic acid, fumaric acid and maleic acid.

In addition to the above methods, the nuclei can also be prepared, for example, by a rolling granulation method, a pancoating method, a fluidized bed coating method, and a melt granulation method, wherein the drug or a mixture of the drug, an excipient, a lubricant and the like is added little by little to inactive carrier particles which become the center of the nuclei, while spraying a binder dissolved in an appropriate solvent such as water, a lower alcohol (for example, methanol, ethanol and the like), etc. As the inactive carrier particles, for example, those prepared from white sugar, lactose, starch, crystalline cellulose and waxes can be used. Their mean particle diameter is preferably about 100 to about 1500 μm.

The surface of the nuclei may be coated with a protective agent in order to separate the coating agent and the drug contained in the nuclei. As the protective agent, for example, the fore-mentioned hydrophilic substance, water-insoluble substance and the like are used. Examples of the protective agent include, preferably, polyethylene glycol, polysaccharides having a hydroxy alkyl group or a carboxyalkyl group, more preferably, hydroxypropylmethylcellulose and hydroxypropylcellulose. The protective agent may contain an acid such as tartaric acid, citric acid, succinic acid,

fumaric acid and maleic acid as a stabilizer, and a lubricant such as talc. When the protective agent is used, the coating amount is about 1 to about 15% (w/w), preferably about 1.0 to about 10% (w/w), more preferably about 2 to about 8% (w/w) based on the nuclei.

The protective agent can be coated by a conventional coating method. Specifically, the protective agent can be coated on the nuclei by spray-coating according to a fluidized bed coating method, a pancoating method and the like.

II. Coating of nuclei with coating agent

The sustained-release preparation is produced by coating the nuclei obtained in the above I with a coating solution in which the above water-insoluble substance, the swelling polymer having pH dependency and hydrophilic substance are dissolved by heating, or dissolved or dispersed in a solvent.

As a coating method of the nuclei with the coating agent, for example, a method of spray-coating and the like are employed.

The composition ratio of the water-insoluble substance and the swelling polymer or the hydrophilic substance in the coating agent solution is appropriately selected so that the contents of respective ingredients in the coating become the above-mentioned contents.

The coating amount of the coating agent (without the coating amount of the protective agent) is about 1 to about 90% (w/w), preferably about 5 to about 50% (w/w), more preferably about 5 to about 35% (w/w) based on the nuclei.

As the solvent of the coating agent, water or an organic solvent can be used alone or a mixture of both can be used. The mixing ratio (water/organic solvent: by weight) of water to the organic solvent upon use of the mixture can be varied within a range of 1 to 100%, preferably 1 to about 30%. The organic solvent is not specifically limited as long as it dissolves the water-insoluble substance, but lower alcohol such as methyl alcohol, ethyl alcohol, isopropyl alcohol and n-butyl alcohol, lower alkanone such as acetone, acetonitrile, chloroform, methylene chloride and the like are used. Among these, the lower alcohol is preferable and in particular, ethyl alcohol and isopropyl alcohol are preferable. Water and the mixed solution of water and an organic solvent are preferably used as the solvent of the coating agent. At this time, an acid such as tartaric acid, citric acid, succinic acid, fumaric acid and maleic acid may be added to the coating agent solution in order to stabilize the coating agent, if necessary.

The coating operation by spray-coating can be carried out by a conventional coating method, and specifically, can be carried out by spray-coating the coating agent on the nuclei, for example by a fluidized bed coating method, a pancoating method and the like. At this time, if necessary, talc, titanium oxide, magnesium stearate, calcium stearate, soft silicic acid anhydride and the like may be added as a lubricant and glycerin fatty acid ester, hardened ricinus, triethyl citrate, cetyl alcohol, stearyl alcohol and the like may be added as a plasticizer.

After coating with the coating agent, an antistatic agent such as talc may be mixed, if necessary.

The rapid-release preparation may be liquid (solution, suspension, emulsion and the like), or solid (granules, pills, tablets and the like). A preparation for oral administration and a preparation for parenteral administration such as an injectable preparation are used but the preparation for oral administration is preferable.

The rapid-release preparation may usually contain a carrier, an additive and an excipient (hereinafter, sometimes, referred to as the excipient) conventionally used in the preparation field, in addition to the drug as the effective ingredient. The excipient of the preparation used is not specifically limited as long as it can be used as the excipient of the preparation. Examples of the excipient for an oral solid preparation includes lactose, starch, corn starch, crystalline cellulose (ABISEL PH 101 and the like: manufactured by Asahi Kasei Corporation), powder sugar, granulated sugar, mannitol, soft silicic acid anhydride, magnesium carbonate, calcium carbonate, L-cysteine and the like, and corn starch and mannitol are preferred. These excipients can be used alone or a combination of 2 or more thereof can be used. The content of the excipient is about 4.5 to about 99.4% (w/w), preferably about 20 to about 98.5% (w/w), more preferably about 30 to about 97% (w/w) based on the total amount of the rapid-release preparation.

The content of the drug in the rapid-release preparation can be appropriately selected from a range of about 0.5 to about 95% (w/w), preferably about 1 to about 60% (w/w) based on the total amount of the rapid-release preparation.

When the rapid-release preparation is an oral solid preparation, it contains usually a disintegrant in addition to the above-mentioned ingredients. Examples of the disintegrant include carboxymethylcellulose calcium, (ECG-505 manufactured by Gotoku Chemical Co., Ltd.), crosscarmerose Sodium (for example, Aczisol manufactured by Asahi Kasei Corporation), Crosspovidone (for example, Coridon CL manufactured by BASF AG), low substituted hydroxypropylcellulose (Shin-Etsu Chemical Co., Ltd.), carboxymethylstarch (Matsutani Chemical Industry Co., Ltd.), carboxymethylstarch sodium (Kimura Sangyo Co., Ltd. EXISPROTAB), partial α-starch (PCS manufactured by Asahi Kasei Corporation) and the like. For example, there can be used a disintegrant which disintegrates granules by water absorption and swelling in contact with water, or forming channels between the effective ingredient and the excipient composing the nuclei. These disintegrants can be used alone or a combination of 2 or more thereof can be used. The amount of the disintegrant to be formulated is appropriately selected according to a particular kind and amount of the drug to be used, releasing preparation design and the like, but is about 0.05 to about 30% (w/w), preferably about 0.5 to about 15% (w/w) based

on the total amount of the rapid-release preparation.

When the rapid-release preparation is an oral solid preparation, it may further contain additives conventionally used in the solid preparation, if necessary, in addition to the above-mentioned composition. Examples of the additives include a binder (for example, sucrose, gelatin, gum arabic, methylcellulose, hydroxypropylcellulose, hydroxypropylmethylcellulose, carboxymethylcellulose, polyvinyl pyrrolidone, plulan, dextrin, and the like), a lubricant (for example, polyethylene glycol, magnesium stearate, talc, soft silicic acid anhydride (for example, AEROSIL (Nippon Aerosil Co.))), a surfactant (for example, anionic surfactants such a sodium alkyl sulfate, nonionic surfactants such polyoxyethylene fatty acid ester, polyoxyethylenesorbitan fatty acid ester, a polyoxyethylene ricinus derivative), a colorant (for example, a tar dye for food, caramel, colcothar, titanium oxide, riboflavins), if necessary, a seasoning agent (a sweetening agent, flavor, and the like), an adsorbing agent, an antiseptic, a wetting agent, an antistatic agent and the like. Further, organic acids such as tartaric acid, citric acid, succinic acid and fumaric acid are added as a stabilizer,

As the above-mentioned binder, hydroxypropylcellulose, polyethylene glycol, polyvinyl pyrrolidone and the like are preferably used.

The rapid-release preparation can be prepared by mixing the above-mentioned respective ingredients, further kneading, if necessary, and molding the mixture according to the production technique of conventional preparations. The above-mentioned mixing is carried out by methods usually used, for example, mixing, kneading and the like. Specifically, for example, when the rapid-release preparation is formed into particles, it can be prepared by mixing them using a vertical granulator, a universal kneader (manufactured by Hata Iron Works Co., Ltd.), a fluidized bed granulator FD-5S (manufactured by Powrec Co.), and then granulating the mixture by a wet extrusion granulation method, a fluidized bed granulation method and the like according to a similar procedure similar as the preparation method of the above-mentioned sustained-release preparation.

The rapid-release preparation and sustained-release preparation thus obtained are as they are, or separately formulated into preparations together with the excipient and the like by a conventional method, and may be used as the preparations which are simultaneously administrated or administrated in combination at arbitrary administration interval (s). Alternatively, they may be appropriately formulated into one preparation for oral administration (for example, granules, fine particles, tablets, capsules and the like) together with the excipient and the like. Both preparations may be formulated into granules or fine particles and filled in the same capsules to prepare a preparation for oral administration.

[3] Sublingual tablet, buccal or intraoral rapid disintegrant and its production

The sublingual tablet, buccal and intraoral rapid disintegrant may be a solid preparation such as tablets or may be oral mucosa adhesive patch (film).

As the sublingual tablet, buccal or intraoral rapid disintegrant, the preparation containing the compound of the present invention or the combined drug and excipient is preferable. Further, it may contain aids such as a lubricant, an isotonizing agent, a hydrophilic carrier, a water dispersing polymer and a stabilizer. Further, it may contain β-cyclodextrin or β-cyclodextrin derivative (hydroxypropyl-β-cyclodextrin and the like) and the like to ease the absorption and to enhance the utilization ratio within a living body.

The above-mentioned excipient includes lactose, white sugar, D-mannitol, starch, crystalline cellulose, light anhydrous silicate and the like. The lubricant includes magnesium stearate, calcium stearate, talc, colloidal silica and the like, and in particular, magnesium stearate and colloidal silica are preferable. The isotonizing agent includes sodium chloride, glucose, fructose, mannitol, sorbitol, lactose, saccharose, glycerin, urea and the like, and in particular, mannitol is preferable. The hydrophilic carrier includes swelling hydrophilic carriers such as crystalline cellulose, ethylcellulose, a crosslinking polyvinyl pyrrolidone, light anhydrous silicate, silicic acid, dicalcium phosphate and calcium carbonate, and in particular, crystalline cellulose (in particular, fine crystalline cellulose) is preferable. The water dispersing polymer includes gum (for example, tragacanth gum, acacia gum, guar gum), arginate (for example, sodium arginate), a cellulose derivative (for example, methylcellulose, carboxymethylcellulose, hydroxymethylcellulose, hydroxypropylcellulose, hydroxypropylmethylcellulose), gelatin, water soluble starch, polyacrylic acid (for example, carbomer), polymethacrylic acid, polyvinyl alcohol, polyethylene glycol, polyvinyl pyrrolidone, polycarbophyll, ascorbate, palmitate and the like, and in particular, hydroxypropylmethylcellulose, polyacrylic acid, arginate, gelatin, carboxymethylcellulose, polyvinyl pyrrolidone and polyethylene glycol are preferable. In particular, hydroxypropylmethylcellulose is preferable. The stabilizer includes cysteine, thiodorbitol, tartaric acid, citric acid, sodium carbonate, ascorbic acid, glycine, sodium sulfite and the like, and in particular, citric acid and ascorbic acid are preferable.

The sublingual tablet, buccal or intraoral rapid disintegrant can be produced by mixing the compound of the present invention or the combined drug and the excipient by a known method. Further, the above-mentioned lubricant, isotonizing agent, hydrophilic carrier, water dispersing polymer, stabilizer, auxiliary substances such as a colorant, a sweetening agent and an antiseptic may be mixed if necessary. The sublingual tablet, buccal or intraoral fast disintegrant is obtained by mixing the above-mentioned ingredients simultaneously or at time difference, and then carrying out pressured tablet compression. In order to obtain appropriate hardness, they may be wetted and swollen before and after

the tablet compression using a solvent such as water or alcohol, if necessary, and dried after molding to be produced.

When they are molded as the mucosa adhesive patch (film), the film is prepared by dissolving the compound of the present invention or the combined drug and the above-mentioned water dispersing polymer (preferably, hydroxypropylcellulose, hydroxypropylmethylcellulose), excipient and the like in a solvent such as water and flow-casting the solution obtained. Further, additives such as a plasticizer, a stabilizer, an antioxidant, a preserving agent, a colorant, a buffer and a sweetening agent may be added. The film may contain glycols such as polyethylene glycol and polypropylene glycol in order to obtain appropriate elasticity to the film and contain an organism adhesive polymer (for example, polycarbophyll, carbopol) in order to enhance the adhesion of the film to oral mucosa lining. The flow casting is attained by pouring the solution on non-adhesive surface, spreading it at a homogeneous thickness (preferably about 10 to 100 μm) with a doctor blade and the like, and then drying the solution to form a film. The film thus formed is dried at room temperature or under heating and cut at a desired surface area.

The preferable intraoral rapid disintegrant includes a solid rapid spread administration agent consisting of a net body of the compound of the present invention or the combined drug and the water-soluble or water dispersing carrier which is inactive with the compound of the present invention or the combined drug. The net body is obtained by subliming a solvent from a solid composition which is composed of a solution which is obtained by dissolving the compound of the present invention or the combined drug in an appropriate solvent.

The composition of the intraoral rapid disintegrant contains preferably a matrix forming agent and a secondary ingredient in addition to the compound of the present invention or the combined drug.

The matrix forming agent includes animal proteins or plant proteins such as gelatins, dextrins, soy beans, wheat and psyllium; rubber substances such as gum arabic, guar gum, agar and xanthene gum; polysaccharides; arginic acids; carboxymethylcelluloses; carrageenans; dextranes; pectines; synthetic polymers such as polyvinyl pyrrolidone; substances induced from gelatin-gum arabic complex and the like. Further, it includes saccharides such as mannitol, dextrose, lactose, galactose and trehalose; cyclic saccharides cyclodextrin; inorganic salts such as sodium phosphate, sodium chloride and aluminum silicate; amino acids having 2 to 12 carbons such as glycine, L-alanine, L-asparagic acid, L-glutamic acid, L-hydroxyproline, L-isoleucine, L-leucine and L-phenylalanine; and the like.

One or more of the matrix forming agents can be introduced in a solution or suspension before solidification. The matrix forming agent may exist by being added in a surfactant and may exist excluding the surfactant. The matrix forming agent can aid to keep the dispersion condition of the compound of the present invention or the combined drug in the solution or dispersion, in addition to form matrix.

The composition may contain secondary ingredients such as a preserving agent, an antioxidant, a surfactant, a thickener, a colorant, a pH adjuster, a sweetening agent, a flavoring or an eating quality masking agent. The appropriate colorant includes red, black and yellow iron oxides and FD & C dyes such as FD & C Blue No. 2 and FD & C Red No. 40 manufactured by Elis & Everard Ltd. The appropriate flavoring includes mint, raspberry, licorice, orange, lemon, grape fruit, caramel, vanilla, cherry and grape flavor and a combination thereof. The appropriate pH adjuster includes citric acid, tartaric acid, phosphoric acid, hydrochloric acid and maleic acid. The sweetening agent includes Aspartame, Acesulfame K and thaumatine. The appropriate eating quality masking agent includes sodium bicarbonate, an ion exchange resin, a cyclodextrin clathrate compound, an adsorbing substance and microcapsulated Apomorphine.

The drug contains usually about 0.1 to about 50% by weight of the compound of the present invention or the combined drug and preferably about 0.1 to about 30% by weight, and is preferably the drug (the above-mentioned sublingual tablet, buccal and the like) which can dissolve 90% by weight or more of the compound of the present invention or the combined drug for about 1 minute to about 60 minutes, preferably for about 1 minutes to about 15 minutes and more preferably for about 2 minutes to about 5 minutes, and preferably the intraoral rapid disintegrant which is disintegrated within 1 to 60 seconds, preferably within 1 to 30 seconds, more preferably within 1 to 10 seconds.

The above-mentioned excipient for the whole preparation is about 10 to about 99% by weight, preferably about 30 to about 90% by weight. The β-cyclodextrin or β-cyclodextrin derivative for the whole preparation is 0 to about 30% by weight. The lubricant for the whole preparation is about 0.01 to about 10% by weight, preferably about 1 to about 5% by weight. The isotonizing agent for the whole preparation is about 0.1 to about 90% by weight, preferably about 10 to about 70% by weight. The hydrophilic carrier for the whole preparation is about 0.1 to about 50% by weight, preferably about 10 to about 30% by weight. The water dispersing polymer for the whole preparation is about 0.1 to about 30% by weight, preferably about 10 to about 25% by weight. The stabilizer for the whole preparation is about 0.1 to about 10% by weight, preferably about 1 to about 5% by weight. The above-mentioned preparation may further contain additives such as a colorant, a sweetening agent and an antiseptic, if necessary.

The dose of the combined drug of the present invention differs depending on the kind of the compound of the present invention, an age, a weight, symptom, a form, administration method, administration term and the like, but for example, as the oral agent of the compound of the present invention and the combined drug, the dose per one time for an adult with overactive bladder (an adult, approximately 60 kg in body weight) is respectively about 0.005 to about 100 mg as an effective ingredient, preferably about 0.05 to about 30 mg, and more preferably about 0.2 to about 10 mg. These can be administrated once, or at several times per a day.

When the pharmaceutical of the present invention is administrated, it may be administrated at the same timing, but after the combined drug is administrated in advance, the compound of the present invention may be administrated, and after the compound of the present invention is administrated, the combined drug may be administrated. When it is administrated at time difference, the time difference differs depending on the effective ingredient, the form of preparation and the administration method, but for example, when the combined drug is administrated in advance, there is mentioned a method in which the compound of the present invention is administrated within 1 minute to 3 days, preferably 10 minutes to 1 day, more preferably 15 minutes to 1 hour after administrating the combined drug. When the compound of the present invention is administrated in advance, there is mentioned a method in which the compound of the present invention is administrated within 1 minute to 1 day, preferably 10 minutes to 6 hours and more preferably 15 minutes to 1 hour after administrating the compound of the present invention.

As the preferable administration method, for example, about 0.001 to 200 mg/kg of the combined drug which was formulated as the preparation for oral administration is orally administrated, and about 0.005 to 100 mg/kg of the compound of the present invention which was formulated as the preparation for oral administration is orally administrated as a dose per day after about 15 minutes.

## Best Mode for Carrying Out the Invention

The present invention will be more specifically illustrated by the following Examples, Preparation Examples, Reference Preparation Examples and the like, but these are merely exemplification and the scope of the present invention is not limited thereto.

### Example 1: Acetylcholine esterase selectivity of various drugs

The AChE inhibitory activity and the BuChE inhibitory activity were determined by a thiocholine method (Ellman method) using acetylcholine esterase of human erythrocyte origin (Sigma, St. Louis, MO, USA) and butyrylcholine esterase of human serum origin (Biogenesis, Poole, UK). Acetylcholine esterase or butyrylcholine esterase was diluted to a concentration of 0.2 IU/mL with 80 mM tris-HCL buffers (pH = 7.4) containing 0.3% bovine serum albumin (BSA) to prepare an enzyme preparation. The test drugs [8-[3-[1-[(3-fluorophenyl)methyl]-4-piperidinyl]-1-oxopropyl]-1,2,5,6-tetrahydro-4H-pyrrolo[3,2,1-ij]quinolin-4-one] (hereinafter, abbreviated as the compound A), neostigigmine, distigmine or iso-OMPA were weighed in a spitz roll with an electronic weighing machine, dimethylsulfoxide (DMSO) was added thereto to dissolve it at a concentration of $10^{-2}$ M, thereby preparing a drug stock solution. The stock solution was diluted at 3-fold common ratio with 10% DMSO and 80 mM tris-HC1 buffers containing 0.3% BSA. On a 96 wells microplate, 20 μL of the drug solution with a given concentration, 30 μL of 80 mM tris-HCl buffers containing 0.3% BSA and 50 μL of the enzyme preparation were distributed, and incubated at room temperature for 1 hour after shaking for 10 seconds. Thereto, 50 μL of 5 mM 5,5-dithio-bis(2-nitrobenzoic acid) (Sigma, St. Louis, MO, USA) and 50 μL of 4 mM acetylthiocholine iodide (Sigma) or 4 mM butyrylthiocholine chloride were added and shaken again, and then the increase of light absorption at 412 nm was immediately measured at 30 second-intervals for 10 minutes with a microplate reader (Spectra rainbow thermo, TECAN, Austria). For the respective drugs, the test was repeated twice. The % of control of the increase in light absorption was calculated at each concentration with respect to the respective drugs.

$$\% \text{ of control} = \text{increase in light absorption of drug/increase in light absorption of control} \times 100$$

The value of 50% inhibitory concentration ($IC_{50}$) was determined by all clinical package program (SAS Institute Japan) using % of control at each concentration determined in each test.

The compound A, neostigigmine and distigmine inhibited the activity of human acetylcholine esterase. The compound A did not show inhibitory activity for human butyrylcholine esterase up to $10^{-5}$ M, while the inhibitory activity was confirmed for neostigigmine and distigmine. The butyrylcholine esterase selective inhibitor, iso-OMPA, showed the inhibitory activity to only butyrylcholine esterase. Table 2 shows $IC_{50}$ value and the selectivity of acetylcholine esterase inhibitory activity in the inhibition of respective enzymes by respective drugs. Acetylcholine esterase selective inhibitory activity of 6700-fold or more was confirmed for the compound A, but neostigigmine and distigmine had scarcely selectivity.

The test of acetylcholine esterase selectivity was also carried out in the same manner as that for the compound A with respect to the compounds B1 to B6 (B1 : 8-(5-[[2-(2-chlorophenyl)ethyl]amino]pentanoyl]-5,6-dihydro-4H-pyrrolo [3,2,1-ij]quinolin-2(1H)-one) hydrochloride, B2 : 5-[5-[[2-(2-chlorophenyl)ethyl](methyl)amino]pentanoyl]-1,3-dihydro-2H-benzoimidazol-2-one) hydrochloride, B3 : 5-[5-[[2-(2-chlorophenyl)ethyl](methyl)amino]pentanoyl]-1,3-dimethyl-1,3-dihydro-2H-benzoimidazol-2-one) hydrochloride, B4 : N-ethyl-5-(5-[[2-(2-methoxyphenyl)ethyl]amino]pentanoyl]-1-indolincarboxamide hydrochloride, B5 : 5-(5-[[2-(2-methoxyphenyl)ethyl]amino]pentanoyl]-1,3-dimethyl-1,3-dihydro-2H-benzoimidazol-2-one) hydrochloride, B6 : 5-(5-[[2-(2-chlorophenyl)ethyl]amino]pentanoyl]-1,3-dimethyl-1,3-dihydro-2H-benzoimidazol-2-one) hydrochloride). Any of the compounds B1 to B6 had high selectivity for acetylcholine esterase inhibitory activity (Table 3).

Table 2 AChE inhibitory activity and BuChE inhibitory activity of various drugs

| Drugs | Acetylcholine esterase $IC_{50}$ (μM)[a] , | Butyrylcholine esterase $IC_{50}$ (μM)[b] | Selectivity b/a |
|---|---|---|---|
| Compound A | 0.0015 | > 10 | > 6700 |
| Neostigigmine | 0.043 | 0.60 | 14 |
| Distigmine | 0.38 | 0.54 | 1.4 |
| iso-OMPA | > 10 | 3.2 | < 0.32 |

$IC_{50}$ value was shown by mean value of twice tests

Table 3 AChE inhibitory activity and BuChE inhibitory activity of various compounds

| Drug | AchE $IC_{50}$ (μmol/L)[a] | BuChE $IC_{50}$ (umol/L)[b] | Selectivity b/a |
|---|---|---|---|
| Compound B1 | 0.056 | 5.7 | 100 |
| Compound B2 | 0.224 | > 10 | > 45 |
| Compound B3 | 0.028 | > 10 | > 350 |
| Compound B4 | 0.118 | > 10 | > 85 |
| Compound B5 | 0.065 | > 10 | > 150 |
| Compound B6 | 0.071 | > 10 | > 140 |

$IC_{50}$ value was shown by mean value of twice tests

Example 2: Effect of various drugs on basic tension of bladder muscle

A Hartley male guinea pig of 5 weeks old (Japan SLC Co.) was used. The animal was killed by beheading and the bladder was removed to prepare muscle segment having a length of about 10 mm to a longitudinal direction and a width of about 4 mm. Four segments were prepared from one animal. Tension was measured with an isometric strain transducer, downloaded in PC (Vectra VE, Hewlett Packard, Palo Alto, CA, USA) through an amplifier and a multi-channel data analyzer (MP-100A-CE, Biopac systems), and analyzed by a proprietary software (Acqknowledge, Biopac systems). The sampling interval of data was 0.2 second. The muscle segments were suspended in a Magnus vessel filled with Krebs solution (20 mL) to which 95% $O_2$ + 5% $CO_2$ was aerated, while loading about a load of 1 g. After an equilibrium term of 20 minutes or more, depolarlizing contraction was induced with 100 mM KCl-Krebs solution and the maximum contraction was measured. Twenty minutes or more after rinsing, the segments were treated with the drugs (the compound A, neostigigmine, pyridostigmine, distigmine, and iso-OMPA or the compound A + iso-OMPA) for 30 minutes. Each of the drugs was dissolved with 100% DMSO or Krebs solution and 20 μL was added dropwise to a Magnus tube so as to be a given concentration. The solvent was added dropwise for a control group.

Difference between the mean value of tension for 30 seconds (marker: for 30 sec before 100 mM KCl) just before 100 mM KCl treatment and the maximum value after treatment was taken as 100%, and the change of contraction tension of the bladder muscle by the drugs was standardized. The difference was determined from the mean value of tension just before the treatment of the drug and for 30 seconds from 30 minutes after the treatment of the drug (marker: for 30 seconds before the drug, and for 30 seconds after 30 minutes from drug).

Neostigigmine and pyridostigmine increased basic tension significantly and concentration-dependently. Distigmine increased tension slightly but significantly. On the other hand, the compound A did not influence the basic tension (Table 4). Butyrylcholine esterase selective inhibitor, iso-OMPA, alone did not influence tension either, but when the compound A was used in combination with iso-OMPA, the significant increase in the basic tension was confirmed (Table 5).

Table 4 Action of various drugs for basic tension

| Drug | Concentration (μM) | Number of sample | Tension (%) |
|---|---|---|---|
| Solvent | - | 8 | -1.30 ± 0.44 |
| Compound A | 0.003 | 8 | -0.55 ± 0.60 |
| | 0.01 | 8 | -0.26 ± 0.38 |
| | 0.03 | 8 | 0.13 ± 0.80 |
| | 0.1 | 8 | -0.74 ± 0.60 |
| Neostigigmine | 0.01 | 8 | -0.95 ± 0.37 |
| | 0.03 | 8 | -0.31 ± 0.25 |
| | 0.1 | 8 | 1.52 ± 0.37* |
| | 0.3 | 8 | 6.87 ± 1.25** |
| | 1 | 8 | 17.78 ± 2.25** |
| Pyridostigmine | 0.3 | 8 | -1.49 ± 0.52 |
| | 1 | 8 | 0.04 ± 0.38 |
| | 3 | 8 | 2.87 ± 0.85* |
| | 10 | 8 | 7.71 ± 1.30** |
| | 30 | 8 | 14.38 ± 1.62** |
| Distigmine | 0.1 | 8 | -1.49 ± 0.30 |
| | 0.3 | 8 | -0.78 ± 0.50 |
| | 1 | 8 | -0.35 ± 0.73 |
| | 3 | 8 | 0.64 ± 0.49* |

Data indicates mean value ± SEM
* P < 0.05, * P < 0.01 for control group (Dunnett calibration)

Table 5 Effect of concomitant use of iso-OMPA and compound A on basic tension

| iso-OMPA (μM) | Treatment Compound A (μM) | Number of sample | Tension (%) |
|---|---|---|---|
| 0 | 0 | 8 | -1.33 ± 0.24 |
| 10 | 0 | 8 | -1.31 ± 0.21 |
| 100 | 0 | 8 | -0.85 ± 0.38 |
| 0 | 0.1 | 8 | -0.47 ± 0.17 |
| 10 | 0.1 | 8 | 1.96 ±0.87* |
| 100 | 0.1 | 8 | 5.43 ± 1.17** |

Data indicates mean value ± SEM
* P < 0.05, * P < 0.01 for control group (Dunnett calibration)

Example 3: Pressure/flow study using guinea pig (compound A)

A Hartley male guinea pig of 5 weeks old (Japan SLC) was used. After urethane anesthesia, the medial incision of the lower abdomen was carried out, and the bladder was exposed. Two injection needles (20G) with which a polyethylene tube (PE-100) was linked were inserted in the bladder, and one needle was used for injection of saline and another needle was used for measurement of the inner pressure of the bladder. The injection of saline was continuously carried out at a rate of 18 mL/h using a syringe pump (SP-100S, JMS, and Hiroshima), the injection was stopped after intermittent urination was confirmed 3 times at minimum, and the saline in the bladder was removed by suction. Injection was started again, and the injection was stopped when the raise of the inner pressure of the bladder just before urination was confirmed. The amount of urine excreted was measured by an electronic weighing machine (HX-400, A&D, and Tokyo) and the inner pressure of the bladder was measured using a pressure transducer (AP641G, NIPPON KODEN Corporation). The analogue data of the weight of urine and the inner pressure of the bladder were inputted in a multi-channel data analyzer (MP-100A-CE, Biopac systems), and analyzed by a proprietary software (Acqknowledge, Biopac

systems). The sampling interval of data was 0.1 second and a low pass filter was applied to the data of discharge amount and urine flow rate at 0.5 Hz in order to remove noise. A delay time of 0.1 second in weighing the urine was rectified, and the urine flow rate was determined by differentiating the value of discharge amount. After a value before twice administration of the drug was measured, the drugs (the compound A, distigmine, neostigigmine and bethanechol) were intravenously administrated. Saline in the bladder was extracted after 10 minutes of administration of the compound A, bethanechol and neostigigmine and after 30 minutes of administration of distigmine, and injection was started again to measure data. The drugs were dissolved with distilled water so that administration volume was 0.5 mL/kg. Parameters (bladder volume, the amount of urination, the maximum urine flow rate, the inner pressure of the bladder at the maximum urine flow, bladder compliance) below were measured. The bladder compliance was calculated from intersection point (the threshold of bladder volume, threshold pressure) which was obtained by determining regression line in the respective curves of the inner pressure of the bladder at collection of urine and urinary reflex. The change rate before and after administration of the drug was calculated for the respective parameters and the calibration of significant difference was carried out by a solvent administration group and Dunnett calibration (Table 6 and Table 7).

The change of the parameters at urination by administration of the compound A, distigmine, neostigigmine and bethanechol was shown in Table 5 and the change of the parameters at collection of urine was shown in Table 6. It was cleared that the compound A increased significantly the amount of urination and the maximum urine flow rate at 0.003 mg/kg or more and enhances the urination function of the bladder without influencing urination pressure (the inner pressure of the bladder at the maximum urine flow). Further, the increase of the bladder volume was confirmed at 0.03 mg/kg, and did not influence the bladder compliance; therefore it was suggested that it did not damage the collection function of the bladder and rather acted as a direction for enhancing the function. On the other hand, both of the carbamate acetylcholine esterase inhibitors of distigmine and neostigigmine did not show clear action for the amount of urination and the maximum urine flow rate and increased urination pressure significantly. It was cleared that these drugs did not improve the elimination function of the bladder because the resistance of urinary tract was enhanced. Further, since Neostigigmine at 0.03 mg/kg or more and Distigmine at 0.1 mg/kg lowered the bladder compliance significantly, it was suggested that they had the collection function of the bladder. It was cleared that bethanechol lowered the amount of urination, bladder volume and bladder compliance significantly and lowered both of the urination function and the collection function of urine.

Table 6 Action of various drugs for parameters at urination

| Drug | Dose (mg/kg, i.v.) | Number of example | Proportion for value before administration (%) Amount of urination | Maximum urine flow rate | At maximum urine flow rate Inner pressure of bladder |
|---|---|---|---|---|---|
| Solvent | | 10 | 86.6 ± 4.4 | 75.3 ± 4.2 | 115.0 ± 5.4 |
| Compound A | | | | | |
| | 0.003 | 10 | 150.2 ± 10.0** | 112.1 ± 9.7* | 117.5 ± 6.2 |
| | 0.01 | 10 | 165.2 ± 18.2** | 124.4 ± 10.3** | 110.9 ± 6.0 |
| | 0.03 | 10 | 164.0 ± 13.4** | 133.5 ± 15.3** | 116.9 ± 8.4 |
| Solvent | | 10 | 73.6 ± 11.5 | 73.2 ± 7.5 | 100.00 ± 5.5 |
| Distigmine | | | | | |
| | 0.03 | 10 | 73.9 ± 4.4 | 75.1 ± 6.8 | 112.7 ± 6.6 |
| | 0.1 | 10 | 89.8 ± 10.3 | 76.4 ± 5.8 | 115.9 ± 4.2 |
| | 0.3 | 9 | 65.8 ± 11.9 | 49.2 ±7.0 | 142.4 ± 6.1** |
| Solvent | | 8 | 90.1 ± 5.5 | 78.9 ± 5.3 | 98.6 ± 6.9 |
| Neostigigmine | | | | | |
| | 0.01 | 7 | 107.7 ± 17.2 | 79.4 ± 11.5 | 112.3 ± 4.2 |
| | 0.03 | 8 | 91.6 ± 14.9 | 67.2 ± 9.9 | 128.5 ± 10.9* |
| | 0.1 | 8 | 58.3 ± 16.4 | 54.0 ± 13.8 | 130.9 ± 8.7* |
| Solvent | | 8 | 95.3 ± 6.1 | 90.4 ± 9.6 | 108.6 ± 4.8 |
| Bethanechol | | | | | |
| | 0.1 | 8 | 74.5 ± 13.1 | 72.8 ± 9.8 | 107.0 ± 7.1 |

Table continued

| Drug | Dose (mg/kg, i.v.) | Number of example | Proportion for value before administration (%) | | |
|---|---|---|---|---|---|
| | | | Amount of urination | Maximum urine flow rate | At maximum urine flow rate Inner pressure of bladder |
| | 0.3 | 8 | 63.5 ± 8.5 | 81.00± 12.1 | 99.2 ± 6.4 |
| | 1 | 7 | 39.1 ± 8.0** | 66.1 ± 12.3 | 104.9 ± 13.0 |

Data indicates mean value ± SEM
* P < 0.05, * P < 0.01 for control group (Dunnett calibration)

Table 7 Action of various drugs for parameters at collection of urine

| Drug | Dose (mg/kg, iv) | Number of example | Proportion for value before administration (%) | |
|---|---|---|---|---|
| | | | Bladder volume | Bladder compliance |
| Solvent Compound A | | 10 | 91.9 ± 3.2 | 82.7 ± 5.6 |
| | 0.003 | 10 | 94.6 ± 5.8 | 75.2 ± 4.8 |
| | 0.01 | 10 | 101.7 ± 5.0 | 82.6 ± 2.6 |
| | 0.03 | 10 | 117.2 ± 9.0* | 95.9 ± 18.0 |
| Solvent | | 10 | 83.9 ± 5.0 | 87.1 ± 7.5 |
| Distigmine | 0.03 | 10 | 80.6 ± 3.3 | 64.6 ± 6.0 |
| | 0.1 | 10 | 83.6 ± 5.9 | 53.4 ± 2.2** |
| | 0.3 | 9 | 80.0 ± 4.5 | 68.3 ± 7.9 |
| Solvent Neostigigmine | | 8 | 97.2 ± 5.3 | 89.6 ± 8.0 |
| | 0.01 | 7 | 81.1 ± 8.9 | 61.5 ± 8.6 |
| | 0.03 | 8 | 86.4 ± 7.5 | 59.4 ± 9.1* |
| | 0.1 | 8 | 66.5 ± 14.7 | 31.7 ± 7.1** |
| Solvent Bethanechol | | 8 | 96.0 ± 4.4 | 93.1 ± 10.4 |
| | 0.1 | 8 | 86.0 ± 2.5 | 75.7 ± 9.8 |
| | 0.3 | 8 | 85.3 ± 6.3 | 62.6 ± 6.8* |
| | 1 | 7 | 66.3 ± 6.0** | 45.3 ± 4.9** |

Data indicates mean value ± SEM
* P < 0.05, * P < 0.01 for control group (Dunnett calibration)

Example 4: Pressure/flow study using guinea pig (compound B)

A parameter (the maximum urine flow rate) at urination and a parameter (bladder compliance) at the collection of urine were also measured by a method similar as Example 3 shown below, with respect to the compounds B1 to B6 ([Table 8]).

A Hartley male guinea pig of 4 to 5 weeks old (Japan SLC) was used. After urethane anesthesia, the medial incision of the lower abdomen was carried out, and the bladder was exposed. Two injection needles (20G) with which a polyethylene tube (PE-100) was linked were inserted in the bladder, and one needle was used for injection of saline and another needle was used for measurement of the inner pressure of the bladder. A catheter was inserted in fernoral vein, and phenylephrine was continuously administrated at a flow rate of 3 μg/animal/min. Saline was continuously injected at a speed of 18 mL/h, the injection was stopped after intermittent urination was confirmed 2 times at minimum, and the saline in the bladder was removed by suction. Injection was started again, and the injection was stopped when the raise of the inner pressure of the bladder just before urination was confirmed. The amount of urine eliminated was measured by an electronic weighing machine and the inner pressure of the bladder was measured using a pressure transducer. The analogue data of the weight of urine and the inner pressure of the bladder were inputted in a multi-channel

data analyzer (MP-100A-CE, Biopac systems), and analyzed by a proprietary software (Acqknowledge, Biopac systems). As the parameters, the maximum urine flow rate and bladder compliance were measured. Saline in the bladder was extracted after 10 min of the intravenous administration of the compound, and injection was started again to carry out measurement. The compounds were dissolved with DMSO so that administration volume was 0.5 mL/kg. The change rate of respective parameters before and after administration of the compounds was calculated and the calibration of significant difference was carried out by a solvent administration group and t calibration.

The change of the parameters by administration of the compounds was shown in (Table 8). Any of the compounds increased the maximum urine flow rate significantly. On the other hand, they did not influence bladder compliance. It was cleared that any of the compounds enhances the urination function without damaging the urination function of the bladder.

Table 8 Action of various compounds for parameters at urination

| Drug | Dose (mg/kg, i.v.) | Number of example | Proportion for value before administration | |
|---|---|---|---|---|
| | | | Maximum urine flow rate | Bladder compliance |
| Solvent | - | 9 | 85.6 ± 8.0 | 93.9 ± 10.8 |
| Compound B1 | 0.1 | 8 | 170.4 ± 27.8** | 105.1 ± 12.9 |
| Compound B2 | 0.3 | 9 | 138.1 ± 13.1** | 95.0 ± 5.1 |
| Compound B3 | 0.1 | 8 | 150.8 ± 19.4** | 94.7 ± 11.1 |
| Compound B4 | 0.3 | 8 | 165.4 ± 14.9** | 109.2 ± 8.1 |
| Compound B5 | 0.03 | 9 | 135.5 ± 19.1** | 95.0 ± 4.3 |
| Compound B6 | 0.1 | 8 | 147.1 ±26.2* | 98.6 ± 6.9 |

Data indicates mean value ± SEM
* P =< 0.05, * P =< 0.01 for solvent administration group (t calibration)

Example 5

A male SD rat (CLEA Japan Inc.) with a body weight of about 400 g or a Hartley male guinea pig with a body weight of 400 to 500 g (Japan SLC) were used. After urethane anesthesia, sanitary cotton preliminarily weighed was inserted in the mouth of the animal and acetylcholine was intravenously administrated. After 5 minutes, the sanitary cotton was taken out to be weighed and the amount of saliva secreted was measured. The drug was intravenously administrated at 10 min before the administration of acetylcholine. The effect of oxybutynin was analyzed by paired t calibration and the concomitant use effect of the compound A was analyzed by William calibration. Acetylcholine promotes the secretion of saliva in the rat and guinea pig and the action was significantly suppressed by preliminary administration of the oxybutynin of an anticholinergic agent (Table 9 and Table 10). The salivation suppressing action of oxybutynin was significantly antagonized by the simultaneous administration of the compound A.

It was grasped according to the above result that the compound A had antagonistic action for the salivation suppressing action by an anticholinergic agent.

Table 9 Action for Ach inductive salivation of oxybutynin and compound A in rat

| Acetylcholine (mg/kg) | Oxybutynin (mg/kg) | Compound A (mg/kg) | Number of example | Amount of saliva secreted (g) |
|---|---|---|---|---|
| 1 | - | - | 10 | 0.27 ± 0.04 |
| 1 | 0.1 | - | 10 | 0.17 ± 0.02* |
| 1 | 0.1 | 0.001 | 5 | 0.22 ± 0.02 |
| 1 | 0.1 | 0.003 | 5 | 0.26 ± 0.02# |
| 1 | 0.1 | 0.01 | 5 | 0.49 ± 0.03# |

Data indicates mean value ± SEM
* P ≤ 0.05 for acetylcholine single administration group (paired t calibration)
# P ≤ 0.025 for oxybutynin single administration group (Williams calibration)

Table 10 Action for Ach inductive salivation of oxybutynin and compound A in guinea pig

| Acetylcholine (mg/kg) | Oxybutynin (mg/kg) | Compound A (mg/kg) | Number of example | Amount of saliva secreted (g) |
|---|---|---|---|---|
| 1 | - | - | 5 | 0.23 ± 0.03 |
| 1 | 1 | - | 5 | 0.11 ± 0.02* |
| 1 | 1 | 0.003 | 5 | 0.17 ± 0.03 |
| 1 | 1 | 0.010 | 5 | 0.27 ± 0.02# |

Data indicates mean value ± SEM
* P ≤ 0.05 for acetylcholine single administration group (paired t calibration)
# ≤ 0.025 for oxybutynin single administration group (Williams calibration)

Example 6

A Hartley male guinea pig with a body weight of about 500 g (Japan SLC Hamamatsu) was used. After urethane anesthesia (1.4 g/kg, i.p.), the medial incision of the lower abdomen was carried out, and the bladder was exposed. Two injection needles (20G) with which a polyethylene tube (PE-100, Becton Dickinson, Franklin Lakes, NJ, USA) was linked were inserted in the bladder, and one needle was used for injection of saline and another needle was used for measurement of the inner pressure of the bladder. Further, a cannula (PE-10, Becton Dickinson) for injection of phenylephrine was inserted in right common jugular vein. After saline in the bladder was removed by suction, the injection of saline was continuously carried out at a rate of 18 mL/h using a syringe pump (Model 220, KD scientific, New Hope, PA, USA), the injection was stopped when the raise of the inner pressure of the bladder just before urination was confirmed. The amount of urine eliminated was measured by an electronic weighing machine (GX-400, A&D, Tokyo) and the inner pressure of the bladder was measured using a pressure transducer (AP641G, NIPPON KODEN Corporation, Tokyo). The analogue data of the weight of urine and the inner pressure of the bladder were inputted in a multi-channel data analyzer (MP-100A-CE, Biopac systems), and analyzed by a proprietary software (Acqknowledge 3.7.1, Biopac systems). The sampling interval of data was 0.1 sec and a low pass filter was applied to the data of discharge amount and urine flow rate at 0.5 Hz in order to remove noise. A delay time of 0.1 sec in weighing the urine was rectified, and the urine flow rate was determined by differentiating the value of discharge amount. The dynamic state parameter of the flow of urine below was measured. A bladder volume, the maximum urine flow rate, urination efficiency (= amount of urination/ bladder volume). The effect of oxybutynin was analyzed by the paired t calibration and the concomitant use effect of the compound A was analyzed by Dunnett calibration.

After administration of oxybutynin (1 mg/kg), the significant increase of bladder volume and the lowering of the maximum urine flow rate and urination efficiency were confirmed (Table 11). Among these actions of oxybutynin, the compound A (0.003, 0.01 mg/kg) acted antagonistically against the lowering of the maximum urine flow rate and urination efficiency but did not influence the increase of bladder volume.

From the above result, it is suggested that the compound A antagonize the change of urination parameter accompanied by the lowering of bladder contraction force at urination by an anticholinergic agent but does not influence the increase of bladder volume, and it was cleared that the compound A can be used as not only a drug for urinary disturbance accompanied by conventional low active urination muscle, but also a drug for reducing the adverse reaction of the anticholinergic agent.

Table 11 Action of drug for various parameters

| Oxybutynin (mg/kg, iv) | Compound A (mg/kg, iv) | Number of example | Proportion for value before administration (%) | | |
|---|---|---|---|---|---|
| | | | Bladder volume | Maximum urine flow rate | Urination efficiency |
| - | - | 9 | 3.24 ± 2.64 | -6.85 ± 3.91 | -7.96 ± 4.59 |
| 1 | - | 9 | 28.87 ± 7.01** | -44.42 ± 9.44** | -66.14 ± 5.91** |
| 1 | 0.003 | 9 | 21.07 ± 4.65 | -34.88 ± 8.22 | -61.67 ± 7.22 |
| 1 | 0.01 | 8 | 24.73 ± 7.05 | -9.04 ± 5.94# | -30.23 ± 10.48## |

Data indicates mean value ± SEM
** P 0.01, for solvent administration group (paired t calibration)
## P 0.01, #P≤ 0.05 for oxybutynin single administration group (Dunnett calibration)

Preparation Example 1

| | |
|---|---|
| (1) The compound A | 10.0 mg |
| (2) Lactose | 60.0 mg |
| (3) Corn starch | 35.0 mg |
| (4) Gelatin | 3.0 mg |
| (5) Magnesium stearate | 2.0 mg |

A mixture of 10.0 mg of the compound A, 60.0 mg of lactose and 35.0 mg of corn starch is granulated through a sieve of 1 mm mesh using 0.03 mL of 10% gelatin aqueous solution (3.0 mg as gelatin) and then, dried at 40°C to be passed through a sieve again. The granules thus obtained are mixed with 2.0 mg of magnesium stearate and compressed. The central tablets obtained are coated with sugarcoating by an aqueous suspension of sucrose, titanium oxide, talc and gum Arabica. The coated tablets are polished with beeswax to obtain coated tablets.

Preparation Example 2

| | |
|---|---|
| (1) The compound A | 10.0 mg |
| (2) Lactose | 70.0 mg |
| (3) Corn starch | 50.0 mg |
| (4) Soluble starch | 7.0 mg |
| (5) Magnesium stearate | 3.0 mg |

After 10.0 mg of the compound A and 3.0 mg of magnesium stearate are granulated with 0.07 mL of soluble starch (7.0 mg as soluble starch), the granules are dried and mixed with 70.0 mg of lactose and 50.0 mg of corn starch. The mixture is compressed to obtain tablets. Preparation Example 3

| | |
|---|---|
| (1) The compound A | 5.0 mg |
| (2) Salt | 20.0 mg |
| (3) Distilled water | 2 mL in total |

To a solution of 5.0 mg of the compound A and 20.0 mg of the salt in distilled water is added water to adjust the total volume to 2.0 mL. The solution is filtered and filled in an ampoule of 2 mL under aseptic conditions. After the ampoule is sterilized, it is sealed to obtain a solution for injection.

Preparation Example 4

An either drug of Preparation Examples 1 to 3 is combined with an either drug of Reference Preparation Examples 1 to 3.

Reference Preparation Example 1

| | |
|---|---|
| (1) Oxybutynin | 10.0 mg |
| (2) Lactose | 60.0 mg |
| (3) Corn starch | 35.0 mg |
| (4) Gelatin | 3.0 mg |
| (5) Magnesium stearate | 2.0 mg |

A mixture of 10.0 mg of oxybutynin, 60.0 mg of lactose and 35.0 mg of corn starch is granulated through a

sieve of 1 mm mesh using 0.03 mL of 10% gelatin aqueous solution (3.0 mg as gelatin) and then, dried at 40°C to be passed through a sieve again. The granules thus obtained are mixed with 2.0 mg of magnesium stearate and compressed. The central tablets obtained are coated with sugarcoating by an aqueous suspension of sucrose, titanium oxide, talc and gum arabic. The coated tablets are polished with beeswax to obtain coated tablets.

Reference Preparation Example 2

| | |
|---|---|
| (1) Oxybutynin | 10.0 mg |
| (2) Lactose | 70.0 mg |
| (3) Corn starch | 50.0 mg |
| (4) Soluble starch | 7.0 mg |
| (5) Magnesium stearate | 3.0 mg |

After 10.0 mg of oxybutynin and 3.0 mg of magnesium stearate are granulated with 0.07 mL of soluble starch (7.0 mg as soluble starch), the granules are dried and mixed with 70.0 mg of lactose and 50.0 mg of corn starch. The mixture is compressed to obtain tablets.

Preparation Example 3

| | |
|---|---|
| (1) Oxybutynin | 5.0 mg |
| (2) Salt | 20.0 mg |
| (3) Distilled water | 2 mL in total |

To a solution of 5.0 mg of oxybutynin and 20.0 mg of the salt in distilled water and water is added water to adjust the total volume to 2.0 mL. The solution is filtered and filled in an ampoule of 2 mL under aseptic conditions. After the ampoule is sterilized, it is sealed to obtain a solution for injection.

Industrial Applicability

Since the compound having AChE inhibitory activity without BuChE inhibitory activity enhances the contraction action of the detrusor muscle only at urination without lowering the bladder compliance at the collection of urine, the compound is extremely useful as a safe and effective preventive or remedy for urinary disturbance having no side effect, and additionally, is also extremely useful as a preventive or remedy for dry mouth induced by the administration of a remedy for urinary disturbance and also a preventive or remedy for overactive bladder not accompanied by dry mouth.

## Claims

1. A preventive or remedy for urinary disturbance comprising a compound having acetylcholine esterase inhibitory activity and being substantially free from butyrylcholine esterase inhibitory activity, which does not inhibit the collection function of urine.

2. A preventive or remedy for dry mouth induced by the administration of a remedy for urinary disturbance, comprising a compound having acetylcholine esterase inhibitory activity and being substantially free from butyrylcholine esterase inhibitory activity.

3. A preventive or remedy for overactive bladder not accompanied by dry mouth comprising a combination of a compound having acetylcholine esterase inhibitory activity and being substantially free from butyrylcholine esterase inhibitory activity, and an anticholinergic agent.

4. The preventive or remedy according to claim 1, 2 or 3, wherein the ratio of a butyrylcholine esterase 50% inhibitory concentration to an acetylcholine esterase 50% inhibitory concentration of the compound is 20 or more.

**5.** The preventive or remedy according to claim 1, 2 or 3, wherein the ratio of a butyrylcholine esterase 50% inhibitory concentration to an acetylcholine esterase 50% inhibitory concentration of the compound is 100 or more.

**6.** The preventive or remedy according to claim 1, wherein urinary disturbance is dysuria.

**7.** The preventive or remedy according to claim 2, wherein the remedy for urinary disturbance is an anticholinergic agent.

**8.** A method for screening a preventive or remedy substance for urinary disturbance not inhibiting the collection function of urine, which comprises measuring and comparing the acetylcholine esterase inhibitory activity and the butyrylcholine esterase inhibitory activity of a test compound.

**9.** A method for preventing or treating urinary disturbance without inhibiting the collection function of urine, which comprises administering an effective amount of a compound having acetylcholine esterase inhibitory activity and being substantially free from butyrylcholine esterase inhibitory activity to a mammal.

**10.** A method for preventing or treating dry mouth induced by the administration of a remedy for urinary disturbance, which comprises administering an effective amount of a compound having acetylcholine esterase inhibitory activity and being substantially free from butyrylcholine esterase inhibitory activity to a mammal.

**11.** A method for preventing or treating overactive bladder without being accompanied by dry mouth, which comprises administering a combination of effective amounts of a compound having acetylcholine esterase inhibitory activity and being substantially free from butyrylcholine esterase inhibitory activity, and an anticholinergic agent to a mammal.

**12.** Use of a compound having acetylcholine esterase inhibitory activity and being substantially free from butyrylcholine esterase inhibitory activity for manufacturing a preventive or remedy for urinary disturbance which does not inhibit the collection function of urine.

**13.** Use of a compound having acetylcholine esterase inhibitory activity and being substantially free from butyrylcholine esterase inhibitory activity for manufacturing a preventive or remedy for dry mouth induced by the administration of a remedy for urinary disturbance.

**14.** Use of a compound having acetylcholine esterase inhibitory activity and being substantially free from butyrylcholine esterase inhibitory activity and an anticholinergic agent for manufacturing a preventive or remedy for overactive bladder not accompanied by dry mouth.

**15.** A preventive or remedy for overactive bladder not accompanied by dry mouth comprising a compound having acetylcholine esterase inhibitory activity and being substantially free from butyrylcholine esterase inhibitory activity.

**16.** A method for preventing or treating overactive bladder without being accompanied by dry mouth, which comprises administering an effective amount of a compound having acetylcholine esterase inhibitory activity and being substantially free from butyrylcholine esterase inhibitory activity to a mammal.

**17.** Use of a compound having acetylcholine esterase inhibitory activity and being substantially free from butyrylcholine esterase inhibitory activity for manufacturing a preventive or remedy for overactive bladder not accompanied by dry mouth.

**INTERNATIONAL SEARCH REPORT**

International application No.
PCT/JP2004/009486

A. CLASSIFICATION OF SUBJECT MATTER
Int.Cl[7] A61K45/00, A61P13/02, 13/10, 43/00

According to International Patent Classification (IPC) or to both national classification and IPC

B. FIELDS SEARCHED

Minimum documentation searched (classification system followed by classification symbols)
Int.Cl[7] A61K45/00, A61P13/02, 13/10, 43/00

Documentation searched other than minimum documentation to the extent that such documents are included in the fields searched

Electronic data base consulted during the international search (name of data base and, where practicable, search terms used)
CAPLUS(STN), REGISTRY(STN), MEDLINE(STN), BIOSIS(STN)

C. DOCUMENTS CONSIDERED TO BE RELEVANT

| Category* | Citation of document, with indication, where appropriate, of the relevant passages | Relevant to claim No. |
|---|---|---|
| X | JP 2000-169373 A (Takeda Chemical Industries, Ltd.),<br>20 June, 2000 (20.06.00),<br>Particularly, Claims; examples<br>& JP 2001-335576 A & JP 2003-192593 A<br>& JP 2003-201237 A & WO 00/18391 A1<br>& EP 1118322 A1 & US 2002/0177593 A1 | 1,4-6,12 |
| E,X | WO 03/057254 A1 (Takeda Chemical Industries, Ltd.),<br>17 July, 2003 (17.07.03),<br>& JP 2003-335701 A | 1,4-6,12 |
| A | JP 7-206854 A (Takeda Chemical Industries, Ltd.),<br>08 August, 1995 (08.08.95),<br>& EP 607864 A1 | 1,4-6,12 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 17 September, 2004 (17.09.04) | 05 October, 2004 (05.10.04) |
| Name and mailing address of the ISA/<br>Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2004/009486

**Box No. II Observations where certain claims were found unsearchable (Continuation of item 2 of first sheet)**

This international search report has not been established in respect of certain claims under Article 17(2)(a) for the following reasons:

1. [X] Claims Nos.: 9-11, 16
because they relate to subject matter not required to be searched by this Authority, namely:
The inventions as set forth in claims 9 to 11 and 16 pertain to methods for treatment of the human body by therapy and thus relate to a subject matter which this International Searching Authority is not required, under the provisions of Article 17(2)(a)(i) of the PCT (continued to extra sheet.)

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
Urinary disturbance largely differs from dry mouth and hyperactive bladder induced by the administration of a remedy for urinary disturbance in diseases causes and drugs to be used for treating the same. It cannot be recognized that a method of screening a substance preventing/treating urinary disturbance without inhibiting the urine collection function is a method particularly appropriate for the production of a preventive/remedy for urinary disturbance without inhibiting the urine collection function. That is, there is no matter common to the inventions according to claims 1, 4 to 6 and 12, the inventions according to claims 2, 7 and 13, the inventions according to claims 3, 14, 15 and 17 and the invention according to claim 8 (continued to extra sheet.)

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [X] No required additional search fees were timely paid by the applicant. Consequently, this international search report is restricted to the invention first mentioned in the claims; it is covered by claims Nos.:
Claims 1, 4 to 6 and 12.

**Remark on Protest**
[ ] The additional search fees were accompanied by the applicant's protest.
[ ] No protest accompanied the payment of additional search fees.

Form PCT/ISA/210 (continuation of first sheet (2)) (January 2004)

INTERNATIONAL SEARCH REPORT

International application No.
PCT/JP2004/009486

Continuation of Box No.II-1 of continuation of first sheet(2)

and Rule 39.1(iv) of the Regulations under the PCT, to search.

Continuation of Box No.III of continuation of first sheet(2)

seemingly being a special technical feature in the meaning within the second sentence of PCT Rule 13.2 and no technical relevancy can be found among these groups of inventions differing from each other in the meaning within PCT Rule 13.

Such being the case, it does not appear that there is a technical relationship between these groups of inventions involving one or more of the same or corresponding special technical features and, therefore, these groups of inventions are not considered as being so linked as to form a single general inventive concept.

<Subject of search>

Claims 1, 4 to 6 and 12 relate to a preventive/remedy for urinary disturbance without inhibiting the urine collection function that contains a compound defined by a desired property "showing an acetylcholine esterase inhibitory activity but substantially having no butyrylcholine esterase inhibitory activity" as the active ingredient. Although claims 1, 4 to 6 and 12 involve any compounds having the above property, it is recognized that only small part of the claimed compounds are supported by the description in the meaning within PCT Article 6 and disclosed therein in the meaning within PCT Article 5.

Although the common technical knowledge at the point of the application is taken into consideration, the scope of the compounds "showing an acetylcholine esterase inhibitory activity but substantially having no butyrylcholine esterase inhibitory activity" cannot be specified. Thus, claims 1, 4 to 6 and 12 do not comply with the requirement for clearness as specified in PCT Article 6 too.

Such being the case, the search was made exclusively on the relationship among an acetylcholine esterase inhibitory activity, a butyrylcholine esterase inhibitory activity and a preventive/remedy for urinary disturbance and preventives/remedies for urinary disturbance containing the compounds (A, B1 to B6) specifically cited in the description as the active ingredient.

Form PCT/ISA/210 (extra sheet) (January 2004)